# EUROPEAN PATENT SPECIFICATION

(11) **EP 4 499 611 B1**
(45) Date of publication and mention of the grant of the patent: **18.02.2026**
(21) Application number: 23720428.4
(22) Date of filing: 30.03.2023
(51) Int. Cl.: C07D 231/14, A61P 37/06, A61K 31/382, C07D 401/04, C07D 401/12, C07D 401/14, C07D 403/04, C07D 403/12, C07D 409/12, C07D 409/14

(54) **MALT1 MODULATORS AND USES THEREOF**
MALT1-MODULATOREN UND VERWENDUNGEN DAVON
MODULATEURS DE MALT1 ET LEURS UTILISATIONS

(30) Priority: 31.03.2022 US 202263325852 P
(43) Date of publication of application: 05.02.2025
(60) Divisional application: 25223292.1
(73) Proprietor: Rarefied Biosciences, Inc., Santa Monica, CA 90403 (US)
(72) Inventor: DECHRISTOPHER, Brian Addison, Cambridge, Massachusetts 02142 (US); BARBE, Guillaume, Cambridge, Massachusetts 02142 (US)
(74) Representative: Müller-Boré & Partner Patentanwälte PartG mbB
(86) International application number: PCT/US2023/016941
(87) International publication number: WO 2023/192506

(56) References cited:
- WO-A1-2018/020474
- WO-A1-2021/138298
- US-A1- 2019 381 019

## Description

### BACKGROUND

Mucosa associated lymphoid tissue lymphoma translocation protein 1 (MALT1) is an intracellular signaling protein, known from innate (e.g., natural killer cells NK, dendritic cells DC, and mast cells) and adaptive immune cells (e.g., T cells and B cells). MALT1 plays an essential role in influencing immune responses. For example, in T cell receptor signaling, MALT1 mediates nuclear factor κB (NFKB) signaling, leading to T cell activation and proliferation. Accordingly, MALT1 is of interest in the mechanism of autoimmune and inflammatory pathologies. In addition, constitutive (dysregulated) MALT1 activity is associated with cancers such as MALT lymphoma and activated B cell-like diffuse large B Cell lymphoma (ABC-DLBCL). Modulators of MALT1 activity may be useful as potential therapeutics.

WO 2018/020474 describes substituted thiazole-pyridine compounds as MALTI inhibitors.

### SUMMARY

Provided herein are compounds designed to act as MALT1 modulators. In some embodiments, such compounds are envisioned to be useful as therapeutic agents for treating autoimmune and inflammatory diseases, disorders, or conditions or cancers.

In one aspect, provided herein are compounds represented by Formula (I): 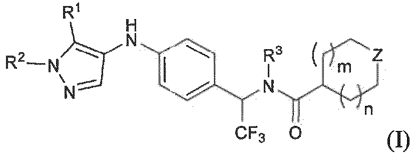 or a stereoisomer and/or a pharmaceutically acceptable salt thereof, wherein:
R¹ is selected from the group consisting of C₁₋₆alkyl, C₁₋₃haloalkyl, C₃₋₆cycloalkyl, and aryl, wherein R¹ may be optionally substituted on one or more available carbons by one, two, three, or more substituents each independently selected from R^{1a};
R² is selected from the group consisting of C₁₋₆alkyl, C₃₋₆cycloalkyl, aryl, and 5-10 membered heteroaryl, wherein R² may be optionally substituted on one or more available carbons by one, two, three, or more substituents each independently selected from R^{2a};
R³ is hydrogen or C₁₋₃alkyl;
R^{1a} is independently, for each occurrence, hydroxyl or -O-C₁₋₃alkyl;
R^{2a} is independently, for each occurrence, selected from the group consisting of hydroxyl, cyano, C₁₋₆alkyl, and -O-C₁₋₃alkyl;
Z is -C(H)(R^{A})-, -SO₂-, or -NR^{A}-;
R^{A} is -C(O)C₁₋₆alkyl, -C(O)OH, or 5-6 membered heteroaryl;
m is 0 or 1; and
n is 0 or 1.

In another aspect, provided herein are compounds represented by Formula (Ia) 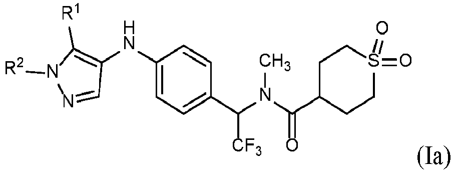 or a stereoisomer and/or a pharmaceutically acceptable salt thereof, wherein:
R¹ is selected from C₁₋₆alkyl, C₁₋₃haloalkyl, or aryl, wherein the C₁₋₆alkyl may be optionally substituted on one or more available carbons by one, two, three, or more substituents each independently selected from R^{1a};
R² is selected from the group consisting of C₁₋₆alkyl, C₃₋₆cycloalkyl, aryl, and 5-10 membered heteroaryl, wherein R² may be optionally substituted on one or more available carbons by one, two, three, or more substituents each independently selected from R^{2a};
R^{1a} is independently, for each occurrence, hydroxyl or -O-C₁₋₃alkyl; and
R^{2a} is independently, for each occurrence, selected from the group consisting of hydroxyl, cyano, C₁₋₆alkyl, and -O-C₁₋₃alkyl.

In another aspect, provided herein are compounds represented by Formula (Ib) 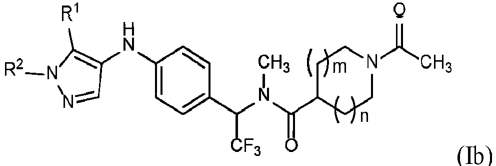 or a stereoisomer and/or a pharmaceutically acceptable salt thereof, wherein:
R¹ is C₁₋₆alkyl, C₁₋₃haloalkyl, or C₃₋₆cycloalkyl, wherein the C₁₋₆alkyl may be optionally substituted with -O-C₁₋₃alkyl;
R² is aryl or 5-6 membered heteroaryl, wherein the aryl may be optionally substituted with cyano;
m is 0 or 1; and
n is 0 or 1.

In another aspect, provided herein are compounds represented by Formula (Ic) 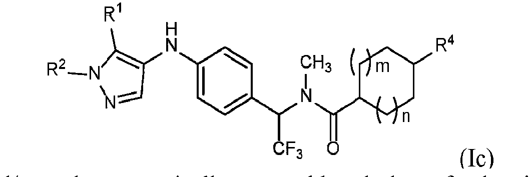 or a stereoisomer and/or a pharmaceutically acceptable salt thereof, wherein:
R¹ is C₁₋₆alkyl or C₁₋₃haloalkyl, wherein the C₁₋₆alkyl may be optionally substituted with -O-C₁₋₃alkyl;
R² is aryl or 5-6 membered heteroaryl, wherein the aryl may be optionally substituted with cyano;
R⁴ is -C(O)OH or 5-6 membered heteroaryl;
m is 0 or 1; and
n is 0 or 1.

In some embodiments, a compound provided herein is selected from a compound set forth in Table 1, or a pharmaceutically acceptable salt thereof.

In another aspect, provided herein is a pharmaceutical composition comprising a compound disclosed herein and a pharmaceutically acceptable carrier. Any references in the description to methods of treatment refer to the compounds and pharmaceutical compositions of the present invention for use in a method for treatment of the human (or animal) body by therapy.

In another aspect, provided herein is a method of treating a cancer in a subject in need thereof, the method comprising administering to the subject a therapeutically effective amount of a compound or a stereoisomer and/or a pharmaceutical composition disclosed herein.

In another aspect, provided herein is a method of treating an autoimmune or inflammatory disorder or disease in a subject in need thereof, the method comprising administering to the subject a therapeutically effective amount of a compound or a pharmaceutical composition disclosed herein.

### DETAILED DESCRIPTION

As generally described herein, the present invention provides compounds designed, for example, to act as MALT1 modulators. In certain embodiments, such compounds are envisioned to be useful as therapeutic agents for treating autoimmune and inflammatory diseases, disorders, or conditions or cancers.

### Definitions

### Chemical definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

It is to be understood that compounds that have the same molecular formula but differ in the nature or sequence of bonding of their atoms or the arrangement of their atoms in space are termed "isomers." Isomers that differ in the arrangement of their atoms in space are termed "stereoisomers." Stereoisomers that are not mirror images of one another are termed "diastereomers" and those that are non-superimposable mirror images of each other are termed "enantiomers." When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The invention additionally encompasses compounds described herein as individual isomers substantially free of other isomers, and alternatively, as mixtures of various isomers.

As used herein a pure enantiomeric compound is substantially free from other enantiomers or stereoisomers of the compound (*i*.*e*., in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the enantiomer. In certain embodiments, the weights are based upon total weight of all enantiomers or stereoisomers of the compound.

In the compositions provided herein, an enantiomerically pure compound can be present with other active or inactive ingredients. For example, a pharmaceutical composition comprising enantiomerically pure R-compound can comprise, for example, about 90% excipient and about 10% enantiomerically pure R-compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of the compound. In certain embodiments, the active ingredient can be formulated with little or no excipient or carrier.

Compound described herein may also comprise one or more isotopic substitutions. For example, H may be in any isotopic form, including ¹H, ²H (D or deuterium), and ³H (T or tritium); C may be in any isotopic form, including ¹²C, ¹³C, and ¹⁴C; O may be in any isotopic form, including ¹⁶O and ¹⁸O; F may be in any isotopic form, including ¹⁸F and ¹⁹F; and the like.

The following terms are intended to have the meanings presented therewith below and are useful in understanding the description and intended scope of the present invention. When describing the invention, which may include compounds and pharmaceutically acceptable salts thereof, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein. The articles "a" and "an" may be used herein to refer to one or to more than one *(i.e.* at least one) of the grammatical objects of the article. By way of example "an analogue" means one analogue or more than one analogue.

When a range of values is listed, it is intended to encompass each value and sub-range within the range. For example, "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

As used herein, "alkyl" refers to a radical of a straight-chain or branched saturated hydrocarbon group, e.g., having 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). Examples of C₁₋₆ alkyl groups include methyl, ethyl, propyl, isopropyl, butyl, isobutyl, pentyl, hexyl, and the like.

As used herein, "alkenyl" refers to a radical of a straight-chain or branched hydrocarbon group having from 2 to 20 carbon atoms, one or more carbon-carbon double bonds (e.g., 1, 2, 3, or 4 carbon-carbon double bonds), and optionally one or more carbon-carbon triple bonds (e.g., 1, 2, 3, or 4 carbon-carbon triple bonds) ("C₂₋₂₀ alkenyl"). In certain embodiments, alkenyl does not contain any triple bonds. In some embodiments, an alkenyl group has 2 to 10 carbon atoms ("C₂₋₁₀ alkenyl"). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂-₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like.

As used herein, "alkynyl" refers to a radical of a straight-chain or branched hydrocarbon group having from 2 to 20 carbon atoms, one or more carbon-carbon triple bonds (e.g., 1, 2, 3, or 4 carbon-carbon triple bonds), and optionally one or more carbon-carbon double bonds (e.g., 1, 2, 3, or 4 carbon-carbon double bonds) ("C₂₋₂₀ alkynyl"). In certain embodiments, alkynyl does not contain any double bonds. In some embodiments, an alkynyl group has 2 to 10 carbon atoms ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like.

As used herein, "alkylene," "alkenylene," "alkynylene," "cycloalkylene," "heterocyclylene," "heteroarylene," and "phenylene" refer to a divalent radical of an alkyl, alkenyl, alkynyl, cycloalkyl, heterocyclyl (e.g., saturated and partially saturated), heteroaryl, and phenyl group respectively.

When a range or number of carbons is provided for a particular "alkylene," "alkenylene," or "alkynylene," group, it is understood that the range or number refers to the range or number of carbons in the linear carbon divalent chain. "Alkylene," "alkenylene," and "alkynylene," groups may be substituted or unsubstituted with one or more substituents as described herein.

As used herein, "aryl" refers to a radical of a monocyclic or polycyclic (e.g., bicyclic or tricyclic) 4n+2 aromatic ring system (e.g., having 6, 10, or 14 π electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, an aryl group has fourteen ring carbon atoms ("C₁₄ aryl"; e.g., anthracyl). Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, and indenyl.

As used herein, "heteroaryl" refers to a radical of a 5-10 membered monocyclic or bicyclic 4n+2 aromatic ring system (e.g., having 6 or 10 electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. For bicyclic heteroaryl groups wherein one ring does not contain a heteroatom (e.g., indolyl, quinolinyl, carbazolyl, and the like), the point of attachment can be on either ring, i.e., either the ring bearing a heteroatom (e.g., 2-indolyl) or the ring that does not contain a heteroatom (e.g., 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Examples of representative heteroaryls include the following: 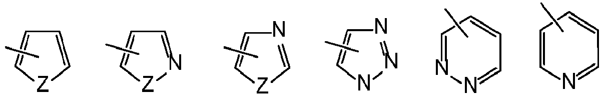 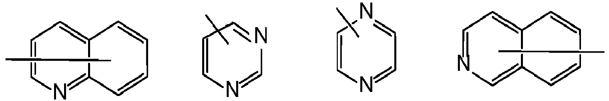 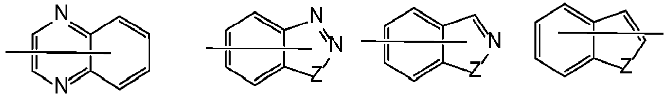 wherein each Z is selected from carbonyl, N, NR⁶⁵, O, and S; and R⁶⁵ is independently hydrogen, C₁₋₈ alkyl, C₃₋₁₀ carbocyclyl, 4-10 membered heterocyclyl, C₆-C₁₀ aryl, and 5-10 membered heteroaryl.

As used herein, "carbocyclyl" or "carbocyclic" refers to a radical of a non-aromatic cyclic hydrocarbon group having from 3 to 10 ring carbon atoms ("C₃₋₁₀ carbocyclyl") and zero heteroatoms in the non-aromatic ring system. In some embodiments, a carbocyclyl group has 3 to 8 ring carbon atoms ("C₃₋₈ carbocyclyl"). In some embodiments, a carbocyclyl group has 3 to 7 ring carbon atoms ("C₃₋₇ carbocycyl"). In some embodiments, a carbocyclyl group has 3 to 6 ring carbon atoms ("C₃₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃),cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1*H*-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated.

The term "cycloalkyl" refers to a monovalent saturated cyclic, bicyclic, or bridged cyclic (e.g., adamantyl) hydrocarbon group of 3-12, 3-8, 4-8, or 4-6 carbons, referred to herein, e.g., as "C₄₋8cycloalkyl," derived from a cycloalkane. Exemplary cycloalkyl groups include, but are not limited to, cyclohexanes, cyclopentanes, cyclobutanes and cyclopropanes.

As used herein, "C₃₋₆ monocyclic cycloalkyl" or "monocyclic C₃₋₆ cycloalkyl" refers to a 3- to 7-membered monocyclic hydrocarbon ring system that is saturated. 3- to 7-membered monocyclic cycloalkyl groups include, without limitation, cyclopropyl, cyclobutyl, cyclopentyl, and cyclohexyl. Where specified as being optionally substituted or substituted, substituents on a cycloalkyl (e.g., in the case of an optionally substituted cycloalkyl) may be present on any substitutable position and, include, *e.g.,* the position at which the cycloalkyl group is attached.

As used herein, "heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. The terms "heterocycle," "heterocyclyl," "heterocyclyl ring," "heterocyclic group," "heterocyclic moiety," and "heterocyclic radical," may be used interchangeably.

In some embodiments, a heterocyclyl group is a 4-7 membered non-aromatic ring system having ring carbon atoms and 1-4 ring heteroatoms, wherein each heteroatom is independently selected from nitrogen, oxygen, and sulfur ("4-7 membered heterocyclyl"). In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, aziridinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

Examples of saturated or partially unsaturated heterocyclic radicals include, without limitation, tetrahydrofuranyl, tetrahydrothienyl, terahydropyranyl, pyrrolidinyl, pyridinonyl, pyrrolidonyl, piperidinyl, oxazolidinyl, piperazinyl, dioxanyl, dioxolanyl, morpholinyl, dihydrofuranyl, dihydropyranyl, dihydropyridinyl, tetrahydropyridinyl, dihydropyrimidinyl, oxetanyl, azetidinyl and tetrahydropyrimidinyl. Where specified as being optionally substituted or substituted, substituents on a heterocyclyl (e.g., in the case of an optionally substituted heterocyclyl) may be present on any substitutable position and, include, *e.g.,* the position at which the heterocyclyl group is attached.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, e.g., heteroalkyl; carbocyclyl, e.g., heterocyclyl; aryl, e.g., heteroaryl; and the like having from 1 to 5, and particularly from 1 to 3 heteroatoms.

As used herein, "cyano" refers to -CN.

The terms "halo" and "halogen" as used herein refer to an atom selected from fluorine (fluoro, -F), chlorine (chloro, -Cl), bromine (bromo, -Br), and iodine (iodo, -I). In certain embodiments, the halo group is either fluoro or chloro.

The term "alkoxy," as used herein, refers to an alkyl group which is attached to another moiety via an oxygen atom (-O(alkyl)). Non-limiting examples include e.g., methoxy, ethoxy, propoxy, and butoxy.

"Haloalkoxy" is a haloalkyl group which is attached to another moiety via an oxygen atom such as, e.g., but are not limited to -OCHF₂ or -OCF₃.

The term "haloalkyl" includes mono, poly, and perhaloalkyl groups substituted with one or more halogen atoms where the halogens are independently selected from fluorine, chlorine, bromine, and iodine. For the group C₁₋₄haloalkyl-O-C₁₋₄alkyl, the point of attachment occurs on the alkyl moiety which is halogenated.

As used herein, "oxo" refers to -C=O.

In general, the term "substituted", whether preceded by the term "optionally" or not, means that at least one hydrogen present on a group (e.g., a carbon or nitrogen atom) is replaced with a permissible substituent, e.g., a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position.

Nitrogen atoms can be substituted or unsubstituted as valency permits, and include primary, secondary, tertiary, and quaternary nitrogen atoms. Exemplary nitrogen atom substituents include, but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, - SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, - P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{cc} groups attached to a nitrogen atom are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc} and R^{dd} are as defined above.

These and other exemplary substituents are described in more detail in the **Detailed Description,** Examples, and **Claims.** The invention is not intended to be limited in any manner by the above exemplary listing of substituents.

### Other Definitions

As used herein, "pharmaceutically acceptable carrier" refers to a non-toxic carrier, adjuvant, or vehicle that does not destroy the pharmacological activity of the compound with which it is formulated. Pharmaceutically acceptable carriers, adjuvants or vehicles that may be used in the compositions described herein include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

As used herein, "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge *et al.,* describes pharmaceutically acceptable salts in detail in J. Pharmaceutical Sciences (1977) 66:1-19. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methane sulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

As used herein, a "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human," "patient," and "subject" are used interchangeably herein.

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition ("prophylactic treatment").

As used herein, the "effective amount" of a compound refers to an amount sufficient to elicit the desired biological response. As will be appreciated by those of ordinary skill in this art, the effective amount of a compound of the invention may vary depending on such factors as the desired biological endpoint, the pharmacokinetics of the compound, the disease being treated, the mode of administration, and the age, health, and condition of the subject An effective amount encompasses therapeutic and prophylactic treatment.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

### Compounds

In one aspect, provided herein are compounds represented by Formula (I): 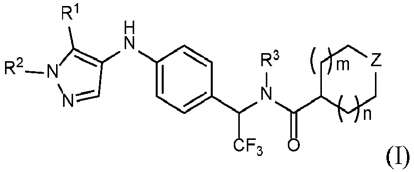 or a stereoisomer and/or a pharmaceutically acceptable salt thereof, wherein:
R¹ is selected from the group consisting of C₁₋₆alkyl, C₁₋₃haloalkyl, C₃₋₆cycloalkyl, and aryl, wherein R¹ may be optionally substituted on one or more available carbons by one, two, three, or more substituents each independently selected from R^{1a};
R² is selected from the group consisting of C₁₋₆alkyl, C₃₋₆cycloalkyl, aryl, and 5-10 membered heteroaryl, wherein R² may be optionally substituted on one or more available carbons by one, two, three, or more substituents each independently selected from R^{2a};
R³ is hydrogen or C₁₋₃alkyl;
R^{1a} is independently, for each occurrence, hydroxyl or -O-C₁₋₃alkyl;
R^{2a} is independently, for each occurrence, selected from the group consisting of hydroxyl, cyano, C₁₋₆alkyl, and -O-C₁₋₃alkyl;
Z is -C(H)(R^{A})-, -SO₂-, or -NR^{A}-;
R^{A} is -C(O)C₁₋₆alkyl, -C(O)OH, or 5-6 membered heteroaryl;
m is 0 or 1; and
n is 0 or 1.

In some embodiments, the compound is represented by Formula (I'): 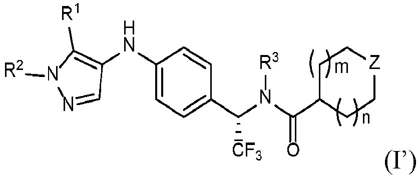 or a stereoisomer and/or a pharmaceutically acceptable salt thereof, wherein:
R¹ is selected from the group consisting of C₁₋₆alkyl, C₁₋₃haloalkyl, C₃₋₆cycloalkyl, and aryl, wherein R¹ may be optionally substituted on one or more available carbons by one, two, three, or more substituents each independently selected from R^{1a};
R² is selected from the group consisting of C₁₋₆alkyl, C₃₋₆cycloalkyl, aryl, and 5-10 membered heteroaryl, wherein R² may be optionally substituted on one or more available carbons by one, two, three, or more substituents each independently selected from R^{2a};
R³ is hydrogen or C₁₋₃alkyl;
R^{1a} is independently, for each occurrence, hydroxyl or -O-C₁₋₃alkyl;
R^{2a} is independently, for each occurrence, selected from the group consisting of hydroxyl, cyano, C₁₋₆alkyl, and -O-C₁₋₃alkyl;
Z is -C(H)(R^{A})-, -SO₂-, or -NR^{A}-;
R^{A} is -C(O)C₁₋₆alkyl, -C(O)OH, or 5-6 membered heteroaryl;
m is 0 or 1; and
n is 0 or 1.

In some embodiments, R¹ is selected from the group consisting of C₁₋₆alkyl, C₁₋₃haloalkyl, C₃₋₆cycloalkyl, and aryl, wherein the C₁₋₆alkyl may be optionally substituted with hydroxyl or -O-C₁₋₃alkyl.

In some embodiments, R¹ is selected from the group consisting of C₁₋₆alkyl, C₁₋₃haloalkyl, C₃₋₆cycloalkyl, and aryl, wherein the C₁₋₆alkyl may be optionally substituted with hydroxyl or -O-CH₃.

In some embodiments, R¹ is selected from the group consisting of -CH(CH₃)₂, CF₃, cyclopropyl, phenyl,

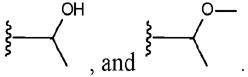

In some embodiments, R² is selected from the group consisting of C₁₋₆alkyl, C₃₋₆cycloalkyl, aryl, and 5-10 membered heteroaryl, wherein the C₁₋₆alkyl and aryl may be optionally substituted with hydroxyl, cyano, C₁₋₆alkyl, or -O-C₁₋₃alkyl.

In some embodiments, R² is selected from the group consisting of C₁₋₆alkyl, C₃₋₆cycloalkyl, aryl, and 5-10 membered heteroaryl, wherein the C₁₋₆alkyl and aryl may be optionally substituted with hydroxyl, cyano, CH₃, or -O-CH₃.

In some embodiments, R² is selected from the group consisting of CH₃, cyclopropyl, - C(H)(CH₃)₂, -CH₂C(CH₃)₃, phenyl, 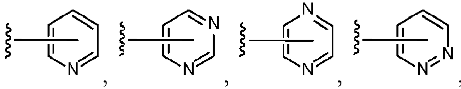 , and

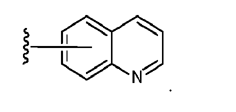

In some embodiments, R² is selected from the group consisting of CH₃, cyclopropyl, - C(H)(CH₃)₂, -CH₂C(CH₃)₃, phenyl,

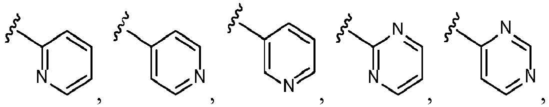

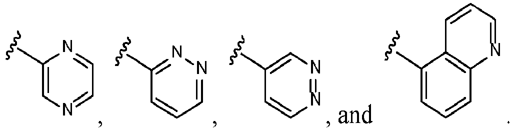

In some embodiments, R² is C₁₋₆alkyl or aryl, wherein the C₁₋₆alkyl and aryl is substituted with hydroxyl, cyano, CH₃, or -O-CH₃.

In some embodiments, R² is C₁₋₆alkyl or phenyl, wherein the C₁₋₆alkyl or phenyl is substituted with hydroxyl, cyano, CH₃, or -O-CH₃.

In some embodiments, R² is selected from the group consisting of

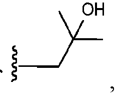

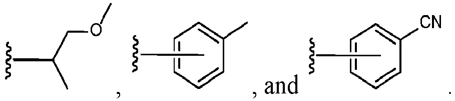

In some embodiments, R² is selected from the group consisting of

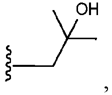

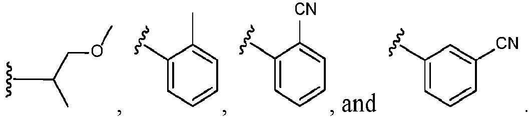

In some embodiments, R² is selected from the group consisting of CH₃, cyclopropyl, - C(H)(CH₃)₂, -CH₂C(CH₃)₃, phenyl, 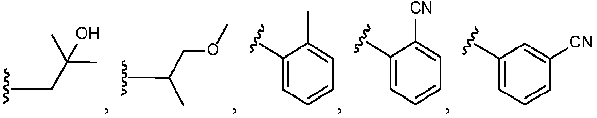 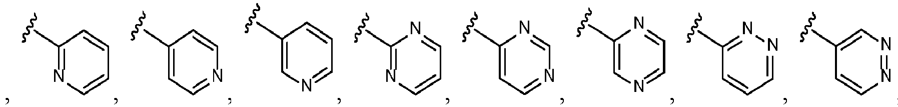 , and

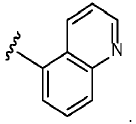

In some embodiments, R³ is hydrogen or CH₃. In some embodiments, R³ is CH₃.

In some embodiments, Z is -SO₂-.

In some embodiments, m is 1 and n is 1.

In some embodiments, Z is -NR^{A}-.

In some embodiments, m is 0 and n is 0. In some embodiments, m is 1 and n is 1.

In some embodiments, R^{A} is -C(O)C₁₋₆alkyl. In some embodiments, R^{A} is -C(O)CH₃.

In some embodiments, Z is -C(H)(R^{A})-.

In some embodiments, m is 0 and n is 0.

In some embodiments, R^{A} is -C(O)OH.

In some embodiments, m is 1 and n is 1.

In some embodiments, R^{A} is -C(O)OH or 5-6 membered heteroaryl. In some embodiments, R^{A} is -C(O)OH or

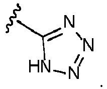

In another aspect, provided herein are compounds represented by Formula (Ia): 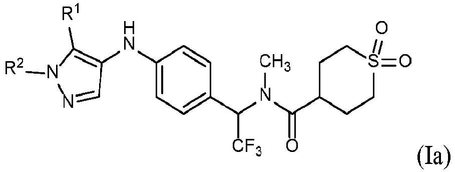 or a stereoisomer and/or a pharmaceutically acceptable salt thereof, wherein:
R¹ is selected from C₁₋₆alkyl, C₁₋₃haloalkyl, or aryl, wherein the C₁₋₆alkyl may be optionally substituted on one or more available carbons by one, two, three, or more substituents each independently selected from R^{1a};
R² is selected from the group consisting of C₁₋₆alkyl, C₃₋₆cycloalkyl, aryl, and 5-10 membered heteroaryl, wherein R² may be optionally substituted on one or more available carbons by one, two, three, or more substituents each independently selected from R^{2a};
R^{1a} is independently, for each occurrence, hydroxyl or -O-C₁₋₃alkyl; and
R^{2a} is independently, for each occurrence, selected from the group consisting of hydroxyl, cyano, C₁₋₆alkyl, and -O-C₁₋₃alkyl.

In some embodiments, the compound is represented by Formula (Ia'): 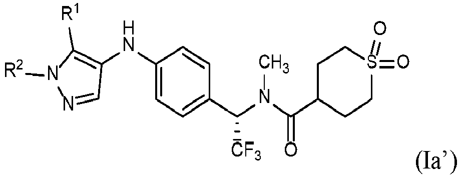 or a stereoisomer and/or a pharmaceutically acceptable salt thereof, wherein:
R¹ is selected from C₁₋₆alkyl, C₁₋₃haloalkyl, or aryl, wherein the C₁₋₆alkyl may be optionally substituted on one or more available carbons by one, two, three, or more substituents each independently selected from R^{1a};
R² is selected from the group consisting of C₁₋₆alkyl, C₃₋₆cycloalkyl, aryl, and 5-10 membered heteroaryl, wherein R² may be optionally substituted on one or more available carbons by one, two, three, or more substituents each independently selected from R^{2a};
R^{1a} is independently, for each occurrence, hydroxyl or -O-C₁₋₃alkyl; and
R^{2a} is independently, for each occurrence, selected from the group consisting of hydroxyl, cyano, C₁₋₆alkyl, and -O-C₁₋₃alkyl.

In some embodiments, R¹ is C₁₋₆alkyl, C₁₋₃haloalkyl, or aryl, wherein the C₁₋₆alkyl may be optionally substituted with hydroxyl or -O-C₁₋₃alkyl.

In some embodiments, R¹ is C₁₋₆alkyl, C₁₋₃haloalkyl, or aryl, wherein the C₁₋₆alkyl may be optionally substituted with hydroxyl or -O-CH₃.

In some embodiments, R¹ is selected from the group consisting of -CH(CH₃)₂, CF₃, phenyl,

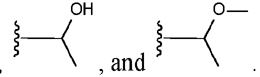

In some embodiments, R² is selected from the group consisting of C₁₋₆alkyl, C₃₋₆cycloalkyl, aryl, and 5-10 membered heteroaryl, wherein the C₁₋₆alkyl and aryl may be optionally substituted with hydroxyl, cyano, C₁₋₆alkyl, or -O-C₁₋₃alkyl.

In some embodiments, R² is selected from the group consisting of C₁₋₆alkyl, C₃₋₆cycloalkyl, aryl, and 5-10 membered heteroaryl, wherein the C₁₋₆alkyl and aryl may be optionally substituted with hydroxyl, cyano, CH₃, or -O-CH₃.

In some embodiments, R² is selected from the group consisting of CH₃, cyclopropyl, - C(H)(CH₃)₂, -CH₂C(CH₃)₃, phenyl, 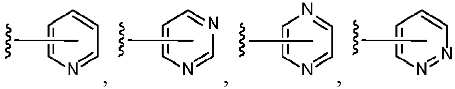 , and

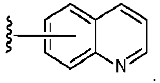

In some embodiments, R² is selected from the group consisting of CH₃, cyclopropyl, - C(H)(CH₃)₂, -CH₂C(CH₃)₃, phenyl,

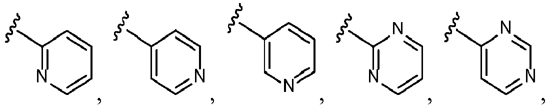

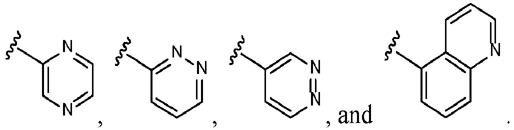

In some embodiments, R² is C₁₋₆alkyl or aryl, wherein the C₁₋₆alkyl and aryl is substituted with hydroxyl, cyano, CH₃, or -O-CH₃.

In some embodiments, R² is C₁₋₆alkyl or phenyl, wherein the C₁₋₆alkyl or phenyl is substituted with hydroxyl, cyano, CH₃, or -O-CH₃.

In some embodiments, R² is selected from the group consisting of

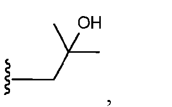

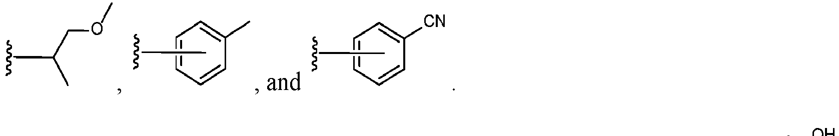

In some embodiments, R² is selected from the group consisting of

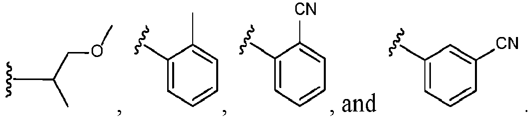

In some embodiments, R² is selected from the group consisting of CH₃, cyclopropyl, - C(H)(CH₃)₂, -CH₂C(CH₃)₃, phenyl, 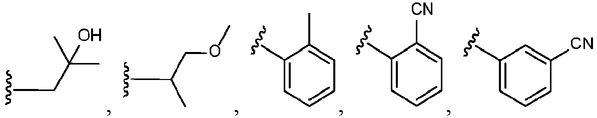 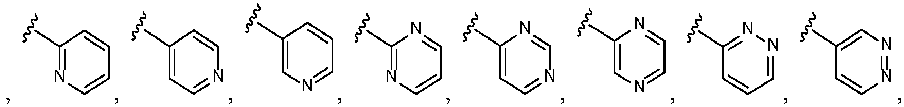 and

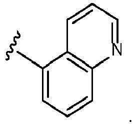

In another aspect, provided herein are compounds represented by Formula (Ib): 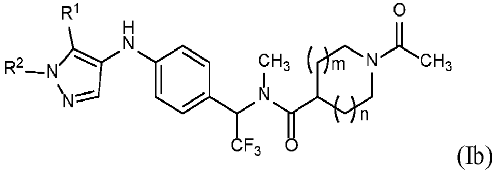 or a stereoisomer and/or a pharmaceutically acceptable salt thereof, wherein:
R¹ is C₁₋₆alkyl, C₁₋₃haloalkyl, or C₃₋₆cycloalkyl, wherein the C₁₋₆alkyl may be optionally substituted with -O-C₁₋₃alkyl;
R² is aryl or 5-6 membered heteroaryl, wherein the aryl may be optionally substituted with cyano;
m is 0 or 1; and
n is 0 or 1.

In some embodiments, the compound is represented by Formula (Ib'): 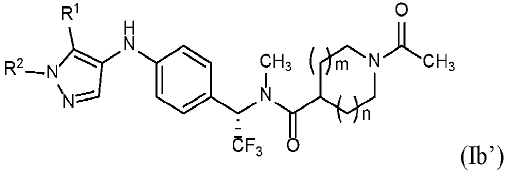 or a stereoisomer and/or a pharmaceutically acceptable salt thereof, wherein:
R¹ is C₁₋₆alkyl, C₁₋₃haloalkyl, or C₃₋₆cycloalkyl, wherein the C₁₋₆alkyl may be optionally substituted with -O-C₁₋₃alkyl;
R² is aryl or 5-6 membered heteroaryl, wherein the aryl may be optionally substituted with cyano;
m is 0 or 1; and
n is 0 or 1.

In some embodiments, R¹ is C₁₋₆alkyl, C₁₋₃haloalkyl, or C₃₋₆cycloalkyl, wherein the C₁₋₆alkyl may be optionally substituted with -O-CH₃.

In some embodiments, R¹ is selected from the group consisting of -CH(CH₃)₂, CF₃, cyclopropyl, and

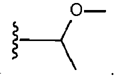

In some embodiments, R² is phenyl or 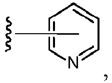 wherein the phenyl is optionally substituted with cyano.

In some embodiments, R² is selected from the group consisting of phenyl,

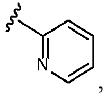

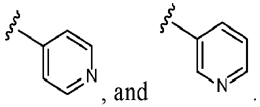

In some embodiments, R² is 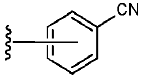 . In some embodiments, R² is 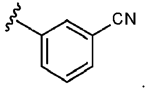 In some embodiments, R² is selected from the group consisting of phenyl,

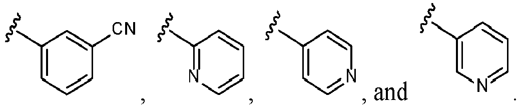

In some embodiments, m is 0 and n is 0. In some embodiments, m is 1 and n is 1.

In another aspect, provided herein are compounds represented by Formula (Ic): 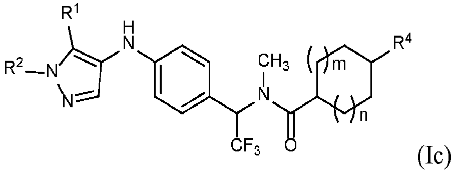 or a stereoisomer and/or a pharmaceutically acceptable salt thereof, wherein:
R¹ is C₁₋₆alkyl or C₁₋₃haloalkyl, wherein the C₁₋₆alkyl may be optionally substituted with -O-C₁₋₃alkyl;
R² is aryl or 5-6 membered heteroaryl, wherein the aryl may be optionally substituted with cyano;
R⁴ is -C(O)OH or 5-6 membered heteroaryl;
m is 0 or 1; and
n is 0 or 1.

In some embodiments, the compound is represented by formula (Ic'): 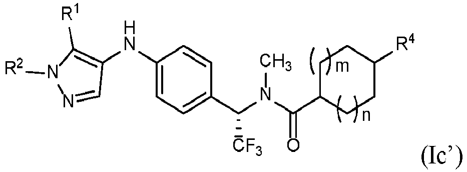 or a stereoisomer and/or a pharmaceutically acceptable salt thereof, wherein:
R¹ is C₁₋₆alkyl or C₁₋₃haloalkyl, wherein the C₁₋₆alkyl may be optionally substituted with -O-C₁₋₃alkyl;
R² is aryl or 5-6 membered heteroaryl, wherein the aryl may be optionally substituted with cyano;
R⁴ is -C(O)OH or 5-6 membered heteroaryl;
m is 0 or 1; and
n is 0 or 1.

In some embodiments, R¹ is C₁₋₆alkyl or C₁₋₃haloalkyl, wherein the C₁₋₆alkyl may be optionally substituted with -O-CH₃.

In some embodiments, R¹ is CF₃ or

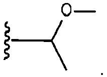

In some embodiments, R² is phenyl, 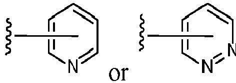 , wherein the phenyl is optionally substituted with cyano.

In some embodiments, R² is selected from the group consisting of phenyl,

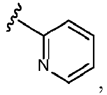

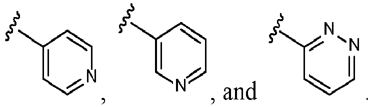

In some embodiments, R² is 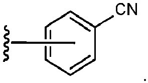 In some embodiments, R² is 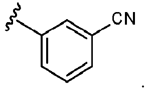 In some embodiments, R² is selected from the group consisting of phenyl,

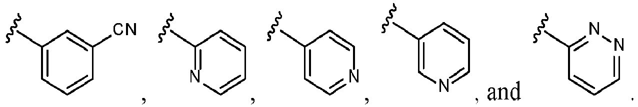

In some embodiments, m is 0 and n is 0.

In some embodiments, R¹ is -C(O)OH.

In some embodiments, the compound is selected from any compound set forth in Table 1, or a pharmaceutically acceptable salt thereof.

**Table 1. List of compounds.**

| **Cmpd No.** | **Structure** |
|---|---|
| 1.1 | 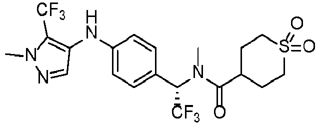 |
| 1.2 | 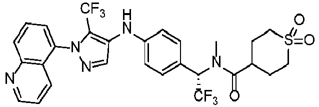 |
| 1.3 | 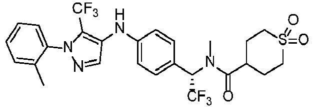 |
| 1.4 | 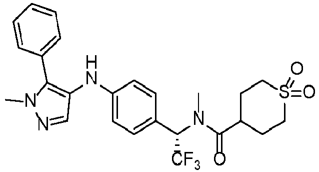 |
| 1.5 | 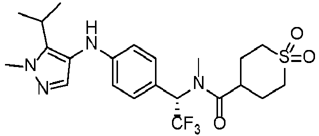 |
| 1.6 | 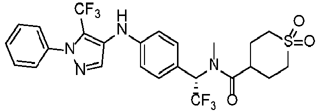 |
| 1.7 | 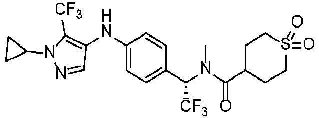 |
| 1.8 | 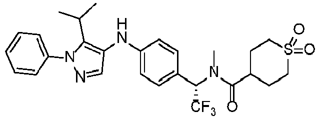 |
| 1.9 | 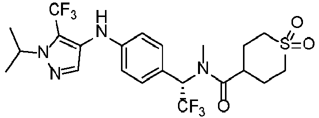 |
| 1.10 | 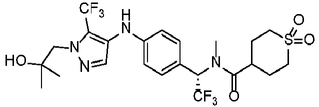 |
| 1.11 | 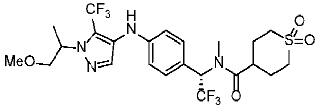 |
| 1.12 | 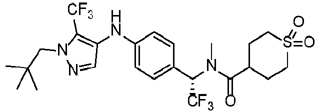 |
| 1.13 | 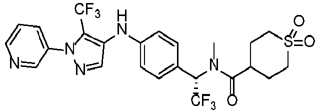 |
| 1.14 | 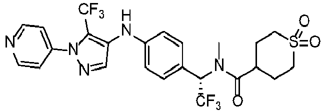 |
| 1.15 | 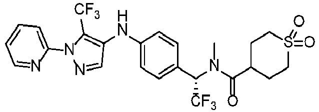 |
| 1.16 | 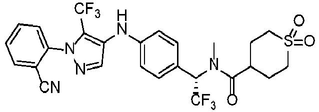 |
| 1.17 | 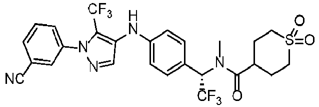 |
| 1.18 | 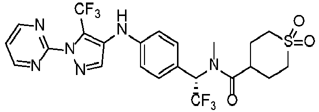 |
| 1.19 | 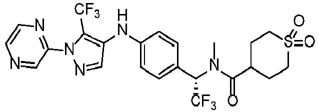 |
| 1.20 | 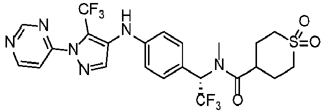 |
| 1.21 | 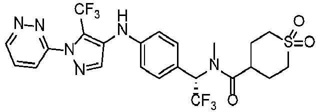 |
| 1.22 | 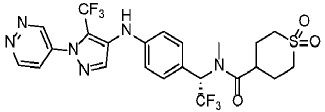 |
| 1.23 | 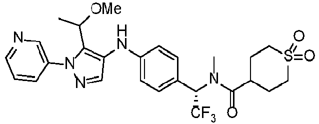 |
| 1.24 | 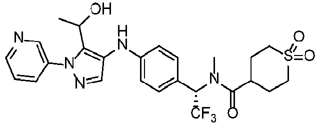 |
| 1.25 | 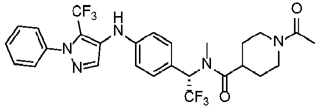 |
| 1.26 | 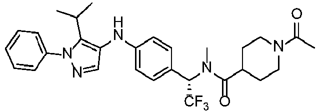 |
| 1.27 | 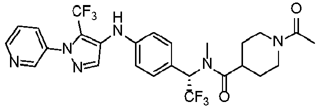 |
| 1.28 | 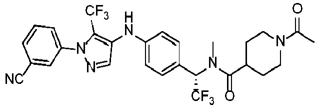 |
| 1.29 | 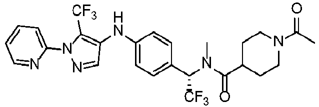 |
| 1.30 | 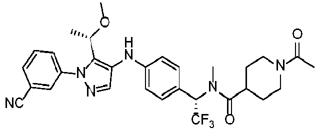 |
| 1.31 | 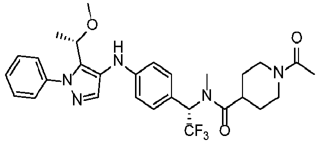 |
| 1.32 | 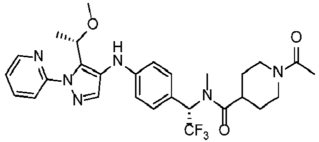 |
| 1.33 | 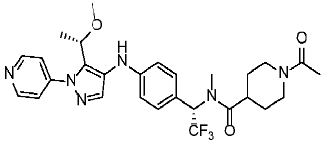 |
| 1.34 | 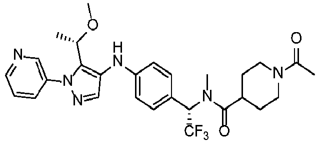 |
| 1.35 | 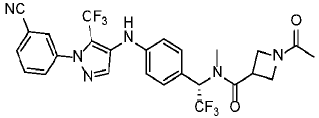 |
| 1.36 | 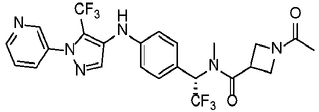 |
| 1.37 | 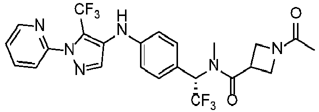 |
| 1.38 | 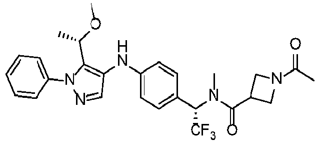 |
| 1.39 | 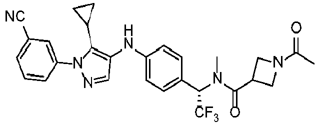 |
| 1.40 | 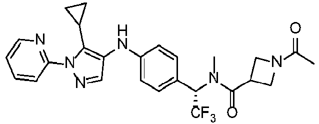 |
| 1.41 | 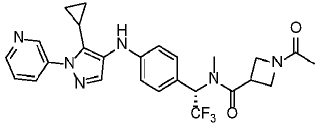 |
| 2.1 | 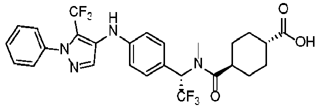 |
| 2.2 | 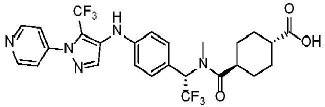 |
| 2.3 | 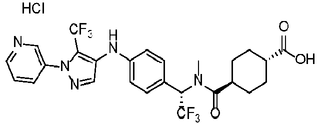 |
| 2.4 | 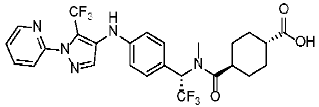 |
| 2.5 | 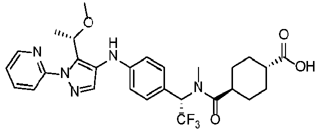 |
| 2.6 | 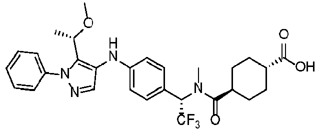 |
| 2.7 | 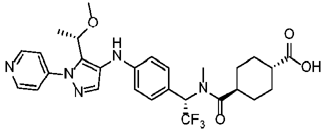 |
| 2.8 | 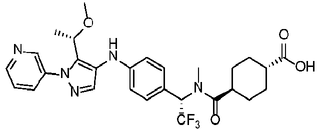 |
| 2.9 | 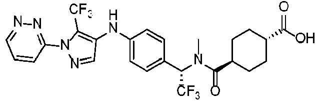 |
| 2.10 | 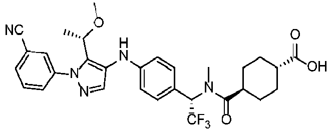 |
| 2.11 | 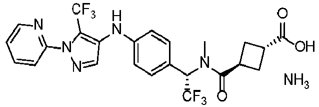 |
| 2.12 | 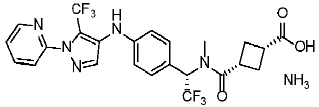 |
| 2.13 | 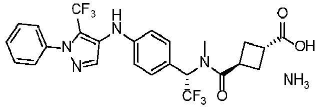 |
| 2.14 | 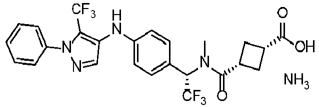 |
| 3.1 | 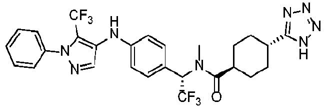 |

### Pharmaceutical Compositions and Routes of Administration

Compounds provided in accordance with the present invention are usually administered in the form of pharmaceutical compositions. This invention therefore provides pharmaceutical compositions that contain, as the active ingredient, one or more of the compounds described, or a pharmaceutically acceptable salt or ester thereof, and one or more pharmaceutically acceptable excipients, carriers, including inert solid diluents and fillers, diluents, including sterile aqueous solution and various organic solvents, permeation enhancers, solubilizers and adjuvants. The pharmaceutical compositions may be administered alone or in combination with other therapeutic agents. Such compositions are prepared in a manner well known in the pharmaceutical art (see, e.g., Remington's Pharmaceutical Sciences, Mace Publishing Co., Philadelphia, Pa. 17th Ed. (1985); and Modern Pharmaceutics, Marcel Dekker, Inc. 3rd Ed. (G. S. Banker & C. T. Rhodes, Eds.)

The pharmaceutical compositions may be administered in either single or multiple doses by any of the accepted modes of administration of agents having similar utilities, for example as described in those patents and patent applications, including rectal, buccal, intranasal and transdermal routes, by intra-arterial injection, intravenously, intraperitoneally, parenterally, intramuscularly, subcutaneously, orally, topically, as an inhalant, or via an impregnated or coated device such as a stent, for example, or an arteryinserted cylindrical polymer.

One mode for administration is parenteral, particularly by injection. The forms in which the novel compositions of the present invention may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles. Aqueous solutions in saline are also conventionally used for injection, but less preferred in the context of the present invention. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating a compound according to the present invention in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterilefiltered solution thereof.

Oral administration is another route for administration of compounds in accordance with the invention. Administration may be via capsule or enteric coated tablets, or the like. In making the pharmaceutical compositions that include at least one compound described herein, the active ingredient is usually diluted by an excipient and/or enclosed within such a carrier that can be in the form of a capsule, sachet, paper or other container. When the excipient serves as a diluent, it can be in the form of a solid, semi-solid, or liquid material (as above), which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl and propylhydroxy-benzoates; sweetening agents; and flavoring agents.

The compositions of the invention can be formulated so as to provide quick, sustained or delayed release of the active ingredient after administration to the patient by employing procedures known in the art. Controlled release drug delivery systems for oral administration include osmotic pump systems and dissolutional systems containing polymer-coated reservoirs or drug-polymer matrix formulations. Examples of controlled release systems are given in U.S. Pat. Nos. 3,845,770; 4,326,525; 4,902,514; and 5,616,345. Another formulation for use in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Pat. Nos. 5,023,252, 4,992,445 and 5,001,139. Such patches may be constructed for continuous, pulsatile, or on demand delivery of pharmaceutical agents.

The compositions are preferably formulated in a unit dosage form. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient (e.g., a tablet, capsule, ampoule). The compounds are generally administered in a pharmaceutically effective amount. It will be understood, however, that the amount of the compound actually administered usually will be determined by a physician, in the light of the relevant circumstances, including the condition to be treated, the chosen route of administration, the actual compound administered and its relative activity, the age, weight, and response of the individual patient, the severity of the patient's symptoms, and the like.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules.

The tablets or pills of the present invention may be coated or otherwise compounded to provide a dosage form affording the advantage of prolonged action, or to protect from the acid conditions of the stomach. For example, the tablet or pill can comprise an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably, the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a facemask tent, or intermittent positive pressure breathing machine. Solution, suspension, or powder compositions may be administered, preferably orally or nasally, from devices that deliver the formulation in an appropriate manner.

In some embodiments, a pharmaceutical composition comprises a disclosed compound, or a stereoisomer and/or pharmaceutically acceptable salt thereof, and a pharmaceutically acceptable carrier.

### Methods of Use

Compounds and compositions described herein are generally useful for modulating MALT1 and are useful for treating diseases or disorders, in particular those susceptible to modulation of proteolytic and/or autoproteolytic activity of MALT1. In some embodiments, the compounds and compositions described herein are useful for inhibiting MALT1. In some embodiments, it is contemplated that the compounds and compositions of the present invention may be useful in the treatment of a disease, a disorder, or a condition characterized by dysregulated NF-kB activation, for example, autoimmune or immunological and inflammatory disorders, allergic disorders, respiratory disorders and oncological disorders.

In typical embodiments, the present invention is intended to encompass the compounds disclosed herein, and the pharmaceutically acceptable salts, tautomeric forms, and polymorphs. Also described are pharmaceutically acceptable esters and prodrugs of such compounds. In some embodiments, the present invention includes a pharmaceutically acceptable addition salt, a solvate (e.g., hydrate) of an addition salt, a tautomeric form, a polymorph, an enantiomer, a mixture of enantiomers, a stereoisomer or mixture of stereoisomers (pure or as a racemic or non-racemic mixture) of a compound described herein, e.g., a compound of Formula I, Ia, Ib, or Ic.

In some embodiments, the autoimmune and inflammatory disorders are selected from arthritis, ankylosing spondylitis, inflammatory bowel disease, ulcerative colitis, gastritis, pancreatitis, Crohn's disease, celiac disease, multiple sclerosis, systemic lupus erythematosus, lupus nephritis, rheumatoid arthritis, rheumatic fever, gout, organ or transplant rejection, acute or chronic graft-versus-host disease, chronic allograft rejection, Behcet's disease, uveitis, psoriasis, psoriatic arthritis, BENTA disease, polymyositis, dermatitis, atopic dermatitis, dermatomyositis, acne vulgaris, myasthenia gravis, hidradenitis suppurativa, Grave's disease, Hashimoto thyroiditis, Sjogren's syndrome, and blistering disorders (e.g., pemphigus vulgaris), antibodymediated vasculitis syndromes, including ANCA.-associated vasculitides, Henoch-Schonlein Purpura, and immune-complex vasculitides (either primary or secondary to infection or cancers).

In some embodiments, the autoimmune or inflammatory disorder or disease is selected from the group consisting of acute graft-versus-host disease, chronic graft-versus-host disease, lupus, scleroderma, psoriatic arthritis, primary sclerosing cholangitis, rheumatoid arthritis, and an inflammatory bowel disease.

In some embodiments, the oncological disorders are selected from carcinoma, sarcoma, lymphoma, leukemia and germ cell tumors, adenocarcinoma, bladder cancer, clear cell carcinoma, skin cancer, brain cancer, cervical cancer, colon cancer, colorectal cancer, endometrial cancer, brain tumors, breast cancer, gastric cancer, germ cell tumors, glioblastoma, hepatic adenomas, Hodgkin's lymphoma, liver cancer, kidney cancer, lung cancer, pancreatic cancer, head/neck/throat cancer, ovarian cancer, dermal tumors, prostate cancer, renal cell carcinoma, stomach cancer, hematologic cancer, medulloblastoma, non-Hodgkin's lymphoma, diffuse large B-cell lymphoma (DLBCL), activated B cell-like diffuse large B Cell lymphoma (ABC-DLBCL), mantle cell lymphoma, marginal zone lymphoma, T cell lymphomas, in particular Sezary syndrome, Mycosis fungoides, cutaneous T-cell lymphoma, T-cell acute lymphoblastic leukemia, melanoma, mucosa-associated lymphoid tissue (MALT) lymphoma, multiple myeloma, plasma cell neoplasm, lentigo maligna melanomas, acral lentiginous melanoma, squamous cell carcinoma, chronic myelogenous leukemia, myeloid leukemia, superficial spreading melanoma, acral lentiginous melanoma, mucosal melanoma, nodular melanoma, polypoid melanoma, desmoplastic melanoma, amelanotic melanoma, soft-tissue melanoma, melanoma with small nevus-like cells, melanoma with features of a Spitz nevus, uveal melanoma, precursor T-cell, leukemia/lymphoma, acute myeloid leukemia, chronic myeloid leukemia, acute lymphocytic leukemia, follicular lymphoma, chronic lymphocytic leukemia/lymphoma, Burkitt's lymphoma, mycosis fungoides, peripheral T-cell lymphoma, nodular sclerosis form of Hodgkin lymphoma, mixed-cellularity subtype of Hodgkin lymphoma, non-small-cell lung cancer, large-cell carcinoma, and small-cell lung carcinoma.

In some embodiments, the oncological disorder is a cancer in the form of a tumor or a blood born cancer. In some embodiments, the tumor is a solid tumor. In some embodiments, the tumor is malignant and/or metastatic. In some embodiments, the tumor is selected from an adenoma, an adenocarcinoma, a blastoma (e.g., hepatoblastoma, glioblastoma, neuroblastoma and retinoblastoma), a carcinoma (e.g., colorectal carcinoma or heptatocellular carcinoma, pancreatic, prostate, gastric, esophageal, cervical, and head and neck carcinomas, and adenocarcinoma), a desmoid tumor, a desmoplastic small round cell tumor, an endocrine tumor, a germ cell tumor, a lymphoma, a leukemia, a sarcoma (e.g, Ewing sarcoma, osteosarcoma, rhabdomyosarcoma, or any other soft tissue sarcoma), a Wilms tumor, a lung tumor, a colon tumor, a lymph tumor, a breast tumor or a melanoma.

In some embodiments, the allergic disorder is selected from contact dermatitis, celiac disease, asthma, hypersensitivity to house dust mites, pollen and related allergens, and berylliosis.

In some embodiments, the respiratory disorder is selected from asthma, bronchitis, chronic obstructive pulmonary disease (COPD), cystic fibrosis, pulmonary edema, pulmonary embolism, pneumonia, pulmonary sarcoidosis, silicosis, pulmonary fibrosis, respiratory failure, acute respiratory distress syndrome, primary pulmonary hypertension and emphysema.

In some embodiments, the compounds and compositions of the present invention may be useful in the treatment of rheumatoid arthritis, systemic lupus erythematosus, vasculitic conditions, allergic diseases, asthma, chronic obstructive pulmonary disease (COPD), acute or chronic transplant rejection, graft versus host disease, cancers of hematopoietic origin or solid tumors, chronic myelogenous leukemia, myeloid leukemia, non-Hodgkin lymphoma or other B cell lymphomas.

### Combination Therapy

A compound of composition described herein may be administered in combination with another agent or therapy. A subject to be administered a compound disclosed herein may have a disease, disorder, or condition, or a symptom thereof, that would benefit from treatment with another agent or therapy.

In some embodiments, the compound of composition described herein may be administered either simultaneously with, or before or after, one or more other therapeutic agent. In some embodiments, the compound of composition described herein may be administered separately, by the same or different route of administration, or together in the same pharmaceutical composition as the other agents.

In some embodiments, the compound described herein may be administered as the sole active ingredient or in conjunction with, e.g., as an adjuvant to, other drugs e.g., immunosuppressive or immunomodulating agents or other anti-inflammatory agents, e.g., for the treatment or prevention of alio- or xenograft acute or chronic rejection or inflammatory or autoimmune disorders, or a chemotherapeutic agent, e.g., a malignant cell anti-proliferative agent. For example, the compounds of the invention may be used in combination with a calcineurin inhibitor, e.g., cyclosporin A or FK 506; a mTOR inhibitor, e.g., rapamycin, 40-0-(2-hydroxyethyl)-rapamycin, biolimus-7 or biolimus-9; an ascomycin having immunosuppressive properties, e.g., ABT-281, ASM981; corticosteroids; cyclophosphamide; azathioprene; methotrexate-, leflunomide; mizoribine; mycophenolic acid or salt; mycophenolate mofetil; or IL-1 beta inhibitor.

In some embodiments, the compound described herein is combined with a co-agent which is a PIK3 inhibitor.

In some embodiments, the compound described herein is combined with co-agetit that influence BTK (Bruton's tyrosine kinase).

For the treatment of oncological diseases, the compound described herein may be used in combination with B-cell modulating agents, e g., Rituximab, Ofatumumab, BTK or SYK inhibitors, inhibitors of PKC, PI3K, PDK, PIM, JAK and mTOR and BH3 mimetics.

### EXAMPLES

The representative examples that follow are intended to help illustrate the invention, and are not intended to, nor should they be construed to, limit the scope of the invention.

The compounds provided herein can be prepared from readily available starting materials using the following general methods and procedures. It will be appreciated that where typical or preferred process conditions (i.e., reaction temperatures, times, mole ratios of reactants, solvents, pressures, etc.) are given, other process conditions can also be used unless otherwise stated. Optimal reaction conditions may vary with the particular reactants or solvent used, but such conditions can be determined by one skilled in the art by routine optimization.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and P. G. M. Wuts, Protecting Groups in Organic Synthesis, Second Edition, Wiley, New York, 1991, and references cited therein.

The compounds provided herein may be isolated and purified by known standard procedures. Such procedures include recrystallization, filtration, flash chromatography, trituration, high pressure liquid chromatography (HPLC), or supercritical fluid chromatography (SFC). Note that flash chromatography may either be performed manually or via an automated system. The compounds provided herein may be characterized by known standard procedures, such as nuclear magnetic resonance spectroscopy (NMR) or liquid chromatography mass spectrometry (LCMS). NMR chemical shifts are reported in part per million (ppm) and are generated using methods well known to those of skill in the art.

### List of Abbreviations:

- EtOAc, EA: ethyl acetate
- THF: tetrahydrofuran
- PE: petroleum ether
- MeOH: methanol
- EtOH: ethanol
- DMF: N,N-dimethylformamide
- FA: formic acid
- DCM: dichloromethane
- MeCN, ACN, CH₃CN: acetonitrile
- xantphos: 4,5-Bis(diphenylphosphino)-9,9-dimethylxanthene
- h, hr, hrs: hour(s)
- Calcd: calculated
- UV: ultraviolet
- rt: room temperature
- DIEA, DIPEA: N,N-Diisopropylethylamine
- Pd₂(dba)₃: tris(dibenzylideneacetone)dipalladium(0)
- Et₃N, TEA: triethylamine
- Cs₂CO₃: cesium carbonate
- INT: intermediate
- t-BuOH: tert-butyl alcohol
- Boc: tert-butyloxycarbonyl
- LiOH.H₂O: lithium hydroxide monohydrate
- NH₄Cl: ammonium chloride
- DPPA: diphenylphosphoryl azide
- NaOH: sodium hydroxide
- (COCl)₂: oxalyl chloride
- NaN₃: sodium azide
- CDI: 1,1'-Carbonyldiimidazole
- BnOH: benzyl alcohol
- Cbz: benzyloxycarbonyl
- SnCl₂: stannous chloride
- HCl: hydrochloric acid
- NaNO₂: sodium nitrite
- KOH: potassium hydroxide
- BH₃.DMS: borane dimethyl sulfide complex
- H₂O₂: hydrogen peroxide
- RuCl₃: ruthenium trichloride
- NaIO₄: sodium periodate
- NaH: sodium hydride
- KNO₃: potassium nitrate
- H₂SO₄: sulfuric acid
- Pd/C: palladium on carbon
- DMF-DMA, (CH₃)₂NCH(OCH₃)₂: N,N-Dimethylformamide dimethyl acetal
- CF₃CH₂OH: 2,2,2-Trifluoroethanol
- N₂H₄: hydrazine
- CH₃(NH)NH₂: methylhydrazine
- MeI: methyl iodide
- LiHMDS: lithium bis(trimethylsilyl)amide
- (n-Bu)₄NOAc: tetrabutylammonium acetate
- TMSCF₃: trimethyl(trifluoromethyl)silane
- AcOH: acetic acid
- PtO₂: platinum oxide
- NaBH₃CN: sodium cyanoborohydride
- CuCN: cuprous cyanide
- MgCl₂: magnesium chloride
- K₂CO₃: potassium carbonate
- CuI: cuprous iodide
- CH₂1₂: diiodomethane
- Pd(PPh₃)₂Cl₂: bis(triphenylphosphine)palladium(II) dichloride
- NaBH₄: sodium borohydride

### Example 1. Preparation of the compounds

Methods for preparing compounds described herein are illustrated in the following synthetic schemes. These schemes are given for the purpose of illustrating the invention. Starting materials shown in the schemes may be obtained from commercial sources or can be prepared from commercially available sources based on procedures described in the literature.

### 1. Compounds Prepared using Scheme 1

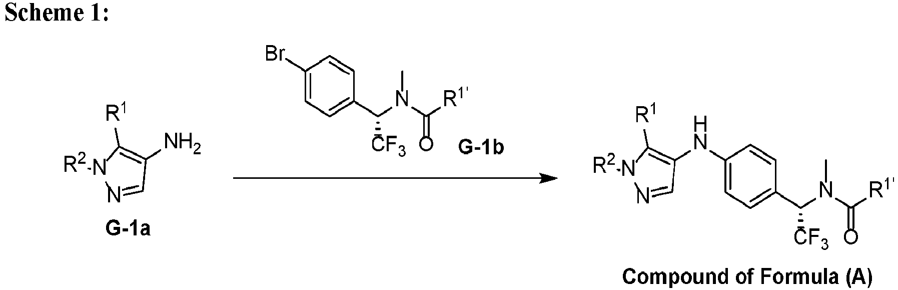

G-la is reacted with R^{1'}-containing carboxamide **G-1b** to provide a compound of Formula (A).

### Exemplary procedure of compounds prepared using scheme 1:

### Synthesis of (S)-N-methyl-N-(2,2,2-trifluoro-1-(4-((1-methyl-5-(trifluoromethyl)-1H-pyrazol-4-yl)amino)phenyl)ethyl)tetrahydro-2H-thiopyran-4-carboxamide 1,1-dioxide (Compound 1.1)

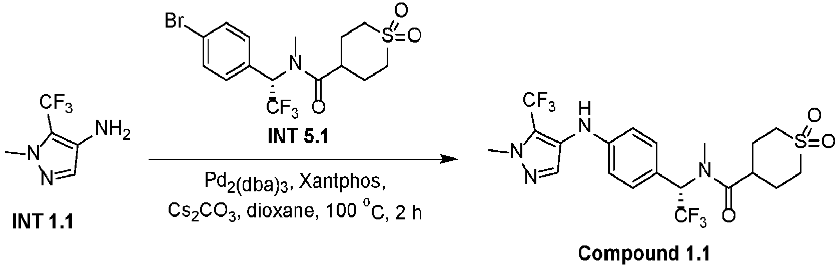

To a mixture of 1-methyl-5-(trifluoromethyl)-1H-pyrazol-4-amine [free base of**INT 1.1]** (50 mg, 302 µmol) and (S)-N-(1-(4-bromophenyl)-2,2,2-trifluoroethyl)-N-methyltetrahydro-2H-thiopyran-4-carboxamide 1,1-dioxide **[INT 5.1]** (155 mg, 362 µmol) in dioxane (2 mL) was added Pd₂(dba)₃ (27.6 mg, 30.2 µmol), xantphos (34.9 mg, 60.4 µmol) and Cs₂CO₃ (295 mg, 906 µmol). The mixture was stirred at 100 °C for 2 hr under N₂ atmosphere, after which it was quenched with water (30 mL) and extracted with EtOAc (3 x 20 mL). The combined organic layers were dried over anhydrous Na₂SO₄ and concentrated under reduced pressure to give the crude product, which was purified by prep-HPLC (column: YMC-Actus Triart C18 150*30 mm*5 µm, condition: 42%-62% CH₃CN in water (0.05% ammonia hydroxide v/v)-ACN, flow rate: 35 mL/min) to give (S)-N-methyl-N-(2,2,2-trifluoro-1-(4-((1-methyl-5-(trifluoromethyl)-1H-pyrazol-4-yl)amino)phenyl)ethyl)tetrahydro-2H-thiopyran-4-carboxamide 1,1-dioxide **[Compound 1.1]** (20.6 mg, 40.1 µmol, 13.3% yield) as a white dry powder. m/z: [M + H]+ Calcd for C20H23F6N403S 513.1; Found 513.2. ¹H NMR (400 MHz, CD₃OD) δ = 7.55 (s, 1H), 7.19 (d, J = 8.4 Hz, 2H), 6.82 (d, J = 8.4 Hz, 2H), 6.57 - 5.95 (m, 1H), 4.00 (s, 3H), 3.30 - 3.09 (m, 5H), 2.98 (s, 3H), 2.39 - 2.10 (m, 4H).

### 2. Compounds Prepared using Scheme 2

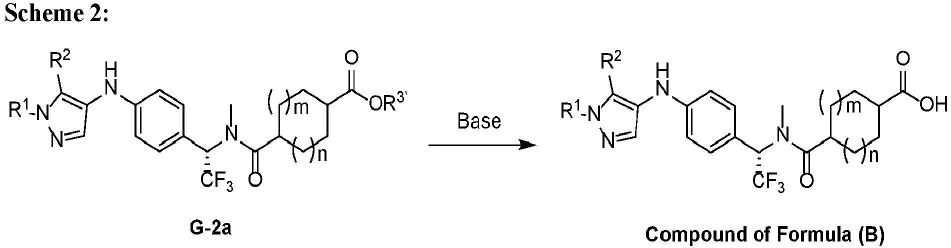

Starting material **G-2a** is treated with base (LiOH.H₂O or NaOH) to provide a compound of Formula (B). R² is phenyl, pyridyl, or pyridazine; R¹ is CF₃ or (S)-methoxyethane; and m and n are each independently 0 or 1. R^{3'} is methyl or benzyl.

### Exemplary procedures of compounds prepared using scheme 2:

### Synthesis of (1S,4r)-4-(methyl((S)-2,2,2-trifluoro-1-(4-((1-phenyl-5-(trifluoromethyl)-1H-pyrazol-4-yl)amino)phenyl)ethyl)carbamoyl)cyclohexane-1-carboxylic acid (Compound 2.1)

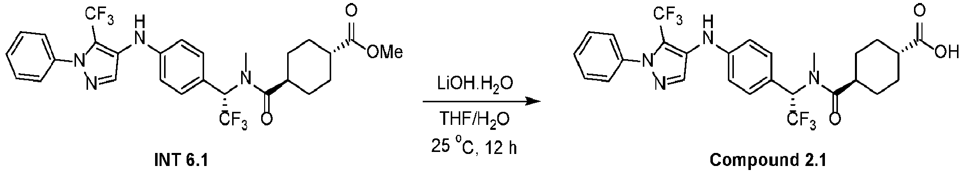

To a mixture of methyl (1r,4r)-4-{methyl[(1S)-2,2,2-trifluoro-1-(4-{[1-phenyl-5-(trifluoromethyl)-1H-pyrazol-4- yl]amino}phenyl)ethyl]carbamoyl}cyclohexane-1-carboxylate **[INT 6.1]** (280 mg, 480 µmol) in THF (3 mL) and H₂O (1 mL) was added LiOH.H₂O (60.4 mg, 1.44 mmol) and the mixture was stirred at 25 °C for 12 hr. The mixture was concentrated under reduced pressure to afford the crude product, which was purified by Prep-HPLC (column: YMCActus Triart C18 150*30 mm*5 µm, table: 50-70% B (A = water(0.225% formic acid)-ACN), B = acetonitrile), flowrate: 35 mL/min, UV Detector 220 nm) to afford (1S,4r)-4-(methyl((S)-2,2,2-trifluoro-1-(4-((1-phenyl-5-(trifluoromethyl)-1H-pyrazol-4-yl)amino)phenyl)ethyl)carbamoyl)cyclohexane-1-carboxylic acid **[Compound 2.1]** (30.5 mg, 53.6 µmol, 11.2% yield) as a white dry powder. m/z: [M + H]+ Calcd for C27H27F6N4O3 569.2 Found 569.1. ¹H NMR (400 MHz, CDCl₃) δ = 7.80 (s, 1H), 7.54 - 7.44 (m, 5H), 7.26 - 7.24 (m, 2H), 6.94 (d, J = 8.8 Hz, 2H), 6.60 (q, J = 9.2 Hz, 1H), 5.51 (s, 1H), 2.90 (s, 3H), 2.62 - 2.53 (m, 1H), 2.42 (tt, J = 3.6, 12.0 Hz, 1H), 2.15 (br t, J = 8.8 Hz, 2H), 2.01 - 1.82 (m, 2H), 1.67 (q, J = 12.8 Hz, 2H), 1.56 - 1.42 (m, 2H).

### Synthesis of (1r,3r)-3-{methyl[(1S)-2,2,2-trifluoro-1-(4-{[1-(pyridin-2-yl)-5-(trifluoromethyl)-1H-pyrazol-4-yl]amino}phenyl)ethyl]carbamoyl}cyclobutane-1-carboxylic acid amine (Compound 2.11) and (1s,3s)-3-{methyl[(1S)-2,2,2-trifluoro-1-(4-{[1-(pyridin-2-yl)-5-(trifluoromethyl)-1H-pyrazol-4-yl]amino}phenyl)ethyl]carbamoyl}cyclobutane-1- carboxylic acid amine (Compound 2.12)

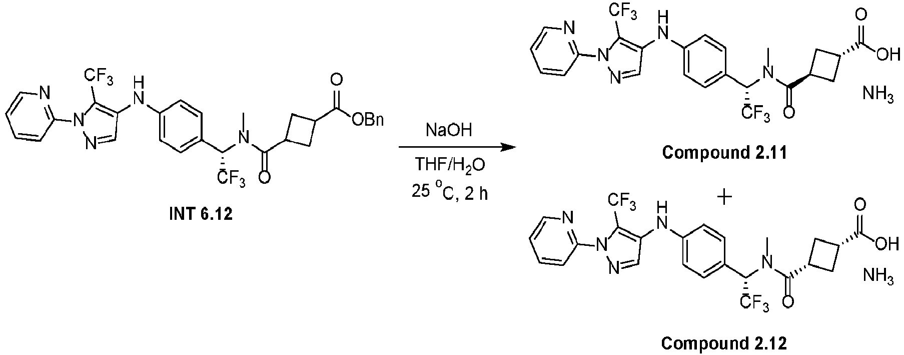

A mixture of benzyl 3-{methyl[(1S)-2,2,2-trifluoro-1-(4-{[1-(pyridin-2-yl)-5-(trifluoromethyl)-1H-pyrazol-4-yl]amino}phenyl)ethyl]carbamoyl}cyclobutane-1-carboxylate **[INT 6.12]** (390 mg, 617 µmol) and sodium hydroxide (98.3 mg, 2.46 mmol) in THF (5 mL) and water (5 mL) was stirred at 25 °C for 2 h. The reaction mixture was combined with another of the same type. The mixture was diluted with water (10 mL), adjusted with 1 N HCl to pH=4, and extracted with EtOAc (50 mL x 2). The combined organic phase was concentrated under reduced pressure to give the crude product, which was purified by Prep-HPLC (column: YMC-Triart Prep C18 150*40mm*7µm, table: 9-49% B (A = water (0.1% NH₃.H₂O and 10 mM NH₄HCO₃), B = acetonitrile), flow rate: 60 mL/min, UV Detector 220 nm) to give 120 mg of an off-white solid. The material was purified by SFC (column: DAICEL CHIRALPAK AS (250 mm*30 mm, 10 µm, table: 25% B (A = CO₂ (0.1% NH₃H₂O EtOH), B = EtOH), flow rate: 60 mL/min, UV Detector 220 nm) to give (1r,3r)-3-{methyl[(1S)-2,2,2-trifluoro-1-(4-{[1-(pyridin-2-yl)-5-(trifluoromethyl)-1H-pyrazol-4-yl]amino}phenyl)ethyl]carbamoyl}cyclobutane-1-carboxylic acid amine **[Compound 2.11]** (41.0 mg, 73.4 µmol, 11.9% yield) and (1s,3s)-3-{methyl[(1S)-2,2,2-trifluoro-1-(4-{[1-(pyridin-2-yl)-5-(trifluoromethyl)-1H-pyrazol-4-yl]amino}phenyl)ethyl]carbamoyl}cyclobutane-1- carboxylic acid amine **[Compound 2.12]** (55.1 mg, 98.6 µmol, 16.0% yield) as white solids.

Compound 2.11: m/z: [M + H]+ Calcd for C24H22F6N5O3 542.2; Found 542.2. ¹H NMR (400 MHz, CDCl₃) δ = 8.58 - 8.48 (m, 1H), 7.89 (dt, J = 2.0, 8.0 Hz, 1H), 7.84 (s, 1H), 7.75 (d, J = 8.8 Hz, 1H), 7.34 (dd, J = 4.0, 6.8 Hz, 1H), 7.31-7.27 (m, 2H), 6.97 (d, J = 8.4 Hz, 2H), 6.57 (q, J = 8.4 Hz, 1H), 5.67 (s, 1H), 3.51 (br t, J = 8.3 Hz, 1H), 3.23 (td, J = 4.4, 9.5 Hz, 1H), 2.78 (s, 3H), 2.74 - 2.65 (m, 2H), 2.64 - 2.49 (m, 2H).

Compound 2.12: m/z: [M + H]+ Calcd for C24H22F6N5O3 542.2; Found 542.2. ¹H NMR (400 MHz, CDCl₃) δ = 8.58 - 8.49 (m, 1H), 7.88 (dt, J = 1.6, 8.0 Hz, 1H), 7.85 (s, 1H), 7.75 (d, J = 8.8 Hz, 1H), 7.34 (dd, J = 4.8, 7.2 Hz, 1H), 7.31-7.27 (m, 2H), 6.97 (d, J = 8.4 Hz, 2H), 6.55 (q, J = 8.8 Hz, 1H), 5.68 (s, 1H), 3.29 (quin, J = 8.8 Hz, 1H), 3.17 (quin, J = 8.8 Hz, 1H), 2.80 (s, 3H), 2.75 - 2.62 (m, 2H), 2.61 - 2.46 (m, 2H).

### 3. Compounds Prepared using Scheme 3

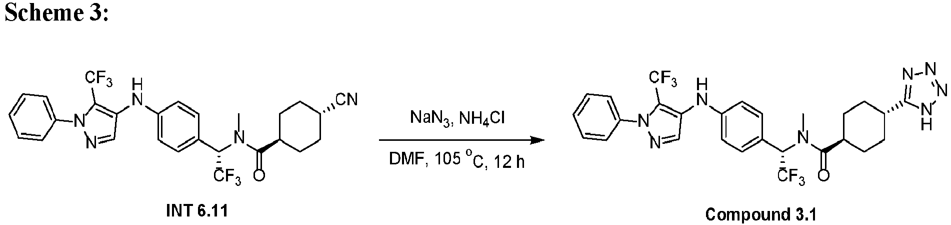

### Synthesis of (1r,48)-N-((S)-1-(4-((4-cyclopropyl-1,5-naphthyridin-3-yl)amino)phenyl)-2,2,2-trifluoroethyl)-N-methyl-4-(1H-tetrazol-5-yl)cyclohexane-1-carboxamide (Compound 3.1)

To a solution of (1r,4r)-4-cyano-N-methyl-N-[(1S)-2,2,2-trifluoro-1-(4-{[1-phenyl-5-(trifluoromethyl)-1H-pyrazol-4-yl]amino}phenyl)ethyl]cyclohexane-1- carboxamide **[INT 6.11]** (100 mg, 181 µmol) and ammonium chloride (14.4 mg, 271 µmol) in DMF (5 mL) was added azidosodium (40 mg, 615 µmol). The reaction mixture was stirred at 105 °C for 12 h under nitrogen. H₂O (10 mL) was added and the mixture was extracted with EtOAc (20 mL x 2). The combined organic layers were washed with brine (20 mL x 2), dried over anhydrous Na₂SO₄ and concentrated under reduced pressure to give crude (1r,45)-N-((S)-1-(4-((4-cyclopropyl-1,5-naphthyridin-3-yl)amino)phenyl)-2,2,2-trifluoroethyl)-N-methyl-4-(1H-tetrazol-5-yl)cyclohexane-1-carboxamide **[Compound 3.1]** (100 mg, 168 µmol, 93.4% yield). The material was purified by prep-HPLC (column: YMC-Triart Prep C18 150*40 mm*7 µm, table: 16-56% B (A = water (ammonia hydroxide), B = acetonitrile), flowrate: 60 mL/min, UV Detector 220 nm) to give (1r,4S)-N-((S)-1-(4-((4-cyclopropyl-1,5-naphthyridin-3-yl)amino)phenyl)-2,2,2-trifluoroethyl)-N-methyl-4-(1H-tetrazol-5-yl)cyclohexane-1-carboxamide **[Compound 3.1]** (3.90 mg, 6.58 µmol, 3.9% yield) as an off-white solid. m/z: [M + H]+ Calcd for C27H27F6N8O 593.2; Found 593.2. ¹H NMR (400 MHz, CDCl₃) δ = 7.81 (s, 1H), 7.49 (br s, 5H), 7.27 (s, 2H), 6.96 (d, J = 8.8 Hz, 2H), 6.67 - 6.51 (m, 1H), 5.60 (s, 1H), 3.22 - 3.15 (m, 1H), 3.03 - 2.85 (m, 3H), 2.79 (br s, 1H), 2.28 (br s, 2H), 2.13 - 1.95 (m, 2H), 1.82 (br s, 4H).

### Synthesis of Exemplary Intermediates

The following exemplary intermediates were synthesized and used for the synthesis of one or more exemplified compounds of the present disclosure.

### Synthesis of 1-methyl-5-(trifluoromethyl)-1H-pyrazol-4-amine hydrochloride [Intermediate 1.1]:

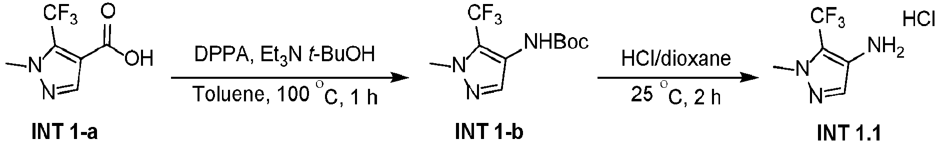

### Synthesis of tert-butyl N-[1-methyl-5-(trifluoromethyl)-1H-pyrazol-4-yl]carbamate [INT 1-b]:

To a solution of 1-methyl-5-(trifluoromethyl)-1H-pyrazole-4-carboxylic acid **[INT 1-a]** (500 mg, 2.57 mmol), 2-methylpropan-2-ol (285 mg, 3.85 mmol), and triethylamine (780 mg, 7.71 mmol) in toluene (5 mL) was added DPPA (1.05 g, 3.85 mmol). The reaction mixture was stirred at 100 °C for 1 h under N₂. The reaction was concentrated under reduced pressure to give the crude product, which was purified by flash chromatography on silica gel (EtOAC/PE= 0/1 to 1/1) to give tert-butyl N-[1-methyl-5-(trifluoromethyl)-1H-pyrazol-4-yl]carbamate **[INT 1-b]** (540 mg, 2.03 mmol, 79% yield) as a white solid. m/z: [M + H]+ Calcd for C10H15F3N3O2 266.1; Found 266.1.

### 1-methyl-5-(trifluoromethyl)-1H-pyrazol-4-amine hydrochloride [INT 1.1]:

A solution of tert-butyl N-[1-methyl-5-(trifluoromethyl)-1H-pyrazol-4-yl]carbamate [INT 1-b] (540 mg, 2.03 mmol) in 4 M HCl/dioxane (10 mL) was stirred at 25 °C for 2 h . The reaction was concentrated under reduced pressure to give 1-methyl-5-(trifluoromethyl)-1H-pyrazol-4-amine hydrochloride **[INT 1.1]** (350 mg, 1.73 mmol, 86% yield) as a brown solid. m/z: [M + H]+ Calcd for C5H7F3N3 166.1; Found 166.1.

### Synthesis of 1-(quinolin-5-yl)-5-(trifluoromethyl)-1H-pyrazol-4-amine [Intermediate 1.2]:

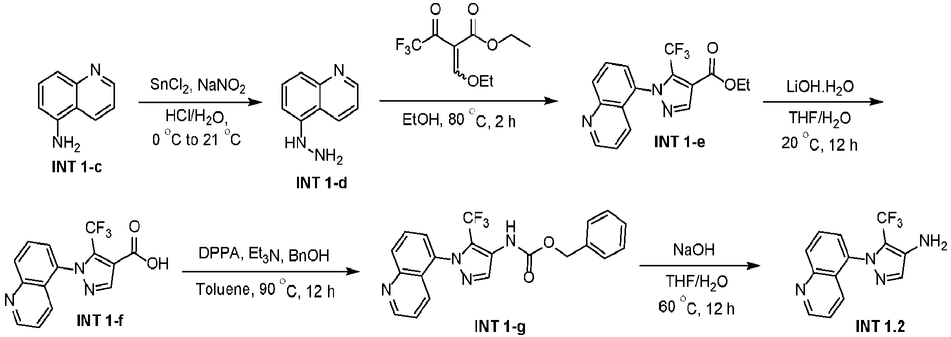

### Synthesis of 5-hydrazinylquinoline [INT 1-d]:

To a solution of quinolin-5-amine **[INT 1-c]** (1 g, 6.93 mmol) in HCl (6 mL) was added a solution of NaNO₂ (525 mg, 7.62 mmol) in H₂O (1.5 mL) dropwise at 0 °C. The mixture was stirred at 0 °C for 30 min and allowed to warm to 21 °C over 30 min until an orange solution was formed. The mixture was cooled to 0 °C and a solution of Tin(II) chloride (3.11 g, 13.8 mmol) dissolved in HCl (3 mL) was added dropwise. A yellow precipitate formed immediately upon addition of the tin salt. The mixture was stirred at 0 °C for 30 minutes and was then allowed to warm to 21 °C with vigorous stirring for 12 hours. The reaction mixture was filtered. The filter cake was washed with cold EtOH (10 mL x 3) and dried under reduced pressure to give crude 5-hydrazinylquinoline [**INT 1-d**] (1.10 g, 6.90 mmol) as a yellow solid. ¹H NMR (400 MHz, DMSO-d6) δ = 10.05 (br s, 1H), 9.28 - 9.18 (m, 2H), 8.05 - 7.96 (m, 2H), 7.92 (d, J = 8.4 Hz, 1H), 7.29 (d, J = 7.6 Hz, 1H).

### Synthesis of ethyl 1-(quinolin-5-yl)-5-(trifluoromethyl)-1H-pyrazole-4-carboxylate [INT 1-e]:

To a solution of ethyl 2-(ethoxymethylene)-4,4,4-trifluoro-3-oxobutanoate (668 mg, 2.78 mmol) in EtOH (5 mL), 5-hydrazinylquinoline [**INT 1-d**] (367 mg, 2.31 mmol) was added. The mixture was stirred at 80 °C for 2 hours. The reaction mixture was concentrated under reduced pressure to give the crude product, which was purified by flash chromatography on silica gel (CH₂Cl₂/MeOH = 100/0 to 95/5) to give ethyl 1-(quinolin-5-yl)-5-(trifluoromethyl)-1H-pyrazole-4-carboxylate [**INT 1-e**] (185 mg, 551 µmol, 23.9% yield) as a brown semisolid. m/z: [M + H]+ Calcd for C16H13F3N3O2 336.1; Found 336.2.

### Synthesis of 1-(quinolin-5-yl)-5-(trifluoromethyl)-1H-pyrazole-4-carboxylic acid [INT 1-f]:

To a solution of ethyl 1-(quinolin-5-yl)-5-(trifluoromethyl)-1H-pyrazole-4-carboxylate [**INT 1-e**] (419 mg, 1.24 mmol) in H₂O (0.5 mL) and THF (3 mL) was added lithium hydroxide monohydrate (104 mg, 2.48 mmol). The mixture was stirred at 21 °C for 2 h, after which it was concentrated under reduced pressure to give a brown solid. Then H₂O (3 mL) was added and the pH of the reaction mixture was adjusted to 5 with 1 M HCl. The mixture was extracted with EtOAc (10 mL x 3). The combined organic layers were washed with brine (2 x 30 mL), dried over anhydrous Na₂SO₄ and concentrated under reduced pressure to give the crude 1-(quinolin-5-yl)-5-(trifluoromethyl)-1H-pyrazole-4-carboxylic acid [**INT 1-f**] (120 mg, 390 µmol, 31.5% yield) as a brown solid. m/z: [M + H]+ Calcd for C14H9F3N3O2 308.1; Found 308.0.

### Synthesis of benzyl (1-(quinolin-5-yl)-5-(trifluoromethyl)-1H-pyrazol-4-yl)carbamate [INT 1-g]:

A solution of 1-(quinolin-5-yl)-5-(trifluoromethyl)-1H-pyrazole-4-carboxylic acid [**INT** 1-f] (180 mg, 585 µmol), benzyl alcohol (75.9 mg, 702 µmol), Et₃N (59.1 mg, 585 µmol) and DPPA (241 mg, 877 µmol) in toluene (2 mL) was stirred at 90 °C for 12 h. The reaction mixture was concentrated under reduced pressure to give the crude product, which was purified by flash chromatography on silica gel (EtOAc/Petroleum ether = 0/1 to 1/3) to give benzyl (1-(quinolin-5-yl)-5-(trifluoromethyl)-1H-pyrazol-4-yl)carbamate_[**INT 1-g**] (100 mg, 242 µmol, 41.4% yield) as a yellow solid. m/z: [M + H]+ Calcd for C21H16F3N4O2 413.1; Found 413.1.

### Synthesis of 1-(quinolin-5-yl)-5-(trifluoromethyl)-1H-pyrazol-4-amine [INT 1.2]:

To a solution of benzyl (1-(quinolin-5-yl)-5-(trifluoromethyl)-1H-pyrazol-4-yl)carbamate [**INT 1-g**] (100 mg, 242 µmol) in THF (2 mL) was added NaOH (96.8 mg, 2.42 mmol) and H₂O (2 mL). The resulting mixture was stirred at 60 °C for 12 h. Water (10 mL) was added and the mixture was extracted with EtOAc (15 mL x 2). The combined organic layers were washed with brine (20 mL), dried over Na₂SO₄ and concentrated under reduced pressure to give the crude product, which was purified by flash chromatography on silica gel (EtOAc/hexane = 0/1 to 1/1) to give 1-(quinolin-5-yl)-5-(trifluoromethyl)-1H-pyrazol-4-amine [**INT 1.2**] (45.0 mg, 161 µmol, 66.8% yield) as a white solid. m/z: [M + H]+ Calcd for C13H10F3N4 279.1; Found 279.1.

### Synthesis of 1-(o-tolyl)-5-(trifluoromethyl)-1H-pyrazol-4-amine [Intermediate 1.3]:

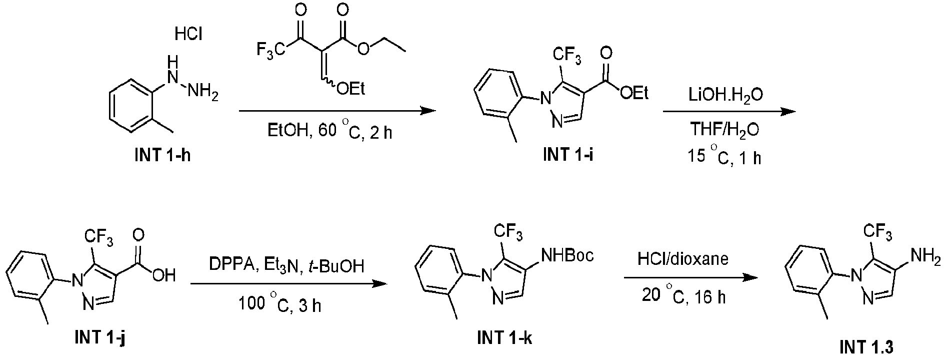

### Synthesis of ethyl 1-(o-tolyl)-5-(trifluoromethyl)-1H-pyrazole-4-carboxylate[INT 1-i]:

To a solution of ethyl 2-(ethoxymethylene)-4,4,4-trifluoro-3-oxobutanoate (3 g, 12.4 mmol) in EtOH (30 mL) was added (2-methylphenyl)hydrazine hydrochloride [**INT 1-h**] (1.96 g, 12.4 mmol) at 25 °C and the reaction mixture was stirred at 60 °C for 2 h. The reaction mixture was concentrated under reduced pressure to give the crude product, which was purified by flash chromatography on silica gel (PE/EA = 1/0 to 3/1) to give ethyl 1-(o-tolyl)-5-(trifluoromethyl)-1H-pyrazole-4-carboxylate [**INT 1-i**] (3.10 g, 10.3 mmol, 84.0% yield) as a yellow oil. m/z: [M + H]+ Calcd for C14H14F3N2O2 299.1; Found 298.9.

### Synthesis of 1-fo-tolyl)-5-(trifluoromethyl)-1H-pyrazole-4-carboxylic acid [INT 1-i]:

To a mixture of ethyl 1-(o-tolyl)-5-(trifluoromethyl)-1H-pyrazole-4-carboxylate [**INT 1-i**] (3.1 g, 10.3 mmol) in H₂O (10 mL) and THF (20 mL) was added lithium hydroxide monohydrate (1.07 g, 25.7 mmol). The reaction mixture was stirred at 15 °C for 1 hour. The reaction mixture was concentrated under reduced pressure. The mixture was then acidified with 1 M HCl to pH=4 and concentrated under reduced pressure to provide crude 1-(o-tolyl)-5-(trifluoromethyl)-1H-pyrazole-4-carboxylic acid [**INT 1-j**] (2.6 g, 9.62 mmol, 93.5% yield). m/z: [M + H]+ Calcd for C12H10F3N2O2 271.1; Found 270.8.

### Synthesis of tert-butyl (1-(o-tolyl)-5-(trifluoromethyl)-1H-pyrazol-4-yl)carbamate [INT 1-k]:

To a mixture of 1-(o-tolyl)-5-(trifluoromethyl)-1H-pyrazole-4-carboxylic acid [**INT 1-**j] (2 g, 7.40 mmol) in t-BuOH (20mL) was added DPPA (3.05 g, 11.1 mmol) and triethylamine (1.49 g, 14.8 mmol). The reaction mixture was stirred at 100 °C under nitrogen for 3 hours. The reaction mixture was concentrated under reduced pressure to give a solid, which was purified by flash chromatography on silica gel (EtOAc/PE = 0/1 to 1/3) to give tert-butyl (1-(o-tolyl)-5-(trifluoromethyl)-1H-pyrazol-4-yl)carbamate [**INT 1-k**] (400 mg, 1.17 mmol, 15.8% yield) as a white solid. m/z: [M + H]+ Calcd for C16H19F3N3O2 342.1; Found 342.0.

### Synthesis of 1-fo-tolyl)-5-(trifluoromethyl)-1H-pyrazol-4-amine [INT 1.3]:

A mixture of tert-butyl (1-(o-tolyl)-5-(trifluoromethyl)-1H-pyrazol-4-yl)carbamate [**INT 1-k**] (220 mg, 644 µmol) in 4 M HCI/dioxane (6 mL) was stirred at 20 °C for 16 h. The reaction mixture was concentrated under reduced pressure to give crude 1-(o-tolyl)-5-(trifluoromethyl)-1H-pyrazol-4-amine [**INT 1.3**] (100 mg, 414 µmol, 70.9% yield). m/z: [M + H]+ Calcd for C11H11F3N3 242.1; Found 241.9.

### Synthesis of 1-methyl-5-phenyl-1H-pyrazol-4-amine [Intermediate 1.4]:

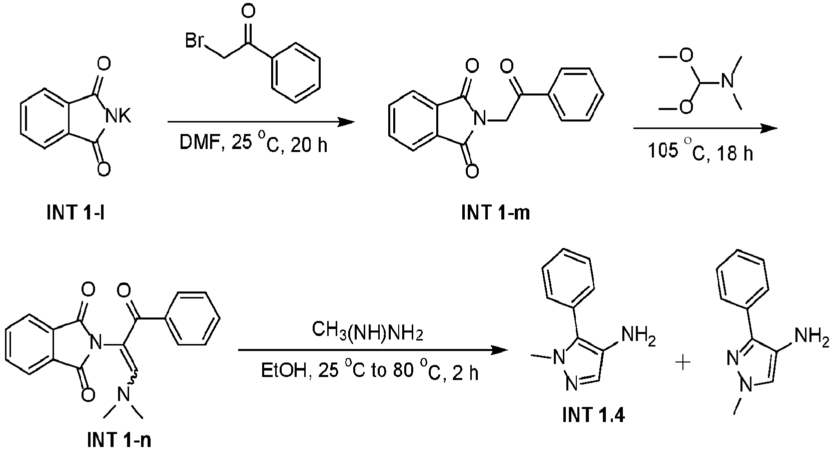

### Synthesis of 2-(2-oxo-2-phenylethyl)isoindoline-1,3-dione [INT 1-m]:

To a solution of potassium 1,3-dioxo-2,3-dihydro-1H-isoindol-2-ide [**INT 1-l**] (3 g, 16.1 mmol) in DMF (10 mL) was added 2-bromo-1-phenylethan-1-one (3.20 g, 16.1 mmol). The reaction mixture was stirred at 25 °C for 20 h. Water (20 mL) was added, and the mixture was extracted with DCM (30 mL x 2). The combined organic layers were washed with brine (40 mL x 2), dried over anhydrous Na₂SO₄ and concentrated under reduced pressure to give the crude product, which was purified by flash chromatography on silica gel (EtOAc/PE = 0/1 to 5/1) to give 2-(2-oxo-2-phenylethyl)isoindoline-1,3-dione [**INT 1-m**] (4.16 g, 15.6 mmol, 97.4% yield) as a white solid. m/z: [M + H]+ Calcd for C16H12NO3 266.1; Found 266.1. ¹H NMR (400 MHz, CDCl₃) δ = 8.05 - 8.00 (m, 2H), 7.96 - 7.86 (m, 2H), 7.79 - 7.74 (m, 2H), 7.67 - 7.62 (m, 1H), 7.56 - 7.50 (m, 2H), 5.15 (s, 2H).

### Synthesis of 2-[1-(dimethylamino)-3-oxo-3-phenylprop-1-en-2-yl]-2,3-dihydro-1H-isoindole-1,3-dione [INT 1-n]:

A suspension of 2-(2-oxo-2-phenylethyl)-2,3-dihydro-1H-isoindole-1,3-dione [**INT 1-m**] (1.0 g, 3.76 mmol) in (dimethoxymethyl)dimethylamine (3 mL, 15.0 mmol) was heated at 105 °C for 18 hr and concentrated under reduced pressure. The resulting amber oil was crystallized from isopropanol (10 mL) and washed with isopropanol (2 x 5 mL) to give 2-[1-(dimethylamino)-3-oxo-3-phenylprop-1-en-2-yl]-2,3-dihydro-1H-isoindole-1,3-dione [**INT 1-n**] (930 mg, 2.90 mmol, 77.5% yield) as a yellow solid. m/z: [M + H]+ Calcd for C19H17N203 321.1; Found 321.1. ¹H NMR (400 MHz, CDCl₃) δ = 7.94 - 7.89 (m, 2H), 7.78 - 7.73 (m, 2H), 7.59 (d, J=6.8 Hz, 2H), 7.45 (s, 1H), 7.43 - 7.35 (m, 3H), 2.99 (s, 6H).

### Synthesis of 1-methyl-5-phenyl-1H-pyrazol-4-amine [INT 1.4]:

To a solution of 2-[1-(dimethylamino)-3-oxo-3-phenylprop-1-en-2-yl]-2,3-dihydro-1H-isoindole-1,3-dione [**INT 1-n**] (930 mg, 2.90 mmol) in EtOH (6 mL) was added methylhydrazine (723 mg, 15.7 mmol). The reaction was stirred at 25 °C for 1 h under N₂. The reaction was then heated to 80 °C for 1 h. The reaction was cooled to 25 °C and concentrated under reduced pressure to give the crude product, which was purified by flash chromatography on silica gel (EtOAc/PE = 0/1 to 1/1) to give a mixture of 1-methyl-5-phenyl-1H-pyrazol-4-amine [**INT 1.4**] (152 mg, 877 µmol, 30.2% yield) and 1-methyl-3-phenyl-1H-pyrazol-4-amine (190 mg, 1.09 mmol, 37.8% yield) as a brown solid. The mixture was purified Prep-HPLC (column: Phenomenex Gemini C18 250*50 mm*10 µm, table:1-41% B (A = water (0.05%NH₃H₂O+10 mM NH₄HCO₃), B = acetonitrile), flow rate: 25 mL/min, UV Detector 220 nm) to afford 1-methyl-5-phenyl-1H-pyrazol-4-amine [**INT 1.4**] (40.0 mg, 230 µmol) as a white solid. m/z: [M + H]+ Calcd for C10H12N3 174.1; Found 174.1.

### Synthesis of 5-isopropyl-1-methyl-1H-pyrazol-4-amine [Intermediate 1.5]:

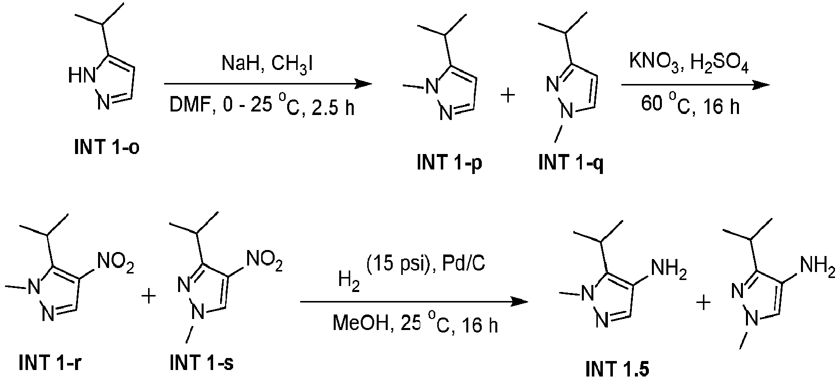

### Synthesis of 1-methyl-5-(propan-2-yl)-1H-pyrazole [INT 1-p] and 1-methyl-3-(propan-2-yl)-1H-pyrazole [INT 1-q]:

To a solution of 5-(propan-2-yl)-1H-pyrazole [**INT 1-o**] (3 g, 27.2 mmol) in DMF (20 mL) was added sodium hydride (1.63 g, 40.8 mmol) at 0 °C and the reaction mixture was stirred at 0 °C for 0.5 h under nitrogen. Iodomethane (11.5 g, 81.6 mmol) was then added and the reaction was allowed to warm to 25 °C over 2 h. The reaction was quenched by adding NH₄Cl (aq) (30 mL) and was extracted with EtOAc (3 x 20 mL). The combined organic layers were washed with brine (2 x 50 mL), dried over anhydrous Na₂SO₄ and concentrated under reduced pressure to give the crude product, which was purified by flash chromatography on silica gel (PE/EtOAc = 1/0 to 1/2) to give a mixture of 1-methyl-5-(propan-2-yl)-1H-pyrazole [**INT 1-p**] 1-methyl-3-(propan-2-yl)-1H-pyrazole [**INT 1-q**] (1.30 g, 5.23 mmol, 19.2% yield) as a colorless oil. m/z: [M + H]+ Calcd for C7H13N2 125.1; Found 125.3.

### Synthesis of 1-methyl-4-nitro-5-(propan-2-yl)-1H-pyrazole [INT 1-r] and 1-methyl-4-nitro-3-(propan-2-yl)-1H-pyrazole [INT 1-s]:

To a mixture of 1-methyl-5-(propan-2-yl)-1H-pyrazole [**INT 1-p**] and 1-methyl-3-(propan-2-yl)-1H-pyrazole [**INT 1-q**] in H₂SO₄ (1 mL) was added KNO₃ (203 mg, 2.01 mmol) and the reaction mixture was stirred at 60 °C for 16 h under nitrogen. The reaction was quenched by adding 1 N NaOH (aq) (10 mL) and was extracted with EtOAc (3 x 10 mL). The combined organic layers were washed with brine (2 x 10 mL), dried over anhydrous Na₂SO₄ and concentrated under reduced pressure to give 1-methyl-4-nitro-5-(propan-2-yl)-1H-pyrazole [**INT 1-r**] and 1-methyl-4-nitro-3-(propan-2-yl)-1H-pyrazole [**INT 1-s**] (100 mg, 295 µmol, 73.5% yield) as a yellow soild. m/z: [M + H]+ Calcd for C7H12N3O2 170.1; Found 170.1.

### Synthesis of 1-methyl-5-(propan-2-yl)-1H-pyrazol-4-amine [INT 1.5]:

To a solution of 1-methyl-4-nitro-5-(propan-2-yl)-1H-pyrazole [**INT 1-r**] and 1-methyl-4-nitro-3-(propan-2-yl)-1H-pyrazole [**INT 1-s**] (1 g, 2.95 mmol) in MeOH (10 mL) was added Pd/C (313 mg, 295 µmol) and the suspension was degassed under vacuum and purged with H₂ several times. The mixture was stirred under H₂ (103.421 kPa, 15 psi) at 25 °C for 16 hours. The reaction mixture was filtered and the filtrate was concentrated under reduced pressure to give 1-methyl-5-(propan-2-yl)-1H-pyrazol-4-amine [INT 1.5] and 1-methyl-3-(propan-2-yl)-1H-pyrazol-4-amine. The mixture was purified by SFC (column: REGIS(S,S)WHELK-O1(250 mm*25 mm, 10 µm), condition: 15%-15% 0.1%NH₃H₂O ETOH,flowrate:60 mL/min) to give 1-methyl-5-(propan-2-yl)-1H-pyrazol-4-amine [**INT 1.5**] (100 mg, 718 µmol) as a blue oil (m/z: [M + H]+ Calcd for C7H14N3 140.1; Found 140.3) and 1-methyl-3-(propan-2-yl)-1H-pyrazol-4-amine (200 mg, 1.43 mmol) as a blue oil ([M + H]+ Calcd for C7H14N3 140.1; Found 140.3).

### Synthesis of 1-phenyl-5-(trifluoromethyl)-1H-pyrazol-4-amine hydrochloride [Intermediate 1.6]:

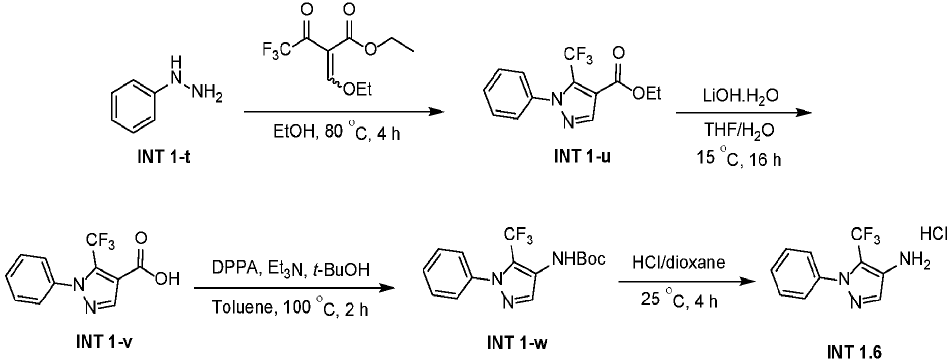

### Synthesis of ethyl 1-phenyl-5-(trifluoromethyl)-1H-pyrazole-4-carboxylate [INT 1-u]:

A mixture of ethyl 2-(ethoxymethylene)-4,4,4-trifluoro-3-oxobutanoate (3 g, 12.4 mmol) and phenylhydrazine [**INT 1-t**] (1.47 g, 13.6 mmol) in EtOH (35 mL) was stirred at 80 °C for 4 hr. The mixture was concentrated under reduced pressure to afford the crude product, which was purified by flash chromatography on silica gel (EtOAc/Petroleum ether = 1/1 to 1/0) to give ethyl 1-phenyl-5-(trifluoromethyl)-1H-pyrazole-4-carboxylate [**INT 1-u**] (2.44 g, 8.58 mmol, 69.3% yield) as a yellow oil. m/z: [M + H]+ Calcd for C13H12F3N2O2 285.1; Found 285. 1. ¹H NMR (400 MHz, CDCl₃) δ = 8.13 (s, 1H), 7.56 - 7.49 (m, 3H), 7.47 - 7.41 (m, 2H), 4.44 - 4.35 (m, 2H), 1.40 (t, J = 7.2Hz, 3H)

### Synthesis of 1-phenyl-5-(trifluoromethyl)-1H-pyrazole-4-carboxylic acid [INT 1-v]:

To a mixture of ethyl 1-phenyl-5-(trifluoromethyl)-1H-pyrazole-4-carboxylate [**INT 1-u**] (2.34 g, 8.23 mmol) in THF (15 mL) and H₂O (5 mL) was added LiOH.H₂O (1.03 g, 24.6 mmol) and the mixture was stirred at 15 °C for 16 hr. The mixture was concentrated under reduced pressure to afford the crude product. H₂O (30 mL) and EtOAc (30 mL) were added and the precipitate was collected by filtration. The aqueous phase was washed with EtOAc (30 mL x 2), acidified with 1 M HCl to pH = 3 and extracted with EtOAc (30 mL x 2). The combined organic layers were dried over Na₂SO₄ and filtered. The filtrate was concentrated to give a yellow solid, which was combined with the precipitate to give 1-phenyl-5-(trifluoromethyl)-1H-pyrazole-4-carboxylic acid [**INT 1-v**] (1.88 g, 7.33 mmol, 89.5% yield) as a yellow solid. m/z: [M + H]+ Calcd for C11H8F3N2O2 257.0; Found 256.9. ¹H NMR (400 MHz, CDCl₃) δ = 8.22 (s, 1H), 7.59 - 7.50 (m, 3H), 7.49 - 7.43 (m, 2H).

### Synthesis of tert-butyl (1-phenyl-5-(trifluoromethyl)-1H-pyrazol-4-yl)carbamate [INT 1-w]:

A mixture of 1-phenyl-5-(trifluoromethyl)-1H-pyrazole-4-carboxylic acid [**INT 1-v**] (400 mg, 1.56 mmol), 2-methylpropan-2-ol (1.73 g, 23.4 mmol), diphenylphosphoryl azide (569 mg, 2.34 mmol) and triethylamine (473 mg, 4.68 mmol) in toluene (4 mL) was stirred at 100 °C for 2 hr. NaHCO₃ (30 mL) was added and the mixture was extracted with EtOAc (30 mL x 2). The combined organic layers were washed with brine (30 mL x 2), dried over anhydrous Na₂SO₄, filtered and concentrated under reduced pressure to give the crude product, which was purified by flash chromatography on silica gel (EtOAc/Petroleum ether = 1/20 to 1/0) to give tert-butyl (1-phenyl-5-(trifluoromethyl)-1H-pyrazol-4-yl)carbamate [**INT 1-w**] (335 mg, 1.02 mmol, 65.6% yield) as a yellow oil. m/z: [M + H]+ Calcd for C15H17F3N3O2 328.1; Found 328.2. ¹H NMR (CDCl₃) δ = 8.27 (br s, 1H), 7.52 - 7.46 (m, 3H), 7.46 - 7.39 (m, 2H), 6.54 (br s, 1H), 1.56 - 1.54 (m, 9H).

### Synthesis of 1-phenyl-5-(trifluoromethyl)-1H-pyrazol-4-amine hydrochloride [INT 1.6]:

A mixture of tert-butyl N-[1-phenyl-5-(trifluoromethyl)-1H-pyrazol-4-yl]carbamate [**INT 1-w**] (235 mg, 717 µmol) in 4 M HCl/dioxane (10 mL, 40.0 mmol) was stirred at 25 °C for 4 hr. The mixture was concentrated under reduced pressure to afford 1-phenyl-5-(trifluoromethyl)-1H-pyrazol-4-amine hydrochloride [**INT 1.6**] (160 mg, 606 µmol, 84.6% yield) as a yellow solid. m/z: [M + H]+ Calcd for C10H9F3N3 228.1; Found 228.0.

### Synthesis of 1-cyclopropyl-5-(trifluoromethyl)-1H-pyrazol-4-amine hydrochloride [Intermediate 1.7]:

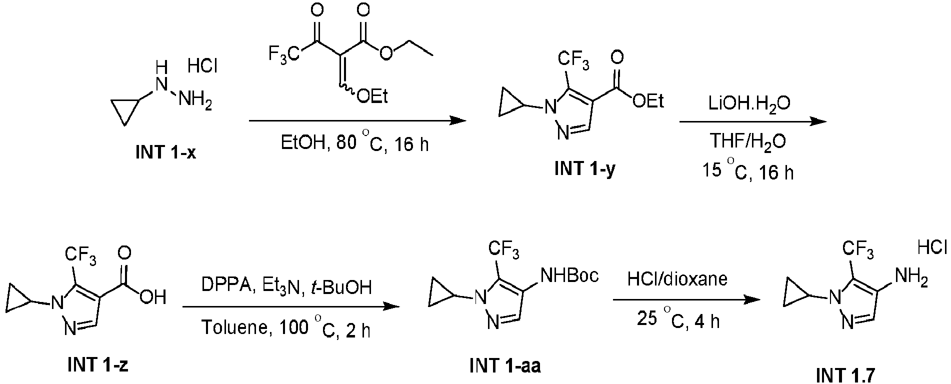

### Synthesis of ethyl 1-cyclopropyl-5-(trifluoromethyl)-1H-pyrazole-4-carboxylate [INT 1-y]:

A mixture of ethyl 2-(ethoxymethylene)-4,4,4-trifluoro-3-oxobutanoate (5 g, 20.8 mmol) and cyclopropylhydrazine hydrochloride [**INT 1-x**] (2.47 g, 22.8 mmol) in EtOH (50 mL) was stirred at 80 °C for 16 hr. The mixture was concentrated under reduced pressure to afford the crude product, which was purified by flash chromatography on silica gel (EtOAc/Petroleum ether = 1/10 to 1/0) to give ethyl 1-cyclopropyl-5-(trifluoromethyl)-1H-pyrazole-4-carboxylate [**INT 1-y**] (608 mg, 2.44 mmol, 11.7% yield) as a yellow oil. m/z: [M + H]+ Calcd for C10H12F3N2O2 249.1; Found 249.1. ¹H NMR (400MHz, CDCl₃) δ = 7.85 (s, 1H), 4.36 - 4.30 (m, 2H), 3.73 (dtt, J = 1.2, 3.6, 7.2 Hz, 1H), 1.39 - 1.34 (m, 3H), 1.33 - 1.29 (m, 2H), 1.17 - 1.10 (m, 2H).

### Synthesis of 1-cyclopropyl-5-(trifluoromethyl)-1H-pyrazole-4-carboxylic acid [INT 1-z]:

To a mixture of ethyl 1-cyclopropyl-5-(trifluoromethyl)-1H-pyrazole-4-carboxylate [**INT 1-y**] (608 mg, 2.44 mmol) in THF (3 mL) and H₂O (1 mL) was added LiOH.H₂O (307 mg, 7.32 mmol) and the mixture was stirred at 15 °C for 16 hr. The mixture was concentrated under reduced pressure to afford the crude product. 1N HCl was added to adjust the pH to 3-4 and the mixture was extracted with EtOAc (50 mL x 2). The combined organic layers were dried over anhydrous Na₂SO₄, filtered and concentrated under reduced pressure to give 1-cyclopropyl-5-(trifluoromethyl)-1H-pyrazole-4-carboxylic acid [**INT 1-z**] (436 mg, 1.98 mmol, 81.1% yield) as a yellow solid. m/z: [M + H]+ Calcd for C8H8F3N2O2 221.0; Found 221.0. ¹H NMR (400 MHz, CDCl₃) δ = 7.95 (s, 1H), 2.17 - 2.11 (m, 1H), 1.38 - 1.32 (m, 2H), 1.20 - 1.14 (m, 2H).

### Synthesis of tert-butyl N-[1-cyclopropyl-5-(trifluoromethyl)-1H-pyrazol-4-yl]carbamate [INT 1-aa]:

A mixture of 1-cyclopropyl-5-(trifluoromethyl)-1H-pyrazole-4-carboxylic acid [**INT 1-z**] (336 mg, 1.52 mmol), 2-methylpropan-2-ol (1.69 g, 22.8 mmol), diphenylphosphoryl azide (554 mg, 2.28 mmol), and triethylamine (461 mg, 4.56 mmol) in toluene (4 mL) was stirred at 100 °C for 2 hr. NaHCO₃ (30 mL) was added and the mixture was extracted with EtOAc (30 mL x 2). The combined organic layers were washed with brine (30 mL x 2), dried over anhydrous Na₂SO₄, filtered and concentrated under reduced pressure to give the crude product, which was purified by flash chromatography on silica gel (EtOAc/Petroleum ether = 1/10 to 1/0) to give tert-butyl N-[1-cyclopropyl-5-(trifluoromethyl)-1H-pyrazol-4-yl]carbamate [**INT 1-aa**] (287 mg, 985 µmol, 64.9% yield) as a colorless oil. m/z: [M + H]+ Calcd for C12H17F3N3O2 292.1; Found 292.1. ¹H NMR (400 MHz, CDCl₃) δ = 7.94 (br s, 1H), 7.43 - 7.36 (m, 1H), 3.55 (tt, J = 3.6, 7.2 Hz, 1H), 1.51 (s, 9H), 1.30 - 1.23 (m, 2H), 1.09 - 1.02 (m, 2H).

### Synthesis of 1-cyclopropyl-5-(trifluoromethyl)-1H-pyrazol-4-amine hydrochloride [INT 1.7]:

A mixture of tert-butyl N-[1-cyclopropyl-5-(trifluoromethyl)-1H-pyrazol-4-yl]carbamate [**INT 1-aa**] (187 mg, 642 µmol) in 4 M HCl/dioxane (10 mL, 40.0 mmol) was stirred at 25 °C for 4 hr. The mixture was concentrated under reduced pressure to afford 1-cyclopropyl-5-(trifluoromethyl)-1H-pyrazol-4-amine hydrochloride [**INT 1.7**] (100 mg, 439 µmol, 68.4% yield) as a brown solid. m/z: [M + H]+ Calcd for C7H9F3N3 192.1; Found 192.0.

### Synthesis of 1-phenyl-5-(propan-2-yl)-1H-pyrazol-4-amine hydrochloride [Intermediate 1.8]:

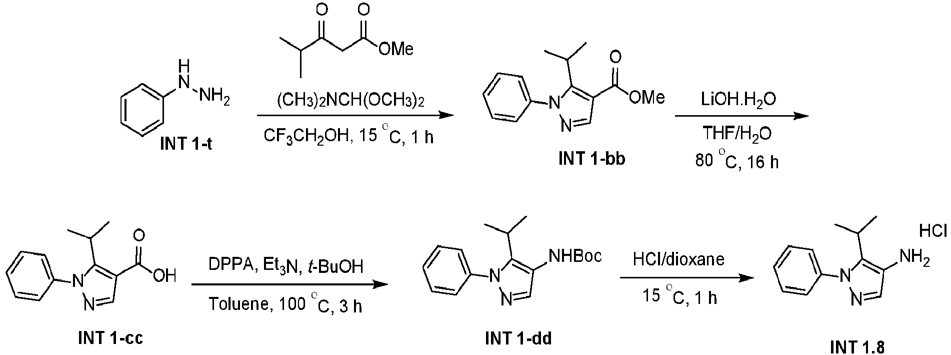

### Synthesis of methyl 1-phenyl-5-(propan-2-yl)-1H-pyrazole-4-carboxylate [INT 1-bb]:

To a mixture of methyl 4-methyl-3-oxopentanoate (1 g, 6.93 mmol) and (dimethoxymethyl)dimethylamine (990 mg, 8.31 mmol) in 2,2,2-trifluoroethanol (5 mL) was added phenylhydrazine [**INT 1-t**] (898 mg, 8.31 mmol) in 2,2,2-trifluoroethanol (5 mL). The reaction mixture was stirred at 15 °C for 1 hour. The reaction was quenched with water (20 mL) and extracted with EtOAc (10 mL x 3). The combined organic layers were dried over anhydrous sodium sulfate and filtered. The filtrate was concentrated to give the crude product, which was purified by flash chromatography on silica gel (0-6% EtOAc in petroleum ether) to give methyl 1-phenyl-5-(propan-2-yl)-1H-pyrazole-4-carboxylate [**INT 1-bb**] (620 mg, 2.53 mmol, 36.6% yield) as an orange solid. m/z: [M + H]+ Calcd for C14H17N2O2 245.1; Found 245.0. ¹H NMR (400 MHz, CDCl₃) δ = 8.03 (s, 1H), 7.55 - 7.48 (m, 3H), 7.40 - 7.34 (m, 2H), 3.87 (s, 3H), 3.34 - 3.21 (m, 1H), 1.36 (d, J = 7.2 Hz, 6H).

### Synthesis of 1-phenyl-5-(propan-2-yl)-1H-pyrazole-4-carboxylic acid [INT 1-cc]:

To a mixture of methyl 1-phenyl-5-(propan-2-yl)-1H-pyrazole-4-carboxylate [**INT 1-bb**] (620 mg, 2.53 mmol) in THF (6 mL) and H₂O (2 mL) was added lithium hydroxide monohydrate (318 mg, 7.58 mmol). The reaction mixture was stirred at 80 °C for 16 hours. The reaction mixture was concentrated, diluted with water (20 mL) and washed with EtOAc (10 mL x 2). The aqueous phase was acidified with 1 M HCl to pH = 3 and extracted with EtOAc (10 mL x 2). The combined organic layers were dried over anhydrous sodium sulfate and filtered. The filtrate was concentrated to give 1-phenyl-5-(propan-2-yl)-1H-pyrazole-4-carboxylic acid [**INT 1-cc**] (460 mg, 1.99 mmol, 79.0%) as a yellow solid. ¹H NMR (400 MHz, CDCl₃) δ = 8.13 (s, 1H), 7.57 - 7.48 (m, 3H), 7.43 - 7.36 (m, 2H), 3.37 - 3.24 (m, 1H), 1.39 (d, J=6.8 Hz, 6H).

### Synthesis of tert-butyl N-[1-phenyl-5-(propan-2-yl)-1H-pyrazol-4-yl]carbamate [INT 1-dd]:

To a mixture of 1-phenyl-5-(propan-2-yl)-1H-pyrazole-4-carboxylic acid [**INT 1-cc**] (410 mg, 1.78 mmol), 2-methylpropan-2-ol (1.97 g, 26.7 mmol) and triethylamine (540 mg, 5.34 mmol) in toluene (5 mL) was added diphenylphosphoryl azide (734 mg, 2.67 mmol). The reaction mixture was stirred at 100 °C under nitrogen for 3 hours. The reaction mixture was diluted with EtOAc (20 mL) and washed with saturated NaHCO₃ (20 mL) and brine (20 mL). The organic layer was dried over anhydrous Na₂SO₄ and filtered. The filtrate was concentrated to give the crude product, which was purified by flash chromatography on silica gel (0-20% EtOAc in petroleum ether) to give tert-butyl N-[1-phenyl-5-(propan-2-yl)-1H-pyrazol-4-yl]carbamate [**INT 1-dd**] (270 mg, 895 µmol, 50.3% yield) as a colorless oil. m/z: [M + H]+ Calcd for C17H24N3O2 302.2; Found 302.1. ¹H NMR (400 MHz, CDCl₃) δ = 7.82 (br s, 1H), 7.55 - 7.44 (m, 3H), 7.41 - 7.36 (m, 2H), 5.81 (br s, 1H), 3.07 (spt, J = 7.2 Hz, 1H), 1.53 (s, 9H), 1.29 (d, J = 7.2 Hz, 6H).

### Synthesis of 1-phenyl-5-(propan-2-yl)-1H-pyrazol-4-amine hydrochloride [INT 1.8]:

A mixture of tert-butyl N-[1-phenyl-5-(propan-2-yl)-1H-pyrazol-4-yl]carbamate [**INT 1-dd**] (270 mg, 895 µmol) in 4 M HCl/dioxane (5 mL, 20 mmol) was stirred at 15 °C for 1 hour. The reaction mixture was concentrated to give 1-phenyl-5-(propan-2-yl)-1H-pyrazol-4-amine hydrochloride [**INT 1.8**] (210 mg, 883 µmol, 99.0% yield) as a yellow solid. ¹H NMR (400 MHz, MeOD) δ = 7.71 (s, 1H), 7.63 - 7.55 (m, 3H), 7.46 - 7.39 (m, 2H), 3.23 - 3.09 (m, 1H), 1.27 (d, J=7.2 Hz, 6H).

### Synthesis of 1-(propan-2-yl)-5-(trifluoromethyl)-1H-pyrazol-4-amine hydrochloride [Intermediate 1.9]:

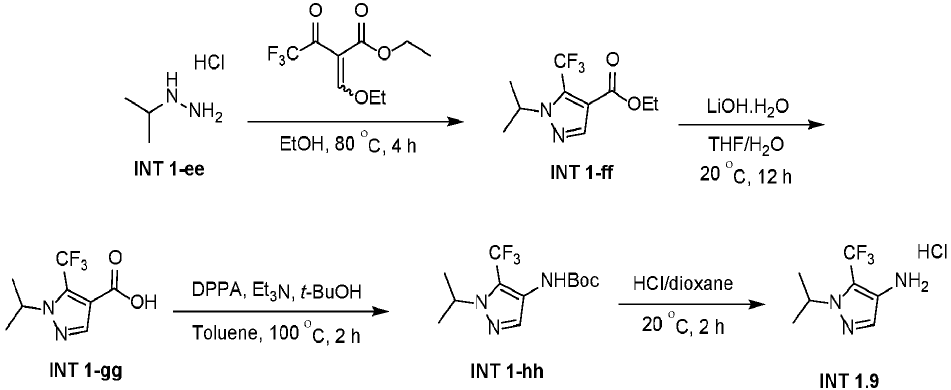

### Synthesis of ethyl 1-(propan-2-yl)-5-(trifluoromethyl)-1H-pyrazole-4-carboxylate [INT 1-ff]:

A mixture of ethyl 2-(ethoxymethylene)-4,4,4-trifluoro-3-oxobutanoate (2 g, 8.32 mmol) and (propan-2-yl)hydrazine hydrochloride [**INT 1-ee**] (920 mg, 8.32 mmol) in EtOH (20 mL) was stirred at 80 °C for 16 hr. The mixture was concentrated under reduced pressure to afford the crude product, which was purified by flash chromatography on silica gel (EtOAc/Petroleum ether = 0/1 to 1/10) to give ethyl 1-(propan-2-yl)-5-(trifluoromethyl)-1H-pyrazole-4-carboxylate [**INT 1-ff**] (1.40 g, 5.59 mmol, 67.3% yield) as a colorless oil. m/z: [M + H]+ Calcd for C10H14F3N2O2 251.1; Found 251.1. ¹H NMR (400 MHz, DMSO-d6) δ = 8.08 (s, 1H), 4.80 (spt, J = 6.4 Hz, 1H), 4.25 (q, J = 7.2 Hz, 2H), 1.45 (d, J = 6.4 Hz, 6H), 1.26 (t, J = 7.2 Hz, 3H).

### Synthesis of 1-(propan-2-yl)-5-(trifluoromethyl)-1H-pyrazole-4-carboxylic acid [INT 1-gg]:

To a solution of ethyl 1-(propan-2-yl)-5-(trifluoromethyl)-1H-pyrazole-4-carboxylate [**INT 1-ff**] (900 mg, 3.59 mmol) in THF (9 mL) and H₂O (3 mL) was added lithium hydroxide monohydrate (448 mg, 10.7 mmol). The reaction mixture was stirred at 20 °C for 12 hr, after which it was concentrated under reduced pressure. The crude product was quenched with water (20 mL) and washed with EtOAc (30 mL x 2). The aqueous phase was then acidified with 1 M HCl to pH =4 and extracted with EtOAc (20 mL x 2). The combined organic layers were washed with brine (50 mL x 2), dried over anhydrous Na₂SO₄, filtered, and concentrated under reduced pressure to give 1-(propan-2-yl)-5-(trifluoromethyl)-1H-pyrazole-4-carboxylic acid [**INT 1-gg**] (250 mg, 1.12 mmol, 31.3% yield) as a colorless oil. m/z: [M + H]+ Calcd for C8H10F3N2O2 223.1; Found 223.0.

### Synthesis of tert-butyl N-[1-(propan-2-yl)-5-(trifluoromethyl)-1H-pyrazol-4-yl]carbamate [INT 1-hh]:

A mixture of 1-(propan-2-yl)-5-(trifluoromethyl)-1H-pyrazole-4-carboxylic acid [**INT 1-gg**] (250 mg, 1.12 mmol), 2-methylpropan-2-ol (1.24 g, 16.8 mmol), diphenylphosphoryl azide (408 mg, 1.68 mmol) and triethylamine (340 mg, 3.36 mmol) in toluene (4 mL) was stirred at 100 °C for 2 hr. The reaction mixture was concentrated under reduced pressure to give the crude product, which was purified by flash chromatography on silica gel (EtOAc/Petroleum ether = 0/1 to 1/20) to give tert-butyl N-[1-(propan-2-yl)-5-(trifluoromethyl)-1H-pyrazol-4-yl]carbamate [**INT 1-hh**] (150 mg, 511 µmol, 45.7% yield) as a yellow oil. m/z: [M + H]+ Calcd for C12H19F3N3O2 294.1 ; Found 294.1.

### Synthesis of 1-(propan-2-yl)-5-(trifluoromethyl)-1H-pyrazol-4-amine hydrochloride [INT 1.9]:

A solution of tert-butyl N-[1-(propan-2-yl)-5-(trifluoromethyl)-1H-pyrazol-4-yl]carbamate [**INT 1-hh**] (150 mg, 511 µmol) in 4 M HCl in dioxane (3 mL) was stirred at 20 °C for 2 hr. The reaction mixture was concentrated under reduced pressure to give 1-(propan-2-yl)-5-(trifluoromethyl)-1H-pyrazol-4-amine hydrochloride [**INT 1.9**] (110 mg, 479 µmol, 94.0% yield). m/z: [M + H]+ Calcd for C7H11F3N3 194.1; Found 193.9.

### Synthesis of 1-[4-amino-5-(trifluoromethyl)-1H-pyrazol-1-yl]-2-methylpropan-2-ol hydrochloride [Intermediate 1.10]:

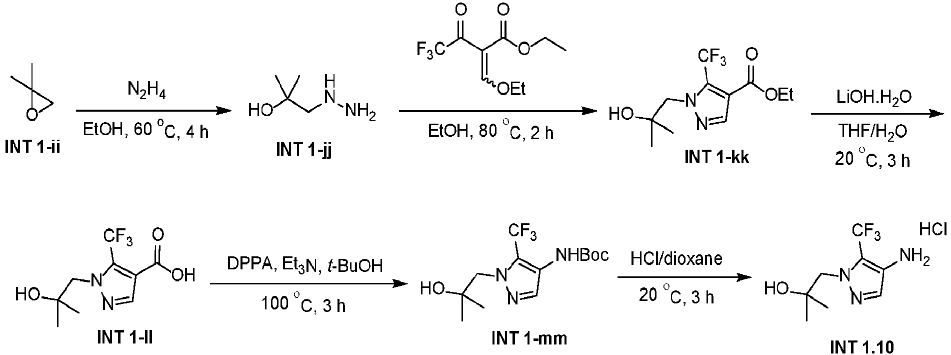

### Synthesis of 1-hydrazinyl-2-methylpropan-2-ol [INT 1-jj]:

To a suspension of 2,2-dimethyloxirane [**INT 1-ii**] (1.0 g, 13.8 mmol) in EtOH (5 mL) was added hydrazine (884 mg, 27.6 mmol). The reaction mixture was stirred at 60 °C for 4 h, after which it was concentrated under reduced pressure to give 1-hydrazinyl-2-methylpropan-2-ol [**INT 1-jj**] (1.24 g, 11.9 mmol, 86.7% yield) as a colorless oil. ¹H NMR (400 MHz, CDCl₃) δ = 3.52 (s, 4H), 2.71 (s, 2H), 1.21 - 1.18 (m, 6H).

### Synthesis of ethyl 1-(2-hydroxy-2-methylpropyl)-5-(trifluoromethyl)-IH-pyrazole-4-carboxylate [INT 1-kk]:

To a solution of ethyl 2-(ethoxymethylene)-4,4,4-trifluoro-3-oxobutanoate (2.0 g, 8.32 mmol) in EtOH (8 mL) was added 1-hydrazinyl-2-methylpropan-2-ol [**INT 1-jj**] (1.03 g, 9.98 mmol). The reaction was stirred at 80 °C for 2 h. The reaction mixture was concentrated under reduced pressure to give the crude product, which was purified by flash chromatography on silica gel (EtOAc/PE = 0/1 to 1/3) to give ethyl 1-(2-hydroxy-2-methylpropyl)-5-(trifluoromethyl)-1H-pyrazole-4-carboxylate [**INT 1-kk**] (1 g, 3.56 mmol, 42.9% yield) as a yellow oil. m/z: [M + H]+ Calcd for C11H16F3N2O3 281.1; Found 280.9. ¹H NMR (400 MHz, CDCl₃) δ = 8.01 (s, 1H), 4.40 - 4.30 (m, 4H), 3.85 (s, 1H), 1.37 (t, J=7.2 Hz, 3H), 1.19 (s, 6H).

### Synthesis of 1-(2-hydroxy-2-methylpropyl)-5-(trifluoromethyl)-1H-pyrazole-4-carboxylic acid [INT 1-l1]:

To a solution of ethyl 1-(2-hydroxy-2-methylpropyl)-5-(trifluoromethyl)-1H-pyrazole-4-carboxylate [**INT 1-kk**] (1.0 g, 3.56 mmol) in THF (5 mL) was added lithium hydroxide monohydrate (298 mg, 7.12 mmol) in H₂O (5 mL). The reaction was stirred at 20 °C for 3 h. The reaction mixture was poured into water (10 mL), acidified with 1 N HCl to pH=5-6 and extracted with EtOAc (20 mL x 2). The combined organic layers were washed with brine (20 mL x 2), dried over anhydrous Na₂SO₄ and concentrated under reduced pressure to give 1-(2-hydroxy-2-methylpropyl)-5-(trifluoromethyl)-1H-pyrazole-4-carboxylic acid [**INT 1-ll**] (710 mg, 2.81 mmol, 79.1% yield) as a yellow solid. m/z: [M + H]+ Calcd for C9H12F3N2O3 253.1; Found 252.9.

### Synthesis of tert-butyl N-[1-(2-hydroxy-2-methylpropyl)-5-(trifluoromethyl)-1H-pyrazol-4-yl]carbamate [INT 1-mm]:

A mixture of 1-(2-hydroxy-2-methylpropyl)-5-(trifluoromethyl)-1H-pyrazole-4-carboxylic acid [**INT 1-ll**] (710 mg, 2.81 mmol), diphenylphosphoryl azide (1.54 g, 5.62 mmol) and triethylamine (1.41 g, 14.0 mmol) in t-BuOH (8 mL) was stirred at 100 °C for 3 h under N₂. The reaction mixture was concentrated under reduced pressure to give the crude product, which was purified by flash chromatography on silica gel (EtOAc/PE = 0/1 to 1/3) to give tert-butyl N-[1-(2-hydroxy-2-methylpropyl)-5-(trifluoromethyl)-1H-pyrazol-4-yl]carbamate [**INT 1-mm**] (600 mg, 1.85 mmol, 66.0% yield) as a yellow oil. m/z: [M + H]+ Calcd for C13H21F3N3O3 324.1; Found 323.9. ¹H NMR (400 MHz, CDCl₃) δ = 8.11 (s, 1H), 6.47 (s, 1H), 4.42 (s, 1H), 4.08 (s, 2H), 1.52 (s, 9H), 1.17 (s, 6H).

### Synthesis of 1-[4-amino-5-(trifluoromethyl)-1H-pyrazol-1-yl]-2-methylpropan-2-ol hydrochloride [INT 1.10]:

A solution of tert-butyl N-[1-(2-hydroxy-2-methylpropyl)-5-(trifluoromethyl)-1H-pyrazol-4-yl]carbamate [**INT 1-mm**] (600 mg, 1.85 mmol) in HCl/Dioxane (5 mL) was stirred at 20 °C for 3 h. The reaction mixture was concentrated under reduced pressure to give 1-[4-amino-5-(trifluoromethyl)-1H-pyrazol-1-yl]-2-methylpropan-2-ol hydrochloride [**INT 1.10**] (480 mg, 1.84 mmol) as a yellow oil. m/z: [M + H]+ Calcd for C8H13F3N3O 224.1; Found 223.9.

### Synthesis of 1-(1-methoxypropan-2-yl)-5-(trifluoromethyl)-1H-pyrazol-4-amine hydrochloride [Intermediate 1.11]:

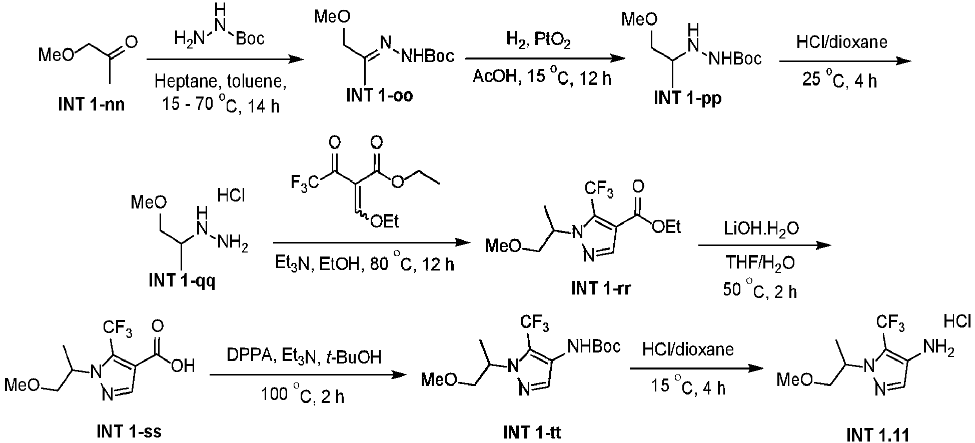

### Synthesis of N'-[(2Z)-1-methoxypropan-2-ylidene](tert-butoxy)carbohydrazide [INT 1-oo]:

To a mixture of 1-methoxypropan-2-one [**INT 1-nn**] (10 g, 113 mmol) in heptane (400 mL) at 50 °C was added (tert-butoxy)carbohydrazide (19.2 g, 146 mmol) in toluene (30 ml). The mixture was stirred at 70 °C for 2 hr. The mixture was then stirred at 15 °C for 12 hr. The precipitate formed was collected, washed with heptane and dried to give N'-[(2Z)-1-methoxypropan-2-ylidene](tert-butoxy)carbohydrazide [**INT 1-oo**] (19.6 g, 96.9 mmol, 85.9% yield) as a white solid. ¹H NMR (400 MHz, CDCl₃) δ = 7.59 (br d, J = 6.8 Hz, 1H), 4.02 (d, J = 2.4 Hz, 2H), 3.32 - 3.28 (m, 3H), 1.84 (s, 3H), 1.50 (d, J = 2.4 Hz, 9H).

### Synthesis of N'-(1-methoxypropan-2-yl)(tert-butoxy)carbohydrazide [INT 1-pp]:

A mixture of N'-[(2Z)-1-methoxypropan-2-ylidene](tert-butoxy)carbohydrazide [**INT 1-oo**] (5 g, 24.7 mmol) and PtO₂ (0.05 g, 220 µmol) in AcOH (50 ml) under H₂ (103.421 kPa, 15 psi) was stirred at 15 °C for 12 h. After filtering the mixture through celite a solution of NaHCO₃ was added to adjust the pH to 8-9. The mixture was extracted with EtOAc (100 mL x 2). The combined organic layers were dried over Na₂SO₄, filtered and concentrated under reduced pressure to give the crude product, which was purified by flash chromatography on silica gel (CH₂Cl₂/MeOH = 1/20 to 1/0) to give N'-(1-methoxypropan-2-yl)(tert-butoxy)carbohydrazide [**INT 1-pp**] (3.25 g, 15.9 mmol, 64.4% yield) as a yellow oil. m/z: [M -56+ H]+ Calcd for C9H21N2O3 149.0; Found 148.9. ¹H NMR (400 MHz, CDCl₃) δ = 6.45 - 5.99 (m, 1H), 4.25 (br s, 1H), 3.34 (s, 3H), 3.31 (t, J=4.4 Hz, 1H), 3.27 - 3.17 (m, 2H), 1.44 (s, 9H), 0.99 (br s, 3H).

### Synthesis of (1-methoxypropan-2-yl)hydrazine hydrochloride [INT 1-qq]:

A mixture of N'-(1-methoxypropan-2-yl)(tert-butoxy)carbohydrazide [**INT 1-pp**] (3.25 g, 15.9 mmol) in 4 M HCl/dioxane (30 mL, 120 mmol) was stirred at 25 °C for 4 hr. The mixture was concentrated under reduced pressure to afford (1-methoxypropan-2-yl)hydrazine hydrochloride [**INT 1-qq**] (2.00 g, 14.2 mmol, 89.6% yield) as a yellow oil. m/z: [M + H]+ Calcd for C4H13N2O 105.1; Found 104.8.

### Synthesis of ethyl 1-(1-methoxypropan-2-yl)-5-(trifluoromethyl)-1H-pyrazole-4-carboxylate [INT 1-rr]:

A mixture of ethyl 2-(ethoxymethylene)-4,4,4-trifluoro-3-oxobutanoate (2 g, 8.32 mmol), (1-methoxypropan-2-yl)hydrazine hydrochloride [**INT 1-qq**] (1.28 g, 9.10 mmol), and triethylamine (841 mg, 8.32 mmol) in EtOH (20 mL) was stirred at 80 °C for 12 hr. The mixture was concentrated under reduced pressure to afford the crude product, which was purified by flash chromatography on silica gel (EtOAc/Petroleum ether = 1/10 to 1/0) to give ethyl 1-(1-methoxypropan-2-yl)-5-(trifluoromethyl)-1H-pyrazole-4-carboxylate [**INT 1-rr**] (990 mg, 3.55 mmol, 42.8% yield) as a yellow oil. m/z: [M + H]+ Calcd for C11H16F3N2O3 281.1; Found 281.1. ¹H NMR (400 MHz, DMSO-d6) δ = 8.11 (s, 1H), 4.88 - 4.77 (m, 1H), 4.25 (q, J=6.8 Hz, 2H), 3.74 - 3.66 (m, 1H), 3.62 - 3.55 (m, 1H), 3.19 - 3.12 (m, 3H), 1.40 (d, J = 6.8 Hz, 3H), 1.27 (t, J = 7.2 Hz, 3H).

### Synthesis of 1-(1-methoxypropan-2-yl)-5-(trifluoromethyl)-1H-pyrazole-4-carboxylic acid [INT 1-ss]:

To a mixture of ethyl 1-(1-methoxypropan-2-yl)-5-(trifluoromethyl)-1H-pyrazole-4-carboxylate [**INT 1-rr**] (890 mg, 3.17 mmol) in THF (6 mL) and H₂O (2 mL) was added LiOH·H₂O (1.06 g, 25.3 mmol) and the mixture was stirred at 50 °C for 2 hr. The mixture was concentrated under reduced pressure to afford the crude product. 1N HCl was added to adjust the pH to 3-4 and the mixture was extracted with EtOAc (50 mL x 2). The combined organic layers were dried over anhydrous Na₂SO₄, filtered and concentrated under reduced pressure to give 1-(1-methoxypropan-2-yl)-5-(trifluoromethyl)-1H-pyrazole-4-carboxylic acid [**INT 1-ss**] (395 mg, 1.56 mmol, 49.4% yield) as a yellow oil. m/z: [M + H]+ Calcd for C9H12F3N2O3 253.1; Found 253.0. ¹H NMR (400 MHz, CDCl₃) δ = 8.07 (s, 1H), 4.88 (qd, J = 6.8, 13.2 Hz, 1H), 3.85 (t, J = 9.2 Hz, 1H), 3.63 (dd, J = 5.2, 10.0 Hz, 1H), 3.30 (s, 3H), 1.52 (d, J = 6.8 Hz, 3H).

### Synthesis of tert-butyl N-[1-(1-methoxypropan-2-yl)-5-(trifluoromethyl)- 1H-pyrazol-4-yl]carbamate [INT 1-tt]:

A mixture of 1-(1-methoxypropan-2-yl)-5-(trifluoromethyl)-1H-pyrazole-4-carboxylic acid [**INT 1-ss**] (295 mg, 1.16 mmol), 2-methylpropan-2-ol (1.28 g, 17.4 mmol), diphenylphosphoryl azide (423 mg, 1.74 mmol) and triethylamine (352 mg, 3.48 mmol) in toluene (3 mL) was stirred at 100 °C for 2 hr. NaHCO₃ (30 mL) was added and the mixture was extracted with EtOAc (30 mL x 2). The combined organic layers were washed with brine (30 mL x 2), dried over anhydrous Na₂SO₄, filtered and concentrated under reduced pressure to give the crude product, which was purified by flash chromatography on silica gel (EtOAc/Petroleum ether = 1/10 to 1/0) to give tert-butyl N-[1-(1-methoxypropan-2-yl)-5-(trifluoromethyl)-1H-pyrazol-4-yl]carbamate [**INT 1-tt**] (208 mg, 643 µmol, 55.4% yield) as a yellow oil. m/z: [M + H]+ Calcd for C13H21F3N303 324.1; Found 324.1. ¹H NMR (400 MHz, CDCl₃) δ = 8.07 (br s, 1H), 6.44 (br s, 1H), 4.55 (sxt, J = 6.8 Hz, 1H), 3.84 (dd, J = 7.6, 9.6 Hz, 1H), 3.60 (dd, J = 5.6, 10.0 Hz, 1H), 3.30 (s, 3H), 1.51 (s, 9H), 1.45 (d, J = 6.8 Hz, 3H).

### 1-(1-methoxypropan-2-yl)-5-(trifluoromethyl)-1H-pyrazol-4-amine hydrochloride [INT 1.11]:

A mixture of tert-butyl N-[1-(1-methoxypropan-2-yl)-5-(trifluoromethyl)-1H-pyrazol-4-yl]carbamate [**INT 1-tt**] (158 mg, 488 µmol) in 4 M HCl/dioxane (15 mL, 60.0 mmol) was stirred at 15 °C for 4 hr. The mixture was concentrated under reduced pressure to afford 1-(1-methoxypropan-2-yl)-5-(trifluoromethyl)-1H-pyrazol-4-amine hydrochloride [**INT 1.11**] (150 mg, 577 µmol) as a yellow oil. m/z: [M + H]+ Calcd for C8H13F3NO 224.1; Found 223.9.

### Synthesis of 1-neopentyl-5-(trifluoromethyl)-1H-pyrazol-4-amine [Intermediate 1.12]:

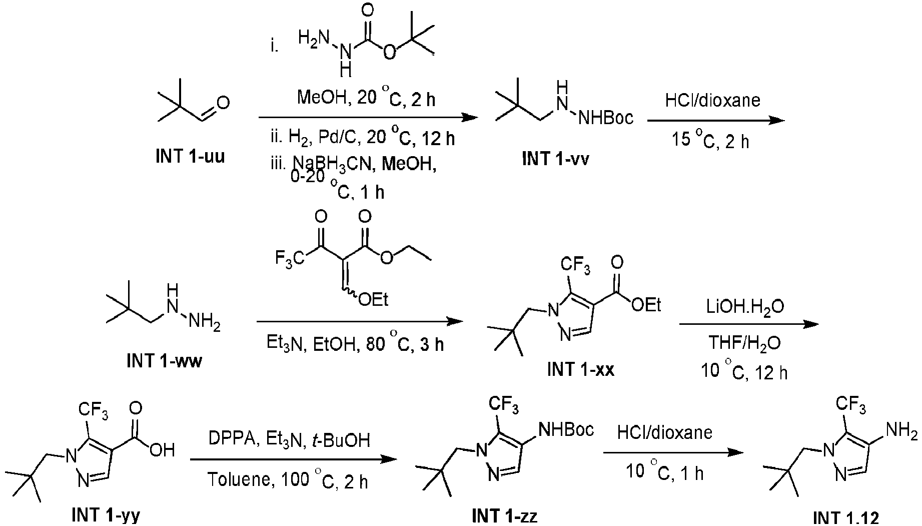

### Synthesis of N'-(2,2-dimethylpropyl)(tertbutoxy)carbohydrazide [INT 1-vv]:

A mixture of 2,2-dimethylpropanal [**INT 1-uu**] (5 g, 58.0 mmol) and (tert-butoxy)carbohydrazide (7.66 g, 58.0 mmol) in MeOH (80 mL) was stirred at 20 °C for 2 hours. Then 10% Pd/C (1 g) was added. The flask was evacuated and backfilled with N₂ three times, then evacuated and backfilled with H₂ three more times. The reaction mixture was stirred at 20 °C under H₂ (103.421 kPa, 15 psi) for 12 h. The reaction was filtered and the filter cake was washed with MeOH (20 mL x 3). The filtrate was cooled to 0 °C, then sodium cyanoborohydride (3.64 g, 58.0 mmol) was added. The mixture was stirred at 20 °C for 1 hour. The reaction was quenched by adding water (80 mL). The mixture was concentrated under reduced pressure to remove MeOH. The residue was diluted with water (100 mL) and was extracted with EtOAc (2 x 150 mL). The combined organic layers were washed with brine (2 x 80 mL), dried over anhydrous Na₂SO₄ and concentrated under reduced pressure to give N'-(2,2-dimethylpropyl)(tertbutoxy)carbohydrazide [**INT 1-vv**] (11.0 g, 54.3 mmol, 94% yield) as a gray solid. m/z: [M -t-Bu+ H]+ Calcd for C6H15N2O2 147.2; Found 147.1. ¹H NMR (400 MHz, CDCl₃) δ = 2.59 (s, 2H), 1.39 (s, 9H), 0.88 (s, 9H).

### Synthesis of (2,2-dimethylpropyl)hydrazine [INT 1-ww]:

N'-(2,2-dimethylpropyl)(tert-butoxy)carbohydrazide [**INT 1-vv**] (6 g, 29.6 mmol) was added to HCl/dioxane (40 mL) at 15 °C and the mixture was stirred for 2 h. The mixture was concentrated under reduced pressure to give (2,2-dimethylpropyl)hydrazine [**INT 1-ww**] (3.02 g, 29.5 mmol). ¹H NMR (400 MHz, DMSO-d6) δ = 2.67 (s, 2H), 0.91 (s, 9H).

### Synthesis of ethyl 1-(2,2-dimethylpropyl)-5-(trifluoromethyl)-1H-pyrazole-4-carboxylate [INT 1-xx]:

To a solution of ethyl 2-(ethoxymethylene)-4,4,4-trifluoro-3-oxobutanoate (8.50 g, 35.4 mmol) in EtOH (100 mL) at 10 °C was added (2,2-dimethylpropyl)hydrazine [**INT 1-ww**] (3.02 g, 29.5 mmol) and Et₃N (5.97 g, 59.0 mmol). The mixture was stirred at 80 °C for 3 h. The reaction was concentrated under reduced pressure, quenched by adding water (50 mL) and was extracted with EtOAc (50 mL x 3). The combined organic layers were washed with brine (50 mL x 2), dried over anhydrous Na₂SO₄ and concentrated under reduced pressure to give the crude product, which was purified by flash chromatography on silica gel (PE/EtOAc= 1/0 to 20/1) to give ethyl 1-(2,2-dimethylpropyl)-5-(trifluoromethyl)-1H-pyrazole-4-carboxylate [**INT 1-xx**] (1.81 g, 6.50 mmol, 22.0% yield) as a colorless oil. m/z: [M + H]+ Calcd for C12H18F3N2O2 279.1; Found 278.9. (400 MHz, CDCl₃) δ = 7.97 (s, 1H), 4.34 (q, J = 8.0 Hz, 2H), 4.19 (s, 2H), 1.37 (t, J = 8.0 Hz, 3H), 0.99 (s, 9H).

### 1-(2,2-dimethylpropyl)-5-(trifluoromethyl)-1H-pyrazole-4-carboxylic acid [INT 1-yy]:

To a solution of ethyl 1-(2,2-dimethylpropyl)-5-(trifluoromethyl)-1H-pyrazole-4-carboxylate [**INT 1-xx**] (1.81 g, 6.50 mmol) in THF (10 mL) and H₂O (3 mL) at 10 °C was added lithium hydroxide monohydrate (1.36 g, 32.5 mmol), then the mixture was stirred for 12 h. The aqueous phase was acidified with 1 M HCl to pH =4 and extracted with EtOAc (30 mL x 2). The combined organic layers were concentrated under reduced pressure to give to give 1-(2,2-dimethylpropyl)-5-(trifluoromethyl)-1H-pyrazole-4-carboxylic acid [**INT 1-yy**] (1.16 g, 4.63 mmol, 71.6% yield) as a white solid. ¹H NMR (400 MHz, DMSO) δ = 8.04 (s, 1H), 4.16 (s, 2H), 0.91 (s, 9H).

### Synthesis of tert-butyl N-[1-(2,2-dimethylpropyl)-5-(trifluoromethyl)-1H-pyrazol-4-yl]carbamate [INT 1-zz]:

A mixture of 1-(2,2-dimethylpropyl)-5-(trifluoromethyl)-1H-pyrazole-4-carboxylic acid [**INT 1-yy**] (1.06 g, 4.23 mmol), 2-methylpropan-2-ol (4.69 g, 63.4 mmol), diphenylphosphoryl azide (1.54 g, 6.34 mmol) and triethylamine (2.13 g, 21.1 mmol) in toluene (10 mL) was stirred at 100 °C for 2 hr. The mixture was worked up with material from two of the same reactions. Water (40 mL) was added and the mixture was extracted with EtOAc (40 mL x 2). The combined organic layers were washed with brine (40 mL x 2), dried over anhydrous Na₂SO₄, filtered and concentrated under reduced pressure to give the crude product, which was purified by flash chromatography on silica gel (Petroleum ether/EtOAc = 1/10 to 20/1) to give tert-butyl N-[1-(2,2-dimethylpropyl)-5-(trifluoromethyl)-1H-pyrazol-4-yl]carbamate [**INT 1-zz**] (1.27 g, 3.95 mmol) as a white solid. ¹H NMR (400 MHz, CDCl₃) δ = 8.02 (s, 1H), 6.43 (s, 1H), 3.94 (s, 2H), 1.52 (s, 9H), 0.99 (s, 9H).

### 1-neopentyl-5-(trifluoromethyl)-1H-pyrazol-4-amine [INT 1.12]:

A solution of tert-butyl N-[1-(2,2-dimethylpropyl)-5-(trifluoromethyl)-1H-pyrazol-4-yl]carbamate [**INT 1-zz**] (500 mg, 1.55 mmol) in HCl/dioxane (10 mL) was stirred at 10 °C for 1 h. TLC showed complete consumption of the starting material and formation of a new spot. The mixture was concentrated under reduced pressure to give 1-neopentyl-5-(trifluoromethyl)-1H-pyrazol-4-amine [**INT 1.12**] (342 mg, 1.54 mmol).

### Synthesis of 1-(pyridin-3-yl)-5-(trifluoromethyl)-1H-pyrazol-4-amine hydrochloride [Intermediate 1.13]:

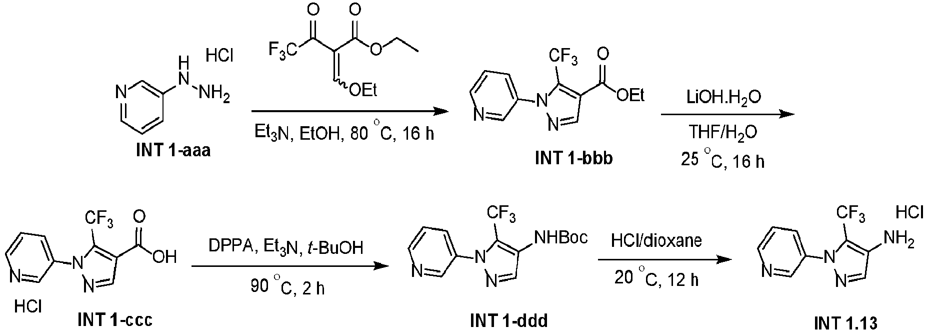

### Synthesis of ethyl 1-(pyridin-3-yl)-5-(trifluoromethyl)-1H-pyrazole-4-carboxylate [INT 1-bbb]:

A solution consisting of ethyl 2-(ethoxymethylidene)-4,4,4-trifluoro-3-oxobutanoate (9.39 g, 39.1 mmol), 3-hydrazinylpyridine hydrochloride [**INT 1-aaa**] (5.7 g, 39.1 mmol), Et₃N (7.91 g, 78.2 mmol) and EtOH (50 mL) was stirred at 80 °C for 16 h. The mixture was concentrated under reduced pressure to afford the crude product, which was purified by flash column chromatography (petroleum ether:ethyl acetate=100:0 to 80:20) to give ethyl 1-(pyridin-3-yl)-5-(trifluoromethyl)-1H-pyrazole-4-carboxylate [**INT 1-bbb**] (3.45 g, 12.0 mmol, 31.0% yield). m/z: [M + H]+ Calcd for C12H11F3N302 286.1; Found 285.9. ¹H NMR (400 MHz, CDCl₃) δ = 8.78 (dd, J = 1.2, 4.8 Hz, 1H), 8.74 (d, J = 2.4 Hz, 1H), 8.18 (s, 1H), 7.84 - 7.76 (m, 1H), 7.50 (dd, J = 4.8, 8.0 Hz, 1H), 4.40 (q, J = 7.2 Hz, 2H), 1.41 (t, J = 7.2 Hz, 3H).

### 1-(pyridin-3-yl)-5-(trifluoromethyl)-1H-pyrazole-4-carboxylic acid hydrochloride [INT 1-ccc]:

To a solution of ethyl 1-(pyridin-3-yl)-5-(trifluoromethyl)-1H-pyrazole-4-carboxylate [**INT 1-bbb**] (3.45 g, 12.0 mmol) in THF (1 mL) was added lithium hydroxide monohydrate (12.0 mL, 24.0 mmol). The mixture was stirred at 25 °C for 16 h. The resulting solution was concentrated to dryness under reduced pressure to afford the crude product, which was poured into water (20 mL) and acidified with 1 M HCl to pH = 5. The resulting solution was filtered and the filter cake was washed water (20 mL) and then dried under reduced pressure to afford 1-(pyridin-3-yl)-5-(trifluoromethyl)-1H-pyrazole-4-carboxylic acid hydrochloride [**INT 1-ccc**] (2.30 g, 7.83 mmol, 65.3% yield) as a white solid. m/z: [M + H]+ Calcd for C10H7F3N3O2 258.0; Found 257.9.

### Synthesis of tert-butyl N-[1-(pyridin-3-yl)-5-(trifluoromethyl)-1H-pyrazol-4-yl]carbamate [INT 1-ddd]:

A solution of 1-(pyridin-3-yl)-5-(trifluoromethyl)-1H-pyrazole-4-carboxylic acid hydrochloride [**INT 1-ccc**] (500 mg, 1.70 mmol), triethylamine (472 µL, 3.40 mmol) and diphenylphosphoryl azide (701 mg, 2.55 mmol) in tert-butanol (2 mL, 21.1 mmol) was stirred at 90 °C for 2 h under N₂. The reaction was quenched by adding water (15 mL) and was extracted with EtOAc (2 x 15 mL). The combined organic layers were washed with brine (2 x 30 mL), dried over anhydrous Na₂SO₄ and concentrated under reduced pressure to give the crude product, which was purified by flash chromatography on silica gel (EtOAc/Petroleum ether = 0/1 to 1/4) to give tert-butyl N-[1-(pyridin-3-yl)-5-(trifluoromethyl)-1H-pyrazol-4-yl]carbamate [**INT 1-ddd**] (443 mg, 1.34 mmol, 79.3% yield) as a white solid. m/z: [M + H]+ Calcd for C14H16F3N4O2 329.1; Found 328.9. ¹H NMR (400 MHz, CDCl₃) δ = 8.80 - 8.69 (m, 2H), 8.35 (br s, 1H), 7.81 (br d, J = 8.0 Hz, 1H), 7.47 (dd, J = 4.8, 8.0 Hz, 1H), 6.60 (br s, 1H), 1.56 (s, 9H).

### 1-(pyridin-3-yl)-5-(trifluoromethyl)-1H-pyrazol-4-amine hydrochloride [INT 1.13]:

A solution of tert-butyl N-[1-(pyridin-3-yl)-5-(trifluoromethyl)-1H-pyrazol-4-yl]carbamate [**INT 1-ddd**] (443 mg, 1.34 mmol) in HCl (4 M in dioxane, 5 mL, 20.0 mmol) was stirred at 20 °C for 12 h. The reaction mixture was concentrated under reduced pressure to give 1-(pyridin-3-yl)-5-(trifluoromethyl)-1H-pyrazol-4-amine hydrochloride [**INT 1.13**] (343 mg, 1.29 mmol, 96.8% yield) as a yellow solid. m/z: [M + H]+ Calcd for C9H8F3N4 229.1; Found 228.9. ¹H NMR (400 MHz, DMSO-d6) δ = 8.81 (d, J = 2.4 Hz, 1H), 8.79 (dd, J = 1.2, 4.8 Hz, 1H), 8.12 (br d, J = 8.4 Hz, 1H), 7.83 (s, 1H), 7.75 (dd, J = 4.8, 8.4 Hz, 1H).

### Synthesis of 1-(pyridin-4-yl)-5-(trifluoromethyl)-1H-pyrazol-4-amine [Intermediate 1.14]:

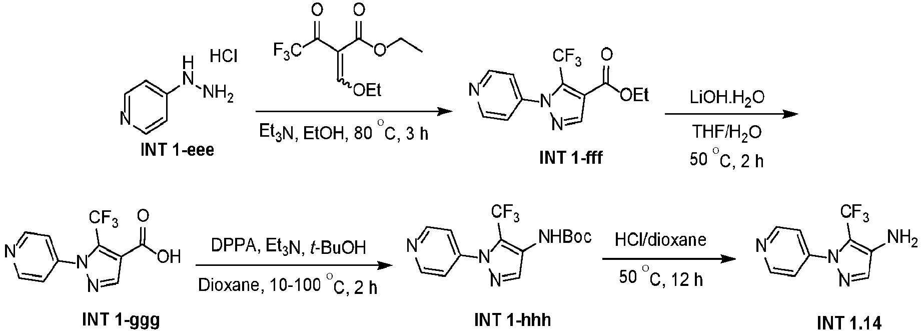

### Synthesis of ethyl 1-(pyridin-4-yl)-5-(trifluoromethyl)-IH-pyrazole-4-carboxylate[INT 1-fff]:

To a solution of ethyl 2-(ethoxymethylidene)-4,4,4-trifluoro-3-oxobutanoate (7.20 g, 30.0 mmol) in EtOH (50 mL) at 10 °C was added 4-hydrazinylpyridine hydrochloride [**INT 1-eee**] (4.37 g, 30.0 mmol) and Et₃N (3.03 g, 30.0 mmol), then the mixture was stirred at 80 °C for 3 h. The reaction was concentrated under reduced pressure, quenched by adding water (50 mL) and was extracted with EtOAc (50 mL x 3). The combined organic layers were washed with brine (50 mL x 2), dried over anhydrous Na₂SO₄ and concentrated under reduced pressure to give the crude product, which was purified by flash chromatography on silica gel (PE/EtOAc= 1/0 to 3/1) to give ethyl 1-(pyridin-4-yl)-5-(trifluoromethyl)-1H-pyrazole-4-carboxylate [**INT 1-fff**] (3.00 g, 10.5 mmol, 35.0% yield) as a yellow solid. m/z: [M + H]+ Calcd for C12H11F3N3O2 286.0; Found 285.9.

### Synthesis of 1-(pyridin-4-yl)-5-(trifluoromethyl)-1H-pyrazole-4-carboxylic acid [INT 1-ggg]:

To a solution of ethyl 1-(pyridin-4-yl)-5-(trifluoromethyl)-1H-pyrazole-4-carboxylate [**INT 1-fff**] (2.5 g, 8.76 mmol) in THF (15 mL) and H₂O (3 mL) at 10 °C was added lithium hydroxide monohydrate (1.83 g, 43.8 mmol), then the mixture was stirred at 50 °C for 2 h. The aqueous phase was acidified with 1 M HCl to pH=4 and extracted with EtOAc (30 mL x 2). The combined organic layers were concentrated under reduced pressure to give 1-(pyridin-4-yl)-5-(trifluoromethyl)-1H-pyrazole-4-carboxylic acid [**INT 1-ggg**] (1.45 g, 5.63 mmol, 64.4% yield) as a white solid. m/z: [M + H]+ Calcd for C10H7F3N3O2 258.0; Found 257.8.

### Synthesis of tert-butyl N-[1-(pyridin-4-yl)-5-(trifluoromethyl)-1H-pyrazol-4-yl]carbamate [INT 1-hhh]:

To a mixture of 1-(pyridin-4-yl)-5-(trifluoromethyl)-1H-pyrazole-4-carboxylic acid [**INT 1-ggg**] (1 g, 3.88 mmol) in t-BuOH (3 mL) and dioxane (5 mL) was added triethylamine (1.96 g, 19.4 mmol) and diphenylphosphoryl azide (1.88 g, 7.76 mmol) at 10 °C, then the mixture was stirred at 100 °C for 2 hr. Water (30 mL) was added and the mixture was extracted with EtOAc (30 mL x 2). The combined organic layers were washed with brine (30 mL x 2), dried over anhydrous Na₂SO₄, filtered and concentrated under reduced pressure to give the crude product, which was purified by flash chromatography on silica gel (Petroleum ether/EtOAc = 1/10 to 3/1) to give tert-butyl N-[1-(pyridin-4-yl)-5-(trifluoromethyl)-1H-pyrazol-4-yl]carbamate [**INT 1-hhh**] (360 mg, 1.09 mmol, 28.3% yield) as a white solid. ¹H NMR (400 MHz, DMSO) δ = 8.78 (d, J = 4.0 Hz, 2H), 8.58 (s, 1H), 8.00 (s, 1H), 7.56 (d, J = 4.0 Hz, 2H), 1.46 (s, 9H).

### Synthesis of 1-(pyridin-4-yl)-5-(trifluoromethyl)-1H-pyrazol-4-amine [INT 1.14]:

A solution of tert-butyl N-[1-(pyridin-4-yl)-5-(trifluoromethyl)-1H-pyrazol-4-yl]carbamate [**INT 1-hhh**] (360 mg, 1.09 mmol) in HCl/dioxane (10 mL) was stirred at 50 °C for 12 h. The mixture was concentrated under reduced pressure to give 1-(pyridin-4-yl)-5-(trifluoromethyl)-1H-pyrazol-4-amine [**INT 1.14**] (248 mg, 1.08 mmol). m/z: [M + H]+ Calcd for C9H8F3N4 229.1; Found 229.1.

### Synthesis of 1-(pyridin-2-yl)-5-(trifluoromethyl)-1H-pyrazol-4-amine [Intermediate 1.15]:

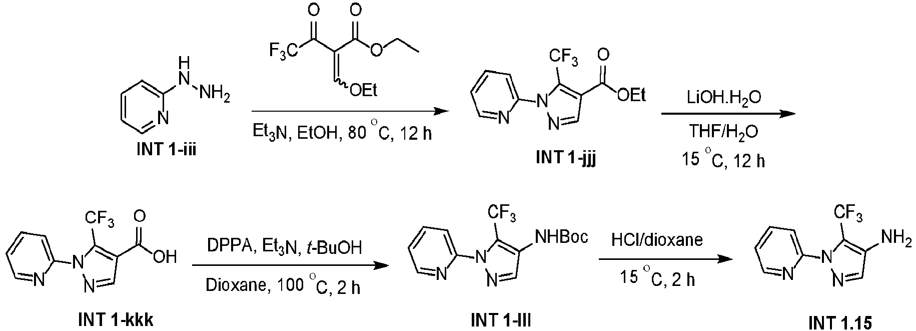

### Synthesis of ethyl 1-(pyridin-2-yl)-5-(trifluoromethyl)-1H-pyrazole-4-carboxylate[INT 1-jjj]:

To a solution of ethyl 2-(ethoxymethylidene)-4,4,4-trifluoro-3-oxobutanoate (5 g, 20.8 mmol) and 2-hydrazinylpyridine [**INT 1-iii**] (2.26 g, 20.8 mmol) in EtOH (30 mL) was added triethylamine (2.10 g, 20.8 mmol). The mixture was stirred at 80 °C for 12 hr. The reaction mixture was concentrated under reduced pressure to give the crude product, which was purified by flash chromatography on silica gel (EtOAc/PE = 0/1 to 10/1) to give ethyl 1-(pyridin-2-yl)-5-(trifluoromethyl)-1H-pyrazole-4-carboxylate [**INT 1-jjj**] (5.00 g, 17.5 mmol, 84.3% yield) as yellow oil. m/z: [M + H]+ Calcd for C12H11F3N3O2 286.1; Found 285.9. ¹H NMR (400 MHz, DMSO-d6) δ = 8.64 - 8.59 (m, 1H), 8.34 (s, 1H). 8.19 - 8.12 (m, 1H), 7.80 (dd, J = 0.8, 8.0 Hz, 1H), 7.68 - 7.63 (m, 1H), 4.33 (q, J = 7.2 Hz, 2H), 1.31 (t, J = 7.2 Hz, 3H).

### Synthesis of 1-(pyridin-2-yl)-5-(trifluoromethyl)-1H-pyrazole-4-carboxylic acid [INT 1-kkk]:

To a mixture of ethyl 1-(pyridin-2-yl)-5-(trifluoromethyl)-1H-pyrazole-4-carboxylate **[INT 1-jjj]** (1 g, 3.50 mmol) in THF (6 mL) and H₂O (2 mL) was added lithium hydroxide monohydrate (440 mg, 10.5 mmol). The reaction mixture was stirred at 15 °C for 12 hours. The reaction mixture was concentrated under reduced pressure. The residue was acidified with 1 M HCl to pH=4. The reaction mixture was quenched by the addition of H₂O (20 mL) and extracted with EtOAc (20 mL x 2). The combined organic layers were washed with brine (20 mL), dried over Na₂SO₄, filtered and concentrated under reduced pressure to give 1-(pyridin-2-yl)-5-(trifluoromethyl)-1H-pyrazole-4-carboxylic acid [**INT 1-kkk**] (800 mg, 3.11 mmol, 88.8% yield) as a yellow solid. ¹H NMR (400 MHz, DMSO-d6) δ = 13.43 (br s, 1H), 8.60 (td, J = 0.8, 4.8 Hz, 1H), 8.28 (s, 1H), 8.14 (dt, J = 2.0, 7.6 Hz, 1H), 7.79 (d, J = 8.0 Hz, 1H), 7.64 (ddd, J = 0.8, 4.8, 7.6 Hz, 1H).

### Synthesis of tert-butyl N-[1-(pyridin-2-yl)-5-(trifluoromethyl)-1H-pyrazol-4-yl]carbamate [INT 1-lll]:

A mixture of 1-(pyridin-2-yl)-5-(trifluoromethyl)-1H-pyrazole-4-carboxylic acid [**INT 1-kkk**] (400 mg, 1.55 mmol), triethylamine (784 mg, 7.75 mmol), and diphenylphosphoryl azide (564 mg, 2.32 mmol) in t-BuOH (3 mL) and dioxane (3 mL) was stirred at 100 °C for 2 hr. The reaction mixture was concentrated under reduced pressure to give the crude product, which was purified by flash chromatography on silica gel (Petroleum ether/EtOAc = 1/0 to 1/1) to give tert-butyl N-[1-(pyridin-2-yl)-5-(trifluoromethyl)-1H-pyrazol-4-yl]carbamate [**INT 1-lll**] (470 mg, 1.43 mmol, 92.5% yield) as a white solid. m/z: [M + H]+ Calcd for C14H16F3N4O2 329.1; Found 328.9. ¹H NMR (400 MHz, DMSO-d6) δ = 9.14 (br s, 1H), 8.56 - 8.51 (m, 1H), 8.06 (dt, J = 1.8, 7.6 Hz, 1H), 7.95 (s, 1H), 7.77 (d, J = 8.0 Hz, 1H), 7.55 - 7.46 (m, 1H), 1.46 (s, 9H).

### Synthesis of 1-(pyridin-2-yl)-5-(trifluoromethyl)-1H-pyrazol-4-amine [INT 1.15]:

To a solution of tert-butyl N-[1-(pyridin-2-yl)-5-(trifluoromethyl)-1H-pyrazol-4-yl]carbamate [**INT 1-lll**] (470 mg, 1.43 mmol) in 4 M HCl/dioxane (4 mL) was stirred at 15 °C for 2 h. The reaction mixture was concentrated under reduced pressure to give 1-(pyridin-2-yl)-5-(trifluoromethyl)-1H-pyrazol-4-amine [**INT 1.15**] (260 mg, 1.13 mmol, 79.7% yield) as a white solid. m/z: [M + H]+ Calcd for C9H8F3N4 229.1; Found 228.9. ¹H NMR (400 MHz, DMSO-d6) δ = 8.48 (dd, J = 1.2, 4.8 Hz, 1H), 8.01 (dt, J = 1.6, 7.6 Hz, 1H), 7.76 - 7.72 (m, 2H), 7.65 (br s, 2H), 7.43 (dd, J = 4.8, 6.8 Hz, 1H).

### Synthesis of 2-[4-amino-5-(trifluoromethyl)-1H-pyrazol-1-yl]benzonitrile [Intermediate 1.16]:

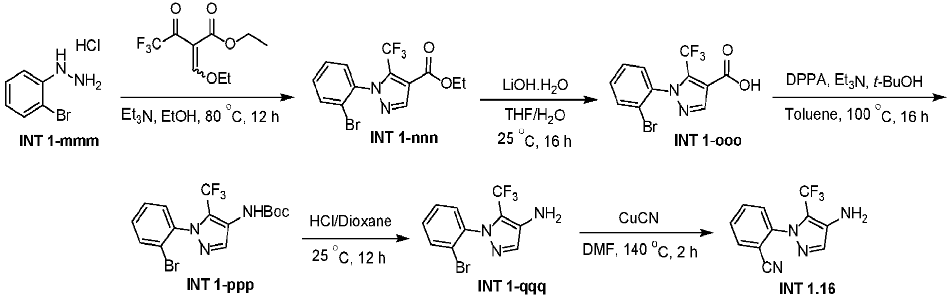

### Synthesis of ethyl 1-(2-bromophenyl)-5-(trifluoromethyl)-1H-pyrazole-4-carboxylate [INT 1-nnn1:

To a solution of (2-bromophenyl)hydrazine hydrochloride [**INT 1-mmm**] (5 g, 22.3 mmol) and ethyl 2-(ethoxymethylidene)-4,4,4-trifluoro-3-oxobutanoate (4.44 g, 18.5 mmol) in EtOH (6 mL) was added triethylamine (2.81 g, 27.8 mmol). The mixture was stirred at 80 °C for 12 hr. The reaction was quenched by adding water (50 mL) and was extracted with EtOAc (3 x 50 mL). The combined organic layers were washed with brine (2 x 50 mL), dried over anhydrous Na₂SO₄ and concentrated under reduced pressure to give the crude product, which was purified by flash chromatography on silica gel (PE/EtOAc = 1/0 to 1/10) to give ethyl 1-(2-bromophenyl)-5-(trifluoromethyl)-1H-pyrazole-4-carboxylate [**INT 1-nnn**] (3.00 g, 8.26 mmol, 44.7% yield) as a red oil. m/z: [M + H]+ Calcd for C13H11BrF3N2O2 363.0; Found 362.9.

### Synthesis of 1-(2-bromophenyl)-5-(trifluoromethyl)-1H-pyrazole-4-carboxylic acid [INT 1-ooo]:

To a solution of ethyl 1-(2-bromophenyl)-5-(trifluoromethyl)-1H-pyrazole-4-carboxylate [**INT 1-nnn**] (2 g, 3.30 mmol) in THF (3 mL) and H₂O (1 mL) was added lithium hydroxide monohydrate (276 mg, 6.60 mmol). The mixture was stirred at 25 °C for 16 hr. The reaction was quenched by adding 1 N HCl (10 mL) and H₂O (20 mL) and was extracted with EtOAc (3 x 20 mL). The combined organic layers were washed with brine (2 x 50 mL), dried over anhydrous Na₂SO₄ and concentrated under reduced pressure to give 1-(2-bromophenyl)-5-(trifluoromethyl)-1H-pyrazole-4-carboxylic acid [**INT 1-ooo**] (800 mg, 2.38 mmol, 72.7% yield) as a red solid. ¹H NMR (400 MHz, DMSO-d6) δ = 13.43 (br s, 1H), 8.31 (s, 1H), 7.88 (dd, J = 1.6, 7.6 Hz, 1H), 7.74 (dd, J = 2.0, 7.6 Hz, 1H), 7.59 (dquin, J = 1.6, 7.6 Hz, 2H).

### Synthesis of tert-butyl N-[1-(2-bromophenyl)-5-(trifluoromethyl)-1H-pyrazol-4-yl]carbamate [INT 1-ppp]:

To a solution of 1-(2-bromophenyl)-5-(trifluoromethyl)-1H-pyrazole-4-carboxylic acid [**INT 1-ooo**] (1.2 g, 3.58 mmol) in toluene (1 mL) was added diphenylphosphoryl azide (1.47 g, 5.37 mmol), triethylamine (1.08 g, 10.7 mmol) and 2-methyl-2-propanol (2.65 g, 35.8 mmol). The reaction mixture was stirred at 100 °C for 16 h under N₂. The reaction was diluted with water (50 mL) and was extracted with EtOAc (100 mL x 2). The combined organic layers were washed with brine (50 mL x 2), dried over anhydrous Na₂SO₄ and concentrated under reduced pressure to give the crude product, which was purified by flash chromatography on silica gel (EtOAc/PE = 0/1 to 1/5) to give tert-butyl N-[1-(2-bromophenyl)-5-(trifluoromethyl)-1H-pyrazol-4-yl]carbamate [**INT 1-ppp**] (400 mg, 984 µmol, 27.5% yield) as a yellow oil. m/z: [M + H]+ Calcd for C15H16BrF3N3O2 406.0; Found 406.1. ¹H NMR (400 MHz, DMSO-d6) δ = 9.04 (br s, 1H), 7.92 (br s, 1H), 7.88 - 7.85 (m, 1H), 7.65 - 7.52 (m, 3H), 1.46 (s, 9H).

### Synthesis of 1-(2-bromophenyl)-5-(trifluoromethyl)-1H-pyrazol-4-amine [INT 1-qqq]:

A mixture of tert-butyl N-[1-(2-bromophenyl)-5-(trifluoromethyl)-1H-pyrazol-4-yl]carbamate [**INT 1-ppp**] (400 mg, 1.13 mmol) in HCl/dioxane (4 mL) was stirred at 25 °C for 12 h under N₂. The reaction was quenched by adding saturated NaHCO₃(aq) (50 mL) and was extracted with EtOAc (3 x 50 mL). The combined organic layers were washed with brine (2 x 50 mL), dried over anhydrous Na₂SO₄ and concentrated under reduced pressure to give 1-(2-bromophenyl)-5-(trifluoromethyl)-1H-pyrazol-4-amine [**INT 1-qqq**] (290 mg, 947 µmol, 96.6% yield) as a yellow oil. m/z: [M + H]+ Calcd for C10H8BrF3N3 306.0; Found 305.8. ¹H NMR (400 MHz, DMSO-d6) δ = 7.81 (d, J = 7.6 Hz, 1H), 7.56 - 7.35 (m, 4H), 4.81 (s, 2H).

### Synthesis of 2-[4-amino-5-(trifluoromethyl)-1H-pyrazol-1-yl]benzonitrile [INT 1.16]:

A mixture of 1-(2-bromophenyl)-5-(trifluoromethyl)-1H-pyrazol-4-amine [**INT 1-qqq**] (240 mg, 784 µmol) and copper cyanide (351 mg, 3.92 mmol) in DMF (3 mL) was stirred at 140 °C for 2 h under N₂. The reaction was combined with another of the same and was quenched by adding NH₃.H₂O (5 mL) and H₂O (20 mL), then the mixture was extracted with EtOAc (3 x 20 mL). The combined organic layers were washed with brine (3 x 20 mL), dried over anhydrous Na₂SO₄ and concentrated under reduced pressure to give the crude product, which was purified by flash chromatography on silica gel (Petroleum ether/EtOAc = 1/0 to 1/1) to give 2-[4-amino-5-(trifluoromethyl)-1H-pyrazol-1-yl]benzonitrile [**INT 1.16**] (160 mg, 634 µmol, 81.2% yield) as a yellow oil. m/z: [M + H]+ Calcd for C11H8F3N4 253.1; Found 252.9. ¹H NMR (400 MHz, DMSO-d6) δ = 8.06 (d, J = 7.6 Hz, 1H), 7.91 - 7.84 (m, 1H), 7.77 - 7.70 (m, 1H), 7.65 (d, J = 8.0 Hz, 1H), 7.47 (s, 1H), 5.01 (s, 2H).

### Synthesis of 3-[4-amino-5-(trifluoromethyl)-1H-pyrazol-1-yl]benzonitrile hydrochloride [Intermediate 1.17]:

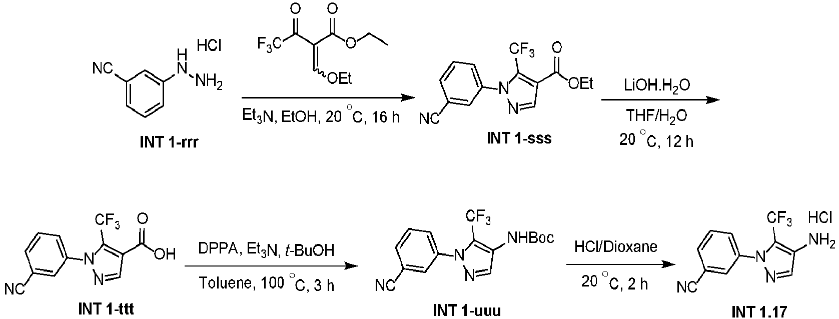

### Synthesis of ethyl 1-(3-cyanophenyl)-5-(trifluoromethyl)-1H-pyrazole-4-carboxylate [INT 1-sss]:

To a solution of ethyl 2-(ethoxymethylidene)-4,4,4-trifluoro-3-oxobutanoate (3.0 g, 12.4 mmol) in EtOH (5 mL) was added 3-hydrazinylbenzonitrile hydrochloride [**INT1-rrr**] (2.10 g, 12.4 mmol) and triethylamine (1.37 g, 13.6 mmol). The reaction was stirred at 20 °C for 16 h. The reaction mixture was concentrated under reduced pressure to give the crude product, which was purified by flash chromatography on silica gel (EtOAc/PE = 0/1 to 1/5) to give ethyl 1-(3-cyanophenyl)-5-(trifluoromethyl)-1H-pyrazole-4-carboxylate [**INT 1-sss**] (350 mg, 1.13 mmol, 9.1% yield) as a red solid. m/z: [M + H]+ Calcd for C14H11F3N302 310.1; Found 310.0. ¹H NMR (400 MHz, CDCl₃) δ = 8.17 (s, 1H), 7.87 - 7.81 (m, 1H), 7.79 (s, 1H), 7.75 - 7.62 (m, 2H), 4.41 (q, J = 7.2 Hz, 2H), 1.41 (t, J = 7.2 Hz, 3H).

### Synthesis of 1-(3-cyanophenyl)-5-(trifluoromethyl)-1H-pyrazole-4-carboxylic acid [INT 1-ttt]:

To a solution of ethyl 1-(3-cyanophenyl)-5-(trifluoromethyl)-1H-pyrazole-4-carboxylate [**INT 1-sss**] (2.35 g, 5.30 mmol) in THF (15 mL) was added lithium hydroxide monohydrate (444 mg, 10.6 mmol) in H₂O (5 mL). The reaction was stirred at 20 °C for 12 h. The reaction mixture was poured into 10 mL of water, acidified with 1 N HCl to pH=3-4 and extracted with EtOAc (20 mL x 2). The combined organic layers were washed with brine (30 mL x 2), dried over anhydrous Na₂SO₄ and concentrated under reduced pressure to give the crude product, which was purified by flash chromatography on silica gel (MeOH/DCM = 0/1 to 1/20) to give 1-(3-cyanophenyl)-5-(trifluoromethyl)-1H-pyrazole-4-carboxylic acid [**INT 1-ttt**] (1.50 g, 5.33 mmol, 100% yield) as a brown solid. ¹H NMR (400 MHz, DMSO-d6) δ = 13.41 (br s, 1H), 8.29 (s, 1H), 8.20 (t, J = 1.6 Hz, 1H), 8.12 - 8.07 (m, 1H), 7.94 (br d, J = 8.8 Hz, 1H), 7.83 - 7.78 (m, 1H).

### Synthesis of tert-butyl N-[1-(3-cyanophenyl)-5-(trifluoromethyl)-1H-pyrazol-4-yl]carbamate [INT 1-uuu]:

A solution of 1-(3-cyanophenyl)-5-(trifluoromethyl)-1H-pyrazole-4-carboxylic acid [**INT 1-ttt**] (1.45 g, 5.15 mmol), triethylamine (781 mg, 7.72 mmol) and diphenylphosphoryl azide (2.83 g, 10.3 mmol) in t-BuOH (6 mL) was stirred at 100 °C for 3 h under N₂. The reaction mixture was concentrated under reduced pressure to give the crude product, which was purified by flash chromatography on silica gel (EtOAc/PE = 0/1 to 1/5) to give tert-butyl N-[1-(3-cyanophenyl)-5-(trifluoromethyl)-1H-pyrazol-4-yl]carbamate [**INT 1-uuu**] (1 g, 2.83 mmol, 55.2% yield) as a white solid. m/z: [M + H]+ Calcd for C16H16F3N4O2 353.1; Found 353.1. ¹H NMR (400 MHz, CDCl₃) δ = 8.32 (br s, 1H), 7.79 - 7.73 (m, 2H), 7.71 (br d, J = 8.0 Hz, 1H), 7.65 - 7.59 (m, 1H), 6.55 (br s, 1H), 1.55 (s, 9H).

### Synthesis of 3-[4-amino-5-(trifluoromethyl)-1H-pyrazol-1-yl]benzonitrile hydrochloride [INT 1.17]:

A solution of tert-butyl N-[1-(3-cyanophenyl)-5-(trifluoromethyl)-1H-pyrazol-4-yl]carbamate [**INT 1-uuu**] (100 mg, 283 µmol) in HCl/Dioxane (1.5 mL) was stirred at 20 °C for 2 h. The reaction was concentrated under reduced pressure to give 3-[4-amino-5-(trifluoromethyl)-1H-pyrazol-1-yl]benzonitrile hydrochloride [**INT 1.17**] (82.0 mg, 284 µmol, 100% yield) as an off-white solid. m/z: [M + H]- Calcd for C11H8F3N4 253.1; Found 253.0. ¹H NMR (400 MHz, DMSO-d6) δ = 8.04 - 7.94 (m, 2H), 7.85 - 7.71 (m, 2H), 7.61 (s, 1H).

### Synthesis of 1-(pyrimidin-2-yl)-5-(trifluoromethyl)-1H-pyrazol-4-amine [Intermediate 1.18]:

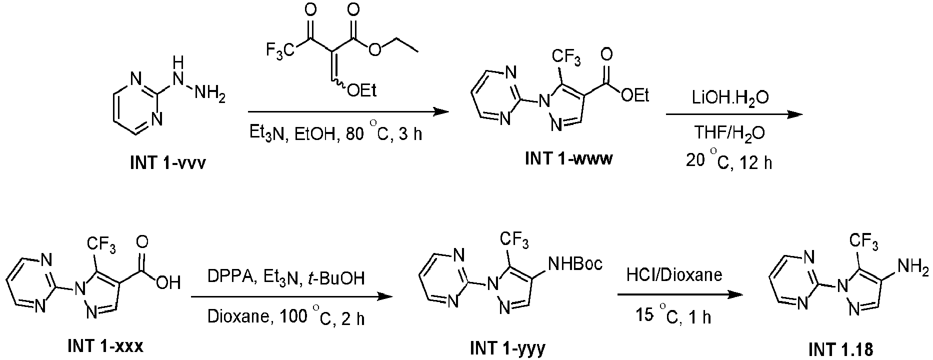

### Synthesis of ethyl 1-(pyrimidin-2-yl)-5-(trifluoromethyl)-1H-pyrazole-4- carboxylate [INT 1-www]:

To a solution of ethyl 2-(ethoxymethylidene)-4,4,4-trifluoro-3-oxobutanoate (5.45 g, 22.7 mmol) in EtOH (10 mL) at 10 °C was added 2-hydrazinylpyrimidine [**INT** 1**-vvv**] (2.5 g, 22.7 mmol) and Et₃N (2.29 g, 22.7 mmol). The mixture was stirred at 80 °C for 3 h. The reaction was concentrated under reduced pressure, quenched by adding water (100 mL) and was extracted with EtOAc (80 mL x 3). The combined organic layers were washed with brine (80 mL x 2), dried over anhydrous Na₂SO₄ and concentrated under reduced pressure to give the crude product, which was purified by flash chromatography on silica gel (PE/EtOAc= 1/0 to 5/1) to give ethyl 1-(pyrimidin-2-yl)-5-(trifluoromethyl)-1H-pyrazole-4- carboxylate [**INT 1-www**] (4.72 g, 16.5 mmol, 72.7% yield) as a yellow oil. ¹H NMR (400 MHz, DMSO) δ = 9.08 (d, J = 4.0 Hz, 2H), 8.38 (s, 1H), 7.83 (t, J = 4.0 Hz, 1H), 4.33 (q, J = 8.0 Hz, 2H), 1.31 (t, J = 8.0 Hz, 3H).

### Synthesis of 1-(pyrimidin-2-yl)-5-(trifluoromethyl)-1H-pyrazole-4-carboxylic acid [INT 1-xxx]:

To a solution of ethyl 1-(pyrimidin-2-yl)-5-(trifluoromethyl)-1H-pyrazole-4-carboxylate [**INT 1-www**] (4 g, 13.9 mmol) in THF (6 mL) and H₂O (2 mL) at 10 °C was added lithium hydroxide monohydrate (2.91 g, 69.5 mmol). The mixture was then stirred at 50 °C for 2 h. The mixture was worked up with another of the same reaction. The mixture was acidified with 1 M HCl to pH =5 and extracted with EtOAc (60 mL x 2). The combined organic layers were washed with brine (40 mL x 2), dried over anhydrous Na₂SO₄ and concentrated under reduced pressure to afford 1-(pyrimidin-2-yl)-5-(trifluoromethyl)-1H-pyrazole-4-carboxylic acid [**INT 1-xxx**] (3.80 g, 14.7 mmol) as a pale yellow solid. ¹H NMR (400 MHz, DMSO) δ = 9.07 (d, J = 4.0 Hz, 2H), 8.30 (s, 1H), 7.82 (t, J = 6.0 Hz, 1H)

### Synthesis of tert-butyl N-[1-(pyrimidin-2-yl)-5-(trifluoromethyl)-1H-pyrazol-4-yl]carbamate [INT 1-yyy]:

To a mixture of 1-(pyrimidin-2-yl)-5-(trifluoromethyl)-1H-pyrazole-4-carboxylic acid [**INT 1-xxx**] (500 mg, 1.93 mmol) in t-BuOH (2 mL) and dioxane (6 mL) was added triethylamine (781 mg, 7.72 mmol) and diphenylphosphoryl azide (1.40 g, 5.79 mmol) at 10 °C. The mixture was then stirred at 100 °C for 2 hr. Water (30 mL) was added and the mixture was extracted with EtOAc (30 mL x 2). The combined organic layers were washed with brine (30 mL x 2), dried over anhydrous Na₂SO₄, filtered and concentrated under reduced pressure to give the crude product, which was purified by flash chromatography on silica gel (Petroleum ether/EtOAc = 1/0 to 3/1) to give tert-butyl N-[1-(pyrimidin-2-yl)-5-(trifluoromethyl)-1H-pyrazol-4-yl]carbamate [**INT 1-yyy**] (340 mg, 1.03 mmol, 53.5% yield) as a white solid. ¹H NMR (400 MHz, DMSO) δ = 9.23 (s, 1H), 8.98 (d, J = 4.0 Hz, 2H), 7.99 (s, 1H), 7.65 (t, J = 4.0 Hz, 1H), 1.47 (s, 9H).

### Synthesis of 1-(pyrimidin-2-yl)-5-(trifluoromethyl)-1H-pyrazol-4-amine [INT 1.18]:

A solution of tert-butyl N-[1-(pyrimidin-2-yl)-5-(trifluoromethyl)-1H-pyrazol-4-yl]carbamate [**INT 1-yyy**] (340 mg, 1.03 mmol) in HCl/dioxane (10 mL) was stirred at 15 °C for 1 h. The mixture was concentrated under reduced pressure to give 1-(pyrimidin-2-yl)-5-(trifluoromethyl)-1H-pyrazol-4-amine [**INT 1.18**] (236 mg, 1.02 mmol, 100% yield) as a brown solid. ¹H NMR (400 MHz, DMSO) δ = 8.86 (d, J = 4.0 Hz, 2H), 7.56 (s, 1H), 7.49 (t, J = 4.0 Hz, 1H).

### Synthesis of 1-(pyrazin-2-yl)-5-(trifluoromethyl)-1H-pyrazol-4-amine hydrochloride [Intermediate 1.19]:

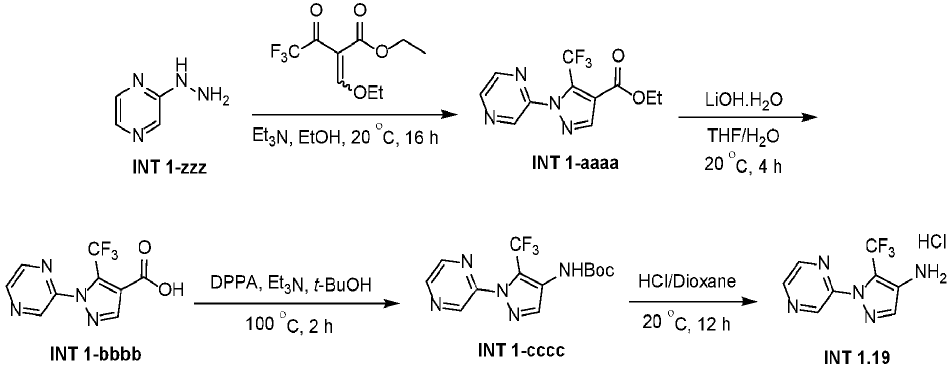

### Synthesis of ethyl 1-(pyrazin-2-yl)-5-(trifluoromethyl)-1H-pyrazole-4-carboxylate [INT 1-aaaa]:

To a solution of 2-hydrazinylpyrazine [**INT 1-zzz**] (2.0 g, 18.1 mmol) in EtOH (15 mL) was added ethyl 2-(ethoxymethylidene)-4,4,4-trifluoro-3-oxobutanoate (4.34 g, 18.1 mmol) and triethylamine (2.01 g, 19.9 mmol). The reaction was stirred at 20 °C for 16 h. The reaction mixture was concentrated under reduced pressure to give the crude product, which was purified by flash chromatography on silica gel (EtOAc/PE = 0/1 to 1/5) to give ethyl 1-(pyrazin-2-yl)-5-(trifluoromethyl)-1H-pyrazole-4-carboxylate [**INT 1-aaaa**] (3.00 g, 10.4 mmol, 57.9% yield) as an oil. m/z: [M + H]+ Calcd for C11H10F3N4O2 287.1; Found 287.1. ¹H NMR (400 MHz, CDCl₃) δ = 9.01 (d, J = 1.2 Hz, 1H), 8.73 (d, J = 2.8 Hz, 1H), 8.59 - 8.54 (m, 1H), 8.18 (s, 1H), 4.40 (q, J = 6.8 Hz, 2H), 1.40 (t, J = 7.2 Hz, 3H).

### Synthesis of 1-(pyrazin-2-yl)-5-(trifluoromethyl)-1H-pyrazole-4-carboxylic acid [INT 1-bbbb]:

To a solution of ethyl 1-(pyrazin-2-yl)-5-(trifluoromethyl)-1H-pyrazole-4-carboxylate **[INT 1-aaaa**] (2.8 g, 9.78 mmol) in THF (12 mL) was added lithium hydroxide monohydrate (818 mg, 19.5 mmol) in H₂O (4 mL). The reaction was stirred at 20 °C for 4 h. The reaction mixture was poured into 20 mL of water, acidified with 1 N HCl to pH=3-4 and extracted with EtOAc (30 mL x 2). The combined organic layers were washed with brine (30 mL x 2), dried over anhydrous Na₂SO₄ and concentrated under reduced pressure to give 1-(pyrazin-2-yl)-5-(trifluoromethyl)-1H-pyrazole-4-carboxylic acid [**INT 1-bbbb**] (2.25 g, 8.71 mmol, 89.2% yield) as a white solid. ¹H NMR (400 MHz, CDCl₃) δ = 13.57 (br s, 1H), 9.14 (s, 1H), 8.92 (d, J = 2.4 Hz, 1H), 8.75 (d, J = 1.2 Hz, 1H), 8.37 (s, 1H).

### Synthesis of tert-butyl N-[1-(pyrazin-2-yl)-5-(trifluoromethyl)-1H-pyrazol-4-yl]carbamate [INT 1-cccc]:

A solution of 1-(pyrazin-2-yl)-5-(trifluoromethyl)-1H-pyrazole-4-carboxylic acid [**INT 1-bbbb**] (2.25 g, 8.71 mmol), triethylamine (1.31 g, 13.0 mmol) and diphenylphosphoryl azide (4.78 g, 17.4 mmol) in t-BuOH (20 mL) was stirred at 100 °C for 2 h under N₂. The reaction mixture was concentrated under reduced pressure to give the crude product, which was purified by flash chromatography on silica gel (EtOAc/PE = 0/1 to 1/5) to give tert-butyl N-[1-(pyrazin-2-yl)-5-(trifluoromethyl)-1H-pyrazol-4-yl]carbamate [**INT 1-cccc**] (2.33 g, 7.07 mmol, 81.4% yield) as a white solid. m/z: [M + H]+ Calcd for C13H15F3N5O2 330.1; Found 330.0. ¹H NMR (400 MHz, CDCl₃) δ = 9.15 (d, J = 0.8 Hz, 1H), 8.56 (d, J = 2.4 Hz, 1H), 8.51 - 8.36 (m, 2H), 6.76 (br s, 1H), 1.54 (s, 9H).

### Synthesis of 1-(pyrazin-2-yl)-5-(trifluoromethyl)-1H-pyrazol-4-amine hydrochloride [INT 1.19]:

A mixture of tert-butyl N-[1-(pyrazin-2-yl)-5-(trifluoromethyl)-1H-pyrazol-4-yl]carbamate [**INT 1-cccc**] (100 mg, 303 µmol) in HCl/Dioxane (5 mL) was stirred at 20 °C for 12 h. The reaction mixture was concentrated under reduced pressure to give 1-(pyrazin-2-yl)-5-(trifluoromethyl)-1H-pyrazol-4-amine hydrochloride [INT 1.19] (81.0 mg, 304 µmol) as a brown solid. m/z: [M + H]+ Calcd for C8H7F3N5 230.1; Found 229.9. ¹H NMR (400 MHz, DMSO-d6) δ = 9.04 (d, J = 1.6 Hz, 1H), 8.59 (d, J = 2.4 Hz, 1H), 8.53 - 8.49 (m, 1H), 7.64 (s, 1H).

### Synthesis of 1-(pyrimidin-4-yl)-5-(trifluoromethyl)-1H-pyrazol-4-amine hydrochloride [Intermediate 1.20]:

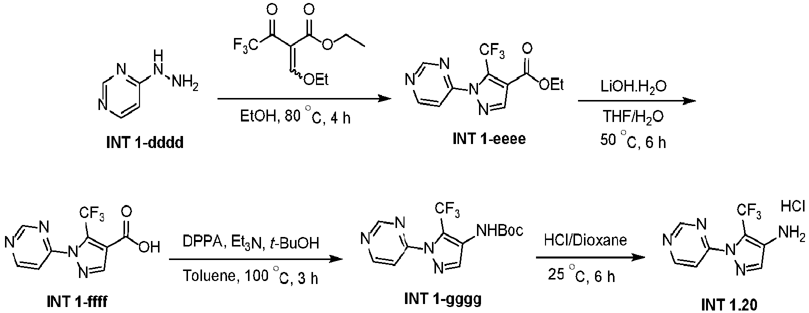

### Synthesis of ethyl 1-(pyrimidin-4-yl)-5-(trifluoromethyl)-1H-pyrazole-4-carboxylate [INT 1-eeee]:

A mixture of ethyl 2-(ethoxymethylidene)-4,4,4-trifluoro-3-oxobutanoate (5 g, 20.8 mmol) and 4-hydrazinylpyrimidine [**INT 1-dddd**] (2.74 g, 24.9 mmol) in EtOH (50 mL) was stirred at 80 °C for 4 hr. The mixture was concentrated under reduced pressure to afford the crude product, which was purified by flash chromatography on silica gel (0-15% EtOAc in Petroleum ether) to give ethyl 1-(pyrimidin-4-yl)-5-(trifluoromethyl)-1H-pyrazole-4-carboxylate [**INT 1-eeee**] (3.90 g, 13.6 mmol, 65.5% yield) as a white solid. m/z: [M + H]+ Calcd for C11H10F3N4O2 287.1; Found 287.0. ¹H NMR (400 MHz, CDCl₃) δ = 9.21 (s, 1H), 8.96 (dd, J = 2.0, 5.6 Hz, 1H), 8.14 (s, 1H), 7.80 (td, J = 1.2, 5.6 Hz, 1H), 4.40 (tq, J = 1.6, 7.2 Hz, 2H), 1.44 - 1.36 (m, 3H).

### Synthesis of 1-(pyrimidin-4-yl)-5-(trifluoromethyl)-1H-pyrazole-4-carboxylic acid [INT 1-ffff]:

To a mixture of ethyl 1-(pyrimidin-4-yl)-5-(trifluoromethyl)-1H-pyrazole-4-carboxylate [**INT 1-eeee**] (2.9 g, 10.1 mmol) in THF (15 mL) and H₂O (5 mL) was added LiOH.H₂O (1.27 g, 30.3 mmol) and the mixture was stirred at 50 °C for 6 hr. The mixture was concentrated under reduced pressure to afford the crude product. H₂O (200 mL) and EtOAc (200 mL) were added and the precipitate was collected by filtration. The aqueous phase was washed with EtOAc (200 mL x 2), acidified with 1 M HCl to pH = 3 and extracted with EtOAc (200 mL x 2). The combined organic layers were dried over Na₂SO₄ and filtered. The filtrate was concentrated to give a yellow solid, which was combined with the precipitate to give 1-(pyrimidin-4-yl)-5-(trifluoromethyl)-1H-pyrazole-4-carboxylic acid [**INT 1-ffff**] (2.10 g, 8.13 mmol, 80.7% yield) as a white solid. ¹H NMR (400 MHz, CDCl₃) δ = 9.23 (s, 1H), 8.99 (d, J = 5.6 Hz, 1H), 8.22 (s, 1H), 7.81 (d, J=4.4 Hz, 1H).

### Synthesis of tert-butyl N-[1-(pyrimidin-4-yl)-5-(trifluoromethyl)-1H-pyrazol-4-yl]carbamate [INT 1-gggg]:

A mixture of 1-(pyrimidin-4-yl)-5-(trifluoromethyl)-1H-pyrazole-4-carboxylic acid [**INT 1-ffff**] (1.6 g, 6.19 mmol), 2-methylpropan-2-ol (6.87 g, 92.8 mmol), diphenylphosphoryl azide (2.25 g, 9.28 mmol) and triethylamine (1.87 g, 18.5 mmol) in toluene (20 mL) was stirred at 100 °C for 3 hr. NaHCO₃ (100 mL) was added and the mixture was extracted with EtOAc (100 mL x 2). The combined organic layers were washed with brine (100 mL x 2), dried over anhydrous Na₂SO₄, filtered and concentrated under reduced pressure to give the crude product, which was purified by flash chromatography on silica gel (0-10% EtOAc in Petroleum ether) to give tert-butyl N-[1-(pyrimidin-4-yl)-5-(trifluoromethyl)-1H-pyrazol-4-yl]carbamate [**INT 1-gggg**] (1.80 g, 5.46 mmol, 88.6% yield) as a white solid. m/z: [M + H]+ Calcd for C13H15F3N5O2 330.1; Found: 330.1. ¹H NMR (400 MHz, CDCl₃) δ = 9.06 (s, 1H), 8.79 (d, J = 5.6 Hz, 1H), 8.49 (br s, 1H), 7.88 (d, J = 5.6 Hz, 1H), 6.82 (br s, 1H), 1.54 (s, 9H).

### Synthesis of 1-(pyrimidin-4-yl)-5-(trifluoromethyl)-1H-pyrazol-4-amine hydrochloride [INT 1.20]:

A mixture of tert-butyl N-[1-(pyrimidin-4-yl)-5-(trifluoromethyl)-1H-pyrazol-4-yl]carbamate [**INT 1-gggg**] (150 mg, 455 µmol) in 4 M HCl/dioxane (10 mL, 40.0 mmol) was stirred at 25 °C for 6 hr. The mixture was concentrated under reduced pressure to afford 1-(pyrimidin-4-yl)-5-(trifluoromethyl)-1H-pyrazol-4-amine hydrochloride [**INT 1.20**] (120 mg, 451 µmol) as a brown solid. m/z: [M + H]+ Calcd for C8H7F3N5 230.1; Found 230.0.

### Synthesis of 1-(pyridazin-3-yl)-5-(trifluoromethyl)-1H-pyrazol-4-amine hydrochloride [Intermediate 1.21]:

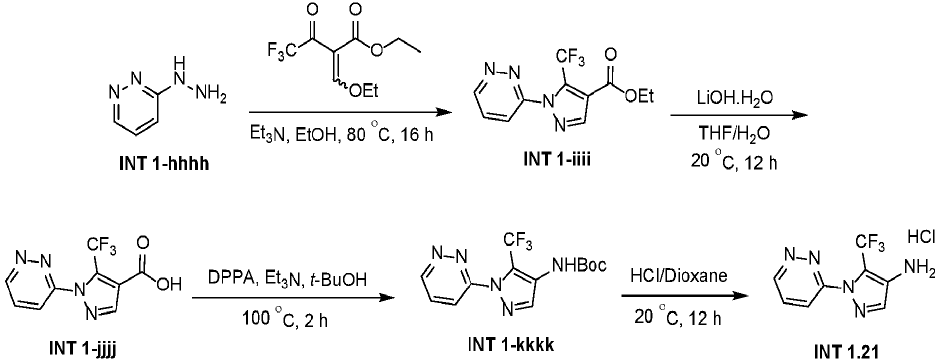

### Synthesis of ethyl 1-(pyridazin-3-yl)-5-(trifluoromethyl)-1H-pyrazole-4-carboxylate[INT 1-iiii]:

A solution consisting of 3-hydrazinylpyridazine [**INT 1-hhhh**] (800 mg, 7.26 mmol), Et₃N (1.46 g, 14.5 mmol), ethyl 2-(ethoxymethylidene)-4,4,4-trifluoro-3-oxobutanoate (1.74 g, 7.26 mmol) and EtOH (50 mL) was stirred at 80 °C for 16 h. The mixture was concentrated under reduced pressure to afford a brown oil, which was purified by flash column chromatography (petroleum ether:ethyl acetate=0:1 to 85:15) to give ethyl 1-(pyridazin-3-yl)-5-(trifluoromethyl)-1H-pyrazole-4-carboxylate [**INT 1-iiii**] (1 g, 3.49 mmol, 48.3% yield) as a white solid. m/z: [M + H]+ Calcd for C11H10F3N4O2 287.1; Found 286.9. ¹H NMR (400 MHz, CDCl₃) δ = 9.36 (dd, J = 1.2, 4.8 Hz, 1H), 8.23 (s, 1H), 7.95 (dd, J = 1.6, 8.8 Hz, 1H), 7.80 (dd, J = 4.8, 8.8 Hz, 1H), 4.46 - 4.41 (m, 2H), 1.45 - 1.41 (m, 3H).

### Synthesis of 1-(pyridazin-3-yl)-5-(trifluoromethyl)-1H-pyrazole-4-carboxylic acid [INT 1-jjjj]:

A mixture of ethyl 1-(pyridazin-3-yl)-5-(trifluoromethyl)-1H-pyrazole-4-carboxylate [**INT 1-iiii**] (800 mg, 2.79 mmol) in THF (9 mL) and H₂O (3 mL) was stirred at 20 °C for 12 h. The reaction was concentrated under reduced pressure to give a residue, which was purified by Prep-HPLC (column: Boston Prime C18 150*30 mm*5 µm, table: 2- 42% B (A = water ( 0.05% HCl), B = acetonitrile), flow rate: 30 mL/min,UV Detector 220 nm) to afford 1-(pyridazin-3-yl)-5-(trifluoromethyl)-1H-pyrazole-4-carboxylic acid [**INT 1-jjjj**] (240 mg, 929 µmol, 33.3% yield) as a yellow solid. m/z: [M + H]+ Calcd for C9H6F3N4O2 259.0; Found 258.9. ¹H NMR (400 MHz, DMSO-d6) δ = 9.46 (dd, J = 1.2, 4.8 Hz, 1H), 8.41 (s, 1H), 8.21 (dd, J = 1.2, 8.8 Hz, 1H), 8.10 (dd, J = 4.8, 8.8 Hz, 1H).

### Synthesis of tert-butyl N-[1-(pyridazin-3-yl)-5-(trifluoromethyl)-1H-pyrazol-4-yl]carbamate [INT 1-kkkk]:

To a solution of 1-(pyridazin-3-yl)-5-(trifluoromethyl)-1H-pyrazole-4-carboxylic acid [**INT 1-jjjj**] (200 mg, 774 µmol) in t-BuOH (3 mL) was added diphenylphosphoryl azide (319 mg, 1.16 mmol) and Et₃N (234 mg, 2.32 mmol). The reaction mixture was stirred at 100 °C for 2 h under N₂, after which it was quenched by adding water (15 mL) and was extracted with EtOAc (2 x 10 mL). The combined organic layers were washed with brine (2 x 20 mL), dried over anhydrous Na₂SO₄ and concentrated under reduced pressure to give the crude product, which was purified by flash chromatography on silica gel (EtOAc/Petroleum ether = 0/1 to 1/4) to give tert-butyl N-[1-(pyridazin-3-yl)-5-(trifluoromethyl)-1H-pyrazol-4-yl]carbamate [**INT 1-kkkk**] (142 mg, 431 µmol, 55.9% yield) as a colorless oil. m/z: [M + H]+ Calcd for C13H15F3N5O2 330.1; Found 330.0. ¹H NMR (400 MHz, CDCl₃) δ = 9.20 (d, J = 4.4 Hz, 1H), 8.51 (br s, 1H), 8.07 (br d, J = 8.8 Hz, 1H), 7.68 (dd, J = 4.8, 8.8 Hz, 1H), 6.79 (br s, 1H), 1.57 (s, 9H).

### Synthesis of 1-(pyridazin-3-yl)-5-(trifluoromethyl)-1H-pyrazol-4-amine hydrochloride [INT 1.21]:

Tert-butyl N-[1-(pyridazin-3-yl)-5-(trifluoromethyl)-1H-pyrazol-4-yl]carbamate [**INT 1-kkkk**] (142 mg, 431 µmol) in HCl (4 M in dioxane, 5 mL, 20.0 mmol) was stirred at 20 °C for 12 h. The mixture was concentrated under reduced pressure to give 1-(pyridazin-3-yl)-5-(trifluoromethyl)-1H-pyrazol-4-amine hydrochloride [**INT 1.21**] (114 mg, 429 µmol) as a yellow solid. m/z: [M + H]+ Calcd for C8H7F3N5 230.1; Found 230.0.

### Synthesis of 1-(pyridazin-4-yl)-5-(trifluoromethyl)-1H-pyrazol-4-amine hydrochloride [Intermediate 1.22]:

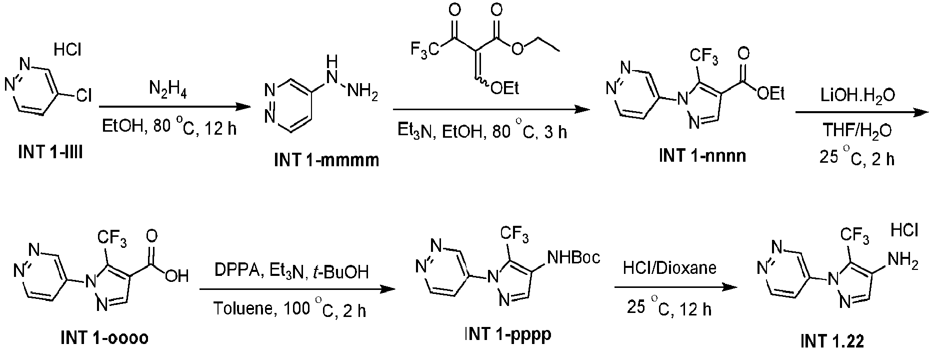

### Synthesis of 4-hydrazinylpyridazine [INT 1-mmmm]:

To a mixture of 4-chloropyridazine hydrochloride [**INT 1-llll**] (900 mg, 5.96 mmol) in EtOH (10 mL) was added NH₂NH₂•H₂O (3.50 g, 59.6 mmol). The reaction mixture was stirred at 80 °C for 12 hours. The reaction mixture was concentrated to give 4-hydrazinylpyridazine [**INT 1-mmmm**] (656 mg, 5.95 mmol) as a brown oil. ¹H NMR (400 MHz, DMSO-d6) δ = 8.59 (d, J = 2.4 Hz, 1H), 8.47 (dd, J = 0.8, 6.0 Hz, 1H), 6.73 (dd, J = 2.8, 6.0 Hz, 1H).

### Synthesis of ethyl 1-(pyridazin-4-yl)-5-(trifluoromethyl)-1H-pyrazole-4-carboxylate[INT 1-nnnn]:

To a mixture of 4-hydrazinylpyridazine [**INT 1-mmmm**] (656 mg, 5.95 mmol) and ethyl 2-(ethoxymethylidene)-4,4,4-trifluoro-3-oxobutanoate (7.13 g, 29.7 mmol) in EtOH (10 mL) was added triethylamine (6.02 g, 59.5 mmol). The reaction mixture was stirred at 80 °C for 3 hours. The reaction mixture was concentrated, diluted with water (100 mL) and extracted with EtOAc (50 mL x 3). The combined organic layers were washed with brine (100 mL), dried over anhydrous sodium sulfate and filtered. The filtrate was concentrated to give the crude product, which was purified by flash chromatography on silica gel (20-25% EtOAc in petroleum ether) to give ethyl 1-(pyridazin-4-yl)-5-(trifluoromethyl)-1H-pyrazole-4-carboxylate [**INT 1-nnnn**] (1 g, 3.49 mmol, 58.8% yield) as brown oil. m/z: [M + H]+ Calcd for C11H10F3N4O2 287.1; Found 287.0. ¹H NMR (400 MHz, CDCl₃) δ = 9.55 - 9.42 (m, 2H), 8.25 (s, 1H), 7.71 (br s, 1H), 4.42 (q, J = 7.2 Hz, 2H), 1.41 (t, J = 7.2 Hz, 3H).

### Synthesis of 1-(pyridazin-4-yl)-5-(trifluoromethyl)-1H-pyrazole-4-carboxylic acid [INT 1-oooo]:

To a mixture of ethyl 1-(pyridazin-4-yl)-5-(trifluoromethyl)-1H-pyrazole-4-carboxylate [**INT 1-nnnn**] (950 mg, 3.31 mmol) in THF (10 mL) and H₂O (3 mL) was added lithium hydroxide monohydrate (416 mg, 9.93 mmol). The reaction mixture was stirred at 25 °C for 2 hours. The reaction mixture was concentrated, diluted with water (50 mL) and washed with EtOAc (50 mL x 2). The aqueous phase was acidified with 1 M HCl to pH = 3 and extracted with EtOAc (50 mL x 3). The combined organic layers were dried over anhydrous sodium sulfate and filtered. The filtrate was concentrated to give 1-(pyridazin-4-yl)-5-(trifluoromethyl)-1H-pyrazole-4-carboxylic acid [**INT 1-oooo**] (530 mg, 2.05 mmol, 62.0% yield) as a yellow solid. m/z: [M + H]+ Calcd for C9H6F3N4O2 259.0; Found 258.9. ¹H NMR (400 MHz, DMSO-d6) δ = 9.58 (d, J = 2.4 Hz, 1H), 9.56 (dd, J = 1.2, 5.6 Hz, 1H), 8.42 (s, 1H), 8.06 (dd, J = 2.4, 5.6 Hz, 1H).

### Synthesis of tert-butyl N-[1-(pyridazin-4-yl)-5-(trifluoromethyl)-1H-pyrazol-4-yl]carbamate [INT 1-pppp]:

To a mixture of 1-(pyridazin-4-yl)-5-(trifluoromethyl)-1H-pyrazole-4-carboxylic acid [**INT 1-oooo**] (500 mg, 1.93 mmol) and 2-methylpropan-2-ol (2.14 g, 28.9 mmol) in toluene (5 mL) were added diphenylphosphoryl azide (795 mg, 2.89 mmol) and triethylamine (585 mg, 5.79 mmol). The reaction mixture was stirred at 100 °C under nitrogen for 2 hours. The mixture was concentrated under reduced pressure to give the crude product, which was purified by flash chromatography on silica gel (EtOAc/PE = 0/1 to 1/3) to give tert-butyl N-[1-(pyridazin-4-yl)-5-(trifluoromethyl)-1H-pyrazol-4-yl]carbamate [**INT 1-pppp**] (250 mg, 759 µmol, 39.3% yield) as a white solid. m/z: [M + H]+ Calcd for C13H15F3N5O2 330.1; Found 330.1. ¹H NMR (400 MHz, CDCl₃) δ = 9.51 (d, J = 2.4 Hz, 1H), 9.32 (dd, J = 0.8, 5.6 Hz, 1H), 8.48 (br s, 1H), 7.66 - 7.59 (m, 1H), 6.69 (br s, 1H), 1.58 - 1.51 (m, 9H).

### Synthesis of 1-(pyridazin-4-yl)-5-(trifluoromethyl)-1H-pyrazol-4-amine hydrochloride [INT 1.22]:

A solution of tert-butyl N-[1-(pyridazin-4-yl)-5-(trifluoromethyl)-1H-pyrazol-4-yl]carbamate [**INT 1-pppp**] (230 mg, 698 µmol) in HCl/Dioxane (5 mL) was stirred at 25 °C for 12 h. The reaction mixture was concentrated under reduced pressure to give 1-(pyridazin-4-yl)-5-(trifluoromethyl)-1H-pyrazol-4-amine hydrochloride [**INT 1.22**] (185 mg, 696 µmol) as a white solid. ¹H NMR (400 MHz, DMSO-d6) δ = 9.47 (d, J = 2.4 Hz, 1H), 9.35 (dd, J = 0.8, 6.0 Hz, 1H), 7.76 - 7.71 (m, 1H), 7.68 (s, 1H).

### Synthesis of 5-(1-methoxyethyl)-1-(pyridin-3-yl)-1H-pyrazol-4-amine [Intermediate 1.23]:

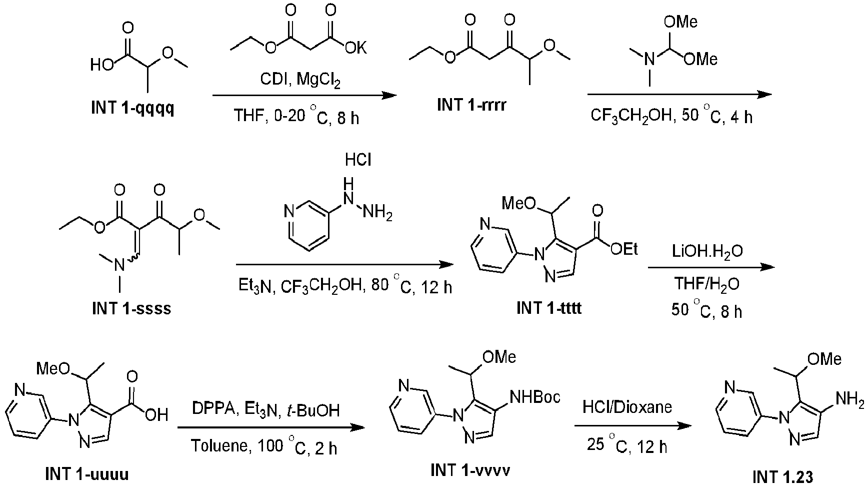

### Synthesis of ethyl 4-methoxy-3-oxopentanoate [INT 1-rrrr]:

To a solution of 2-methoxypropanoic acid [**INT 1-qqqq**] (5 g, 48.0 mmol) in THF (50 mL) was added CDI (9.32 g, 57.5 mmol) at 0 °C, and the mixture was stirred at 20 °C for 2 hours. Magnesium chloride (4.57 g, 48.0 mmol) and ethyl potassium malonate (12.2 g, 72.0 mmol) were added to the reaction solution, and the mixture was stirred at 20 °C for 6 hours. 1 N HCl (200 mL) was then added and the mixture was extracted with EtOAc (2 x 200 mL). The combined organic layers were washed with brine (2 x 200 mL), dried over anhydrous Na₂SO₄ and concentrated under reduced pressure to give the crude product ethyl 4-methoxy-3-oxopentanoate [**INT 1-rrrr**] (6.15 g, 35.3 mmol, 73.5% yield) as a yellow oil. ¹H NMR (400 MHz, CDCl₃) δ = 4.24 - 4.10 (m, 2H), 3.79 (q, J = 6.8 Hz, 1H), 3.60 - 3.47 (m, 2H), 3.40 - 3.29 (m, 3H), 1.37 - 1.17 (m, 6H).

### Synthesis of ethyl 2-((dimethylamino)methylene)-4-methoxy-3-oxopentanoate [INT 1-ssss]:

A mixture of ethyl 4-methoxy-3-oxopentanoate [**INT 1-rrrr**] (6.15 g, 35.3 mmol) and (dimethoxymethyl)dimethylamine (8.41 g, 70.6 mmol) in 2,2,2-trifluoroethanol (60 mL) was stirred at 50 °C for 4 hrs. The mixture was concentrated under reduced pressure to afford the crude product ethyl 2-((dimethylamino)methylene)-4-methoxy-3-oxopentanoate [**INT 1-ssss**].

### Synthesis of ethyl 5-(1-methoxyethyl)-1-(pyridin-3-yl)-1H-pyrazole-4-carboxylate [INT 1-tttt]:

To a mixture of ethyl 2-((dimethylamino)methylene)-4-methoxy-3-oxopentanoate [**INT 1-ssss**] (5.38 g, 23.4 mmol) and triethylamine (11.8 g, 117 mmol) in 2,2,2-trifluoroethanol (50 mL) was added 3-hydrazinylpyridine hydrochloride (11.9 g, 81.9 mmol). The reaction mixture was stirred at 80 °C for 12 hours. The mixture was concentrated under reduced pressure to afford the crude product. Brine (200 mL) was added and the mixture was extracted with EtOAc (200 mL x 2). The combined organic layers were washed with brine (200 mL x 2), dried over anhydrous Na₂SO₄, filtered and concentrated under reduced pressure to give the crude product, which was purified by flash chromatography on silica gel (0-40% EtOAc in Petroleum ether) to give ethyl 5-(1-methoxyethyl)-1-(pyridin-3-yl)-1H-pyrazole-4-carboxylate [**INT 1-tttt**] (4.40 g, 15.9 mmol, 68.3% yield) as a yellow oil m/z: [M + H]+ Calcd for C14H18N3O3 276.1; Found 276.1. ¹H NMR (400 MHz, CDCl₃) δ = 8.80 (d, J = 2.4 Hz, 1H), 8.70 (d, J = 4.4 Hz, 1H), 8.08 (s, 1H), 7.91 - 7.87 (m, 1H), 7.43 (dd, J = 4.8, 8.4 Hz, 1H), 5.44 (q, J = 6.8 Hz, 1H), 4.39 - 4.31 (m, 2H), 3.27 - 3.21 (m, 3H), 1.39 (t, J = 7.2 Hz, 3H), 1.34 (d, J = 6.8 Hz, 3H).

### Synthesis of 5-(1-methoxyethyl)-1-(pyridin-3-yl)-1H-pyrazole-4-carboxylic acid [INT 1-uuuu]:

To a mixture of ethyl 5-(1-methoxyethyl)-1-(pyridin-3-yl)-1H-pyrazole-4-carboxylate [**INT 1-tttt**] (4.4 g, 15.9 mmol) in THF (30 mL) and H₂O (10 mL) was added LiOH·H₂O (2.00 g, 47.7 mmol) and the mixture was stirred at 50 °C for 8 hr. The mixture was concentrated under reduced pressure to afford the crude product. H₂O (200 mL) and EtOAc (200 mL) was added and the precipitate was collected by filtration. The aqueous phase was washed with EtOAc (100 mL x 2), acidified with 1 M HCl to pH = 3 and extracted with EtOAc (100 mL x 2). The combined organic layers were dried over Na₂SO₄ and filtered. The filtrate was concentrated and combined with the precipitate to give 5-(1-methoxyethyl)-1-(pyridin-3-yl)-1H-pyrazole-4-carboxylic acid [**INT 1-uuuu**] (2.80 g, 11.3 mmol, 71.2% yield) as a white solid. m/z: [M + H]+ Calcd for C12H14N3O3 248.1; Found 248.1. ¹H NMR (400 MHz, DMSO-d6) δ = 8.80 - 8.66 (m, 2H), 8.06 (s, 1H), 8.02 - 7.96 (m, 1H), 7.59 (dd, J = 4.8, 8.0 Hz, 1H), 5.30 (q, J = 6.8 Hz, 1H), 3.08 (s, 3H), 1.28 (d, J = 6.8 Hz, 3H).

### Synthesis of tert-butyl N-[5-(1-methoxyethyl)-1-(pyridin-3-yl)-1H-pyrazol-4-yl]carbamate [INT 1-vvvv]:

A mixture of 5-(1-methoxyethyl)-1-(pyridin-3-yl)-1H-pyrazole-4-carboxylic acid [**INT 1-uuuu**] (2.8 g, 11.3 mmol), 2-methylpropan-2-ol (12.5 g, 169 mmol), diphenylphosphoryl azide (4.11 g, 16.9 mmol) and triethylamine (3.43 g, 33.9 mmol) in toluene (20 mL) was stirred at 100 °C for 2 hr. Saturated aq. NaHCO₃ solution (200 mL) was added and the mixture was extracted with EtOAc (100 mL x 2). The combined organic layers were washed with brine (100 mL x 2), dried over anhydrous Na₂SO₄, filtered and concentrated under reduced pressure to give the crude product, which was purified by flash chromatography on silica gel (0-10% EtOAc in Petroleum ether) to give tert-butyl N-[5-(1-methoxyethyl)-1-(pyridin-3-yl)-1H-pyrazol-4-yl]carbamate [**INT 1-vvvv**] (2.50 g, 7.85 mmol, 69.6% yield) as a white solid. m/z: [M + H]+ Calcd for C16H23N4O3 319.2; Found 319.2. ¹H NMR (400 MHz, CDCl₃) δ = 8.66 (br s, 2H), 8.20 (br s, 1H), 7.82 - 7.74 (m, 1H), 7.45 (dd, J = 5.2, 8.0 Hz, 1H), 6.88 (br s, 1H), 4.48 (q, J = 6.8 Hz, 1H), 3.28 (s, 3H), 1.54 (s, 9H), 1.50 (d, J = 6.8 Hz, 3H).

### Synthesis of 5-(1-methoxyethyl)-1-(pyridin-3-yl)-1H-pyrazol-4-amine [INT 1.23]:

A mixture of tert-butyl N-[5-(1-methoxyethyl)-1-(pyridin-3-yl)-1H-pyrazol-4-yl]carbamate [**INT 1-vvvv**] (500 mg, 1.57 mmol) in 4 M HCl/dioxane (10 mL, 40.0 mmol) was stirred at 25 °C for 12 hr. The mixture was concentrated under reduced pressure to afford a yellow solid, which was purified by Prep-HPLC (column: Boston Prime C18 150*30 mm*5 µm, table: 0-30% B (A = water (ammonia hydroxide v/v)-ACN), B = acetonitrile), flowrate: 20 mL/min, UV Detector 220 nm) to afford 5-(1-methoxyethyl)-1-(pyridin-3-yl)-1H-pyrazol-4-amine [**INT 1.23**] (185 mg, 847 µmol, 53.9% yield) as a white solid. [M + H]+ Calcd for C11H15N4O 219.1; Found:219.0. ¹H NMR (400 MHz, CDCl₃) δ = 8.68 (d, J = 2.3 Hz, 1H), 8.63 (d, J = 4.8 Hz, 1H), 7.78 (dd, J = 1.5, 8.0 Hz, 1H), 7.47 - 7.42 (m, 1H), 7.42 - 7.39 (m, 1H), 4.49 (q, J = 6.5 Hz, 1H), 3.27 (d, J = 1.3 Hz, 3H), 3.13 - 2.80 (m, 2H), 1.53 (d, J = 6.5 Hz, 3H).

### Synthesis of 1-[4-amino-1-(pyridin-3-yl)-1H-pyrazol-5-yl]ethan-1-ol [Intermediate 1.24]:

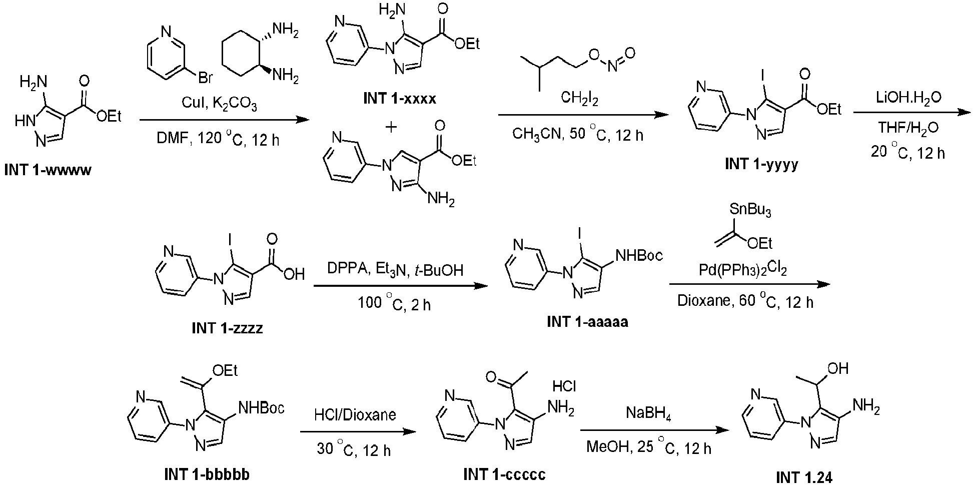

### Synthesis of ethyl 5-amino-1-(pyridin-3-yl)-1H-pyrazole-4-carboxylate [INT 1-xxxx]:

A suspension of ethyl 5-amino-1H-pyrazole-4-carboxylate [**INT 1-wwww**] (18.5 g, 119 mmol), 3-bromopyridine (15.6 g, 99.1 mmol), CuI (942 mg, 4.95 mmol), K₂CO₃ (27.3 g, 198 mmol) and trans-1,2-diaminocyclohexane (1.13 g, 9.91 mmol) in DMF (100 mL) was stirred at 120 °C for 12 h under N₂. The reaction mixture was filtered. The filtrate was diluted with water (30 mL) and was extracted with EtOAc (2 x 20 mL). The combined organic layers were washed with brine (2 x 50 mL), dried over anhydrous Na₂SO₄ and concentrated under reduced pressure to give the crude product, which was purified by flash chromatography on silica gel (EtOAc/Petroleum ether = 1/4 to 1/1) to give a mixture of ethyl 5-amino-1-(pyridin-3-yl)-1H-pyrazole-4-carboxylate [**INT 1-xxxx**] (10.1 g, 43.4 mmol, 43.9% yield) and ethyl 3-amino-1-(pyridin-3-yl)-1H-pyrazole-4-carboxylate (5.20 g, 22.3 mmol, 22.6% yield) as a yellow oil. m/z: [M + H]+ Calcd for C11H13N4O2 233.1; Found 233.2.

### Synthesis of ethyl 5-iodo-1-(pyridin-3-yl)-1H-pyrazole-4-carboxylate [INT 1-yyyy]:

Ethyl 5-amino-1-(pyridin-3-yl)-1H-pyrazole-4-carboxylate [**INT 1-xxxx**] (6.78 g, 29.2 mmol) was suspended in acetonitrile (100 mL) at ambient temperature. Diiodomethane (46.8 g, 175 mmol) was added followed by isopentyl nitrite (10.2 g, 87.6 mmol). The reaction was heated at 50 °C for 12 hours, after which it was combined with another of the same type and partitioned between water (500 mL) and ethyl acetate (300 mL). The aqueous layer was extracted with ethyl acetate (300 mL x 2). The combined organic layers were dried over anhydrous sodium sulfate. After removal of solvents under reduced pressure, the crude material was purified using silica gel column chromatography (10 - 25% ethyl acetate in Petroleum ether). Compound ethyl 5-iodo-1-(pyridin-3-yl)-1H-pyrazole-4-carboxylate [**INT 1-yyyy**] (2.25 g, 6.55 mmol) was obtained as a yellow solid. m/z: [M + H]+ Calcd for C11H11IN3O2 344.0; Found 343.9. ¹H NMR (400 MHz, CDCl₃) δ = 8.76 (d, J = 2.4 Hz, 1H), 8.68 (d, J = 4.8 Hz, 1H), 8.14 (s, 1H), 7.86 - 7.78 (m, 1H), 7.43 (dd, J = 4.8, 8.0 Hz, 1H), 4.35 - 4.29 (m, 2H), 1.33 (t, J = 7.2 Hz, 3H).

### Synthesis of 5-iodo-1-(pyridin-3-yl)-1H-pyrazole-4-carboxylic acid [INT 1-zzzz]:

A solution of ethyl 5-iodo-1-(pyridin-3-yl)-1H-pyrazole-4-carboxylate [**INT 1-yyyy**] (156 mg, 454 µmol) and lithium hydroxide monohydrate (28.5 mg, 681 µmol) in a mixture of THF (3 mL) and H₂O (1 mL) was stirred at 20 °C for 12 h. The mixture was adjusted to pH=4 by 1 N HCl. The resulting suspension was filtered and washed with water (5 mL x 2). The filter cake was dried to obtained 5-iodo-1-(pyridin-3-yl)-1H-pyrazole-4-carboxylic acid [**INT 1-zzzz**] (56.0 mg, 177 µmol, 39.1% yield) as a yellow solid. m/z: [M + H]+ Calcd for C9H7IN3O2 316.0; Found 315.9. ¹H NMR (400 MHz, DMSO-d6) δ = 12.81 (br s, 1H), 8.83 - 8.70 (m, 2H), 8.20 (s, 1H), 8.11 - 8.01 (m, 1H), 7.66 (dd, J = 4.8, 8.0 Hz, 1H).

### Synthesis of tert-butyl N-[5-iodo-1-(pyridin-3-yl)-1H-pyrazol-4-yl]carbamate [INT 1-aaaaal:

To a solution of 5-iodo-1-(pyridin-3-yl)-1H-pyrazole-4-carboxylic acid [**INT 1-zzzz**] (2.2 g, 6.98 mmol) in t-BuOH (20 mL) were added diphenylphosphoryl azide (2.86 g, 10.4 mmol) and Et₃N (2.11 g, 20.9 mmol). The reaction mixture was stirred at 100 °C for 2 h under N₂. The reaction was quenched by adding water (100 mL) and was extracted with EtOAc (2 x 30 mL). The combined organic layers were washed with brine (2 x 50 mL), dried over anhydrous Na₂SO₄ and concentrated under reduced pressure to give the crude product, which was purified by flash chromatography on silica gel (EtOAc/Petroleum ether = 0/1 to 1/1) to give tert-butyl N-[5-iodo-1-(pyridin-3-yl)-1H-pyrazol-4-yl]carbamate [**INT 1-aaaaa**] (1.80 g, 4.66 mmol, 66.9% yield) as a white solid. m/z: [M + H]+ Calcd for C13H16IN4O2 387.0; Found 387.0. ¹H NMR (400 MHz, CDCl₃) δ = 8.89 (d, J = 1.6 Hz, 1H), 8.68 (br d, J = 4.4 Hz, 1H), 8.20 (br s, 1H), 7.96 (br d, J = 8.0 Hz, 1H), 7.49 (dd, J = 4.8, 8.0 Hz, 1H), 6.15 (br s, 1H), 1.56 (s, 9H).

### Synthesis of tert-butyl N-[5-(1-ethoxyethenyl)-1-(pyridin-3-yl)-1H-pyrazol-4-yl]carbamate [INT 1-bbbbb]:

A mixture of tert-butyl N-[5-iodo-1-(pyridin-3-yl)-1H-pyrazol-4-yl]carbamate [**INT 1-aaaaa**] (500 mg, 1.29 mmol), Pd(PPh₃)₂Cl₂ (90.5 mg, 129 µmol), tributyl(1-ethoxyethenyl)stannane (1 g, 2.76 mmol) and dioxane (10 mL) under N₂ was stirred at 60 °C for 12 h. The mixture was quenched with sat. KF solution (100 mL) and stirred at 25 °C for 1 h. The aqueous layer was extracted with ethyl acetate (20 mL x 3). The combined organic layers were dried over sodium sulfate, filtered and concentrated. The residue was purified by flash column chromatography on silica gel (30% ethyl acetate in PE) to give tert-butyl N-[5-(1-ethoxyethenyl)-1-(pyridin-3-yl)-1H-pyrazol-4-yl]carbamate [**INT 1-bbbbb**] (400 mg, 1.21 mmol, 93.8% yield) as a brown gum. m/z: [M + H]+ Calcd for C17H23N4O3 331.2; Found 331.1. ¹H NMR (400 MHz, DMSO-d6) δ = 8.64 (d, J = 2.4 Hz, 1H), 8.57 (dd, J = 1.3, 4.7 Hz, 1H), 8.49 (br s, 1H), 7.88 - 7.84 (m, 1H), 7.81 (br s, 1H), 7.54 (dd, J = 4.8, 8.2 Hz, 1H), 4.66 (d, J = 2.6 Hz, 1H), 4.62 (d, J = 2.6 Hz, 1H), 3.68 (q, J = 7.1 Hz, 2H), 1.45 (s, 9H), 0.86 (t, J = 7.0 Hz, 3H).

### Synthesis of 1-[4-amino-1-(pyridin-3-yl)-1H-pyrazol-5-yl]ethan-1-one hydrochloride [INT 1-ccccc ]:

Tert-butyl N-[5-(1-ethoxyethenyl)-1-(pyridin-3-yl)-1H-pyrazol-4-yl]carbamate [**INT 1-bbbbb**] (400 mg, 1.21 mmol) was dissolved in 1 M HCl (10 mL) and the mixture was stirred at 25 °C for 3 h. The mixture was adjusted to pH 8 with aqueous sat. NaHCO₃ and extracted with ethyl acetate (20 mL x 2). The combined organic layers were dried over Na₂SO₄, filtered and concentrated. The residue was diluted with 4 N HCl in dioxane (5 mL) and stirred at 30 °C for 12 h. The mixture was concentrated to give 1-[4-amino-1-(pyridin-3-yl)-1H-pyrazol-5-yl]ethan-1-one hydrochloride [**INT 1-ccccc**] (280 mg, 1.17 mmol, 97.2% yield) as a brown solid. m/z: [M + H]+ Calcd for C10H11N4O 203.1; Found 203.0.

### Synthesis of 1-[4-amino-1-(pyridin-3-yl)-1H-pyrazol-5-yl]ethan-1-ol [INT 1.24]:

A solution of 1-[4-amino-1-(pyridin-3-yl)-1H-pyrazol-5-yl]ethan-1-one [free base of **INT 1-ccccc**] (180 mg, 0.8901 mmol) in methanol (5 mL) was cooled to 0 °C. Sodium borohydride (101 mg, 2.67 mmol) was added and the reaction mixture was stirred at 0-25 °C for 12 h. MeOH was removed and the residue was diluted with water (10 mL). The reaction mixture's pH was adjusted to 8-9 with aqueous sat. NaHCO₃ and the resulting mixture was extracted with ethyl acetate (15 mL x 3). The combined organic layers were dried over Na₂SO₄, filtered and concentrated to give crude 1-[4-amino-1-(pyridin-3-yl)-1H-pyrazol-5-yl]ethan-1-ol **[INT 1.24]** (180 mg, 0.8813 mmol, 99.4% yield) as brown gum. m/z: [M + H]+ Calcd for C10H13N4O 205.1; Found 204.8. ¹H NMR (400 MHz, DMSO-d6) δ = 8.78 (d, J = 2.4 Hz, 1H), 8.61 (dd, J = 1.3, 4.7 Hz, 1H), 8.01 - 7.97 (m, 1H), 7.58 (dd, J = 4.7, 7.8 Hz, 1H), 7.30 (s, 1H), 5.47 (br s, 1H), 4.94 - 4.80 (m, 1H), 4.45 - 4.02 (m, 2H), 1.41 (d, J = 6.8 Hz, 3H).

### Synthesis of (S)-3-(4-amino-5-(1-methoxyethyl)-1H-pyrazol-1-yl)benzonitrile [Intermediate 1.25]:

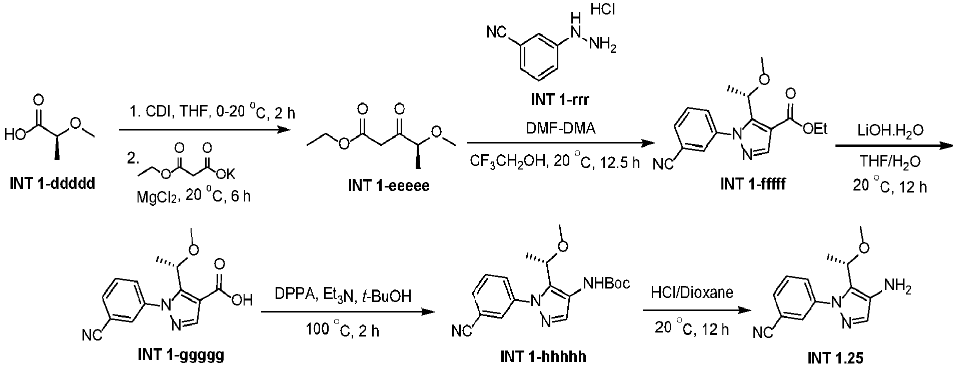

### Synthesis of ethyl (4S)-4-methoxy-3-oxopentanoate [INT 1-eeeee]:

To a solution of (2S)-2-methoxypropanoic acid [**INT 1-ddddd]** (5 g, 48.0 mmol) in THF (50 mL) was added 1,1'-carbonyldiimidazole (9.32 g, 57.5 mmol) at 0 °C, and the mixture was stirred at 20 °C for 2 hours. Magnesium chloride (4.57 g, 48.0 mmol) and 1-ethyl 3-potassium propanedioate (12.2 g, 72.0 mmol) were added to the reaction solution, and the mixture was stirred at 20 °C for 6 hours. The reaction was quenched by adding aqueous 1 N HCl (200 mL) and was extracted with EtOAc (3 x 200 mL). The combined organic layers were washed with brine (2 x 100 mL), dried over anhydrous Na₂SO₄ and concentrated under reduced pressure to give ethyl (4S)-4-methoxy-3-oxopentanoate [**INT 1-eeeee**] (8.00 g, 45.9 mmol, 95.6% yield) as a yellow oil. ¹H NMR (400 MHz, CDCl₃) δ = 4.24 - 4.06 (m, 2H), 3.86 - 3.70 (m, 1H), 3.62 - 3.47 (m, 2H), 3.39 - 3.32 (m, 3H), 1.32 - 1.23 (m, 6H).

### Synthesis of ethyl 1-(3-cyanophenyl)-5-[(1S)-1-methoxyethyl]-1H-pyrazole-4-carboxylate [INT 1- fffff]:

To a mixture of ethyl (4S)-4-methoxy-3-oxopentanoate [**INT 1-eeeee**] (1.5 g, 8.61 mmol) in 2,2,2-trifluoroethanol (15 mL) was added N,N-dimethylformamide dimethyl acetal (1.22 g, 10.3 mmol). The mixture was stirred at 20 °C for 30 min. 3-hydrazinylbenzonitrile hydrochloride [**INT 1-rrr**] (1.74 g, 10.3 mmol) was added and the mixture was stirred at 20 °C for 12 h. The mixture was concentrated to give the crude product, which was purified by flash chromatography on silica gel (EtOAc/PE = 0/1 to 1/3) to give ethyl 1-(3-cyanophenyl)-5-[(1S)-1-methoxyethyl]-1H-pyrazole-4-carboxylate [**INT 1-fffff**] (1.95 g, 6.51 mmol, 75.8% yield) as an orange solid. m/z: [M + H]+ Calcd for C16H18N3O3 300.1; Found 300.0. ¹H NMR (400 MHz, CDCl₃) δ = 8.06 (s, 1H), 7.90 (t, J = 1.6 Hz, 1H). 7.87 - 7.82 (m, 1H), 7.74 (td, J = 1.6, 8.0 Hz, 1H), 7.62 - 7.57 (m, 1H), 5.47 (q, J = 6.8 Hz, 1H), 4.40 - 4.29 (m, 2H), 3.27 (s, 3H), 1.39 (t, J = 7.2 Hz, 3H), 1.33 - 1.30 (m, 3H).

### Synthesis of 1-(3-cyanophenyl)-5-[(1S)-1-methoxyethyl]-1H-pyrazole-4-carboxylic acid [INT 1-ggggg]:

To a solution of ethyl 1-(3-cyanophenyl)-5-[(1S)-1-methoxyethyl]-1H-pyrazole-4-carboxylate [**INT 1-fffff**] (1.8 g, 6.01 mmol) in THF (15 mL) was added lithium hydroxide monohydrate (302 mg, 7.21 mmol) in H₂O (5 mL). The reaction was stirred at 20 °C for 12 h. The reaction mixture was poured into 10 mL of water, acidified with 1 N HCl to pH=5-6 and extracted with EtOAc (30 mL x 2). The combined organic layers were washed with brine (30 mL x 2), dried over anhydrous Na₂SO₄ and concentrated under reduced pressure to give the crude product, which was purified by flash chromatography on silica gel (MeOH/DCM = 0/1 to 1/20) to give 1-(3-cyanophenyl)-5-[(1S)-1-methoxyethyl]-1H-pyrazole-4-carboxylic acid [**INT 1-ggggg**] (1.20 g, 4.42 mmol, 73.6% yield) as a yellow solid. ¹H NMR (400 MHz, DMSO-d6) δ = 12.76 (br s, 1H), 8.07 - 8.03 (m, 2H), 8.00 (d, J = 8.0 Hz, 1H), 7.90 - 7.86 (m, 1H), 7.77 - 7.71 (m, 1H), 5.29 (q, J = 6.8 Hz, 1H), 3.08 (s, 3H), 1.28 (d, J = 6.8 Hz, 3H).

### Synthesis of tert-butyl N-[1-(3-cyanophenyl)-5-[(1S)-1-methoxyethyl]-1H-pyrazol-4-yl]carbamate [INT 1-hhhhh]:

A solution of 1-(3-cyanophenyl)-5-[(1S)-1-methoxyethyl]-1H-pyrazole-4-carboxylic acid [**INT 1-ggggg**] (1.2 g, 4.42 mmol), triethylamine (670 mg, 6.63 mmol) and diphenylphosphoryl azide (2.43 g, 8.84 mmol) in t-BuOH (15 mL) was stirred at 100 °C for 2 h under N₂. The reaction mixture was concentrated under reduced pressure to give the crude product, which was purified by flash chromatography on silica gel (EtOAc/PE = 0/1 to 1/3) to give tert-butyl N-[1-(3-cyanophenyl)-5-[(1S)-1-methoxyethyl]-1H-pyrazol-4-yl]carbamate **[INT 1-hhhhh]** (1 g, 2.92 mmol, 66.2% yield) as a yellow oil. m/z: [M + H]+ Calcd for C18H23N4O3 343.2; Found 343.2. ¹H NMR (400 MHz, CDCl₃) δ = 8.18 (br s, 1H), 7.73 - 7.67 (m, 2H), 7.67 - 7.57 (m, 2H), 6.86 (br s, 1H), 4.49 (q, J = 6.8 Hz, 1H), 3.28 (s, 3H), 1.54 (s, 9H), 1.50 (d, J = 6.8 Hz, 3H).

### Synthesis of (S)-3-(4-amino-5-(1-methoxyethyl)-1H-pyrazol-1-yl)benzonitrile [INT 1.25]:

A mixture of tert-butyl N-[1-(3-cyanophenyl)-5-[(1S)-1-methoxyethyl]-1H-pyrazol-4-yl]carbamate [**INT 1-hhhhh**] (500 mg, 1.46 mmol) in 4 M HCl/Dioxane (8 mL) was stirred at 20 °C for 12 h. The reaction mixture was poured into 10 mL of water, basified with NH₃•H₂O to pH=9-10 and extracted with EtOAc (30 mL x 2). The combined organic layers were washed with brine (30 mL x 2), dried over anhydrous Na₂SO₄ and concentrated under reduced pressure to give the crude product, which was purified by flash chromatography on silica gel (EtOAc/PE = 0/1 to 1/3) to give (S)-3-(4-amino-5-(1-methoxyethyl)-1H-pyrazol-1-yl)benzonitrile [**INT 1.25**] (100 mg, 412 µmol, 28.3% yield) as a yellow solid. m/z: [M - OCH3]+ Calcd for C12H11N4 211.1; Found 210.9. ¹H NMR (400 MHz, CDCl₃) δ = 7.76 - 7.73 (m, 1H), 7.69 - 7.64 (m, 2H), 7.62 - 7.55 (m, 1H), 7.39 (s, 1H), 4.50 (q, J = 6.8 Hz, 1H), 3.28 (s, 3H), 1.53 (d, J = 6.4 Hz, 3H).

### Synthesis of 5-[(1S)-1-methoxyethyl]-1-phenyl-1H-pyrazol-4-amine hydrochloride [Intermediate 1.26]:

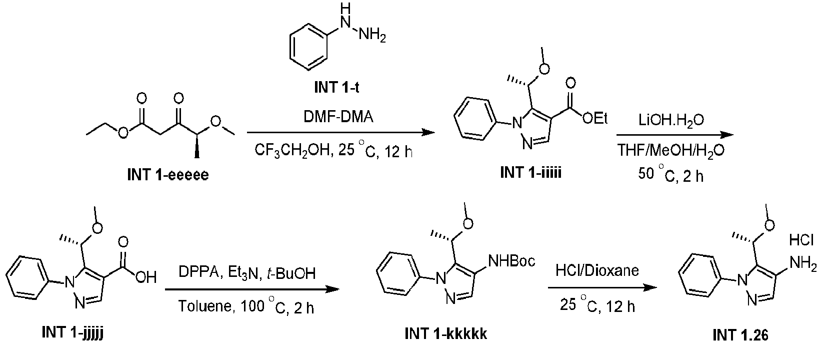

### Synthesis of ethyl 5-(1S)-1-methoxyethyl]-1-phenyl-1H-pyrazole-4-carboxylate [INT 1-iiiii]:

To a mixture of phenylhydrazine [**INT 1-t**] (1.5 g, 13.8 mmol) and N,N-dimethylformamide dimethyl acetal (1.96 g, 16.5 mmol) in 2,2,2-trifluoroethanol (20 mL) was added ethyl (4S)-4-methoxy-3-oxopentanoate [**INT 1-eeeee**] (2.87 g, 16.5 mmol) in 2,2,2-trifluoroethanol (10 mL). The reaction mixture was stirred at 25 °C for 12 hours. The reaction was quenched with water (100 mL) and extracted with EtOAc (100 mL x 3). The combined organic layers were dried over anhydrous sodium sulfate and filtered. The filtrate was concentrated to give the crude product, which was purified by flash chromatography on silica gel (0-15% EtOAc in petroleum ether) to give ethyl 5-[(1S)-1-methoxyethyl]-1-phenyl-1H-pyrazole-4-carboxylate [**INT 1-iiiii**] (2.00 g, 7.29 mmol, 52.9% yield) as a brown oil. m/z: [M + H]+ Calcd for C15H19N2O3 275.1; Found 275.1. ¹H NMR (400 MHz, CDCl₃) δ = 8.05 (s, 1H), 7.53 - 7.43 (m, 5H), 5.34 (q, J = 6.8 Hz, 1H), 4.40 - 4.29 (m, 2H), 3.25 (s, 3H), 1.39 (t, J = 7.2 Hz, 3H), 1.35 (d, J = 6.8 Hz, 3H).

### Synthesis of 5-[(1S)-1-methoxyethyll-1-phenyl-1H-pyrazole-4-carboxylic acid [INT 1-jjjjj]:

To a mixture of ethyl 5-[(1S)-1-methoxyethyl]-1-phenyl-1H-pyrazole-4-carboxylate [**INT 1-iiiii**] (1.8 g, 6.56 mmol) in THF (5 mL), MeOH (5 mL) and H₂O (5 mL) was added lithium hydroxide monohydrate (822 mg, 19.6 mmol). The reaction mixture was stirred at 50 °C for 2 hours, after which it was concentrated, diluted with water (50 mL) and washed with EtOAc (100 mL x 2). The aqueous phase was acidified with aqueous 1 M HCl to pH = 3 and extracted with EtOAc (100 mL x 2). The combined organic layers were dried over anhydrous sodium sulfate and filtered. The filtrate was concentrated to give 5-[(1S)-1-methoxyethyl]-1-phenyl-1H-pyrazole-4-carboxylic acid **[INT 1-jjjjj]** (950 mg, 3.85 mmol, 59.0% yield) as an orange solid. [M + H]+ Calcd for C13H15N2O3 247.1; Found: 247.2. ¹H NMR (400 MHz, CDCl₃) δ = 8.18 (d, J = 1.2 Hz, 1H), 7.51 (br d, J = 2.0 Hz, 3H), 7.48 - 7.42 (m, 2H), 5.13 - 5.00 (m, 1H), 3.32 (d, J = 3.6 Hz, 3H), 1.46 (t, J = 5.6 Hz, 3H).

### Synthesis of tert-butyl N-{5-[(1S)-1-methoxyethyl]-1-phenyl-1H-pyrazol-4-yl}carbamate [INT 1-kkkkk]:

To a mixture of 5-[(1S)-1-methoxyethyl]-1-phenyl-1H-pyrazole-4-carboxylic acid **[INT 1-jjjjj]** (850 mg, 3.45 mmol), 2-methylpropan-2-ol (3.83 g, 51.7 mmol) and triethylamine (1.04 g, 10.3 mmol) in toluene (10 mL) was added diphenylphosphoryl azide (1.42 g, 5.17 mmol). The reaction mixture was stirred at 100 °C under nitrogen for 2 hours. The reaction mixture was diluted with EtOAc (100 mL x 3) and washed with aqueous saturated NaHCO₃ (100 mL) and brine (100 mL). The organic layer was dried over anhydrous sodium sulfate and filtered. The filtrate was concentrated to give the crude product, which was purified by flash chromatography on silica gel (0-13% EtOAc in petroleum ether) to give tert-butyl N-{5-[(1S)-1-methoxyethyl]-1-phenyl-1H-pyrazol-4-yl}carbamate **[INT 1-kkkkk]** (770 mg, 2.42 mmol, 70.6% yield) as a yellow solid. m/z: [M + H]+ Calcd for C17H24N3O3 318.2; Found 318.1. ¹H NMR (400 MHz, CDCl₃) δ = 8.15 (br s, 1H), 7.52 - 7.33 (m, 5H), 6.90 (br s, 1H), 4.48 (q, J = 6.4 Hz, 1H), 3.27 (s, 3H), 1.54 (br s, 9H), 1.48 (d, J = 6. 4 Hz, 3H).

### Synthesis of 5-[(1S)-1-methoxyethyl]-1-phenyl-1H-pyrazol-4-amine hydrochloride [INT 1.26]:

A mixture of tert-butyl N-{5-[(1S)-1-methoxyethyl]-1-phenyl-1H-pyrazol-4-yl}carbamate **[INT 1-kkkkk]** (150 mg, 472 µmol) in 4 M HCl/dioxane (10 mL, 40.0 mmol) was stirred at 25 °C for 12 hr. The mixture was concentrated under reduced pressure to afford 5-[(1S)-1-methoxyethyl]-1-phenyl-1H-pyrazol-4-amine hydrochloride **[INT 1.26]** (119 mg, 469 µmol) as a brown oil. m/z: [M + H]+ Calcd for C12H16N30 218.1; Found 217.9.

### Synthesis of 5-[(1S)-1-methoxyethyl]-1-(pyridin-2-yl)-1H-pyrazol-4-amine hydrochloride [Intermediate 1.27]:

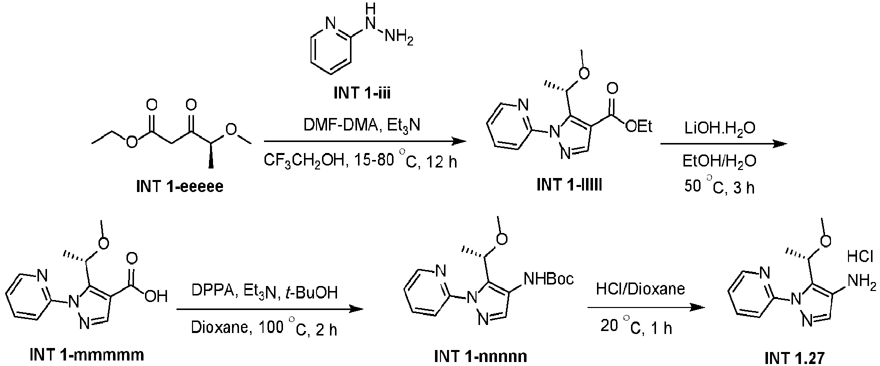

### Synthesis of ethyl 5-[(1S)-1-methoxyethyl]-1-(pyridin-2-yl)-1H-pyrazole-4-carboxylate [INT 1-lllll]:

To a mixture of ethyl (4S)-4-methoxy-3-oxopentanoate **[INT 1-eeeee]** (3 g, 17.2 mmol) and triethylamine (2.08 g, 20.6 mmol) in 2,2,2-trifluoroethanol (50 mL) was added N,N-dimethylformamide dimethyl acetal (10.2 g, 85.9 mmol) and 2-hydrazinylpyridine **[INT 1-iii]** (2.24 g, 20.6 mmol) at 15 °C. The reaction mixture was stirred at 80 °C for 12 hours. The mixture was concentrated, and the resulting residue was quenched with water (60 mL) and extracted with EtOAc (50 mL x 3). The combined organic layers were dried over anhydrous sodium sulfate and filtered. The filtrate was concentrated to give the crude product, which was purified by flash chromatography on silica gel (PE/EtOAc= 1/0 to 5/1) to give ethyl 5-[(1S)-1-methoxyethyl]-1-(pyridin-2-yl)-1H-pyrazole-4-carboxylate **[INT 1-lllll]** (1.30 g, 4.72 mmol, 27.4% yield) as a yellow oil. ¹H NMR (400 MHz, DMSO) δ = 8.59 - 8.53 (m, 1H), 8.10 - 8.01 (m, 2H), 7.67 - 7.61 (m, 1H), 7.58 - 7.52 (m, 1H), 5.19 (q, J = 8.0 Hz, 1H), 4.26 (q, J = 8.0 Hz, 2H), 2.91 (s, 3H), 1.61 (d, J = 8.0 Hz, 3H), 1.30 (t, J = 8.0 Hz, 3H).

### Synthesis of 5-[(1S)-1-methoxyethyl]-1-(pyridin-2-yl)-1H-pyrazole-4-carboxylic acid [INT 1-mmmmm]:

A solution of ethyl 5-[(1S)-1-methoxyethyl]-1-(pyridin-2-yl)-1H-pyrazole-4-carboxylate **[INT 1-lllll**] (1.3 g, 4.72 mmol) and lithium hydroxide monohydrate (990 mg, 23.6 mmol) in EtOH (10 mL) and H₂O (3 mL) was stirred at 50 °C for 3 h. The reaction was acidified with aqueous 1 M HCl to pH = 4 and extracted with EtOAc (25 mL x 2). The combined organic layers were concentrated under reduced pressure to give to 5-[(1S)-1-methoxyethyl]-1-(pyridin-2-yl)-1H-pyrazole-4-carboxylic acid **[INT 1-mmmmm]** (946 mg, 3.82 mmol, 81.5% yield) as a pale yellow solid. ¹H NMR (400 MHz, DMSO) δ = 8.59 - 8.50 (m, 1H), 8.10 - 7.96 (m, 2H), 7.65 - 7.58 (m, 1H), 7.57 - 7.51 (m, 1H), 5.22 (q, J = 8.0 Hz, 1H), 2.90 (s, 3H), 1.59 (d, J = 8.0 Hz, 3H).

### Synthesis of tert-butyl N-{5-[(1S)-1-methoxyethyl]-1-(pyridin-2-yl)-1H-pyrazol-4-yl}carbamate [INT 1-nnnnn]:

To a mixture of 5-[(1S)-1-methoxyethyl]-1-(pyridin-2-yl)-1H-pyrazole-4-carboxylic acid **[INT 1-mmmmm]** (946 mg, 3.82 mmol) in t-BuOH (3 mL) and dioxane (9 mL) was added triethylamine (1.92 g, 19.0 mmol) and diphenylphosphoryl azide (2.77 g, 11.4 mmol) at 10 °C. The mixture was then stirred at 100 °C for 2 hr. Water (30 mL) was added and the mixture was extracted with EtOAc (30 mL x 2). The combined organic layers were washed with brine (30 mL x 2), dried over anhydrous Na₂SO₄, filtered and concentrated under reduced pressure to give the crude product, which was purified by flash chromatography on silica gel (Petroleum ether/EtOAc = 1/0 to 17/3) to give tert-butyl N-{5-[(1S)-1-methoxyethyl]-1-(pyridin-2-yl)-1H-pyrazol-4-yl}carbamate **[INT 1-nnnnn]** (265 mg, 832 µmol, 21.9% yield) as a white solid. m/z: [M + H]+ Calcd for C16H23N4O3 319.2; Found 319.3. ¹H NMR (400 MHz, DMSO) δ = 8.49 - 8.45 (m, 1H), 7.99 - 7.81 (m, 4H), 7.40 - 7.35 (m, 1H), 5.35 (q, J = 8.0 Hz, 1H), 3.15 (s, 3H), 1.52 (d, J = 8.0 Hz, 3H), 1.46 (s, 9H).

### Synthesis of 5-[(1S)-1-methoxyethyl]-1-(pyridin-2-yl)-1H-pyrazol-4-amine hydrochloride [INT 1.27]:

A solution of tert-butyl N-{5-[(1S)-1-methoxyethyl]-1-(pyridin-2-yl)-1H-pyrazol-4-yl}carbamate **[INT 1-nnnnn]** (265 mg, 832 µmol) in HCl/dioxane (10 mL) was stirred at 20 °C for 1 h. The mixture was concentrated under reduced pressure to give 5-[(1S)-1-methoxyethyl]-1-(pyridin-2-yl)-1H-pyrazol-4-amine hydrochloride **[INT 1.27]** (211 mg, 828 µmol) as a black solid. ¹H NMR (400 MHz, CDCl₃) δ = 8.41 - 8.32 (m, 1H), 7.86 (d, J = 8.0 Hz, 1H), 7.80 - 7.71 (m, 1H), 7.35 (s, 1H), 7.15 - 7.08 (m, 1H), 5.62 (q, J = 8.0 Hz, 1H), 3.35 (s, 3H), 1.62 (s, 3H).

### Synthesis of 5-[(1S)-1-methoxyethyl]-1-(pyridin-4-yl)-1H-pyrazol-4-amine [Intermediate 1.28]:

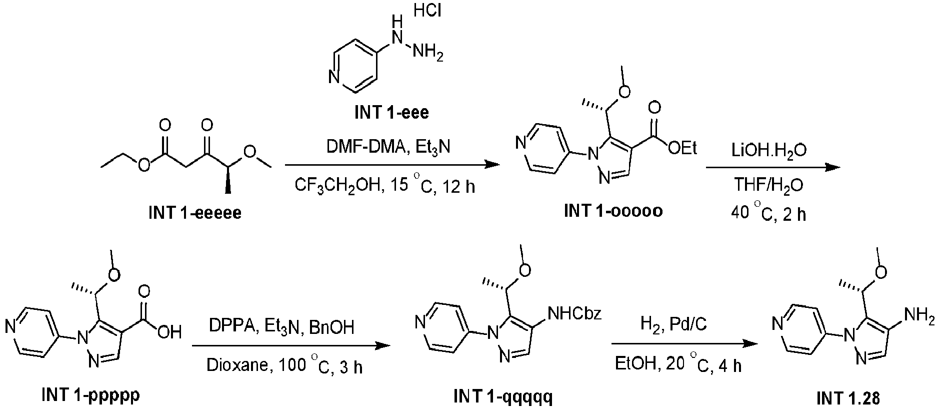

### Synthesis of ethyl 5-[(1S)-1-methoxyethyl]-1-(pyridin-4-yl)-1H-pyrazole-4-carboxylate [INT 1-ooooo]:

To a mixture of ethyl (4S)-4-methoxy-3-oxopentanoate **[INT 1-eeeee]** (1.7 g, 9.75 mmol) and N,N-dimethylformamide dimethyl acetal (1.39 g, 11.7 mmol) in 2,2,2-trifluoroethanol (30 mL) were added 4-hydrazinylpyridine hydrochloride **[INT 1-eee]** (1.70 g, 11.7 mmol) and triethylamine (1.18 g, 11.7 mmol). The reaction mixture was stirred at 15 °C for 12 hrs. The mixture was concentrated to give the crude product. The mixture was worked up with another of the same type. The residue was quenched with water (30 mL) and extracted with EtOAc (30 mL x 3). The combined organic layers were dried over anhydrous sodium sulfate and filtered. The filtrate was concentrated to give the crude product, which was purified by flash chromatography on silica gel (PE/EtOAc= 1/0 to 5/1) to give ethyl 5-[(1S)-1-methoxyethyl]-1-(pyridin-4-yl)-1H-pyrazole-4-carboxylate **[INT 1-ooooo]** (1.80 g, 6.53 mmol, 67.1% yield) as a yellow oil. ¹H NMR (400 MHz, DMSO) δ = 8.80 - 8.71 (m, 2H), 8.14 (s, 1H), 7.67 - 7.64 (m, 2H), 5.29 (q, J = 8.0 Hz, 1H), 4.28 (q, J = 8.0 Hz, 2H), 3.10 (s, 3H), 1.36 (d, J = 8.0 Hz, 3H), 1.31 (t, J = 8.0 Hz, 3H).

### Synthesis of 5-[(1S)-1-methoxyethyl]-1-(pyridin-4-yl)-1H-pyrazole-4-carboxylic acid [INT 1-ppppp]:

To a solution of ethyl 5-[(1S)-1-methoxyethyl]-1-(pyridin-4-yl)-1H-pyrazole-4-carboxylate **[INT 1-ooooo]** (1.76 g, 6.39 mmol) in THF (20 mL) and H₂O (4 mL) at 10 °C was added lithium hydroxide monohydrate (1.33 g, 31.9 mmol). The mixture was stirred at 40 °C for 2 h. The mixture was acidified with aqueous 1 M HCl to pH = 5 and extracted with EtOAc (60 mL x 2). The combined organic layers were washed with brine (30 mL x 2), dried over anhydrous Na₂SO₄ and concentrated under reduced pressure to afford 5-[(1S)-1-methoxyethyl]-1-(pyridin-4-yl)-1H-pyrazole-4-carboxylic acid **[INT 1-ppppp]** (1.57 g, 6.34 mmol) as a pale yellow solid. ¹H NMR (400 MHz, DMSO) δ = 8.66 (d, J = 4.0 Hz, 2H), 7.89 (s, 1H), 7.74 (d, J = 4.0 Hz, 2H), 6.04 (q, J = 4.0 Hz, 1H), 3.19 (s, 3H), 1.21 (d, J = 8.0 Hz, 3H).

### Synthesis of benzyl N-{5-[(1S)-1-methoxyethyl]-1-(pyridin-4-yl)-1H-pyrazol-4-yl}carbamate [INT 1-qqqqq]:

To a mixture of 5-[(1S)-1-methoxyethyl]-1-(pyridin-4-yl)-1H-pyrazole-4-carboxylic acid **[INT 1-ppppp]** (1.25 g, 5.05 mmol) in BnOH (3 mL) and dioxane (12 mL) was added diphenylphosphoryl azide (3.67 g, 15.1 mmol) and triethylamine (2.55 g, 25.2 mmol) at 20 °C. The mixture was then stirred at 100 °C for 3 h under N₂. The reaction was quenched by adding water (40 mL) and was extracted with EtOAc (30 mL x 2). The combined organic layers were washed with brine (40 mL x 2), dried over anhydrous Na₂SO₄ and concentrated under reduced pressure to give the crude product, which was purified by flash chromatography on silica gel (PE/EtOAc= 1/0 to 5/1) to give benzyl N-{5-[(1S)-1-methoxyethyl]-1-(pyridin-4-yl)-1H-pyrazol-4-yl}carbamate **[INT 1-qqqqq]** (390 mg, 1.10 mmol, 22.0% yield) as a pale yellow oil. m/z: [M + H]+ Calcd for C19H21N4O3 353.2; Found 353.2.

### Synthesis of 5-[(1S)-1-methoxyethyl]-1-(pyridin-4-yl)-1H-pyrazol-4-amine [INT 1.28]:

To a mixture of 10% Pd/C (40 mg) in EtOH (40 mL) at 20 °C was added benzyl N-{5-[(1S)-1-methoxyethyl]-1-(pyridin-4-yl)-1H-pyrazol-4-yl}carbamate **[INT 1-qqqqq]** (390 mg, 1.10 mmol). The mixture was then stirred for 4 h under H₂ (103.421 kPa, 15 psi). The mixture was filtered and the filtrate was concentrated under reduced pressure to give 5-[(1S)-1-methoxyethyl]-1-(pyridin-4-yl)-1H-pyrazol-4-amine **[INT 1.28]** (200 mg, 916 µmol, 83.3% yield) as a white solid. ¹H NMR (400 MHz, DMSO) δ = 8.60 (d, J = 8.0 Hz, 2H), 7.60 (d, J = 8.0 Hz, 2H), 7.36 (s, 1H), 4.71 (q, J = 8.0 Hz, 1H), 4.24 (s, 2H), 3.18 (s, 3H), 1.37 (d, J = 8.0 Hz, 3H).

### Synthesis of 5-[(1S)-1-methoxyethyl]-1-(pyridin-3-yl)-1H-pyrazol-4-amine hydrochloride [Intermediate 1.29]:

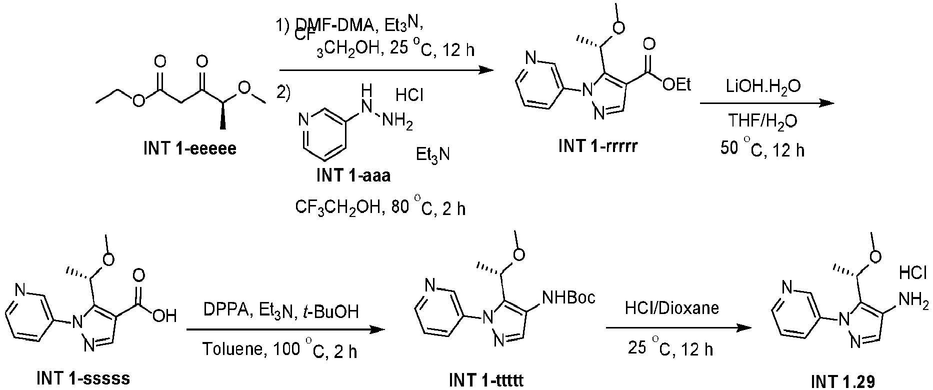

### Synthesis of ethyl 5-[(1S)-1-methoxyethyl]-1-(pyridin-3-yl)-1H-pyrazole-4-carboxylate [INT 1-rrrrr]:

A mixture of ethyl (4S)-4-methoxy-3-oxopentanoate **[INT 1-eeeee]** (3 g, 17.2 mmol) and N,N-dimethylformamide dimethyl acetal (3.07 g, 25.8 mmol) in 2,2,2-trifluoroethanol (20 mL) was stirred at 25 °C for 12 hours. The mixture was concentrated under reduced pressure to afford the crude ethyl (2Z,4S)-2-[(dimethylamino)methylidene]-4-methoxy-3-oxopentanoate, which was used directly in the next step.

To a mixture of ethyl (2Z,4S)-2-[(dimethylamino)methylidene]-4-methoxy-3-oxopentanoate (3.94 g, 17.1 mmol) and triethylamine (8.65 g, 85.5 mmol) in 2,2,2-trifluoroethanol (20 mL) was added 3-hydrazinylpyridine hydrochloride **[INT 1-aaa]** (7.46 g, 51.3 mmol). The reaction mixture was stirred at 80 °C for 2 hours. The mixture was concentrated under reduced pressure to afford the crude product. Brine (200 mL) was added and the mixture was extracted with EtOAc (200 mL x 2). The combined organic layers were washed with brine (200 mL x 2), dried over anhydrous Na₂SO₄, filtered and concentrated under reduced pressure to give the crude product, which was purified by flash chromatography on silica gel (0-40% EtOAc in Petroleum ether) to give ethyl 5-[(1S)-1-methoxyethyl]-1-(pyridin-3-yl)-1H-pyrazole-4-carboxylate **[INT 1-rrrrr]** (2.20 g, 7.99 mmol, 46.8% yield) as a yellow oil. m/z: [M + H]+ Calcd for C14H18N3O3 276.1; Found 276.1. ¹H NMR (400 MHz, CDCl₃) δ = 8.80 (br s, 1H), 8.70 (br d, J = 3.6 Hz, 1H), 8.11 - 8.05 (m, 1H), 7.94 - 7.86 (m, 1H), 7.44 (dd, J = 4.8, 8.0 Hz, 1H), 5.49 - 5.39 (m, 1H), 4.42 - 4.29 (m, 2H), 3.30 - 3.20 (m, 3H), 1.42 - 1.36 (m, 3H), 1.36 - 1.31 (m, 3H).

### Synthesis of 5-[(1S)-1-methoxyethyl]-1-(pyridin-3-yl)-1H-pyrazole-4-carboxylic acid [INT 1-sssss]:

To a mixture of ethyl 5-[(1S)-1-methoxyethyl]-1-(pyridin-3-yl)-1H-pyrazole-4-carboxylate **[INT 1-rrrrr]** (2.2 g, 7.99 mmol) in THF (15 mL) and H₂O (5 mL) was added lithium hydroxide monohydrate (1 g, 23.9 mmol). The reaction mixture was stirred at 50 °C for 12 hours, after which it was concentrated, diluted with water (100 mL) and washed with EtOAc (200 mL x 2). The aqueous phase was acidified with aqueous 1 M HCl to pH = 3 and extracted with EtOAc (200 mL x 2). The combined organic layers were dried over anhydrous sodium sulfate and filtered. The filtrate was concentrated to give 5-[(1S)-1-methoxyethyl]-1-(pyridin-3-yl)-1H-pyrazole-4-carboxylic acid **[INT 1-sssss]** (1.30 g, 5.25 mmol, 65.9% yield) as a white solid. [M + H]+ Calcd for C12H14N3O3 248.1; Found 248.2. ¹H NMR (400 MHz, CDCl₃) δ = 8.83 (d, J = 1.6 Hz, 1H), 8.76 (br d, J = 4.0 Hz, 1H), 8.20 (s, 1H), 7.94 (td, J = 1.6, 8.0 Hz, 1H), 7.51 (dd, J = 4.8, 8.0 Hz, 1H), 5.30 (q, J = 6.8 Hz, 1H), 3.30 (s, 3H), 1.43 (d, J = 6.8 Hz, 3H).

### Synthesis of tert-butyl N-{5-[(1S)-1-methoxyethyl]-1-(pyridin-3-yl)-1H-pyrazol-4-yl}carbamate [INT 1-ttttt]:

To a mixture of 5-[(1S)-1-methoxyethyl]-1-(pyridin-3-yl)-1H-pyrazole-4-carboxylic acid **[INT 1-sssss]** (1.2 g, 4.85 mmol), 2-methylpropan-2-ol (5.38 g, 72.7 mmol) and diphenylphosphoryl azide (2.00 g, 7.27 mmol) in toluene (10 mL) was added triethylamine (1.46 g, 14.5 mmol). The reaction mixture was stirred at 100 °C under nitrogen for 2 hours. The reaction mixture was diluted with EtOAc (100 mL x 3) and washed with saturated NaHCO₃ (100 mL) and brine (100 mL). The organic layer was dried over anhydrous sodium sulfate and filtered. The filtrate was concentrated to give the crude product, which was purified by flash chromatography on silica gel (0-40% EtOAc in petroleum ether) to give tert-butyl N-{5-[(1S)-1-methoxyethyl]-1-(pyridin-3-yl)-1H-pyrazol-4-yl}carbamate **[INT 1-ttttt]** (490 mg, 1.53 mmol, 31.8% yield) as a white solid. m/z: [M + H]+ Calcd for C16H23N4O3 319.2; Found 319.1. ¹H NMR (400 MHz, CDCl₃) δ = 8.76 - 8.65 (m, 2H), 8.21 (br s, 1H), 7.99 - 7.78 (m, 1H), 7.59 - 7.43 (m, 1H), 6.86 (br d, J = 12.8 Hz, 1H), 4.56 - 4.45 (m, 1H), 3.29 (d, J = 6.8 Hz, 3H), 1.55 (s, 9H), 1.52 - 1.48 (m, 3H).

### Synthesis of 5-[(1S)-1-methoxyethyl]-1-(pyridin-3-yl)-1H-pyrazol-4-amine hydrochloride [INT 1.29]:

A mixture of tert-butyl N-{5-[(1S)-1-methoxyethyl]-1-(pyridin-3-yl)-1H-pyrazol-4-yl}carbamate **[INT 1-ttttt]** (490 mg, 1.53 mmol) in 4 M HCl/dioxane (10 mL, 40.0 mmol) was stirred at 25 °C for 12 hr. The mixture was concentrated under reduced pressure to afford 5-[(1S)-1-methoxyethyl]-1-(pyridin-3-yl)-1H-pyrazol-4-amine hydrochloride **[INT 1.29]** (389 mg, 1.52 mmol) as a yellow solid. m/z: [M+H]+ Calcd for C11H15N4O 219.1; Found :219.1.

### Synthesis of 3-(4-amino-5-cyclopropyl-1H-pyrazol-1-yl)benzonitrile hydrochloride [Intermediate 1.30]:

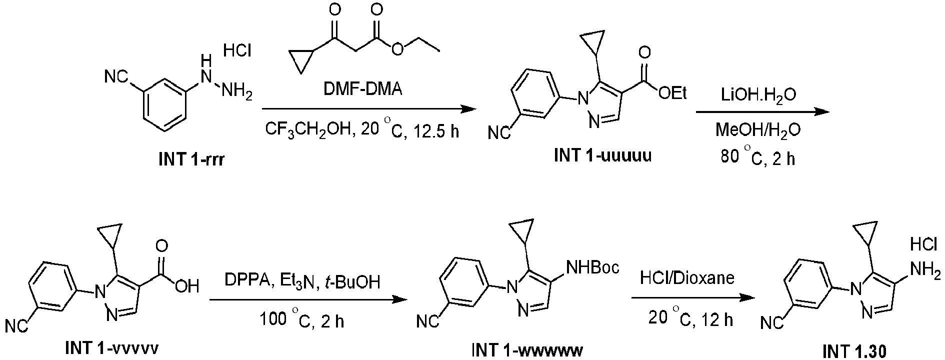

### Synthesis of ethyl 1-(3-cyanophenyl)-5-cyclopropyl-1H-pyrazole-4-carboxylate [INT 1-uuuuu]:

To a mixture of ethyl 3-cyclopropyl-3-oxopropanoate (2.0 g, 12.8 mmol) in 2,2,2-trifluoroethanol (15 mL) was added N,N-dimethylformamide dimethyl acetal (1.82 g, 15.3 mmol). The mixture was stirred at 20 °C for 30 min. 3-hydrazinylbenzonitrile hydrochloride **[INT 1-rrr]** (2.59 g, 15.3 mmol) was added and the mixture was stirred at 20 °C for 12 h. The mixture was concentrated to give the crude product. The residue was quenched with water (40 mL) and extracted with EtOAc (30 mL x 3). The combined organic layers were washed with brine (40 mL x 2), dried over anhydrous Na₂SO₄ and concentrated under reduced pressure to give the crude product, which was purified by flash chromatography on silica gel (EtOAc/PE = 0/1 to 1/5) to give ethyl 1-(3-cyanophenyl)-5-cyclopropyl-1H-pyrazole-4-carboxylate **[INT 1-uuuuu]** (2.00 g, 7.10 mmol, 55.5% yield) as an orange solid. m/z: [M + H]+ Calcd for C16H16N3O2 282.1; Found 282.0. ¹H NMR (400 MHz, CDCl₃) δ = 8.04 (s, 1H), 7.90 (s, 1H), 7.85 (d, J = 8.0 Hz, 1H), 7.75 - 7.70 (m, 1H), 7.67 - 7.59 (m, 1H), 4.34 (q, J = 7.2 Hz, 2H), 1.66 (br s, 1H), 1.39 (t, J = 7.2 Hz, 3H), 1.05 - 0.98 (m, 2H), 0.59 (q, J = 5.6 Hz, 2H).

### Synthesis of 1-(3-cyanophenyl)-5-cyclopropyl-1H-pyrazole-4-carboxylic acid [INT 1-vvvvv]:

To a solution of ethyl 1-(3-cyanophenyl)-5-cyclopropyl-1H-pyrazole-4-carboxylate **[INT 1-uuuuu]** (1.5 g, 5.33 mmol) in MeOH (6 mL) was added lithium hydroxide monohydrate (335 mg, 7.99 mmol) in H₂O (2 mL). The reaction was stirred at 80 °C for 2 h. The reaction mixture was poured into 10 mL of water, acidified with aqueous 1 N HCl to pH=3-4 and extracted with EtOAc (20 mL x 2). The combined organic layers were washed with brine (20 mL x 2), dried over anhydrous Na₂SO₄ and concentrated under reduced pressure to give the crude product, which was purified by flash chromatography on silica gel (MeOH/DCM = 0/1 to 1/10) to give 1-(3-cyanophenyl)-5-cyclopropyl-1H-pyrazole-4-carboxylic acid **[INT 1-vvvvv]** (740 mg, 2.92 mmol, 55.2% yield) as an off-white solid. ¹H NMR (400 MHz, DMSO-d6) δ = 12.38 (s, 1H), 8.18 - 8.15 (m, 1H), 7.99 (s, 1H), 7.98 - 7.92 (m, 2H), 7.79 - 7.73 (m, 1H), 2.22 - 2.12 (m, 1H), 0.86 - 0.81 (m, 2H), 0.47 - 0.41 (m, 2H).

### Synthesis of tert-butyl N-[1-(3-cyanophenyl)-5-cyclopropyl-1H-pyrazol-4-yl]carbamate [INT 1-wwwww]:

A solution of 1-(3-cyanophenyl)-5-cyclopropyl-1H-pyrazole-4-carboxylic acid **[INT 1-vvvvv]** (640 mg, 2.52 mmol), triethylamine (382 mg, 3.78 mmol) and diphenylphosphoryl azide (1.38 g, 5.04 mmol) in *t*-BuOH (8 mL) was stirred at 100 °C for 2 h under N₂. The reaction mixture was concentrated under reduced pressure to give the crude product, which was purified by flash chromatography on silica gel (EtOAc/PE = 0/1 to 1/5) and by Prep HPLC (column : YMC Triart 30*150 mm*7 µm, table: 30 - 70% B (A = water(ammonia hydroxide v/v)-(ACN-THF 50/50), B = acetonitrile), flow rate: 50 mL/min, UV Detector 220 nm) to afford tert-butyl N-[1-(3-cyanophenyl)-5-cyclopropyl-1H-pyrazol-4-yl]carbamate **[INT 1-wwwww]** (110 mg, 339 µmol, 13.4% yield) as a white solid. m/z: [M + H]+ Calcd for C18H21N4O2 325.2; Found 325.1. ¹H NMR (400 MHz, CDCl₃) δ = 8.00 - 7.91 (m, 2H), 7.91 - 7.86 (m, 1H), 7.66 - 7.60 (m, 1H), 7.60 - 7.54 (m, 1H), 6.19 (s, 1H), 1.88 - 1.76 (m, 1H), 1.54 (s, 9H), 1.00 - 0.93 (m, 2H), 0.52 - 0.46 (m, 2H).

### Synthesis of 3-(4-amino-5-cyclopropyl-1H-pyrazol-1-yl)benzonitrile hydrochloride [INT 1.30]:

A solution of tert-butyl N-[1-(3-cyanophenyl)-5-cyclopropyl-1H-pyrazol-4-yl]carbamate **[INT 1-wwwww]** (110 mg, 339 µmol) in HCl/Dioxane (3 mL) was stirred at 20 °C for 12 h. The reaction mixture was concentrated under reduced pressure to give 3-(4-amino-5-cyclopropyl-1H-pyrazol-1-yl)benzonitrile hydrochloride **[INT 1.30]** (89.0 mg, 341 µmol) as an off-white solid. m/z: [M + H]+ Calcd for C13H13N4 225.1; Found 225.0. ¹H NMR (400 MHz, DMSO-d6) δ = 10.22 (br s, 2H), 8.18 (s, 1H), 8.02 (br d, J = 8.0 Hz, 1H), 7.94 (br d, J = 7.6 Hz, 1H), 7.79 (s, 1H), 7.78 - 7.72 (m, 1H), 2.15 - 2.03 (m, 1H), 0.91 - 0.80 (m, 2H), 0.61 - 0.47 (m, 2H).

### Synthesis of 5-cyclopropyl-1-(pyridine-2-yl)-1H-pyrazol-4-amine hydrochloride [Intermediate 1.31]:

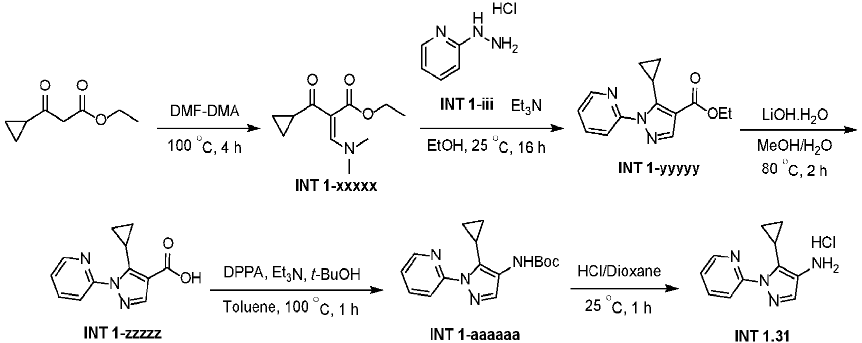

### Synthesis of ethyl (2Z)-2-[(Z)-cyclopropanecarbonyl]-3-(dimethylamino)prop-2-enoate [INT 1-xxxxx]:

A mixture of ethyl 3-cyclopropyl-3-oxopropanoate (20 g, 128 mmol) and N,N-dimethylformamide dimethyl acetal (30.5 g, 256 mmol) was stirred at 100 °C for 4 h. The mixture was concentrated under reduced pressure to give ethyl (2Z)-2-[(Z)-cyclopropanecarbonyl]-3-(dimethylamino)prop-2-enoate **[INT 1-xxxxx]** (27.0 g, 127 mmol). m/z: [M + H]+ Calcd for C11H18NO3 212.1; Found 212.1.

### Synthesis of ethyl 5-cyclopropyl-1-(pyridin-2-yl)-1H-pyrazole-4-carboxylate [INT 1-yyyyy]:

A solution of ethyl (2Z)-2-[(Z)-cyclopropanecarbonyl]-3-(dimethylamino)prop-2-enoate **[INT 1-xxxxx]** (5 g, 21.3 mmol), 2-hydrazinylpyridine hydrochloride [HCl salt **of INT 1-iii]** (3.71 g, 25.5 mmol), and Et₃N (4.30 g, 42.6 mmol) in EtOH (50 mL) was stirred at 25 °C for 16 h. The reaction was concentrated under reduced pressure to give the crude product, which was purified by flash chromatography on silica gel (EtOAc/PE = 0/1 to 1/3) to give ethyl 5-cyclopropyl-1-(pyridin-2-yl)-1H-pyrazole-4-carboxylate **[INT 1-yyyyy]** (2.80 g, 10.8 mmol, 51.0% yield) as a yellow oil. m/z: [M + H]+ Calcd for C14H16N3O2 258.1; Found 258.0. ¹H NMR (400 MHz, CDCl₃) δ = 8.57 (dd, J = 1.0, 4.8 Hz, 1H), 8.02 (s, 1H), 7.87 (dt, J = 1.8, 7.8 Hz, 1H), 7.64 (d, J = 8.1 Hz, 1H), 7.41 - 7.30 (m, 1H), 4.32 (q, J = 7.1 Hz, 2H), 2.29 (tt, J = 5.6, 8.6 Hz, 1H), 1.37 (t, J = 7.1 Hz, 3H), 1.02 - 0.85 (m, 2H), 0.68 - 0.50 (m, 2H).

### Synthesis of 5-cyclopropyl-1-(pyridin-2-yl)-1H-pyrazole-4-carboxylic acid [INT 1-zzzzz]:

A solution of ethyl 5-cyclopropyl-1-(pyridin-2-yl)-1H-pyrazole-4-carboxylate **[INT 1-yyyyy]** (2.5 g, 9.71 mmol) and lithium hydroxide monohydrate (1.01 g, 24.2 mmol) in THF (10 mL), H₂O (10 mL) and CH₃OH (10 mL) was stirred at 25 °C for 16 h. The reaction was concentrated under reduced pressure, and the resulting solution was acidified with aqueous 1 M HCl to pH=6. The resulting suspension was filtered and the filter cake was concentrated under reduced pressure to give 5-cyclopropyl-1-(pyridin-2-yl)-1H-pyrazole-4-carboxylic acid **[INT 1-zzzzz]** (1.90 g, 8.28 mmol, 85.5% yield) as a white solid. m/z: [M + H]+ Calcd for C12H12N3O2 230.1; Found 230.0. ¹H NMR (400 MHz, DMSO-d6) δ = 12.41 (br s, 1H), 8.67 - 8.52 (m, 1H), 8.06 (dt, J = 2.0, 7.8 Hz, 1H), 7.96 (s, 1H), 7.69 (d, J = 8.1 Hz, 1H), 7.53 (ddd, J = 1.0, 4.9, 7.5 Hz, 1H), 2.25 (tt, J = 5.6, 8.7 Hz, 1H), 0.86 - 0.78 (m, 2H), 0.60 - 0.53 (m, 2H).

### Synthesis of tert-butyl N-[5-cyclopropyl-1-(pyridin-2-yl)-1H-pyrazol-4-yl]carbamate [INT 1-aaaaaa] :

A solution of 5-cyclopropyl-1-(pyridin-2-yl)-1H-pyrazole-4-carboxylic acid **[INT 1-zzzzz]** (1.8 g, 7.85 mmol), DPPA (3.21 g, 11.7 mmol), and Et₃N (2.37 g, 23.5 mmol) in toluene (20 mL) was stirred at 25 °C for 15 min. 2-methylpropan-2-ol (581 mg, 7.84 mmol) was added and the reaction mixture was stirred at 100 °C for 1 h. The reaction was quenched by adding water (30 mL) and was extracted with EtOAc (2 x 50 mL). The combined organic layers were washed with brine (2 x 50 mL), dried over anhydrous Na₂SO₄ and concentrated under reduced pressure to give the crude product, which was purified by flash chromatography on silica gel (EtOAc/PE = 0/1 to 1/3) to give tert-butyl N-[5-cyclopropyl-1-(pyridin-2-yl)-1H-pyrazol-4-yl]carbamate **[INT 1-aaaaaa]** (800 mg, 2.66 mmol, 34.0% yield) as a colorless oil. m/z: [M + H]+ Calcd for C16H21N4O2 301.2; Found 301.2. ¹H NMR (400 MHz, CDCl₃) δ = 8.47 (dd, J = 1.0, 4.8 Hz, 1H), 7.97 (br s, 1H), 7.84 - 7.76 (m, 1H), 7.75 - 7.66 (m, 1H), 7.21 (ddd, J = 1.0, 4.9, 7.2 Hz, 1H), 6.28 (br s, 1H), 2.14 (ddd, J = 2.7, 5.5, 8.2 Hz, 1H), 1.53 (s, 9H), 0.96 - 0.81 (m, 2H), 0.54 - 0.39 (m, 2H).

### Synthesis of 5-cyclopropyl-1-(pyridine-2-yl)-1H-pyrazol-4-amine hydrochloride [INT 1.31]:

A solution of tert-butyl N-[5-cyclopropyl-1-(pyridin-2-yl)-1H-pyrazol-4-yl]carbamate **[INT 1-aaaaaa]** (700 mg, 2.33 mmol) in 4 M HCl in dioxane (10 mL) was stirred at 25 °C for 1 h. The reaction was concentrated under reduced pressure to give 5-cyclopropyl-1-(pyridine-2-yl)-1H-pyrazol-4-amine hydrochloride **[INT 1.31]** (550 mg, 2.32 mmol, 99.8% yield) as a yellow solid. m/z: [M + H]+ Calcd for C11H13N4201.1; Found 201.1.

### Synthesis of 5-cyclopropyl-1-(pyridin-3-yl)-1H-pyrazol-4-amine hydrochloride [Intermediate 1.32]:

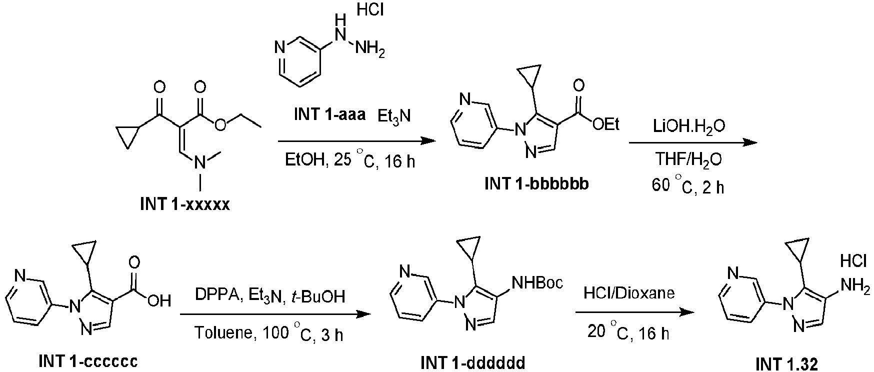

### Synthesis of ethyl 5-cyclopropyl-1-(pyridin-3-yl)-1H-pyrazole-4-carboxylate [INT 1-bbbbbb]:

A solution of ethyl (2Z)-2-[(Z)-cyclopropanecarbonyl]-3-(dimethylamino)prop-2-enoate **[INT 1-xxxxx]** (5g, 23.6 mmol), 3-hydrazinylpyridine hydrochloride **[INT 1-aaa]** (4.12 g, 28.3 mmol), and Et₃N (4.76 g, 47.2 mmol) in EtOH (50 mL) was stirred at 25 °C for 16 h. The reaction was concentrated under reduced pressure to give the crude product, which was purified by flash chromatography on silica gel (EtOAc/PE = 0/1 to 1/1) to give ethyl 5-cyclopropyl-1-(pyridin-3-yl)-1H-pyrazole-4-carboxylate **[INT 1-bbbbbb]** (1.70 g, 6.60 mmol, 28.0% yield) as a yellow oil. m/z: [M + H]+ Calcd for C14H16N3O2 258.1; Found 258.1. ¹H NMR (400 MHz, CDCl₃) δ = 8.85 (d, J = 2.2 Hz, 1H), 8.65 (dd, J = 1.2, 4.7 Hz, 1H), 8.04 (s, 1H), 7.94 - 7.83 (m, 1H), 7.45 (dd, J = 4.8, 8.2 Hz, 1H), 4.32 (q, J = 7.2 Hz, 2H), 2.01 - 1.88 (m, 1H), 1.37 (t, J = 7.2 Hz, 3H), 1.05 - 0.90 (m, 2H), 0.66 - 0.48 (m, 2H).

### Synthesis of 5-cyclopropyl-1-(pyridin-3-yl)-1H-pyrazole-4-carboxylic acid [INT 1-cccccc]:

To a mixture of ethyl 5-cyclopropyl-1-(pyridin-3-yl)-1H-pyrazole-4-carboxylate **[INT 1-bbbbbb]** (1.7 g, 6.60 mmol) in THF (6 mL) and H₂O (2 mL) was added lithium hydroxide monohydrate (826 mg, 19.7 mmol). The reaction mixture was stirred at 60 °C for 2 hours. The reaction mixture was concentrated under reduced pressure. The resulting residue was acidified with aqueous 1 M HCl to pH =4. The reaction mixture was quenched by addition of H₂O (20 mL) and extracted with EtOAc (20 mL x 2). The combined organic layers were washed with brine (20 mL), dried over Na₂SO₄, filtered and concentrated under reduced pressure to afford 5-cyclopropyl-1-(pyridin-3-yl)-1H-pyrazole-4-carboxylic acid **[INT 1-cccccc]** (1 g, 4.36 mmol, 66.2% yield) as a yellow solid. m/z: [M + H]+ Calcd for C12H12N3O2 230.1; Found 230.1.

### Synthesis of tert-butyl (5-cyclopropyl-1-(pyridin-3-yl)-1H-pyrazol-4-yl)carbamate [INT 1-ddddddl:

To a mixture of 5-cyclopropyl-1-(pyridin-3-yl)-1H-pyrazole-4-carboxylic acid **[INT 1-cccccc]** (500 mg, 2.18 mmol) in toluene (3mL) and t-BuOH (2 mL) was added diphenylphosphoryl azide (899 mg, 3.27 mmol) and triethylamine (661 mg, 6.54 mmol). The reaction mixture was stirred at 100 °C under nitrogen for 3 hours. The reaction mixture was concentrated to give a residue which was purified by flash chromatography on silica gel (0-50% EtOAc in petroleum ether) to give tert-butyl (5-cyclopropyl-1-(pyridin-3-yl)-1H-pyrazol-4-yl)carbamate **[INT 1-dddddd]** (500 mg, 1.66 mmol, 76.4% yield) as a yellow oil. m/z: [M + H]+ Calcd for C16H21N4O2 301.2; Found 301.2.

### Synthesis of 5-cyclopropyl-1-(pyridin-3-yl)-1H-pyrazol-4-amine hydrochloride [INT 1.32]:

A solution of tert-butyl N-[5-cyclopropyl-1-(pyridin-3-yl)-1H-pyrazol-4-yl]carbamate **[INT 1-dddddd]** (500 mg, 1.66 mmol) in 4 M HCI/dioxane (5 mL) was stirred at 20 °C for 16 h. The reaction mixture was concentrated under reduced pressure to give 5-cyclopropyl-1-(pyridin-3-yl)-1H-pyrazol-4-amine hydrochloride **[INT 1.32]** (250 mg, 1.24 mmol, 75.3% yield) as a yellow solid. m/z: [M + H]+ Calcd for C11H13N4 201.1; Found 201.0.

### Synthesis of (R)-N-((S)-1-(4-bromophenyl)-2,2,2-trifluoroethyl)-2-methylpropane-2-sulfinamide [Intermediate 2.1]:

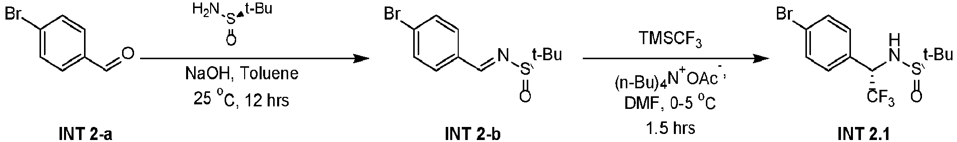

### Synthesis of (R,E)-N-(4-bromobenzylidene)-2-methylpropane-2-sulfinamide [INT 2-b]:

To a solution of 4-bromobenzaldehyde **[INT 2-a]** (100 g, 541 mmol, 1.0 eq) in toluene (500 mL) was added (R)-2-methylpropane-2-sulfinamide (72.1 g, 595 mmol, 1.1 eq) at 25 °C. The mixture was stirred at 25 °C for 15 mins. Then to above reaction was added NaOH (21.6 g, 541 mmol, 1.0 eq) and the mixture was stirred at 25 °C for 12 hours. Na₂SO₄ (50 g) was added to the mixture and stirred for 20 mins. Four reaction mixtures were combined and filtered through celite to give the filtrate, which was concentrated in vacuum to give the crude product as an oil. The crude product was dissolved in petroleum ether (1.0 L) and stirred at -50 °C for 1.0 hour, filtered to give (R,E)-N-(4-bromobenzylidene)-2-methylpropane-2-sulfinamide **[INT 2-b]** (620 g, 2.15 mol, 99.5% yield) as a solid.

### Synthesis of (R)-N-((S)-1-(4-bromophenyl)-2,2,2-trifluoroethyl)-2-methylpropane-2-sulfinamide [INT 2.1]:

To a solution of (R,E)-N-(4-bromobenzylidene)-2-methylpropane-2-sulfinamide **[INT 2-b]** (206 g, 715 mmol, 1.0 eq) and tetrabutylammonium acetate (216 g, 715 mmol, 218 mL, 1.0 eq) in DMF (1.4 L) was added TMSCF₃ (259 g, 1.82 mol, 2.5 eq) at 0 °C. The mixture was stirred at 5 °C for 1.5 hours. This process was repeated 2 times and the three reaction mixtures were combined for work-up. The mixture was poured into saturated NH₄Cl solution (13.0 L) and stirred for 10 mins to give a suspension. The suspension was filtered and the filter cake was washed with water (5.0 L). The filter cake was triturated with MTBE/ Petroleum ether (v/v = 1:4, 2.0 L) and to give the product as a solid and the mother liquid was concentrated in vacuum to give the crude product as an oil which was purified by column chromatography on silica gel with petroleum ether/ethyl acetate (10/1-1/1) to give (R)-N-((S)-1-(4-bromophenyl)-2,2,2-trifluoroethyl)-2-methylpropane-2-sulfinamide **[INT 2.1]** (389 g, 1.09 mol, 50.6% yield) as a solid. ¹H NMR (400 MHz, CDCl₃) δ = 1.25 (s, 9H), 3.64 (d, J = 6.40 Hz, 1H), 4.79-4.83 (m, 1H), 7.32 (d, J = 8.40 Hz, 2H), 7.56 (d, J = 6.40 Hz, 2H).

### Synthesis of (S)-1-(4-bromophenyl)-2,2,2-trifluoro-N-methylethan-1-amine [Intermediate 3.1]:

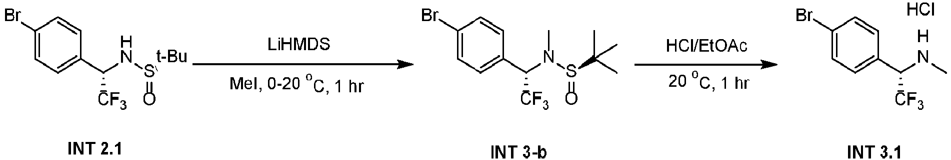

### Synthesis of (R)-N-((S)-1-(4-bromophenyl)-2,2,2-trifluoroethyl)-N,2-dimethylpropane-2-sulfinamide [INT 3-b]:

To a solution of LiHMDS (1.0 M, 838 mL, 3.0 eq) was added (R)-N-((S)-1-(4-bromophenyl)-2,2,2-trifluoroethyl)-2-methylpropane-2-sulfinamide **[INT 2.1]** (100 g, 279 mmol, 1.0 eq) at 0-10 °C and the resulting mixture was stirred at 0-10 °C for 0.5 hour. To the above mixture was added MeI (119 g, 838 mmol, 52.1 mL, 3.0 eq) at 0-10 °C and the reaction was stirred at 25 °C for 1 hour. The process was repeated 2 times and the three combined reaction mixtures were poured into saturated aqueous NH₄Cl (3.0 L) and diluted with EtOAc (1.0 L). The mixture was separated to give the organic layer and the aqueous layer was extracted with EtOAc (500 mL). The combined organic layer was washed with saturated NaCl (1.0 L) and dried with Na₂SO₄, filtered and concentrated in vacuum to give the crude product as an oil. The crude product was purified by column chromatography on silica gel with petroleum ether/ethyl acetate (15/1- 1/1) to give (R)-N-((S)-1-(4-bromophenyl)-2,2,2-trifluoroethyl)-N,2-dimethylpropane-2-sulfinamide **[INT 3-b]** (161 g, 432.5 mmol, 51.6% yield) as an oil.

### Synthesis of (S)-1-(4-bromophenyl)-2,2,2-trifluoro-N-methylethan-1-amine hydrochloride [INT 3.1]:

To the mixture of (R)-N-((S)-1-(4-bromophenyl)-2,2,2-trifluoroethyl)-N,2-dimethylpropane-2-sulfinamide **[INT 3-b]** (202 g, 543 mmol, 1.0 eq) in EtOAc (600 mL) was added HCl/EtOAc (4.0 M, 2.02 L, 14.9 eq) slowly. The above mixture was stirred at 20 °C for 1 hour. The reaction mixture was filtered to give a solid and eluted with EtOAc (200 mL) and the mother liquid was concentrated in vacuum to give a solid. The solid was purified by column chromatography on silica gel with petroleum ether/ethyl acetate (10/1-1/0) and combined with the filter cake and concentrated by oil pump at 45 °C for 1 hour to remove the solvent residue to give (S)-1-(4-bromophenyl)-2,2,2-trifluoro-N-methylethan-1-amine hydrochloride **[INT 3.1]** (115 g, 378 mmol, 69.6% yield, 100% purity, HCl) as a solid. ¹H NMR (400 MHz, DMSO-d₆) δ = 2.45 (s, 3H), 5.51 (s, 1H), 7.62 (d, J = 8.4 Hz, 2H), 7.78 (d, J = 8.40 Hz, 2H), 10.59 (s, 2H).

SFC: Rt = 0.776 min, 99.98% ee; Column: Chiralpak AD-3, 100×4.6 mm,I.D., 3 µm; Mobile phase: A: CO₂, B: MeOH (0.05%IPAm); Gradient: A: B=97:3; Flow rate: 3 mL/min; Column temp.: 35 °C.

LCMS: Rt = 1.755 min, 100.0% purity, m/z = 268.0, 270.0 (M+1)+. The gradient was 5%B in 0.40min and 5-95% B at 0.4-3.0min, hold on 95% B for 1.00min, and then 95-5% B in 0.01min, the flow rate was 1.0 ml/min. Mobile phase A was 0.037% Trifluoroacetic Acid in water, mobile phase B was 0.018% Trifluoroacetic Acid in acetonitrile. The column used for chromatography was a Kinetex C18 50*2.1 mm column (5 µm particles). Detection methods are diode array (DAD) as well as positive electrospray ionization. MS range was 100-1000.

### Synthesis of Tetrahydro-2H-thiopyran-4-carbonyl chloride 1,1-dioxide [Intermediate 4.1]:

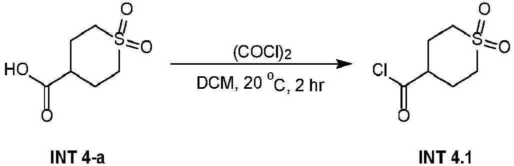

To a solution of tetrahydro-2H-thiopyran-4-carboxylic acid 1,1-dioxide **[INT 4-a]** (41.0 g, 230 mmol, 1.0 eq) in DCM (410 mL) was added (COCl)₂ (58.4 g, 460 mmol, 40.3 mL, 2.0 eq) and DMF (168 mg, 2.30 mmol, 177 µL, 0.01 eq) at 0 °C under N₂. The mixture was warmed to 20 °C and stirred at 20 °C for 2 hours. The reaction mixture was concentrated in vacuo to give the crude product tetrahydro-2H-thiopyran-4-carbonyl chloride 1,1-dioxide **[INT 4.1]** (46.5 g, crude) as a solid.

### Synthesis of 1-acetylpiperidine-4-carbonyl chloride [Intermediate 4.2]:

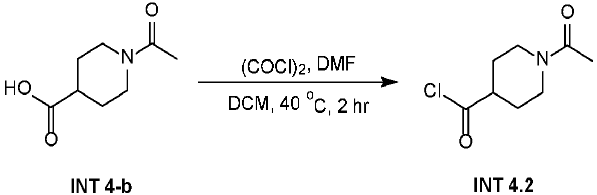

To a mixture of 1-acetylpiperidine-4-carboxylic acid **[INT 4-b]** (1 g, 5.84 mmol) in dichloromethane (10 mL) was added oxalyl dichloride (2.20 g, 17.5 mmol) and DMF (42.6 mg, 584 µmol) slowly and the mixture was stirred at 40 °C for 2 hr. The mixture was concentrated under reduced pressure to afford the crude 1-acetylpiperidine-4-carbonyl chloride **[INT 4.2]** (1.10 g, 5.80 mmol) as a green oil.

### Synthesis of 1-acetylazetidine-3-carbonyl chloride (Intermediate 4.3):

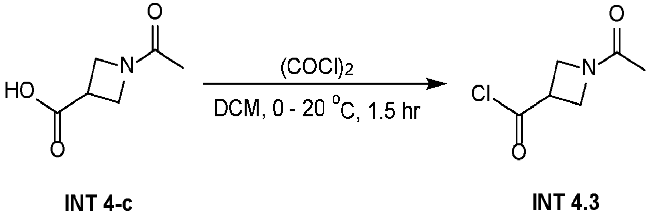

To a solution of 1-acetylazetidine-3-carboxylic acid **[INT 4-c]** (300 mg, 2.09 mmol) in DCM (4 mL) was added oxalyl chloride (397 mg, 3.13 mmol) at 0 °C. The mixture was stirred at 20 °C for 1.5 hr. The reaction was concentrated under reduced pressure to give 1-acetylazetidine-3-carbonyl chloride **[INT 4.3]** (337 mg, 2.08 mmol).

### Synthesis of Methyl (1r,4r)-4-(chlorocarbonyl)cyclohexane-1-carboxylate (Intermediate 4.4):

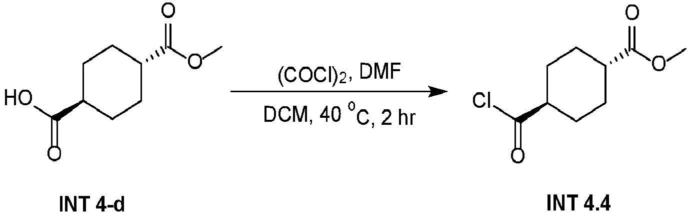

To a mixture of (1r,4r)-4-(methoxycarbonyl)cyclohexane-1-carboxylic acid [INT 4-d] (1.45 g, 7.78 mmol) in dichloromethane (10 mL) was added oxalyl dichloride (2.93 g, 23.3 mmol) and DMF (56.8 mg, 778 µmol) slowly and the mixture was stirred at 40 °C for 2 hr. The mixture was concentrated under reduced pressure to afford the crude methyl (1r,4r)-4-(chlorocarbonyl)cyclohexane-1-carboxylate [INT 4.4] (1.59 g, 7.76 mmol) as a yellow gum.

### Synthesis of methyl (1r,3r)-3-(carbonochloridoyl)cyclobutane-1-carboxylate (Intermediate 4.5):

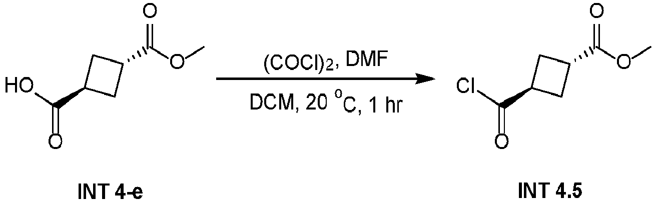

To a mixture of (1r,3r)-3-(methoxycarbonyl)cyclobutane-1-carboxylic acid **[INT 4-e]** (100 mg, 632 µmol) in CH₂Cl₂ (2 mL) was added DMF (one drop) and oxalic dichloride (239 mg, 1.89 mmol) at 20 °C. The mixture was stirred at 20 °C for 1 h. The reaction was concentrated under reduced pressure to give the crude methyl (1r,3r)-3-(carbonochloridoyl)cyclobutane-1-carboxylate **[INT 4.5]** (111 mg, 628 µmol) as a pale-yellow oil.

### Synthesis of benzyl 3-(chlorocarbonyl)cyclobutane-1-carboxylate (Intermediate 4.6):

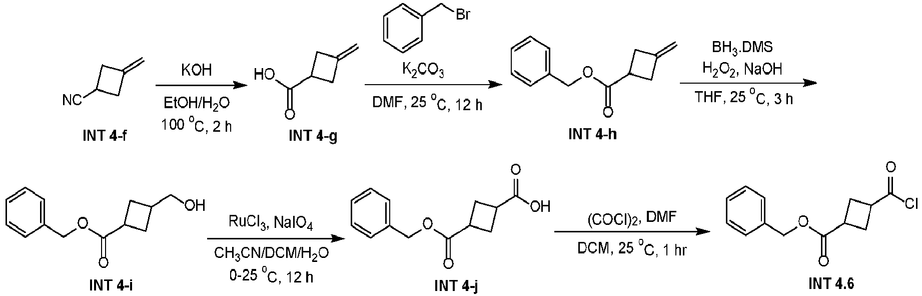

### Synthesis of 3-methylidenecyclobutane-1-carboxylic acid [INT 4-g]]:

To a mixture of 3-methylidenecyclobutane-1-carbonitrile **[INT 4-f]** (20 g, 214 mmol) in EtOH (100 mL) and water (100 mL) was added potassium hydroxide (48.0 g, 856 mmol). The mixture was stirred at 100 °C for 2 h. The ethanol was removed under reduced pressure, after which the solution was cooled to below 10 °C and acidified with concentrated HCl to pH = 1. The mixture was extracted with EtOAc (300 mL x 2) and the combined organic extracts were dried over anhydrous sodium sulfate and concentrated under reduced pressure to give 3-methylidenecyclobutane-1-carboxylic acid **[INT 4-g]** (23.0 g, 205 mmol, 96.2% yield) as a yellow oil. ¹H NMR (400 MHz, CDCl₃) δ = 9.51 (br s, 1H), 4.82 (quin, J = 2.4 Hz, 2H), 3.25 - 3.12 (m, 1H), 3.08 - 2.89 (m, 4H).

### Synthesis of benzyl 3-methylidenecyclobutane-1-carboxylate [INT 4-h]]:

To a mixture of 3-methylidenecyclobutane-1-carboxylic acid **[INT 4-g]** (13 g, 115 mmol) in DMF (120 mL) was added potassium carbonate (23.7 g, 172 mmol) and (bromomethyl)benzene (21.5 g, 126 mmol). The mixture was stirred at 25 °C for 12 h, after which it was combined with another of the same type. The mixture was diluted with water (200 mL) and extracted with ethyl acetate (250 mL x 2). The combined organic phase was washed with saturated brine (100 mL x 3), dried over anhydrous Na₂SO₄ and concentrated under reduced pressure to give the crude product, which was purified by flash chromatography on silica gel (EtOAc/PE = 0/1 to 10/1) to give benzyl 3-methylidenecyclobutane-1-carboxylate **[INT 4-h]** (33.0 g, 163 mmol) as a pale yellow oil. ¹H NMR (400 MHz, CDCl₃) δ = 7.43 - 7.31 (m, 5H), 5.16 (s, 2H), 4.83 (quin, J = 2.4 Hz, 2H), 3.26 - 3.13 (m, 1H), 3.10 - 2.86 (m, 4H).

### Synthesis of benzyl 3-(hydroxymethyl)cyclobutane-1-carboxylate [INT 4-i]]:

To a mixture of benzyl 3-methylidenecyclobutane-1-carboxylate **[INT 4-h]** (20 g, 98.8 mmol) in THF (200 mL) was added borane dimethylsulfide (29.5 mL, 296 mmol) dropwise under an ice-salt bath. The mixture was stirred at 25 °C for 1 h. Then sodium hydroxide (20 mL, 60.0 mmol) and hydrogen peroxide (18 mL, 98.8 mmol) were added under an ice-salt bath. The mixture was stirred at 25 °C for another 2 h. The reaction was quenched with saturated sodium sulfite solution (100 mL), diluted with water (50 mL) and extracted with ethyl acetate (2 x 200 mL). The organic phases were washed with saturated brine (2 x 100 mL), dried over anhydrous Na₂SO₄ and concentrated under reduced pressure to give the crude product, which was purified by flash chromatography on silica gel (PE/EtOAc=1:0 to 1/3) to give benzyl 3-(hydroxymethyl)cyclobutane-1-carboxylate **[INT 4-i]** (6.50 g, 29.5 mmol, 29.9% yield) as a pale yellow oil. ¹H NMR (400 MHz, CDCl₃) δ = 7.41 - 7.30 (m, 5H), 5.14 (d, J = 9.2 Hz, 2H), 3.67 (d, J = 6.4 Hz, 1H), 3.60 (d, J = 6.0 Hz, 1H), 3.22 - 3.05 (m, 1H), 2.64 - 2.28 (m, 3H), 2.14 - 2.01 (m, 2H).

### Synthesis of 3-[(benzyloxy)carbonyl]cyclobutane-1-carboxylic acid [INT 4-i]]:

To a mixture of benzyl 3-(hydroxymethyl)cyclobutane-1-carboxylate **[INT 4-i]** (4.5 g, 20.4 mmol) in CH₃CN (30 mL), water (30 mL) and CH₂Cl₂ (30 mL) was added sodium periodate (13.0 g, 61.1 mmol) and ruthenium(III) chloride (421 mg, 2.03 mmol) at 0 °C. The mixture was raised to 25 °C and reacted for 12 hours. The reaction was poured into 1 N HCl (100 mL). The mixture was concentrated under reduced pressure to give a residue. The residue was diluted with water (50 mL) and extracted with EtOAc (100 mL x 2). The combined organic phase was washed with brine (80 mL), dried over anhydrous Na₂SO₄ and concentrated under reduced pressure to give the crude product, which was purified by flash chromatography on silica gel (EtOAc/PE = 0/1to 1/3) to give 3-[(benzyloxy)carbonyl]cyclobutane-1-carboxylic acid **[INT 4-j]** (3.36 g, 14.3 mmol, 70.4% yield) as a brown oil. m/z: [M + H]+ Calcd for C13H15O4 235.1; Found 235.1. ¹H NMR (400 MHz, CDCl₃) δ = 7.44 - 7.30 (m, 5H), 5.15 (d, J = 8.0 Hz, 2H), 3.37 - 3.04 (m, 2H), 2.69 - 2.44 (m, 4H).

### Synthesis of benzyl 3-(chlorocarbonyl)cyclobutane-1-carboxylate [INT 4.6]]:

To a mixture of 3-[(benzyloxy)carbonyl]cyclobutane-1-carboxylic acid **[INT 4-j]** (3.36 g, 14.3 mmol) in CH₂Cl₂ (80 mL) was added DMF (one drop) and oxalyl chloride (3.62 g, 28.6 mmol) at 25 °C. The mixture was stirred at 25 °C for 1 h. The reaction was concentrated under reduced pressure to give the crude benzyl 3-(chlorocarbonyl)cyclobutane-1-carboxylate **[INT 4.6]** (3.61 g, 14.2 mmol) as a black oil.

### Synthesis of (S)-N-(1-(4-bromophenyl)-2,2,2-trifluoroethyl)-N-methyltetrahydro-2H-thiopyran-4-carboxamide 1,1-dioxide [Intermediate 5.1]:

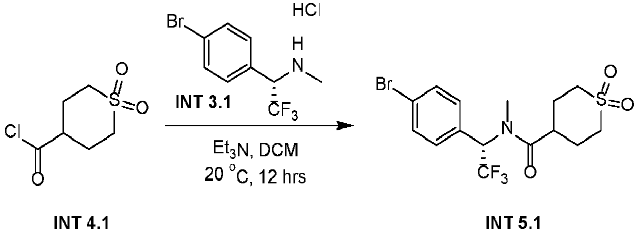

To a solution of (S)-1-(4-bromophenyl)-2,2,2-trifluoro-N-methylethan-1-amine hydrochloride [INT 3.1] (39.0 g, 128 mmol, 1.0 eq, HCl) and TEA (45.7 g, 451 mmol, 62.8 mL, 3.5 eq) in DCM (200 mL) was added tetrahydro-2H-thiopyran-4-carbonyl chloride 1,1-dioxide **[INT 4.1]** (45.3 g, 231 mmol, 1.8 eq) at 0-10 °C. The mixture was stirred at 20 °C for 12 hours. The mixture was separated to give the organic layer, and the aqueous layer was extracted with DCM (100 mL). The combined organic layer was concentrated in vacuum to give the crude product as an oil. The crude product was purified by column chromatography on silica gel with petroleum ether/ethyl acetate (15/1~3/1) to give (S)-N-(1-(4-bromophenyl)-2,2,2-trifluoroethyl)-N-methyltetrahydro-2H-thiopyran-4-carboxamide 1,1-dioxide **[INT 5.1]** (26.0 g, 60.7 mmol, 47.4% yield, 100% purity) as a solid. ¹H NMR (400 MHz, CDCl₃) δ = 2.25-2.37 (m, 1H), 2.38-2.40 (m, 3H), 2.88-3.00 (m, 6H), 3.30-3.31 (m, 1H), 3.22-3.45 (m, 1H), 6.56-6.63 (m, 1H), 7.23 (d, J = 8.00 Hz, 2H), 7.55 (d, J = 8.40 Hz, 2H).

SFC: Rt = 1.21 min, 100.0% ee; Column: Chiralpak AD-3, 50×4.6 mm I.D., 3 µm; Mobile phase: A: CO₂, B: MeOH (0.05%IPAm, v/v); Flow rate: 3.4 mL/min; Column temp.: 35 °C.

LCMS: Rt = 2.431 min, 100% purity, m/z = 428.0, 430.0(M+1)+. The gradient was 5%B in 0.40 min and 5-95% B at 0.4-3.0 min, hold on 95% B for 1.00 min, and then 95-5%B in 0.01 min, the flow rate was 1.0 ml/min. Mobile phase A was 0.037% Trifluoroacetic Acid in water, mobile phase B was 0.018% Trifluoroacetic Acid in acetonitrile. The column used for chromatography was a Kinetex C18 50*2.1mm column (5 µm particles). Detection methods are diode array (DAD) as well as positive electrospray ionization.MS range was 100-1000.

### Synthesis of 1-acetyl-N-[(1S)-1-(4-bromophenyl)-2,2,2-trifluoroethyl]-N-methylpiperidine-4-carboxamide [Intermediate 5.2]:

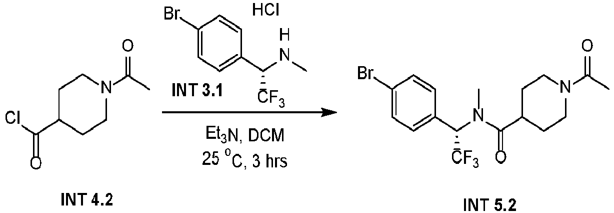

To a mixture of [(1S)-1-(4-bromophenyl)-2,2,2-trifluoroethyl](methyl)amine hydrochloride **[INT 3.1]** (500 mg, 1.64 mmol) and Et₃N (829 mg, 8.20 mmol) in dichloromethane (10 mL) was added 1-acetylpiperidine-4-carbonyl chloride **[INT 4.2]** (550 mg, 2.90 mmol) and the mixture was stirred at 25 °C for 3 hr. The mixture was concentrated under reduced pressure to give the crude product, which was purified by flash chromatography on silica gel (methanol/dichloromethane = 0/1 to 1/20) to give 1-acetyl-N-[(1S)-1-(4-bromophenyl)-2,2,2-trifluoroethyl]-N-methylpiperidine-4-carboxamide **[INT 5.2]** (680 mg, 1.61 mmol, 98.5% yield) as a yellow solid. m/z: [M+H]+ Calcd for C17H21BrF3N2O2 421.1, 423.1; Found 423.1. ¹H NMR (400 MHz, CDCl₃) δ = 7.55 (d, J = 8.4 Hz, 2H), 7.24 (d, J = 8.4 Hz, 2H), 6.62 (q, J = 8.8 Hz, 1H), 4.05 - 3.82 (m, 1H), 3.23 - 3.04 (m, 1H), 2.90 (s, 3H), 2.87 - 2.71 (m, 2H), 2.70 - 2.43 (m, 1H), 2.13 (s, 3H), 1.92 - 1.63 (m, 4H).

### Synthesis of 1-acetyl-N-[(1S)-1-(4-bromophenyl)-2,2,2-trifluoroethyl]-N-methylazetidine-3-carboxamide (Intermediate 5.3):

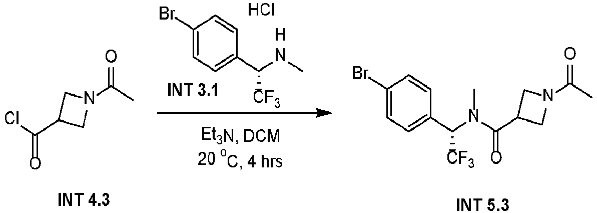

To a solution of 1-acetylazetidine-3-carbonyl chloride **[INT 4.3]** (300 mg, 1.85 mmol) and [(1S)-1-(4-bromophenyl)-2,2,2-trifluoroethyl](methyl)amine hydrochloride **[INT 3.1]** (563 mg, 1.85 mmol) in CH₂Cl₂ (3 mL) was added triethylamine (561 mg, 5.55 mmol). The mixture was stirred at 20 °C for 4 h. The reaction was concentrated under reduced pressure to give the crude product, which was purified by flash chromatography on silica gel (EtOAc/PE = 0/1 to 1/5) to give 1-acetyl-N-[(1S)-1-(4-bromophenyl)-2,2,2-trifluoroethyl]-N-methylazetidine-3-carboxamide **[INT 5.3]** (250 mg, 635 µmol, 34.3% yield) as a yellow oil. m/z: [M + H]+ Calcd for C15H17BrF3N2O2 393.0, 395.0; Found 394.8.

### Synthesis of Methyl (1S,4r)-4-(((S)-1-(4-bromophenyl)-2,2,2-trifluoroethyl)(methyl)carbamoyl)cyclohexane-1-carboxylate (Intermediate 5.4):

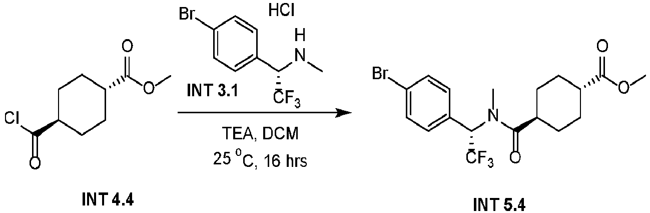

To a mixture of methyl (1r,4r)-4-(carbonochloridoyl)cyclohexane-1-carboxylate **[INT 4.4]** (1.59 g, 7.76 mmol) and Et₃N (2.65 g, 26.2 mmol) in dichloromethane (6 mL) was added a solution of [(1S)-1-(4-bromophenyl)-2,2,2-trifluoroethyl](methyl)amine hydrochloride **[INT 3.1]** (1.6 g, 5.25 mmol) in dichloromethane (6 mL) and the mixture was stirred at 25 °C for 16 hr. Water (30 mL) was added and the mixture was extracted with dichloromethane (30 mL x 2). The combined organic layers were washed with brine (30 mL), dried over anhydrous Na₂SO₄, filtered and concentrated under reduced pressure to give the crude product which was purified by flash chromatography on silica gel (EtOAc/Petroleum ether = 1/10 to 1/5) to give methyl (1S,4r)-4-(((S)-1-(4-bromophenyl)-2,2,2-trifluoroethyl)(methyl)carbamoyl)cyclohexane-1-carboxylate **[INT 5.4]** (1.10 g, 2.52 mmol, 32.5% yield) as yellow oil. m/z: [M+H]+ Calcd for C18H22BrF3NO3 436.1, 438.1; Found 438.0.

### Synthesis of methyl (1S,3r)-3-(((S)-1-(4-bromophenyl)-2,2,2-trifluoroethyl)(methyl)carbamoyl)cyclobutane-1-carboxylate (Intermediate 5.5):

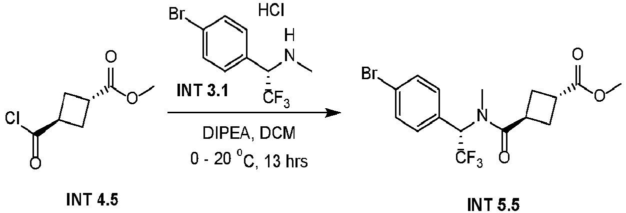

To a mixture of methyl (Ir,3r)-3-(carbonochloridoyl)cyclobutane-1-carboxylate **[INT 4.5]** (111 mg, 628 µmol) and [(1S)-1-(4-bromophenyl)-2,2,2-trifluoroethyl](methyl)amine hydrochloride **[INT 3.1]** (120 mg, 394 µmol) in CH₂Cl₂ (2 mL) was added N,N-diisopropylethylamine (254 mg, 1.97 mmol) at 0 °C. The mixture was stirred at 0 °C for 1 hour and then was stirred at 20 °C for 12 h. The reaction was diluted with CH₂Cl₂ (50 mL) and 1 N HCl (20 mL). The organic phase was separated and washed with brine (20 mL), dried over anhydrous Na₂SO₄ and concentrated under reduced pressure to give the crude product, which was purified by flash chromatography on silica gel (EtOAc/PE = 0/1 to 1/3) to give methyl (1S,3r)-3-(((S)-1-(4-bromophenyl)-2,2,2-trifluoroethyl)(methyl)carbamoyl)cyclobutane-1-carboxylate **[INT 5.5]** (84.8 mg, 207 µmol, 53.0% yield (53% purity)) as a yellow oil. m/z: [M + H]+ Calcd for C16H18BrF3NO3 408.0; Found 408.0.

### Synthesis of benzyl 3-{[(1S)-1-(4-bromophenyl)-2,2,2-trifluoroethyl] (methyl)carbamoyl}cyclobutane-1-carboxylate (Intermediate 5.6):

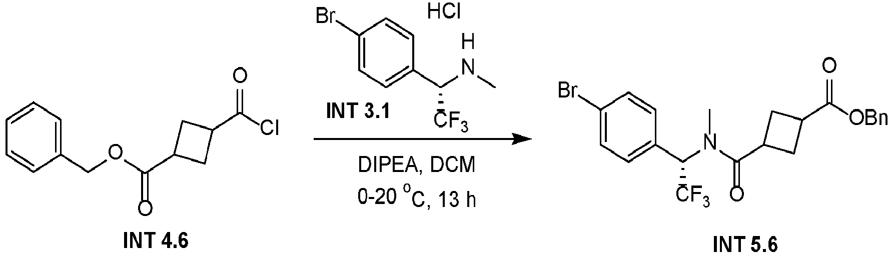

To a mixture of benzyl 3-(carbonochloridoyl)cyclobutane-1-carboxylate **[INT 4.6]** (3.61 g, 14.2 mmol) and [(1S)-1-(4-bromophenyl)-2,2,2-trifluoroethyl](methyl)amine hydrochloride **[INT 3.1]** (2.5 g, 8.20 mmol) in CH₂Cl₂ (100 mL) was added N,N-diisopropylethylamine (5.28 g, 40.9 mmol) at 0 °C. The mixture was stirred at 0 °C for 1 hour and stirred at 20 °C for 12 h. The reaction was diluted with CH₂Cl₂ (100 mL) and 1 N HCl (50 mL). The organic phase was separated and washed with brine (50 mL), dried over anhydrous Na₂SO₄ and concentrated under reduced pressure to give the crude product, which was purified by flash chromatography on silica gel (EtOAc/PE = 0/1 to 1/3). The resulting product was further purified by Prep-HPLC (column: YMC-Triart Prep C18 150*40 mm*7 µm, table: 48-68% B (A = water (0.025% FA), B = acetonitrile), flow rate: 60 mL/min, UV Detector 220 nm) to give benzyl 3-{[(1S)-1-(4-bromophenyl)-2,2,2-trifluoroethyl] (methyl)carbamoyl}cyclobutane-1-carboxylate **[INT 5.6]** (760 mg, 1.56 mmol, 19.1% yield) as a yellow oil. m/z: [M + H]+ Calcd for C22H22BrF3NO3 484.1; Found 484.0. ¹H NMR (400 MHz, CDCl₃) δ = 7.60 - 7.51 (m, 2H), 7.41 - 7.31 (m, 5H), 7.29 - 7.19 (m, 2H), 6.66 - 6.34 (m, 1H), 5.18 - 5.11 (m, 2H), 3.57 - 3.07 (m, 2H), 2.77 - 2.73 (m, 3H), 2.70 - 2.43 (m, 4H).

### Synthesis of (1r,4r)-N-[(1S)-1-(4-bromophenyl)-2,2,2- trifluoroethyl]-4-cyano-N-methylcyclohexane-1-carboxamide (Intermediate 5.7):

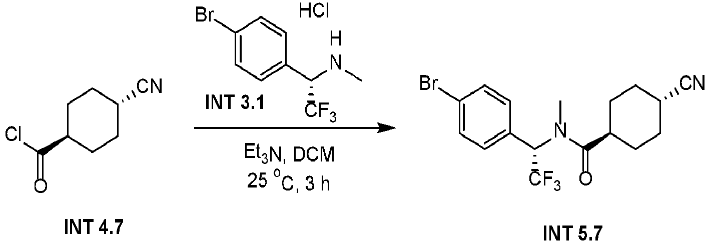

A mixture of [(1S)-1-(4-bromophenyl)-2,2,2-trifluoroethyl](methyl)amine hydrochloride **[INT 3.1]** (350 mg, 1.14 mmol) and triethylamine (576 mg, 5.70 mmol) in CH₂Cl₂ (5 mL) was stirred at 25 °C for 20 min. A solution of (1r,4r)-4-cyanocyclohexane-1-carbonyl chloride **[INT 4.7]** (391 mg, 2.28 mmol) in CH₂Cl₂ (5 mL) was added and the reaction was stirred at 25 °C for 3 hr. The mixture was concentrated under reduced pressure to give the crude product, which was purified by flash chromatography on silica gel (0-30% EtOAc in petroleum ether) to give (1r,4r)-N-[(1S)-1-(4-bromophenyl)-2,2,2- trifluoroethyl]-4-cyano-N-methylcyclohexane-1-carboxamide **[INT 5.7]** (300 mg, 743 µmol, 65.3% yield) as a white solid. ¹H NMR (400 MHz, CDCl₃) δ = 7.57 - 7.51 (m, 2H), 7.23 (d, J = 8.4 Hz, 2H), 6.60 (q, J = 8.8 Hz, 1H), 2.87 (s, 3H), 2.68 - 2.59 (m, 1H), 2.58 - 2.48 (m, 1H), 2.32 - 2.19 (m, 2H), 2.01 - 1.93 (m, 1H), 1.92 - 1.84 (m, 1H), 1.72 - 1.62 (m, 4H).

### Synthesis of methyl (1r,4r)-4-{methyl[(1S)-2,2,2-trifluoro-1-(4-{[1-phenyl-5-(trifluoromethyl)-1H-pyrazol-4-yl]amino}phenyl)ethyl]carbamoyl}cyclohexane-1-carboxylate [Intermediate 6.1]:

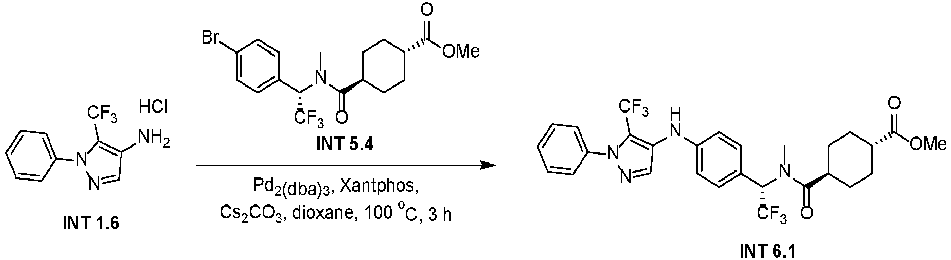

A mixture of 1-phenyl-5-(trifluoromethyl)-1H-pyrazol-4-amine hydrochloride **[INT 1.6]** (140 mg, 531 µmol), methyl (1r,4r)-4-{[(1S)-1-(4-bromophenyl)-2,2,2-trifluoroethyl](methyl)carbamoyl}cyclohexane-1-carboxylate **[INT 5.4]** (231 mg, 531 µmol), xantphos (30.7 mg, 53.1 µmol), Pd₂(dba)₃ (48.6 mg, 53.1 µmol) and Cs₂CO₃ (518 mg, 1.59 mmol) in dioxane (3 mL) was stirred at 100 °C for 3 hr under N₂ atmosphere. Brine (50 mL) was added and the mixture was extracted with EtOAc (50 mL x 2). The combined organic layers were washed with brine (50 mL x 2), dried over anhydrous Na₂SO₄, filtered and concentrated under reduced pressure to give the crude product, which was purified by flash chromatography on silica gel (0-8% EtOAc in petroleum ether) to give methyl (1r,4r)-4-{methyl[(1S)-2,2,2-trifluoro-1-(4-{[1-phenyl-5-(trifluoromethyl)-1H-pyrazol-4-yl]amino}phenyl)ethyl]carbamoyl}cyclohexane-1-carboxylate [INT 6.1] (152 mg, 260 µmol, 49.1% yield) as a brown solid. m/z: [M + H]+ Calcd for C28H29F6N4O3 583.2; Found 583.2.

### Synthesis of methyl (1r,4r)-4-{methyl[(1S)-2,2,2-trifluoro-1-(4-{[1-(pyridin-4-yl)-5-(trifluoromethyl)-1H-pyrazol-4-yl]amino}phenyl)ethyl]carbamoyl}cyclohexane-1-carboxylate [Intermediate 6.2]:

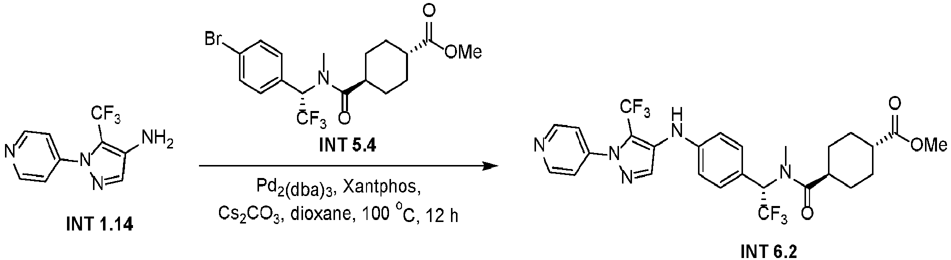

A mixture of 1-(pyridin-4-yl)-5-(trifluoromethyl)-1H-pyrazol-4-amine **[INT 1.14]** (80 mg, 350 µmol), methyl (1r,4r)-4-{[(1S)-1-(4-bromophenyl)-2,2,2-trifluoroethyl](methyl)carbamoyl}cyclohexane-1-carboxylate **[INT 5.4]** (152 mg, 350 µmol), Pd₂(dba)₃ (32.0 mg, 35.0 µmol), xantphos (40.5 mg, 70.0 µmol) and Cs₂CO₃ (228 mg, 700 µmol) in dioxane (3 mL) was stirred at 100 °C for 12 h under N₂. The mixture was worked up with another reaction of the same type. The reaction was quenched by adding water (20 mL) and was extracted with EtOAc (20 mL x 2). The combined organic layers were washed with brine (20 mL x 2), dried over anhydrous Na₂SO₄ and concentrated under reduced pressure to give the crude product, which was purified by flash chromatography on silica gel (PE/EtOAc= 1/0 to 1/1) to give methyl (1r,4r)-4-{methyl[(1S)-2,2,2-trifluoro-1-(4-{[1-(pyridin-4-yl)-5-(trifluoromethyl)-1H-pyrazol-4-yl]amino}phenyl)ethyl]carbamoyl}cyclohexane-1-carboxylate **[INT 6.2]** (115 mg, 197 µmol) as a pale yellow oil. m/z: [M + H]+ Calcd for C27H28F6N5O3 584.2; Found 584.1.

### Synthesis of methyl (1r,4r)-4-{methyl[(1S)-2,2,2-trifluoro-1-(4-{[1-(pyridin-3-yl)-5-(trifluoromethyl)-1H-pyrazol-4- yl]amino}phenyl)ethyl]carbamoyl}cyclohexane-1-carboxylate [Intermediate 6.3]:

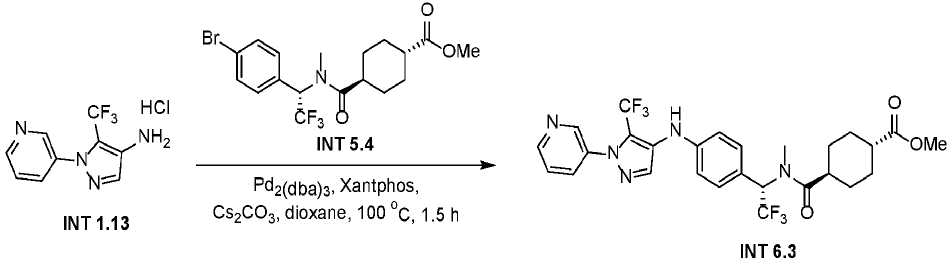

A suspension of 1-(pyridin-3-yl)-5-(trifluoromethyl)-1H-pyrazol-4-amine hydrochloride **[INT 1.13]** (150 mg, 566 µmol), methyl (1r,4r)-4-{[(1S)-1-(4- bromophenyl)-2,2,2-trifluoroethyl](methyl)carbamoyl}cyclohexane-1-carboxylate **[INT 5.4]** (246 mg, 566 µmol), Cs₂CO₃ (550 mg, 1.69 mmol), Pd₂(dba)₃ (25.9 mg, 28.3 µmol) and xantphos (32.7 mg, 56.6 µmol) in dioxane (2 mL) was stirred at 100 °C for 1.5 h under N₂. The mixture was concentrated under reduced pressure to give the crude product, which was purified by flash chromatography on silica gel (MeOH/Dichloromethane = 0/1 to 1/99) to give methyl (1r,4r)-4-{methyl[(1S)-2,2,2-trifluoro-1-(4-{[1-(pyridin-3-yl)-5-(trifluoromethyl)-1H-pyrazol-4-yl]amino}phenyl)ethyl]carbamoyl}cyclohexane-1-carboxylate **[INT 6.3]** (231 mg, 396 µmol, 70.0% yield) as a yellow oil. m/z: [M + H]+ Calcd for C27H28F6N5O3 584.2; Found 584.1. ¹H NMR (400 MHz, CDCl₃) δ = 8.80 (d, J = 2.8 Hz, 1H), 8.74 (dd, J = 1.2, 4.8 Hz, 1H), 7.91 - 7.81 (m, 2H), 7.49 (dd, J = 4.8, 8.0 Hz, 1H), 7.30 (s, 1H), 6.97 (d, J = 8.8 Hz, 2H), 6.61 (q, J = 8.8 Hz, 1H), 5.59 (s, 1H), 3.71 (s, 3H), 2.91 (s, 3H), 2.63 - 2.51 (m, 1H), 2.39 (tt, J = 3.6, 12.0 Hz, 1H), 2.11 (br t, J = 8.8 Hz, 2H), 1.96 - 1.83 (m, 2H), 1.70 - 1.61 (m, 2H), 1.56 - 1.45 (m, 2H).

### Synthesis of methyl (1r,4r)-4-{methyl[(1S)-2,2,2-trifluoro-1-(4-{[1-(pyridin-2-yl)-5-(trifluoromethyl)-1H-pyrazol-4-yl]amino}phenyl)ethyl]carbamoyl}cyclohexane-1-carboxylate [Intermediate 6.4]:

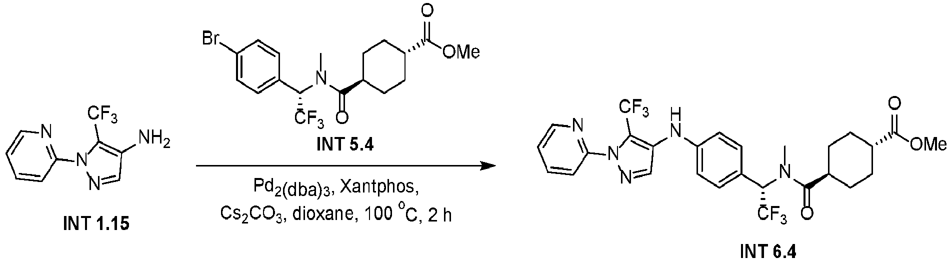

To a solution of 1-(pyridin-2-yl)-5-(trifluoromethyl)-1H-pyrazol-4-amine **[INT 1.15]** (100 mg, 438 µmol), methyl (1r,4r)-4-{[(1S)-1-(4-bromophenyl)-2,2,2-trifluoroethyl](methyl)carbamoyl}cyclohexane-1-carboxylate **[INT 5.4]** (191 mg, 438 µmol), Cs₂CO₃ (426 mg, 1.31 mmol) and xantphos (50.6 mg, 87.6 µmol) in dioxane (3 mL) was added Pd₂(dba)₃ (40.1 mg, 43.8 µmol) and the reaction mixture was stirred at 100°C for 2 h under N₂. The reaction mixture was concentrated under reduced pressure to give the crude product, which was purified by flash chromatography on silica gel (EtOAc/PE= 0/1 to 1/3) to give methyl (1r,4r)-4-{methyl[(1S)-2,2,2-trifluoro-1-(4-{[1-(pyridin-2-yl)-5-(trifluoromethyl)-1H-pyrazol-4-yl]amino}phenyl)ethyl]carbamoyl}cyclohexane-1-carboxylate **[INT 6.4]** (170 mg, 291 µmol, 66.6% yield) as a yellow oil. m/z: [M + H]+ Calcd for C27H28F6N5O3 584.2; Found 584.1.

### Synthesis of methyl (1r,4r)-4-{methyl[(1S)-2,2,2-trifluoro-1-[4-({5-[(1S)-1-methoxyethyl]-1-(pyridin-2-yl)-1H-pyrazol-4-yl}amino)phenyl]ethyl]carbamoyl}cyclohexane-1-carboxylate [Intermediate 6.5]:

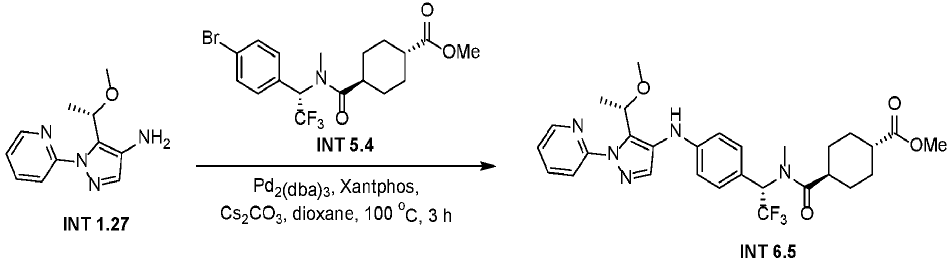

A mixture of 5-[(1S)-1-methoxyethyl]-1-(pyridin-2-yl)-1H-pyrazol-4-amine [free base of **INT 1.27]** (75 mg, 343 µmol), methyl (1r,4r)-4-{[(1S)-1-(4-bromophenyl)-2,2,2-trifluoroethyl](methyl)carbamoyl}cyclohexane-1-carboxylate **[INT 5.4]** (149 mg, 343 µmol), Pd₂(dba)₃ (31.3 mg, 34.3 µmol), xantphos (39.6 mg, 68.6 µmol) and Cs₂CO₃ (223 mg, 686 µmol) in dioxane (3 mL) was stirred at 100 °C for 3 h under N₂. The reaction was quenched by adding water (20 mL) and was extracted with EtOAc (20 mL x 2). The combined organic layers were washed with brine (20 mL x 2), dried over anhydrous Na₂SO₄ and concentrated under reduced pressure to give the crude product, which was purified by flash chromatography on silica gel (PE/EtOAc= 1/0 to 5/1) to give methyl (1r,4r)-4-{methyl[(1S)-2,2,2-trifluoro-1-[4-({5-[(1S)-1-methoxyethyl]-1-(pyridin-2-yl)-1H-pyrazol-4-yl}amino)phenyl]ethyl]carbamoyl}cyclohexane-1-carboxylate **[INT 6.5]** (85.0 mg, 148 µmol, 43.3% yield) as a pale yellow oil. ¹H NMR (400 MHz, DMSO) δ = 8.50 (d, J = 4.0 Hz, 1H), 8.05 - 7.97 (m, 1H), 7.84 - 7.77 (m, 1H), 7.45 - 7.36 (m, 1H), 7.20 (s, 1H), 7.10 (d, J = 8.0 Hz, 2H), 6.86 (d, J = 8.0 Hz, 2H), 6.41 (q, J = 8.0 Hz, 1H), 5.29 (q, J = 8.0 Hz, 1H), 3.58 (s, 3H), 3.06 (s, 3H), 2.87 (s, 3H), 2.73 - 2.65 (m, 1H), 1.96 - 1.64 (m, 5H), 1.52 (d, J = 6.0 Hz, 3H), 1.48 - 1.37 (m, 4H).

### Synthesis of methyl (1r,4r)-4-{methyl[(1S)-2,2,2-trifluoro-1-[4-({S-[(1S)-1-methoxyethyl]-1-phenyl-1H-pyrazol-4-yl}amino)phenyl]ethyl]carbamoyl}cyclohexane-1-carboxylate [Intermediate 6.6]:

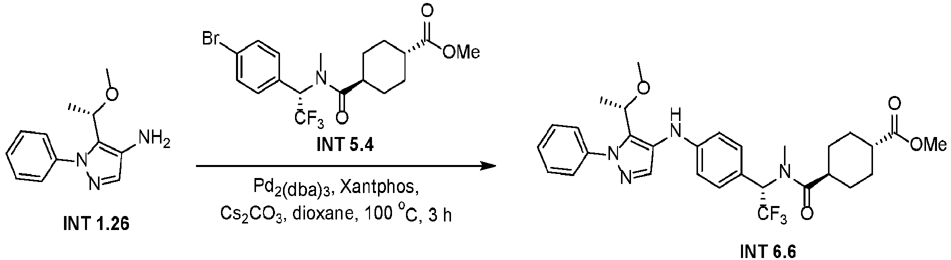

A mixture of 5-[(1S)-1-methoxyethyl]-1-phenyl-1H-pyrazol-4-amine [free base **of INT 1.26]** (100 mg, 460 µmol), methyl (1r,4r)-4-{[(1S)-1-(4-bromophenyl)-2,2,2-trifluoroethyl](methyl)carbamoyl}cyclohexane-1-carboxylate **[INT 5.4]** (200 mg, 460 µmol), xantphos (26.6 mg, 46.0 µmol), Pd₂(dba)₃ (42.1 mg, 46.0 µmol) and Cs₂CO₃ (449 mg, 1.38 mmol) in dioxane (1 mL) was stirred at 100 °C for 3 hr under N₂ atmosphere. Brine (20 mL) was added and the mixture was extracted with EtOAc (50 mL x 2). The combined organic layers were washed with brine (20 mL x 2), dried over anhydrous Na₂SO₄, filtered and concentrated under reduced pressure to give the crude product, which was purified by flash chromatography on silica gel (0-20% EtOAc in Petroleum ether) to give methyl (1r,4r)-4-{ methyl[(1 S)-2,2,2-trifluoro-1-[4-({5-[(1 S)-1-methoxyethyl]-1-phenyl-1H-pyrazol-4-yl}amino)phenyl]ethyl]carbamoyl}cyclohexane-1-carboxylate **[INT 6.6]** (230 mg, 401 µmol, 87.4% yield) as a white solid. m/z: [M + H]+ Calcd for C30H36F3N4O4 573.3; Found 573.3.

### Synthesis of methyl (1r,4r)-4-{methyl[(1S)-2,2,2-trifluoro-1-[4-({5-[(1S)-1-methoxyethyl]-1-(pyridin-4-yl)-1H-pyrazol-4-yl}amino)phenyl]ethyl]carbamoyl}cyclohexane-1-carboxylate [Intermediate 6.7]:

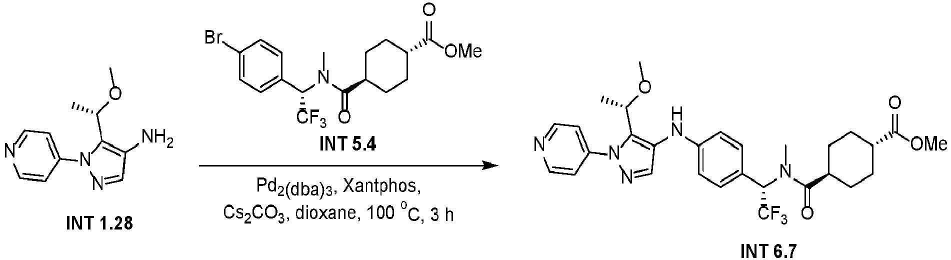

A mixture of 5-[(1S)-1-methoxyethyl]-1-(pyridin-4-yl)-1H-pyrazol-4-amine **[INT 1.28]** (100 mg, 458 µmol), methyl (1r,4r)-4-{[(1S)-1-(4-bromophenyl)-2,2,2-trifluoroethyl](methyl)carbamoyl}cyclohexane-1-carboxylate **[INT 5.4]** (199 mg, 458 µmol), Pd₂(dba)₃ (41.9 mg, 45.8 µmol), xantphos (53.0 mg, 91.6 µmol) and Cs₂CO₃ (298 mg, 916 µmol) in dioxane (3 mL) was stirred at 100 °C for 3 h under N₂. The reaction was quenched by adding water (20 mL) and was extracted with EtOAc (20 mL x 2). The combined organic layers were washed with brine (20 mL x 2), dried over anhydrous Na₂SO₄ and concentrated under reduced pressure to give the crude product, which was purified by flash chromatography on silica gel (PE/EtOAc= 1/0 to 1/1) to give methyl (1r,4r)-4-{ methyl[(1S)-2,2,2-trifluoro-1-[4-({5-[(1S)-1-methoxyethyl]-1-(pyridin-4-yl)-1H-pyrazol-4-yl}amino)phenyl]ethyl]carbamoyl}cyclohexane-1-carboxylate **[INT 6.7]** (230 mg, 400 µmol, 87.7% yield) as a pale yellow oil. m/z: [M + H]+ Calcd for C29H35F3N5O4 574.3; Found 574.3. ¹H NMR (400 MHz, DMSO) δ = 8.71 (d, J = 8.0 Hz, 2H), 7.85 (s, 1H), 7.78 - 7.73 (m, 2H), 7.55 (s, 1H), 7.11 (d, J = 8.0 Hz, 2H), 6.78 (d, J = 8.0 Hz, 2H), 6.41 (q, J = 8.0 Hz, 1H), 4.65 (q, J = 8.0 Hz, 1H), 3.59 (s, 3H), 3.17 (s, 3H), 2.86 (s, 2.5H), 2.74 - 2.67 (m, 1H), 2.62 (s, 0.5H), 2.33 - 2.29 (m, 1H), 1.96 - 1.87 (m, 2H), 1.83 - 1.69 (m, 2H), 1.47 - 1.36 (m, 4H), 1.33 (d, J = 4.0 Hz, 3H).

### Synthesis of methyl (1S,4r)-4-(methyl((S)-2,2,2-trifluoro-1-(4-((5-((S)-1-methoxyethyl)-1-(pyridin-3-yl)-1H-pyrazol-4-yl)amino)phenyl)ethyl)carbamoyl)cyclohexane-1-carboxylate [Intermediate 6.8]:

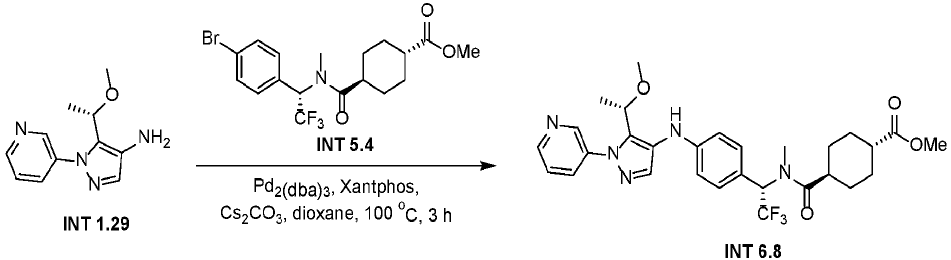

A mixture of 5-[(1S)-1-methoxyethyl]-1-(pyridin-3-yl)-1H-pyrazol-4-amine [free base of **INT 1.29]** (20 mg, 91.6 µmol), methyl (1r,4r)-4-{[(1S)-1-(4-bromophenyl)-2,2,2-trifluoroethyl](methyl)carbamoyl}cyclohexane-1-carboxylate **[INT 5.4]** (39.9 mg, 91.6 µmol), xantphos (5.30 mg, 9.16 µmol), Pd₂(dba)₃ (8.38 mg, 9.16 µmol) and Cs₂CO₃ (89.2 mg, 274 µmol) in dioxane (1 mL) was stirred at 100 °C for 3 hr under N₂ atmosphere. The mixture was concentrated under reduced pressure to afford methyl (1S,4r)-4-(methyl((S)-2,2,2-trifluoro-1-(4-((5-((S)-1-methoxyethyl)-1-(pyridin-3-yl)-1H-pyrazol-4-yl)amino)phenyl)ethyl)carbamoyl)cyclohexane-1-carboxylate **[INT 6.8]** (50.0 mg, 87.1 µmol, 95.2% yield). m/z: [M + H]+ Calcd for C29H35F3N5O4 574.3; Found 574.2.

### Synthesis of methyl (1S,4r)-4-(methyl((S)-2,2,2-trifluoro-1-(4-((1-(pyridazin-3-yl)-5-(trifluoromethyl)-1H-pyrazol-4-yl)amino)phenyl)ethyl)carbamoyl)cyclohexane-1-carboxylate [Intermediate 6.9]:

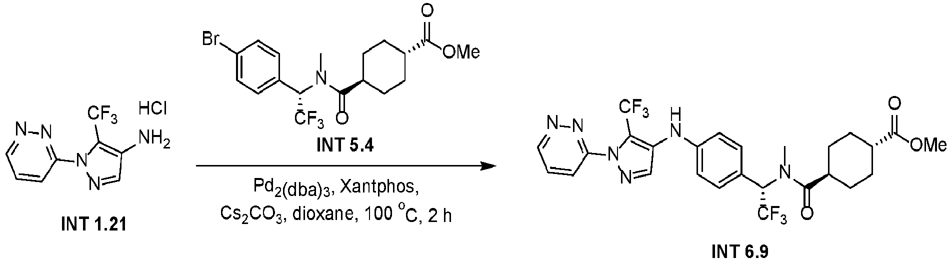

A suspension of 1-(pyridazin-3-yl)-5-(trifluoromethyl)-1H-pyrazol-4-amine hydrochloride **[INT 1.21]** (500 mg, 1.88 mmol), methyl (1r,4r)-4-{[(1S)-1-(4-bromophenyl)-2,2,2-trifluoroethyl](methyl)carbamoyl}cyclohexane-1-carboxylate **[INT 5.4]** (820 mg, 1.88 mmol), Cs₂CO₃ (1.83 g, 5.64 mmol), Pd₂(dba)₃ (86.0 mg, 94.0 µmol) and xantphos (108 mg, 188 µmol) in dioxane (10 mL) was stirred at 100 °C for 2 h under N₂. The reaction mixture was concentrated to provide methyl (1S,4r)-4-(methyl((S)-2,2,2-trifluoro-1-(4-((1-(pyridazin-3-yl)-5-(trifluoromethyl)-1H-pyrazol-4-yl)amino)phenyl)ethyl)carbamoyl)cyclohexane-1-carboxylate **[INT 6.9].** m/z: [M + H]+ Calcd for C26H27F6N6O3 585.2; Found 585.1.

### Synthesis of methyl (1r,4r)-4-{[(1S)-1-(4-{[1-(3-cyanophenyl)-5-[(1S)-1-methoxyethyl]-1H-pyrazol-4-yl]amino}phenyl)-2,2,2-trifluoroethyl](methyl)carbamoyl}cyclohexane-1-carboxylate [Intermediate 6.10]:

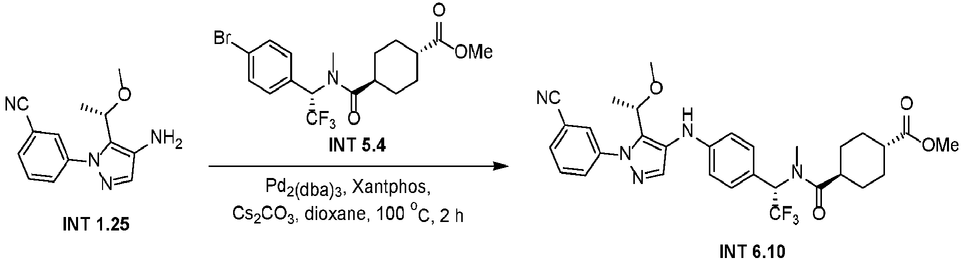

To a mixture of 3-{4-amino-5-[(1S)-1-methoxyethyl]-1H-pyrazol-1-yl}benzonitrile **[INT 1.25]** (160 mg, 660 µmol) in dioxane (5 mL) was added methyl (1r,4r)-4-{[(1S)-1-(4-bromophenyl)-2,2,2-trifluoroethyl](methyl)carbamoyl}cyclohexane-1-carboxylate **[INT 5.4]** (316 mg, 726 µmol), xantphos (114 mg, 198 µmol), Cs₂CO₃ (645 mg, 1.98 mmol) and Pd₂(dba)₃ (60.4 mg, 66.0 µmol). The reaction was stirred at 100°C for 2 h under N₂. The mixture was concentrated under reduced pressure to give the crude product, which was purified by flash chromatography on silica gel (EtOAc/PE = 0/1 to 1/1) to give methyl (1r,4r)-4-{[(1S)-1-(4-{[1-(3-cyanophenyl)-5-[(1S)-1-methoxyethyl]-1H-pyrazol-4-yl]amino}phenyl)-2,2,2-trifluoroethyl](methyl)carbamoyl}cyclohexane-1-carboxylate **[INT 6.10]** (250 mg, 418 µmol, 63.4% yield) as a yellow solid. m/z: [M + H]+ Calcd for C31H35F3N5O4 598.3; Found 598.1.

### Synthesis of (1r,4r)-4-cyano-N-methyl-N-[(1S)-2,2,2-trifluoro-1-(4-{[1-phenyl-5-(trifluoromethyl)-1H-pyrazol-4-yl]amino}phenyl)ethyl]cyclohexane-1-carboxamide [Intermediate 6.11]:

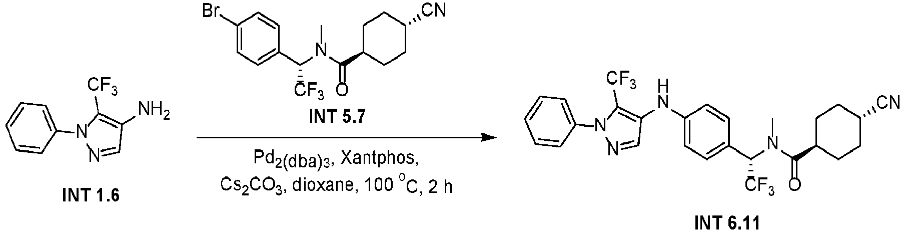

To a mixture of 1-phenyl-5-(trifluoromethyl)-1H-pyrazol-4-amine [free base **of INT 1.6]** (100 mg, 440 µmol) in dioxane (5 mL) was added (1r,4r)-N-[(1S)-1-(4-bromophenyl)-2,2,2-trifluoroethyl]-4-cyano-N-methylcyclohexane-1-carboxamide **[INT 5.6]** (195 mg, 484 µmol), xantphos (76.3 mg, 132 µmol), Cs₂CO₃ (430 mg, 1.32 mmol) and Pd₂(dba)₃ (40.2 mg, 44.0 µmol). The reaction was stirred at 100 °C for 2 h under N₂. The reaction mixture was concentrated under reduced pressure to give the crude product, which was purified by flash chromatography on silica gel (EtOAc/PE = 0/1 to 1/3) to give (1r,4r)-4-cyano-N-methyl-N-[(1S)-2,2,2-trifluoro-1-(4-{[1-phenyl-5-(trifluoromethyl)-1H-pyrazol-4-yl]amino}phenyl)ethyl]cyclohexane-1-carboxamide **[INT 6.11]** (100 mg, 181 µmol, 41.4% yield) as a yellow solid. m/z: [M + H]+ Calcd for C27H26F6N5O 550.2; Found 550.4.

### Synthesis of benzyl 3-{methyl[(1S)-2,2,2-trifluoro-1-(4-{[1-(pyridin-2-yl)-5-(trifluoromethyl)-1H-pyrazol-4-yl]amino}phenyl)ethyl]carbamoyl}cyclobutane-1-carboxylate [Intermediate 6.12]:

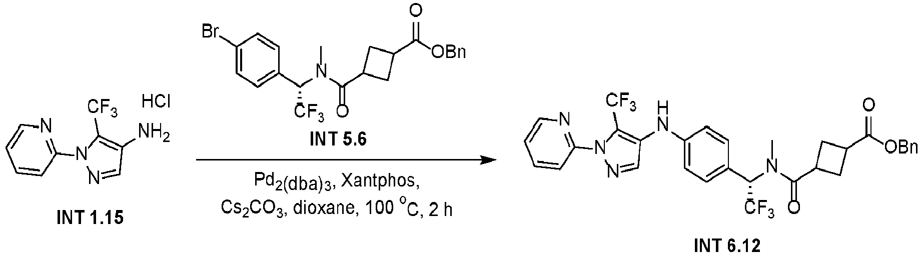

A mixture of benzyl 3-{[(1S)-1-(4-bromophenyl)-2,2,2-trifluoroethyl](methyl)carbamoyl}cyclobutane-1-carboxylate **[INT 5.6]** (300 mg, 619 µmol), 1-(pyridin-2-yl)-5-(trifluoromethyl)-1H-pyrazol-4-amine hydrochloride [HCl salt **of INT 1.15]** (163 mg, 619 µmol), Pd₂(dba)₃ (56.6 mg, 61.9 µmol), xantphos (71.1 mg, 123 µmol) and Cs₂CO₃ (602 mg, 1.85 mmol) in dioxane (10 mL) was stirred at 100 °C for 2 hours under N₂ atmosphere. The reaction was quenched by adding water (40 mL) and was extracted with EtOAc (2 x 80 mL). The combined organic layers were washed with brine (2 x 50 mL), dried over anhydrous Na₂SO₄ and concentrated under reduced pressure to give benzyl 3-{methyl[(1S)-2,2,2-trifluoro-1-(4-{[1-(pyridin-2-yl)-5-(trifluoromethyl)-1H-pyrazol-4-yl]amino}phenyl)ethyl]carbamoyl}cyclobutane-1-carboxylate **[INT 6.12]** (390 mg, 617 µmol) as a black oil. m/z: [M + H]+ Calcd for C31H28F6N5O3 632.2; Found 632.3.

### Synthesis of benzyl 3-{methyl[(1S)-2,2,2-trifluoro-1-(4-{[1-phenyl-5-(trifluoromethyl)-1H-pyrazol-4-yl]amino}phenyl)ethyl]carbamoyl}cyclobutane-1-carboxylate [Intermediate 6.13]:

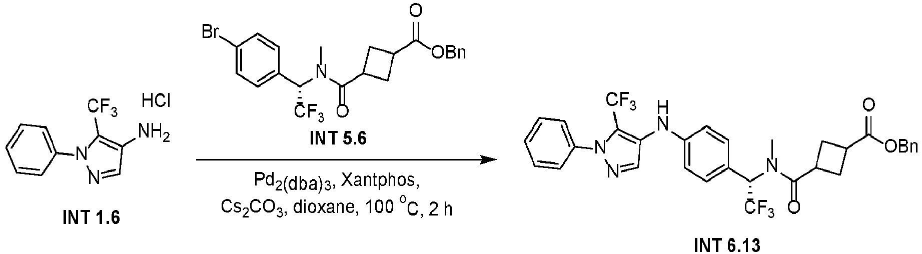

A mixture of benzyl 3-{[(1S)-1-(4-bromophenyl)-2,2,2-trifluoroethyl](methyl)carbamoyl}cyclobutane-1-carboxylate **[INT 5.6]** (650 mg, 805 µmol), 1-phenyl-5-(trifluoromethyl)-1H-pyrazol-4-amine hydrochloride **[INT 1.6]** (150 mg, 568 µmol), Pd₂(dba)₃ (52.0 mg, 56.8 µmol), xantphos (65.3 mg, 113 µmol) and Cs₂CO₃ (553 mg, 1.70 mmol) in dioxane (10 mL) was stirred at 100 °C for 2 hours under N₂ atmosphere. The reaction was quenched by adding water (50 mL) and was extracted with EtOAc (2 x 80 mL). The combined organic layers were washed with brine (60 mL), dried over anhydrous Na₂SO₄ and concentrated under reduced pressure to give benzyl 3-{methyl[(1S)-2,2,2-trifluoro-1-(4-{[1-phenyl-5-(trifluoromethyl)-1H-pyrazol-4-yl]amino}phenyl)ethyl]carbamoyl}cyclobutane-1-carboxylate **[INT 6.13]** (330 mg, 523 µmol, 92.1% yield) as a brown oil. m/z: [M + H]+ Calcd for C32H29F6N4O3 631.2; Found 631.2.

### Exemplary Compounds of Formula (I)

The following compounds in **Table 2** were synthesized according to **Schemes 1-3** as described above with the identified intermediates.

**Table 2.**

| **Cmpd No.** | **Structure** | **Scheme** | **Intermed.** | **LCMS** | **¹H NMR** |
|---|---|---|---|---|---|
| 1.1 | 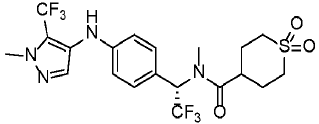 | 1 | INT 1.1 | [M + H]+ C20H23F 6N4O3S | (400 MHz, CD₃OD) δ = 7.55 (s, 1H), 7.19 (d, J=8.4 Hz, 2H), 6.82 (d, J=8.4 Hz, 2H), 6.57 - 5.95 (m, 1H), 4.00 (s, 3H), 3.30 - 3.09 (m, 5H), 2.98 (s, 3H), 2.39 - 2.10 (m, 4H) |
| | | | INT 2.1 | | |
| | | | INT 3.1 | 513.1 | |
| | | | INT 4.1 | 513.2 | |
| | | | INT 5.1 | | |
| 1.2 | 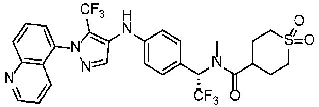 | 1 | INT 1.2 | [M +H]+ C28H26F 6N5O3S | (400 MHz, CDCl₃) δ = 9.01 (dd, J=1.6, 4.0 Hz, 1H), 8.33 (d, J=8.8 Hz, 1H), 7.95 (s, 1H), 7.83 (t, J=8.0 Hz, 1H), 7.67 (br d, J=6.8 Hz, 2H), 7.47 (dd, J=4.0, 8.4 Hz, 1H), 7.31 (br d, J=8.0 Hz, 2H), 7.02 (d, J=8.8 Hz, 2H), 6.60 (q, J=8.8 Hz, 1H), 5.64 (s, 1H), 3.47 (br d, J=10.9 Hz, 1H), 3.35 (br dd, J=2.9, 8.1 Hz, 1H), 3.06 - 2.98 (m, 2H), 2.95 (s, 4H), 251 - 232 (m, 3H), 2.31 - 2.21 (m, 1H) |
| | | | INT 2.1 | | |
| | | | INT 3.1 | 626.2 | |
| | | | INT 4.1 | 626.3 | |
| | | | INT 5.1 | | |
| 1.3 | 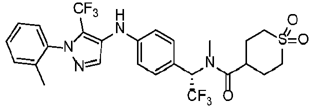 | 1 | INT 1.3 | [M+H]+ C26H27F 6N4O3S | (400 MHz, DMSO-d6) δ = 7.99 - 7.92 (m, 2H), 7.53 - 7.47 (m, 1H), 7.47 - 7.43 (m, 1H), 7.43 - 7.36 (m, 2H), 7.29 - 7.16 (m, 2H), 6.94 - 6.86 (m, 2H), 6.48 - 6.05 (m, 1H), 3.33 - 3.06 (m, 6H), 2.93 - 2.64 (m, 3H), 2.12 - 2.02 (m, 3H), 2.01 (s, 3H) |
| | | | INT 2.1 | | |
| | | | INT 3.1 | 589.2 | |
| | | | INT 4.1 | 589.3 | |
| | | | INT 5.1 | | |
| 1.4 | 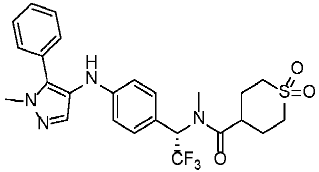 | 1 | INT 1.4 | [M + H]+ C25H28F 3N4O3S | (400 MHz, DMSO-d6) δ = 7.55 - 7.33 (m, 7H), 7.02 (d, J=8.4 Hz, 2H), 6.61 (d, J=8.8 Hz, 2H), 6.39 - 5.92 (m, 1H), 3.79 (s, 3H), 3.28 - 3.06 (m, 5H), 2.86 - 2.57 (m, 3H), 2.10 - 1.91 (m, 4H) |
| | | | INT 2.1 | | |
| | | | INT 3.1 | 521.2 | |
| | | | INT 4.1 | 521.2 | |
| | | | INT 5.1 | | |
| 1.5 | 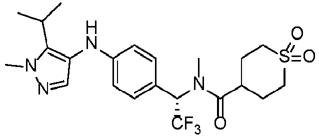 | 1 | INT 1.5 | [M + H]+ C22H30F 3N4O3S | (400 MHz, DMSO-d6) δ = 7.22 - 7.19 (m, 1H), 7.19 - 7.15 (m, 1H), 7.14 - 7.02 (m, 2H), 6.62 - 6.53 (m, 2H), 6.42 - 5.97 (m, 1H), 3.77 (s, 3H), 3.25 - 3.08 (m, 5H), 3.07 - 3.02 (m, 1H), 2.87 (s, 3H), 2.14 - 1.91 (m, 4H), 1.19 (d, J=7.2 Hz, 6H) |
| | | | INT 2.1 | | |
| | | | INT 3.1 | 487.2 | |
| | | | INT 4.1 | 487.1 | |
| | | | INT 5.1 | | |
| 1.6 | 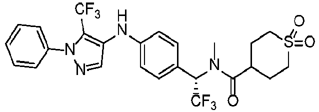 | 1 | INT 1.6 | [M + H]+ C25H25F 6N4O3S | (400 MHz, CDCl₃) δ = 7.80 (s, 1H), 7.56 - 7.46 (m, 5H), 7.28 (br s, 1H), 7.26 (br s, 1H), 6.95 (d, J=8.8 Hz, 2H), 6.58 (q, J=9.2 Hz, 1H), 5.53 (s, 1H), 3.47 (br d, J=11.2 Hz, 1H), 3.40 - 3.32 (m, 1H), 3.07 - 2.94 (m, 3H), 2.93 (s, 3H), 2.51 - 2.32 (m, 3H), 2.32 - 2.21 (m, 1H) |
| | | | INT 2.1 | | |
| | | | INT 3.1 | 575.1 | |
| | | | INT 4.1 | 575.1 | |
| | | | INT 5.1 | | |
| 1.7 | 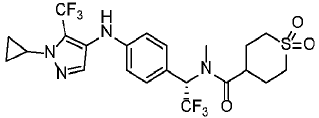 | 1 | INT 1.7 | [M + H]+ C22H25F 6N4O3S | (400MHz, CDCl₃) δ = 7.50 (s, 1H), 7.22 (d, J=8.4 Hz, 2H), 6.84 (d, J=8.8 Hz, 2H), 6.55 (q, J=8.8 Hz, 1H), 5.40 (s, 1H), 3.62 (tt, J=3.6, 7.2 Hz, 1H), 3.46 (br d, J=10.8 Hz, 1H), 3.40 - 3.30 (m, 1H), 3.04 - 2.92 (m, 3H), 2.91 (s, 3H), 2.48 - 2.33 (m, 3H), 2.30 - 2.19 (m, 1H), 1.36 - 1.27 (m, 2H), 1.14 - 1.06 (m, 2H) |
| | | | INT 2.1 | | |
| | | | INT 3.1 | 539.1 | |
| | | | INT 4.1 | 539.3 | |
| | | | INT 5.1 | | |
| 1.8 | 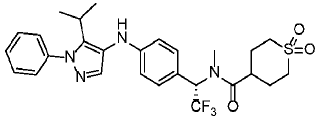 | 1 | INT 1.8 | [M + H]+ C27H32F 3N4O3S | (400 MHz, CDCl₃) δ = 7.56 - 7.40 (m, 6H), 7.17 (d, J=8.4 Hz, 2H), 6.70 (d, J=8.8 Hz, 2H), 6.54 (q, J=9.2 Hz, 1H), 4.96 (s, 1H), 3.53 - 3.43 (m, 1H), 3.42 - 3.32 (m, 1H), 3.13 - 3.04 (m, 1H), 3.04 - 2.89 (m, 6H), 2.49 - 2.31 (m, 3H), 2.30 - 2.19 (m, 1H), 1.25 (d, J=7.2 Hz, 6H) |
| | | | INT 2.1 | | |
| | | | INT 3.1 | 549.2 | |
| | | | INT 4.1 | 549.2 | |
| | | | INT 5.1 | | |
| 1.9 | 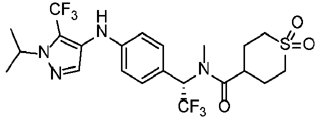 | 1 | INT 1.9 | [M + H]+ C22H27F 6N4O3S | (400 MHz, DMSO-d6) δ = 7.78 - 7.66 (m, 2H), 7.25 - 7.08 (m, 2H), 6.82 - 6.73 (m, 2H), 6.47 - 6.03 (m, 1H), 4.59 (td, J=6.4, 12.8 Hz, 1H), 3.30 - 3.03 (m, 5H), 2.91 - 2.61 (m, 3H), 2.13 - 1.92 (m, 4H), 1.45 (d, J=6.4 Hz, 6H) |
| | | | INT 2.1 | | |
| | | | INT 3.1 | 541.2 | |
| | | | INT 4.1 | 541.2 | |
| | | | INT 5.1 | | |
| 1.10 | 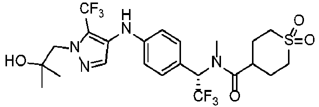 | 1 | INT 1.10 | [M + H]+ C23H29F 6N4O4S | (400MHz, DMSO-d6) δ = 7.81 - 7.60 (m, 2H), 7.25 - 7.07 (m, 2H), 6.83 - 6.69 (m, 2H), 6.46 - 5.97 (m, 1H), 4.75 (s, 1H), 4.11 (s, 2H), 3.28 - 3.03 (m, 5H), 2.94 - 2.59 (m, 3H), 2.16 - 1.89 (m, 4H), 1.13 (s, 6H) |
| | | | INT 2.1 | | |
| | | | INT 3.1 | 571.2 | |
| | | | INT 4.1 | 571.2 | |
| | | | INT 5.1 | | |
| 1.11 | 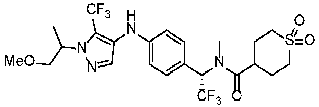 | 1 | INT 1.11 | [M + H]+ C23H29F 6N4O4S | (400 MHz, CDCl₃) δ = 7.63 (s, 1H), 7.21 (d, J=8.4 Hz, 2H), 6.84 (d, J=8.8 Hz, 2H), 6.55 (q, J=9.2 Hz, 1H), 5.38 (br s, 1H), 4.75 - 4.60 (m, 1H), 3.90 (dd, J=8.4, 9.6 Hz, 1H), 3.62 (dd, J=5.2, 10.0 Hz, 1H), 3.53 - 3.43 (m, 1H), 3.39 - 3.32 (m, 4H), 3.04 - 2.88 (m, 6H), 2.48 - 2.30 (m, 3H), 2.30 - 2.19 (m, 1H), 1.50 (d, J=6.8 Hz, 3H) |
| | | | INT 2.1 | | |
| | | | INT 3.1 | 571.2 | |
| | | | INT 4.1 | 571.1 | |
| | | | INT 5.1 | | |
| 1.12 | 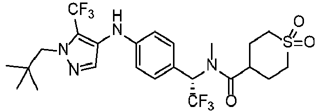 | 1 | INT 1.12 | [M + H]+ C24H31F 6N4O3S | 400MHz, DMSO) δ = 7.78 - 7.69 (m, 2H), 7.24 - 7.10 (m, 2H), 6.80 - 6.71 (m, 2H), 6.38 (q, J=8.0 Hz, 1H), 4.00 (s, 2H), 3.26 - 3.05 (m, 5H), 2.88 (s, 2.5H), 2.62 (s, 0.5H), 2.13 - 1.92 (m, 4H), 0.96 (s, 9H) |
| | | | INT 2.1 | | |
| | | | INT 3.1 | 569.2 | |
| | | | INT 4.1 | 569.2 | |
| | | | INT 5.1 | | |
| 1.13 | 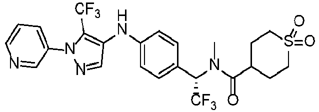 | 1 | INT 1.13 | [M + H]+ C24H24F 6N5O3S | (400 MHz, CDCl₃) δ = 8.79 (d, J=2.0 Hz, 1H), 8.74 (d, J=4.0 Hz, 1H), 7.86 (s, 1H), 7.83 (br d, J=8.0 Hz, 1H), 7.48 (dd, J=4.8, 8.0 Hz, 1H), 7.29 (s, 1H), 6.97 (d, J=8.4 Hz, 2H), 6.66 - 6.19 (m, 1H), 5.62 (s, 1H), 3.53 - 3.43 (m, 1H), 3.40 - 3.31 (m, 1H), 3.06 - 2.97 (m, 2H), 2.96 - 2.91 (m, 4H), 2.50 - 2.31 (m, 3H), 2.30 - 2.20 (m, 1H) |
| | | | INT 2.1 | | |
| | | | INT 3.1 | 576.1 | |
| | | | INT 4.1 | 576.2 | |
| | | | INT 5.1 | | |
| 1.14 | 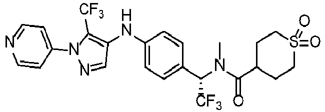 | 1 | INT 1.14 | [M + H]+ C24H24F 6N5O3S | (400 MHz, CDCl₃) δ = 8.82 - 8.74 (m, 2H), 8.21 (s, 1H), 8.11 (s, 1H), 7.61 (d, J=8.0 Hz, 2H), 7.20 (d, J=8.0 Hz, 2H), 6.97 (d, J=8.0 Hz, 2H), 6.27 (q, J=8.0 Hz, 1H), 3.28 - 3.06 (m, 5H), 2.90 (s, 2.5H), 2.64 (s, 0.5H), 2.14 - 1.93 (m, 4H) |
| | | | INT 2.1 | | |
| | | | INT 3.1 | 576.1 | |
| | | | INT 4.1 | 576.2 | |
| | | | INT 5.1 | | |
| 1.15 | 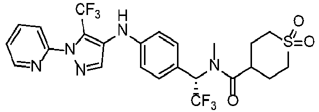 | 1 | INT 1.15 | [M + H]+ C24H24F 6N5O3S | (400 MHz, DMSO-d6) δ = 8.54 (td, J=0.8, 4.8 Hz, 1H), 8.20 - 8.03 (m, 3H), 7.81 (d, J=8.0 Hz, 1H), 7.54 - 7.48 (m, 1H), 7.30 - 7.19 (m, 2H), 7.04 - 6.90 (m, 2H), 6.43 (q, J=9.2 Hz, 1H), 3.31 - 3.07 (m, 5H), 2.94 - 2.64 (m, 3H), 2.16 - 2.08 (m, 1H), 2.07 - 1.94 (m, 3H) |
| | | | INT 2.1 | | |
| | | | INT 3.1 | 576.1 | |
| | | | INT 4.1 | 576.1 | |
| | | | INT 5.1 | | |
| 1.16 | 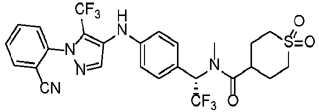 | 1 | INT 1.16 | [M + H]+ C26H24F 6N5O3S | (400 MHz, DMSO-d6) δ = 8.18 - 8.08 (m, 3H), 8.00 - 7.92 (m, 1H), 7.88 - 7.80 (m, 2H), 7.20 (br d, J=8.4 Hz, 2H), 6.93 (d, J=8.8 Hz, 2H), 6.50 - 6.00 (m, 1H), 3.27 - 3.10 (m, 5H), 2.90 (s, 3H), 2.11 - 1.97 (m, 4H) |
| | | | INT 2.1 | | |
| | | | INT 3.1 | 600.1 | |
| | | | INT 4.1 | 600.1 | |
| | | | INT 5.1 | | |
| 1.17 | 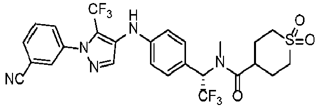 | 1 | INT 1.17 | [M + H]+ C26H24F 6N5O3S | (400 MHz, DMSO-d6) δ = 8.11 (d, J=9.6 Hz, 2H), 8.07 - 8.00 (m, 2H), 7.92 (br d, J=8.4 Hz, 1H), 7.84 - 7.76 (m, 1H), 7.30 - 7.14 (m, 2H), 7.02 - 6.89 (m, 2H), 6.49 - 6.05 (m, 1H), 3.31 - 3.06 (m, 5H), 2.95 - 2.60 (m, 3H), 2.16 - 1.92 (m, 4H) |
| | | | INT 2.1 | | |
| | | | INT 3.1 | 600.1 | |
| | | | INT 4.1 | 600.1 | |
| | | | INT 5.1 | | |
| 1.18 | 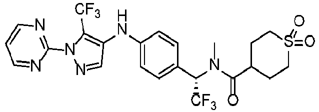 | 1 | INT 1.18 | [M + H]+ C23H23F 6N6O3S | (400 MHz, DMSO) δ = 8.94 (d, J=4.0 Hz, 2H), 8.77 (s, 1H), 7.87 (s, 1H), 7.60 (t, J=4.0 Hz, 1H), 7.18 (d, J=8.0 Hz, 2H), 6.94 (d, J=8.0 Hz, 2H), 6.26 (q, J=8.0 Hz, 1H), 3.25 - 3.07 (m, 5H), 2.89 (s, 2.5H), 2.64 (s, 0.5H), 2.12 - 1.94 (m, 4H) |
| | | | INT 2.1 | | |
| | | | INT 3.1 | 577.1 | |
| | | | INT 4.1 | 577.2 | |
| | | | INT 5.1 | | |
| 1.19 | 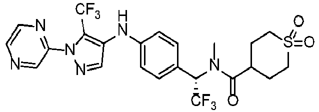 | 1 | INT 1.19 | [M + H]+ C23H23F 6N6O3S | (400 MHz, DMSO-d6) δ = 9.14 (d, J=1.2 Hz, 1H), 8.75 (d, J=2.8 Hz, 1H), 8.67 - 8.59 (m, 1H), 8.33 - 8.22 (m, 1H), 8.18 (s, 1H), 7.34 - 7.14 (m, 2H), 7.04 - 6.93 (m, 2H), 6.50 - 6.07 (m, 1H), 3.30 - 3.05 (m, 5H), 2.92 - 2.63 (m, 3H), 2.14 - 1.93 (m, 4H) |
| | | | INT 2.1 | | |
| | | | INT 3.1 | | |
| | | | INT 4.1 | 577.1 | |
| | | | INT 5.1 | 577.2 | |
| 1.20 | 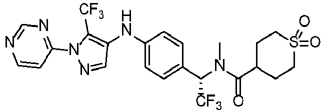 | 1 | INT 1.20 | [M + H]+ C23H23F 6N6O3S | (400 MHz, CDCl₃) δ = 9.09 (s, 1H), 8.82 (d, J=5.6 Hz, 1H), 7.92 (s, 1H), 7.87 (dd, J=1.2, 5.6 Hz, 1H), 7.30 (d, J=8.4 Hz, 2H), 7.02 (d, J=8.4 Hz, 2H), 6.59 (q, J=8.8 Hz, 1H), 5.89 (s, 1H), 3.53 - 3.42 (m, 1H), 3.40 - 3.30 (m, 1H), 3.05 - 2.94 (m, 3H), 2.93 (s, 3H), 2.50 - 2.21 (m, 4H) |
| | | | INT 2.1 | | |
| | | | INT 3.1 | 577.1 | |
| | | | INT 4.1 | 577.3 | |
| | | | INT 5.1 | | |
| 1.21 | 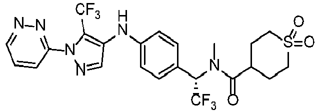 | 1 | INT 1.21 | [M + H]+ C23H23F 6N6O3S | (400 MHz, CDCl₃) δ = 9.23 (d, J=4.8 Hz, 1H), 8.05 (d, J=8.8 Hz, 1H), 7.96 (s, 1H), 7.70 (dd, J=4.8, 8.8 Hz, 1H), 7.31 (br d, J=8.4 Hz, 2H), 7.03 (d, J=8.4 Hz, 2H), 6.60 (q, J=8.8 Hz, 1H), 5.83 (br s, 1H), 3.58 - 3.30 (m, 2H), 3.08 - 2.91 (m, 6H), 2.51 - 2.24 (m, 4H) |
| | | | INT 2.1 | | |
| | | | INT 3.1 | 577.1 | |
| | | | INT 4.1 | 577.3 | |
| | | | INT 5.1 | | |
| 1.22 | 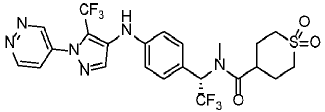 | 1 | INT 1.22 | [M + H]+ C23H23F 6N6O3S | (400MHz, DMSO-d6) δ = 9.55 (d, J=2.4 Hz, 1H), 9.47 (d, J=5.6 Hz, 1H), 8.42 - 8.32 (m, 1H), 8.23 (s, 1H), 7.94 - 7.89 (m, 1H), 7.32 - 7.18 (m, 2H), 7.08 - 6.96 (m, 2H), 6.50 - 6.07 (m, 1H), 3.28 - 3.08 (m, 5H), 2.94 - 2.64 (m, 3H), 2.14 - 1.95 (m, 4H) |
| | | | INT 2.1 | | |
| | | | INT 3.1 | 577.1 | |
| | | | INT 4.1 | 577.2 | |
| | | | INT 5.1 | | |
| 1.23 | 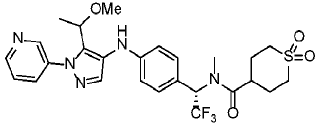 | 1 | INT 1.23 | [M + H]+ C26H31F 3N5O4S | (400 MHz, CDCl₃) δ = 8.79 (br s, 1H), 8.70 (br d, J=4.0 Hz, 1H), 7.93 (br d, J=7.6 Hz, 1H), 7.79 (s, 1H), 7.51 (dd, J=4.8, 7.6 Hz, 1H), 7.22 (br d, J=8.4 Hz, 2H), 6.86 (d, J=8.4 Hz, 2H), 6.55 (q, J=9.2 Hz, 1H), 5.67 (br s, 1H), 4.52 (q, J=6.8 Hz, 1H), 3.54 - 3.43 (m, 1H), 3.40 - 3.30 (m, 1H), 3.26 (s, 3H), 3.06 - 2.88 (m, 6H), 2.50 - 2.30 (m, 3H), 2.25 (br s, 1H), 1.46 (d, J=6.8 Hz, 3H) |
| | | | INT 2.1 | | |
| | | | INT 3.1 | 566.2 | |
| | | | INT 4.1 | 566.4 | |
| | | | INT 5.1 | | |
| 1.24 | 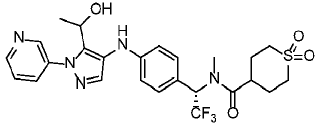 | 1 | INT 1.24 | [M + H]+ C25H29F 3N5O4S | (400 MHz, CDCl₃) δ = 8.79 (d, J=2.4 Hz, 1H), 8.68 (dd, J=1.2, 4.8 Hz, 1H), 7.96 - 7.89 (m, 1H), 7.72 (s, 1H), 7.48 (dd, J=4.8, 8.0 Hz, 1H), 7.22 (br d, J=8.4 Hz, 2H), 6.84 (d, J=8.8 Hz, 2H), 6.55 (q, J=9.2 Hz, 1H), 5.63 (d, J=2.0 Hz, 1H), 5.03 (q, J=6.4 Hz, 1H), 3.56 - 3.29 (m, 2H), 3.04 - 2.96 (m, 2H), 2.92 (s, 3H), 2.58 - 2.18 (m, 5H), 1.54 (dd, J=1.2, 6.8 Hz, 3H) |
| | | | INT 2.1 | | |
| | | | INT 3.1 | 552.2 | |
| | | | INT 4.1 | 552.2 | |
| | | | INT 5.1 | | |
| 1.25 | 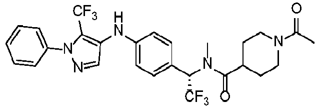 | 1 | INT 1.6 | [M + H]+ C27H28F 6N5O2 | (400 MHz, CD₃OD) δ = 7.84 (s, 1H), 7.62 - 7.55 (m, 3H), 7.54 - 7.50 (m, 2H), 7.33 - 7.21 (m, 2H), 7.01 - 6.92 (m, 2H), 6.51 (q, J=9.2 Hz, 1H), 4.61 (br s, 1H), 4.56 (br d, J=12.8 Hz, 1H), 4.00 (br d, J=10.8 Hz, 1H), 3.25 - 2.97 (m, 4H), 2.82 - 2.73 (m, 1H), 2.13 (d, J=1.6 Hz, 3H), 1.94 - 1.60 (m, 4H) |
| | | | INT 2.1 | | |
| | | | INT 3.1 | 568.2 | |
| | | | INT 4.2 | 568.2 | |
| | | | INT 5.2 | | |
| 1.26 | 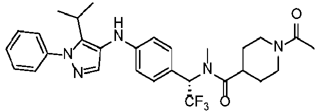 | 1 | INT 1.8 | [M + H]+ C29H35F 3N5O2 | (400 MHz, CDCl₃) δ = |
| | | | INT 2.1 | | 7.57 - 7.39 (m, |
| | | | INT 3.1 | 542.3 | 6H), 7.22 - |
| | | | INT 4.2 | 542.2 | 7.12 (m, 2H), 6.69 (d, J=8.4 |
| | | | INT 5.2 | | Hz, 2H), 6.55 (q, J=9.2 Hz, 1H), 5.13 - 4.86 (m, 1H), 4.66 - 4.56 (m, 1H), 3.91 (br d, J=13.6 Hz, 1H), 3.22 - 3.01 (m, 2H), 2.92 (d, J=2.0 Hz, 3H), 2.85 - 2.63 (m, 2H), 2.12 (s, 3H), 1.91 - 1.69 (m, 4H), 1.25 (s, 3H), 1.24 (s, 3H) |
| 1.27 | 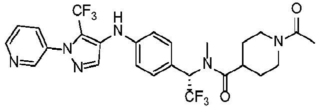 | 1 | INT 1.13 | [M + H]+ C26H27F 6N6O2 | (400 MHz, DMSO-d6) δ = 8.86 - 8.72 (m, 2H), 8.15 - 8.00 (m, 3H), 7.65 (dd, J=4.8, 8.0 Hz, 1H), 7.28 - 7.13 (m, 2H), 7.03 - 6.90 (m, 2H), 6.51 - 6.17 (m, 1H), 4.37 (br d, J=12.8 Hz, 1H), 3.83 (br d, J=10.4 Hz, 1H), 3.18 - 2.95 (m, 2H), 2.91 (s, 2H), 2.69 - 2.61 (m, 1H), 2.59 (br s, 1H), 2.00 (s, 3H), 1.81 - 1.62 (m, 2H), 1.60 - 1.29 (m, 2H) |
| | | | INT 2.1 | | |
| | | | INT 3.1 | 569.2 | |
| | | | INT 4.2 | 569.1 | |
| | | | INT 5.2 | | |
| 1.28 | 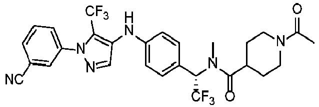 | 1 | INT 1.17 | [M + H]+ C28H27F 6N6O2 | (400 MHz, DMSO-d6) δ = 8.11 (br d, J=13.6 Hz, 2H), 8.07 - 8.00 (m, 2H), 7.92 (br d, J=8.4 Hz, 1H), 7.85 - 7.75 (m, 1H), 7.30 - 7.13 (m, 2H), 7.03 - 6.88 (m, 2H), 6.53 - 6.14 (m, 1H), 4.37 (br d, J=12.4 Hz, 1H), 3.83 (br d, J=11.6 Hz, 1H), 3.17 - 2.94 (m, 2H), 2.94 - 2.60 (m, 4H), 2.00 (s, 3H), 1.83 - 1.60 (m, 2H), 1.59 - 1.29 (m, 2H) |
| | | | INT 2.1 | | |
| | | | INT 3.1 | 593.2 | |
| | | | INT 4.2 | 593.1 | |
| | | | INT 5.2 | | |
| 1.29 | 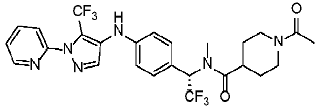 | 1 | INT 1.15 | [M + H]+ C26H27F 6N6O2 | (400 MHz, DMSO-d6) δ = 8.56 - 8.51 (m, 1H), 8.17 - 8.12 (m, 1H), 8.11 - 8.05 (m, 2H), 7.81 (d, J=8.0 Hz, 1H), 7.51 (ddd, J=0.8, 4.8, 7.2 Hz, 1H), 7.33 - 7.17 (m, 2H), 7.02 - 6.92 (m, 2H), 6.52 - 6.16 (m, 1H), 4.38 (br d, J=13.2 Hz, 1H), 3.84 (br d, J=11.6 Hz, 1H), 3.20 - 3.07 (m, 1H), 3.06 - 2.96 (m, 1H), 2.93 - 2.59 (m, 4H), 2.00 (s, 3H), 1.81 - 1.63 (m, 2H), 1.60 - 1.47 (m, 1H), 1.38 (dquin, J=4.0, 12.0 Hz, 1H) |
| | | | INT 2.1 | | |
| | | | INT 3.1 | 569.2 | |
| | | | INT 4.2 | 569.2 | |
| | | | INT 5.2 | | |
| 1.30 | 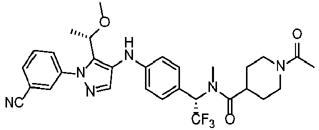 | 1 | INT 1.25 | [M - OCH3]+ C29H30F 3N6O2 | (400 MHz, DMSO-d6) δ = 8.10 - 8.05 (m, 1H), 8.00 - 7.90 (m, 2H), 7.81 - 7.69 (m, 2H), 7.55 - 7.46 (m, 1H), 7.22 - 7.06 (m, 2H), 6.86 - 6.72 (m, 2H), 6.47 - 6.09 (m, 1H), 4.53 (q, J=6.8 Hz, 1H), 4.47 - 4.31 (m, 1H), 3.82 (d, J=10.8 Hz, 1H), 3.15 - 2.92 (m, 5H), 2.92 - 2.55 (m, 4H), 1.99 (s, 3H), 1.80 - 1.60 (m, 2H), 1.58 - 1.31 (m, 2H), 1.27 (d, J=6.8 Hz, 3H) |
| | | | INT 2.1 | | |
| | | | INT 3.1 | | |
| | | | INT 4.2 | 551.2 | |
| | | | INT 5.2 | 551.3 | |
| 1.31 | 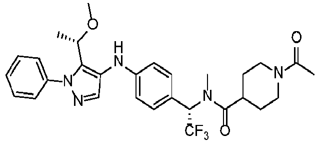 | 1 | INT 1.26 | [M + Na]+ C29H34F 3N5NaO | (400 MHz, CDCl₃) δ = 7.75 (s, 1H), 7.55 - 7.41 (m, 5H), 7.22 (br t, J=7.2 Hz, 2H), 6.88 (br d, J=8.4 Hz, 2H), 6.65 - 6.47 (m, 1H), 5.91 - 5.41 (m, 1H), 4.60 (br s, 1H), 4.50 (q, J=6.8 Hz, 1H), 3.92 (br d, J=13.6 Hz, 1H), 3.23 (s, 3H), 3.20 - 3.11 (m, 1H), 2.93 (br s, 3H), 2.87 - 2.64 (m, 2H), 2.12 (s, 3H), 1.88 (br s, 2H), 1.76 (br d, J=4.4 Hz, 2H), 1.47 (br d, J=6.4 Hz, 3H) |
| | | | INT 2.1 | | |
| | | | INT 3.1 | | |
| | | | INT 4.2 | 3 580.3 | |
| | | | INT 5.2 | 580.4 | |
| 1.32 | 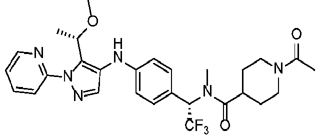 | 1 | INT 1.27 | [M + Na]+ C28H33F 3N6NaO 3 | (400 MHz, DMSO) δ = 8.51 (dd, J=2.0, 4.0 Hz, 1H), 8.06 - 7.97 (m, 1H), 7.85 - 7.77 (m, 2H), 7.44 - 7.37 (m, 1H), 7.21 (s, 1H), 7.19 - 7.08 (m, 2H), 6.86 (d, J=8.0 Hz, 2H), 6.29 (q, J=8.0 Hz, 1H), 5.30 (q, J=8.0 Hz, 1H), 4.42 - 4.32 (m, 1H), 3.88 - 3.77 (m, 1H), 3.16 - 3.07 (m, 1H), 3.06 (s, 3H), 3.04 - 2.94 (m, 1H), 2.91 (s, 2.5H), 2.63 (s, 1H), 2.59 (s, 0.5H), 1.99 (s, 3H), 1.79 - 1.54 (m, 3H), 1.52 (d, J=8.0 Hz, 3H), 1.49 - 1.29 (m, 1H) |
| | | | INT 2.1 | | |
| | | | INT 3.1 | | |
| | | | INT 4.2 | 581.2 | |
| | | | INT 5.2 | 581.3 | |
| 1.33 | 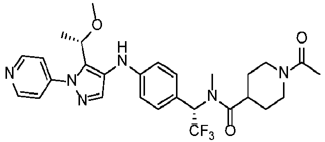 | 1 | INT 1.28 | [M + H]+ C28H34F 3N6O3 | (400 MHz, DMSO) δ = 8.75 - 8.66 (m, 2H), 7.85 (s, 1H), 7.79 - 7.71 (m, 2H), 7.55 (s, 1H), 7.13 (d, J=8.0 Hz, 2H), 6.79 (d, J=8.0 Hz, 2H), 6.28 (q, J=8.0 Hz, 1H), 4.66 (q, J=8.0 Hz, 1H), 4.41 - 4.32 (m, 1H), 3.87 - 3.77 (m, 1H), 3.17 (s, 3H), 3.15 - 2.94 (m, 2H), 2.90 (s, 2.5H), 2.65 - 2.60 (m, 1H), 2.58 (s, 0.5H), 1.99 (s, 3H), 1.79 - 1.36 (m, 4H), 1.34 (d, J=8.0 Hz, 3H) |
| | | | INT 2.1 | | |
| | | | INT 3.1 | 559.3 | |
| | | | INT 4.2 | 559.3 | |
| | | | INT 5.2 | | |
| 1.34 | 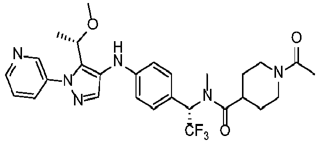 | 1 | INT 1.29 | [M + H]+ C28H34F 3N6O3 | (400 MHz, CDCl₃) δ = 8.76 (d, J=2.4 Hz, 1H), 8.69 (d, J=4.4 Hz, 1H), 7.87 (br d, J=8.4 Hz, 1H), 7.79 (s, 1H), 7.47 (dd, J=4.8, 8.0 Hz, 1H), 7.26 - 7.18 (m, 2H), 6.86 (d, J=8.8 Hz, 2H), 6.57 (q, J=9.2 Hz, 1H), 5.67 (br s, 1H), 4.66 - 4.56 (m, 1H), 4.51 (q, J=6.8 Hz, 1H), 3.91 (br d, J=13.2 Hz, 1H), 3.25 (s, 3H), 3.19 - 3.05 (m, 1H), 2.93 (br s, 3H), 2.86 - 2.61 (m, 2H), 2.12 (s, 3H), 1.92 - 1.82 (m, 2H), 1.81 - 1.74 (m, 2H), 1.46 (d, J=6.8 Hz, 3H) |
| | | | INT 2.1 | | |
| | | | INT 3.1 | 559.3 | |
| | | | INT 4.2 | 559.2 | |
| | | | INT 5.2 | | |
| 1.35 | 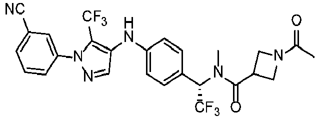 | 1 | INT 1.17 | [M + H]+ C26H23F 6N6O2 | (400 MHz, DMSO-d6) δ = 8.11 (d, J=10.0 Hz, 2H), 8.07 - 8.01 (m, 2H), 7.92 (br d, J=8.4 Hz, 1H), 7.85 - 7.76 (m, 1H), 7.28 - 7.18 (m, 2H), 7.00 - 6.91 (m, 2H), 6.49 - 5.72 (m, 1H), 4.41 - 4.16 (m, 2H), 4.12 - 3.97 (m, 1H), 3.93 - 3.78 (m, 2H), 2.72 (s, 3H), 1.76 (d, J=3.6 Hz, 3H) |
| | | | INT 2.1 | | |
| | | | INT 3.1 | 565.2 | |
| | | | INT 4.3 | 565.1 | |
| | | | INT 5.3 | | |
| 1.36 | 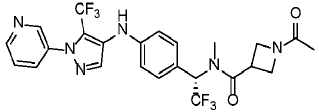 | 1 | INT 1.13 | [M + H]+ C24H23F 6N6O2 | (400 MHz, CDCl₃) δ = 8.84 - 8.70 (m, 2H), 7.90 - 7.82 (m, 2H), 7.49 (dd, J = 4.8, 8.0 Hz, 1H), 7.34 - 7.18 (m, 2H), 7.05 - 6.93 (m, 2H), 6.55 (q, J = 8.8 Hz, 1H), 5.74 (br d, J = 5.6 Hz, 1H), 4.64 - 4.52 (m, 1H), 4.36 - 4.22 (m, 2H), 4.20 - 4.02 (m, 1H), 3.71 - 3.55 (m, 1H), 2.78 (d, J = 6.8 Hz, 3H), 1.91 (d, J = 3.6 Hz, 3H) |
| | | | INT 2.1 | | |
| | | | INT 3.1 | 541.2 | |
| | | | INT 4.3 | 541.2 | |
| | | | INT 5.3 | | |
| 1.37 | 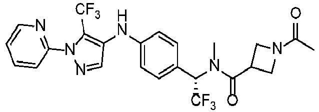 | 1 | INT 1.15 | [M + H]+ C24H23F 6N6O2 | (400 MHz, DMSO-d6) δ = 8.54 (dd, J=1.2, 4.8 Hz, 1H), 8.19 - 8.13 (m, 1H), 8.11 - 8.04 (m, 2H), 7.81 (d, J=8.4 Hz, 1H), 7.51 (dd, J=5.2, 7.2 Hz, 1H), 7.29 - 7.19 (m, 2H), 6.95 (d, J=8.4 Hz, 2H), 6.41 (q, J=9.2 Hz, 1H), 4.40 - 4.17 (m, 2H), 4.13 - 3.97 (m, 1H), 3.93 - 3.78 (m, 2H), 2.74 - 2.69 (m, 3H), 1.76 (d, J=3.6 Hz, 3H) |
| | | | INT 2.1 | | |
| | | | INT 3.1 | 541.2 | |
| | | | INT 4.3 | 541.3 | |
| | | | INT 5.3 | | |
| 1.38 | 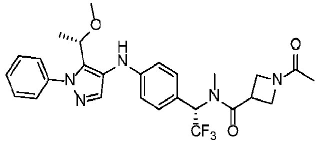 | 1 | INT 1.26 | [M + H]+ C27H31F 3N5O3 | (400 MHz, CDCl₃) δ = 7.75 (d, J=2.0 Hz, 1H), 7.55 - 7.42 (m, 5H), 7.23 (br t, J=7.8 Hz, 2H), 6.89 (br d, J=7.8 Hz, 2H), 6.56 - 6.45 (m, 1H), 5.73 (br s, 1H), 4.62 - 4.53 (m, 1H), 4.50 (q, J=6.8 Hz, 1H), 4.32 - 4.04 (m, 3H), 3.67 - 3.54 (m, 1H), 3.23 (s, 3H), 2.76 (d, J=6.8 Hz, 3H), 1.91 (d, J=2.4 Hz, 3H), 1.47 (d, J=6.8 Hz, 3H) |
| | | | INT 2.1 | | |
| | | | INT 3.1 | 530.2 | |
| | | | INT 4.3 | 530.3 | |
| | | | INT 5.3 | | |
| 1.39 | 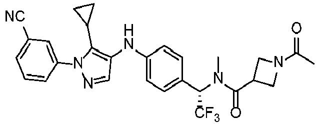 | 1 | INT 1.30 | [M + H]+ C28H28F 3N6O2 | (400 MHz, DMSO-d6) δ = 8.21 - 8.17 (m, 1H), 8.08 - 8.03 (m, 1H), 7.87 (d, J=7.6 Hz, 1H), 7.77 - 7.71 (m, 1H), 7.65 (s, 1H), 7.61 - 7.55 (m, 1H), 7.20 - 7.09 (m, 2H), 6.82 - 6.71 (m, 2H), 6.44 - 5.69 (m, 1H), 4.38 - 4.16 (m, 2H), 4.13 - 3.98 (m, 1H), 3.93 - 3.78 (m, 2H), 2.71 (s, 3H), 2.04 - 1.94 (m, 1H), 1.76 (d, J=3.2 Hz, 3H), 0.77 - 0.69 (m, 2H), 0.48 - 0.42 (m, 2H) |
| | | | INT 2.1 | | |
| | | | INT 3.1 | 537.2 | |
| | | | INT 4.3 | 537.3 | |
| | | | INT 5.3 | | |
| 1.40 | 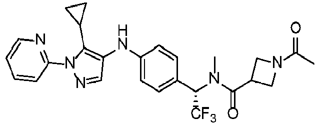 | 1 | INT 1.31 | [M + H]+ C26H28F 3N6O2 | (400 MHz, DMSO-d6) δ = 8.52 (dd, J=1.1, 4.8 Hz, 1H), 8.04 - 7.97 (m, 1H), 7.77 (d, J=8.3 Hz, 1H), 7.61 (s, 1H), 7.52 - 7.46 (m, 1H), 7.41 (dd, J=5.1, 7.0 Hz, 1H), 7.14 (br dd, J=4.0, 8.3 Hz, 2H), 6.70 (d, J=8.6 Hz, 2H), 6.44 - 5.67 (m, 1H), 4.37 - 4.15 (m, 2H), 4.11 - 3.96 (m, 1H), 3.93 - 3.77 (m, 2H), 2.72 - 2.68 (m, 3H), 2.38 - 2.28 (m, 1H), 1.75 (d, J=2.9 Hz, 3H), 0.75 - 0.67 (m, 2H), 0.58 (br d, J=4.8 Hz, 2H) |
| | | | INT 2.1 | | |
| | | | INT 3.1 | 513.2 | |
| | | | INT 4.3 | 513.3 | |
| | | | INT 5.3 | | |
| 1.41 | 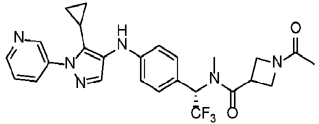 | 1 | INT 1.32 | [M + H]+ C26H28F 3N6O2 | (400 MHz, DMSO-d6) δ = 8.94 (d, J=2.4 Hz, 1H), 8.61 (dd, J=1.2, 4.4 Hz, 1H), 8.17 - 8.11 (m, 1H), 7.65 (s, 1H), 7.61 - 7.52 (m, 2H), 7.19 - 7.09 (m, 2H), 6.81 - 6.72 (m, 2H), 6.38 (q, J=9.6 Hz, 1H), 4.36 - 4.17 (m, 2H), 4.12 - 3.98 (m, 1H), 3.91 - 3.79 (m, 2H), 2.71 (s, 3H), 1.98 - 1.89 (m, 1H), 1.76 (s, 3H), 0.78 - 0.68 (m, 2H), 0.49 (br d, J=5.2 Hz, 2H) |
| | | | INT 2.1 | | |
| | | | INT 3.1 | 513.2 | |
| | | | INT 4.3 | 513.3 | |
| | | | INT 5.3 | | |
| 2.1 | 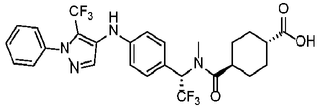 | 2 | INT 1.6 | [M + H]+ C27H27F 6N4O3 | (400 MHz, CDCl₃) δ = 7.80 (s, 1H), 7.54 - 7.44 (m, 5H), 7.26 - 7.24 (m, 2H), 6.94 (d, J=8.8 Hz, 2H), 6.60 (q, J=9.2 Hz, 1H), 5.51 (s, 1H), 2.90 (s, 3H), 2.62 **-** 2.53 (m, 1H), 2.42 (tt, J=3.6, 12.0 Hz, 1H), 2.15 (br t, J=8.8 Hz, 2H), 2.01 - 1.82 (m, 2H), 1.67 (q, J=12.8 Hz, 2H), 1.56 - 1.42 (m, 2H) |
| | | | INT 2.1 | | |
| | | | INT 3.1 | 569.2 | |
| | | | INT 4.4 | 569.1 | |
| | | | INT 5.4 | | |
| | | | INT 6.1 | | |
| 2.2 | 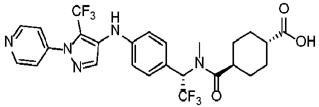 | 2 | INT 1.14 | [M + H]+ C26H26F 6N5O3 | (400 MHz, DMSO) δ = 8.78 (d, J=4.0 Hz, 2H), 8.20 (s, 1H), 8.11 (s, 1H), 7.61 (d, J=4.0 Hz, 2H), 7.18 (d, J=8.0 Hz, 2H), 6.97 (d, J=8.0 Hz, 2H), 6.30 (q, J=8.0 Hz, 1H), 2.87 (s, 2.5H), 2.73 - 2.66 (m, 1H), 2.62 (s, 0.5H), 2.25 - 2.15 (m, 1H), 1.99 - 1.87 (m, 2H), 1.83 - 1.69 (m, 2H), 1.52 - 1.31 (m, 4H) |
| | | | INT 2.1 | | |
| | | | INT 3.1 | 570.2 | |
| | | | INT 4.4 | 570.4 | |
| | | | INT 5.4 | | |
| | | | INT 6.2 | | |
| 2.3 | 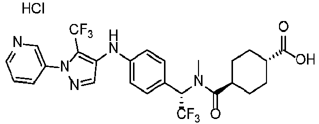 | 2 | INT 1.13 | [M + H]+ C26H26F 6N5O3 | (400 MHz, CDCl₃) δ = 8.87 (d, J=2.0 Hz, 1H), 8.78 (dd, J=1.2, 4.8 Hz, 1H), 8.01 (br d, J=8.4 Hz, 1H), 7.90 (s, 1H), 7.63 (dd, J=4.8, 8.0 Hz, 1H), 7.32 - 7.26 (m, 2H), 6.99 (d, J=8.8 Hz, 2H), 6.62 (q, J=9.2 Hz, 1H), 5.92 - 5.45 (m, 2H), 2.92 (s, 3H), 2.64 - 2.52 (m, 1H), 2.42 (tt, J=3.2, 12.0 Hz, 1H), 2.17 (br t, J=8.8 Hz, 2H), 1.98 (br d, J=12.8 Hz, 1H), 1.88 (br d, J=13.2 Hz, 1H), 1.75 - 1.61 (m, 2H), 1.60 - 1.44 (m, 2H) |
| | | | INT 2.1 | | |
| | | | INT 3.1 | 570.2 | |
| | | | INT 4.4 | 570.1 | |
| | | | INT 5.4 | | |
| | | | INT 6.3 | | |
| 2.4 | 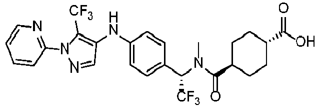 | 2 | INT 1.15 | [M + H]+ C26H26F 6N5O3 | (400 MHz, DMSO-d6) δ = 8.47 (dd, J=1.2, 4.8 Hz, 1H), 8.10 - 8.06 (m, 1H), 8.04 - 7.99 (m, 2H), 7.75 (d, J=8.0 Hz, 1H), 7.48 - 7.42 (m, 1H), 7.21 - 7.08 (m, 2H), 6.94 - 6.86 (m, 2H), 6.44 - 6.05 (m, 1H), 2.81 (s, 3H), 2.62 (br d, J=3.6 Hz, 1H), 2.14 (br s, 1H), 1.94 - 1.80 (m, 2H), 1.79 - 1.57 (m, 2H), 1.44 - 1.25 (m, 4H) |
| | | | INT 2.1 | | |
| | | | INT 3.1 | 570.2 | |
| | | | INT 4.4 | 570.3 | |
| | | | INT 5.4 | | |
| | | | INT 6.4 | | |
| 2.5 | 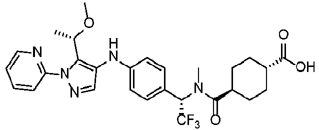 | 2 | INT 1.27 | [M + H]+ C28H33F 3N5O4 | (400 MHz, DMSO) δ = 8.50 (d, J=4.0 Hz, 1H), 8.06 - 7.96 (m, 1H), 7.85 - 7.75 (m, 2H), 7.45 - 7.36 (m, 1H), 7.23 - 7.06 (m, 3H), 6.91 - 6.80 (m, 2H), 6.25 (q, J=8.0 Hz, 1H), 5.29 (q, J=8.0 Hz, 1H), 3.06 (s, 3H), 2.87 (s, 2.5H), 2.70 - 2.65 (m, 1H), 2.62 (s, 0.5H), 2.25 - 2.13 (m, 1H), 1.99 - 1.86 (m, 2H), 1.82 - 1.63 (m, 2H), 1.52 (d, J=6.0 Hz, 3H), 1.49 - 1.30 (m, 4H) |
| | | | INT 2.1 | | |
| | | | INT 3.1 | 560.2 | |
| | | | INT 4.4 | 560.2 | |
| | | | INT 5.4 | | |
| | | | INT 6.5 | | |
| 2.6 | 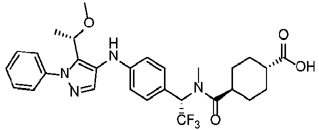 | 2 | INT 1.26 | [M + H]+ C29H34F 3N4O4 | (400 MHz, CDCl₃) δ = 7.76 (s, 1H), 7.55 - 7.48 (m, 2H), 7.48 - 7.42 (m, 3H), 7.22 (d, J=8.4 Hz, 2H), 6.88 (d, J=8.8 Hz, 2H), 6.58 (q, J=9.2 Hz, 1H), 5.71 (br s, 1H), 4.50 (q, J=6.8 Hz, 1H), 3.23 (s, 3H), 2.91 (s, 3H), 2.63 - 2.52 (m, 1H), 2.43 (tt, J=3.6, 12.0 Hz, 1H), 2.21 - 2.10 (m, 2H), 2.03 - 1.82 (m, 2H), 1.75 - 1.60 (m, 2H), 1.55 - 1.44 (m, 5H) (400 MHz, DMSO) δ = 8.71 (d, J=8.0 Hz, 2H), 7.85 (s, 1H), 7.75 (d, J=8.0 Hz, 2H), 7.54 (s, 1H), 7.11 (d, J=8.0 Hz, 2H), 6.78 (d, J=8.0 Hz, 2H), 6.09 (q, J=8.0 Hz, 1H), 4.65 (q, J=8.0 Hz, 1H), 3.17 (s, 3H), 2.86 (s, 2.5H), 2.72 - 2.65 (m, 1H), 2.61 (s, 0.5H), 2.25 - 2.13 (m, 1H), 1.97 - 1.68 (m, 4H), 1.49 - 1.36 (m, 4H), 1.33 (d, J=8.0 Hz, 3H) |
| | | | INT 2.1 | | |
| | | | INT 3.1 | 559.2 | |
| | | | INT 4.4 | 559.3 | |
| | | | INT 5.4 | | |
| | | | INT 6.6 | | |
| 2.7 | 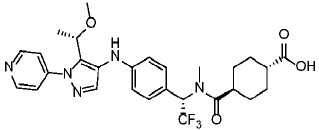 | 2 | INT 1.28 | [M + H]+ C28H33F 3N5O4 | |
| | | | INT 2.1 | | |
| | | | INT 3.1 | 560.2 | |
| | | | INT 4.4 | 560.3 | |
| | | | INT 5.4 | | |
| | | | INT 6.7 | | |
| 2.8 | 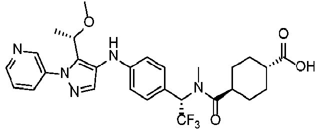 | 2 | INT 1.29 | [M + H]+ C28H33F 3N5O4 | (400 MHz, CDCl₃) δ = 8.78 (s, 1H), 8.69 (br d, J=4.4 Hz, 1H), 7.89 (br d, J=7.6 Hz, 1H), 7.80 (s, 1H), 7.48 (dd, J=5.6, 7.6 Hz, 1H), 7.22 (br d, J=7.6 Hz, 2H), 6.86 (br d, J=8.4 Hz, 2H), 6.63 - 6.53 (m, 1H), 5.64 (br s, 1H), 4.51 (q, J=6.8 Hz, 1H), 3.26 (s, 3H), 2.91 (s, 3H), 2.58 (br s, 1H), 2.48 - 2.37 (m, 1H), 2.16 (br s, 2H), 2.02 - 1.79 (m, 2H), 1.67 (br d, J=12.4 Hz, 2H), 1.52 (br d, J=12.0 Hz, 2H), 1.46 (br d, J=6.8 Hz, 3H) |
| | | | INT 2.1 | | |
| | | | INT 3.1 | 560.2 | |
| | | | INT 4.4 | 560.3 | |
| | | | INT 5.4 | | |
| | | | INT 6.8 | | |
| 2.9 | 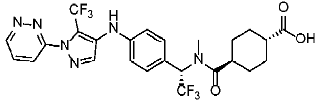 | 2 | INT 1.21 | [M + H]+ C25H25F 6N6O3 | (400 MHz, DMSO-d6) δ = 9.31 (dd, J=1.2, 4.8 Hz, 1H), 8.29 - 8.23 (m, 1H), 8.20 (s, 1H), 8.16 (dd, J=1.2, 8.8 Hz, 1H), 7.99 (dd, J=4.8, 8.8Hz, 1H), 7.28 - 7.14 (m, 2H), 7.04 - 6.93 (m, 2H), 6.44 (q, J=9.6 Hz, 1H), 2.87 (s, 3H), 2.71 - 2.62 (m, 1H), 2.19 (br s, 1H), 1.99 - 1.85 (m, 2H), 1.82 - 1.64 (m, 2H), 1.53 - 1.29 (m, 4H) |
| | | | INT 2.1 | | |
| | | | INT 3.1 | 571.2 | |
| | | | INT 4.4 | 571.2 | |
| | | | INT 5.4 | | |
| | | | INT 6.9 | | |
| 2.10 | 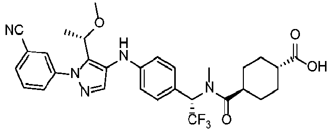 | 2 | INT 1.25 | [M + H]+ C30H33F 3N5O4 | (400 MHz, DMSO-d6) δ = 8.11 - 8.05 (m, 1H), 7.99 - 7.91 (m, 2H), 7.80 - 7.70 (m, 2H), 7.50 (s, 1H), 7.20 - 7.06 (m, 2H), 6.92 - 6.72 (m, 2H), 6.46 - 6.05 (m, 1H), 4.54 (q, J=6.8 Hz, 1H), 3.12 (s, 3H), 2.86 (s, 4H), 2.19 (br s, 1H), 2.00 - 1.85 (m, 2H), 1.83 - 1.64 (m, 2H), 1.50 - 1.31 (m, 4H), 1.27 (d, J=6.8 Hz, 3H) |
| | | | INT 2.1 | | |
| | | | INT 3.1 | 584.2 | |
| | | | INT 4.4 | 584.3 | |
| | | | INT 5.4 | | |
| | | | INT 6.10 | | |
| 2.11 | 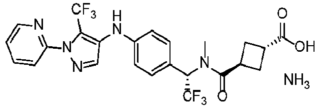 | 2 | INT 1.15 | [M + H]+ C24H22F 6N5O3 | (400 MHz, CDCl₃) δ = 8.58 - 8.48 (m, 1H), 7.89 (dt, J = 2.0, 8.0 Hz, 1H), 7.84 (s, 1H), 7.75 (d, J = 8.8 Hz, 1H), 7.34 (dd, J = 4.0, 6.8 Hz, 1H), 7.31-7.27 (m, 2H), 6.97 (d, J = 8.4 Hz, 2H), 6.57 (q, J = 8.4 Hz, 1H), 5.67 (s, 1H), 3.51 (br t, J = 8.3 Hz, 1H), 3.23 (td, J = 4.4, 9.5 Hz, 1H), 2.78 (s, 3H), 2.74 - 2.65 (m, 2H), 2.64 - 2.49 (m, 2H) |
| | | | INT 2.1 | | |
| | | | INT 3.1 | 542.2 | |
| | | | INT 4.6 | 542.2 | |
| | | | INT 5.6 | | |
| | | | INT 6.12 | | |
| 2.12 | 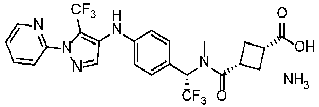 | 2 | INT 1.15 | [M + H]+ C24H22F 6N5O3 | (400 MHz, CDCl₃) δ = 8.58 - 8.49 (m, 1H), 7.88 (dt, J = 1.6, 8.0 Hz, 1H), 7.85 (s, 1H), 7.75 (d, J = 8.8 Hz, 1H), 7.34 (dd, J = 4.8, 7.2 Hz, 1H), 7.31-7.27 (m, 2H), 6.97 (d, J = 8.4 Hz, 2H), 6.55 (q, J = 8.8 Hz, 1H), 5.68 (s, 1H), 3.29 (quin, J = 8.8 Hz, 1H), 3.17 (quin, J = 8.8 Hz, 1H), 2.80 (s, 3H), 2.75 - 2.62 (m, 2H), 2.61 - 2.46 (m, 2H) |
| | | | INT 2.1 | | |
| | | | INT 3.1 | | |
| | | | INT 4.6 | 542.2 | |
| | | | INT 5.6 | 542.2 | |
| | | | INT 6.12 | | |
| 2.13 | 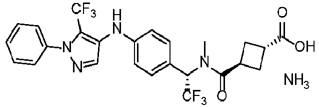 | 2 | INT 1.6 | [M + H]+ C25H23F 6N4O3 | (400 MHz, CDCl₃) δ = 7.81 (s, 1H), 7.54 - 7.45 (m, 5H), 7.29 - 7.27 (m, 2H), 6.99 - 6.93 (m, 2H), 6.61 - 6.51 (m, 1H), 5.54 (br s, 1H), 3.56 - 3.46 (m, 1H), 3.29 - 3.17 (m, 1H), 2.78 (s, 3H), 2.75 - 2.64 (m, 2H), 2.63 - 2.48 (m, 2H) |
| | | | INT 2.1 | | |
| | | | INT 3.1 | 541.2 | |
| | | | INT 4.6 | 541.2 | |
| | | | INT 5.6 | | |
| | | | INT 6.13 | | |
| 2.14 | 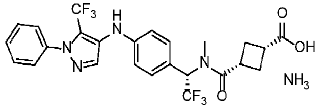 | 2 | INT 1.6 | [M + H]+ C25H23F 6N4O3 | (400 MHz, CDCl₃) δ = 7.81 (s, 1H), 7.49 (br s, 5H), 7.29 - 7.27 (m, 2H), 6.95 (d, J=8.4 Hz, 2H), 6.54 (q, J=8.4 Hz, 1H), 5.56 (br s, 1H), 3.35 - 3.23 (m, 1H), 3.21 - 3.08 (m, 1H), 2.80 (s, 3H), 2.75 - 2.60 (m, 2H), 2.59 - 2.46 (m, 2H) |
| | | | INT 2.1 | | |
| | | | INT 3.1 | 541.2 | |
| | | | INT 4.6 | 541.2 | |
| | | | INT 5.6 | | |
| | | | INT 6.13 | | |
| 3.1 | 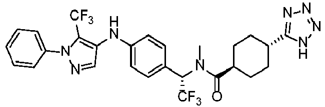 | 3 | INT 1.6 | [M + H]+ C27H27F 6N8O | (400 MHz, CDCl₃) δ = 7.81 (s, 1H), 7.49 (br s, 5H), 7.27 (s, 2H), 6.96 (d, J=8.8 Hz, 2H), 6.67 - 6.51 (m, 1H), 5.60 (s, 1H), 3.22 - 3.15 (m, 1H), 3.03 - 2.85 (m, 3H), 2.79 (br s, 1H), 2.28 (br s, 2H), 2.13 - 1.95 (m, 2H), 1.82 (br s, 4H) |
| | | | INT 2.1 | | |
| | | | INT 3.1 | 593.2 | |
| | | | INT 4.7 | 593.2 | |
| | | | INT 5.7 | | |
| | | | INT 6.11 | | |

### Example 2. MALT1 Biochemical Assay

Inhibitor potency was evaluated by measuring enzymatic activity of full length MALT1 at varying concentrations of compound. The enzymatic assay consists of a single substrate reaction that monitors the release of a fluorescent dye upon cleavage of the peptide substrate. The peptide substrate has the following sequence: Ac-Leu-Arg-Ser-Arg-Rh110-dPro (custom synthesis from WuXi AppTec, Shanghai, China). The assay buffer consists of 50 mM Hepes, pH 7.5, 0.8 M sodium citrate, 1 mM DTT, 0.004% tween-20, and 0.005% bovine serum albumin (BSA). Steady-state kinetic analysis of peptide substrate binding resulted in a Michaelis-Menten constant (*K*_{M}) of 150 µM. The assay was performed in a 384-well F-bottom polypropylene, black microplate (Greiner Bio_ One, Catalog no. 781209) at 15 nM enzyme and 30 µM peptide substrate. The reaction was quenched after 60 minutes with the addition of iodoacetate at a final concentration of 10 mM. Total fluorescence was measured using an Envision (PerkinElmer) with fluorescence excitation at 485 nm and emission at 520 nm.

For potency determination, 1 µL of serially diluted compound (in 100% DMSO) was pre-incubated with 40 µL of enzyme for 30 minutes. The reaction was initiated with the addition of 10 µL of peptide substrate. The relative fluorescence units were transformed to percent inhibition by using 0% and 100% inhibition controls as reference. The 100% inhibition control consisted of 1 µM final concentration of (S)-1-(5-chloro-6-(2H-1,2,3-triazol-2-yl)pyridin-3-yl)-3-(2-chloro-7-(1-methoxyethyl)pyrazolo[1,5-a]pyrimidin-6-yl)urea (IC₅₀ = 15 nM), while the 0% inhibition control consisted of 2% DMSO. IC₅₀ values were calculated by fitting the concentration-response curves to a four-parameter logistic equation in GraphPad Prism.

Results from this assay are summarized in **Table 3** below. In this table, "A" indicates IC₅₀ of less than 0.1 µM; "B" indicates IC₅₀ from 0.1 µM up to 1 µM; and "C" indicates IC₅₀ of greater than 1 µM. "N/A" indicates not tested. For a compound that was tested in more than one experiment, the result shown represents the mean value.

**Table 3.**

| **Compound** | **IC₅₀ (µM)** | **Compound** | **IC₅₀ (µM)** |
|---|---|---|---|
| 1.1 | C | 1.41 | A |
| 1.2 | B | 2.1 | A |
| 1.3 | C | 2.2 | B |
| 1.4 | C | 2.3 | B |
| 1.5 | C | 2.4 | A |
| 1.6 | B | 2.5 | A |
| 1.7 | B | 2.6 | B |
| 1.8 | B | 2.7 | B |
| 1.9 | B | 2.8 | B |
| 1.10 | C | 2.9 | A |
| 1.11 | C | 2.10 | A |
| 1.12 | C | 2.11 | A |
| 1.13 | A | 2.12 | A |
| 1.14 | B | 2.13 | A |
| 1.15 | A | 2.14 | A |
| 1.16 | B | 3.1 | C |
| 1.17 | A | | |
| 1.18 | C | | |
| 1.19 | B | | |
| 1.20 | A | | |
| 1.21 | A | | |
| 1.22 | A | | |
| 1.23 | B | | |
| 1.24 | A | | |
| 1.25 | B | | |
| 1.26 | B | | |
| 1.27 | A | | |
| 1.28 | A | | |
| 1.29 | B | | |
| 1.30 | A | | |
| 1.31 | B | | |
| 1.32 | A | | |
| 1.33 | B | | |
| 1.34 | B | | |
| 1.35 | B | | |
| 1.36 | A | | |
| 1.37 | A | | |
| 1.38 | B | | |
| 1.39 | B | | |
| 1.40 | A | | |

### Example 3. Jurkat IL-2 Assay

Inhibition was determined in a cell-based assay using Jurkat (ATCC, clone E6.1), an immortalized T-cell line, exposed to a dose response of compound and assessed for viability, and IL-2 inhibition by ELISA. Cells were cultured in RPMI/10% FBS (Invitrogen 11875093, Atlanta Biologicals S12450H), maintained below 3E6/mL and only used in assays while below passage 25. Compounds were stamped by ECHO onto 384w plates (PerkinElmer Culturplate, 6007680). The cells were plated in fresh media on top of compound and incubated for 30 minutes before stimulation with soluble anti-CD3/28/2 (Stemcell, 10970) for 24 hours. Supernatant was collected and assessed for IL-2 (MSD, 384w, L21SA-1). To assess viability of cells treated with compound, cells were lysed with CTG reagent (Promega, G7570), and measured by luminometer. IL-2 curves were calculated as percent of DMSO (100%) and signal-inhibiting (0%, (S)-1-(5-chloro-6-(2H-1,2,3-triazol-2-yl)pyridin-3-yl)-3-(2-chloro-7-(1-methoxyethyl)pyrazolo[1,5-a]pyrimidin-6-yl)urea) controls. IC₅₀s calculated using 4-parameter fit in GraphPad Prism.

Results from this assay are summarized in **Table 4** below. In this table, "A" indicates IC₅₀ of less than 0.1 µM; "B" indicates IC₅₀ from 0.1 µM up to 1 µM; and "C" indicates IC₅₀ of greater than 1 µM. "N/A" indicates not tested. For a compound that was tested in more than one experiment, the result shown represents the mean value.

**Table 4.**

| **Compound** | **IC₅₀ (µM)** | **Compound** | **IC₅₀ (µM)** |
|---|---|---|---|
| 1.1 | C | 1.41 | B |
| 1.2 | B | 2.1 | A |
| 1.3 | B | 2.2 | B |
| 1.4 | C | 2.3 | A |
| 1.5 | C | 2.4 | A |
| 1.6 | B | 2.5 | B |
| 1.7 | B | 2.6 | B |
| 1.8 | B | 2.7 | C |
| 1.9 | B | 2.8 | B |
| 1.10 | C | 2.9 | A |
| 1.11 | C | 2.10 | B |
| 1.12 | C | 2.11 | A |
| 1.13 | A | 2.12 | B |
| 1.14 | A | 2.13 | A |
| 1.15 | A | 2.14 | B |
| 1.16 | B | 3.1 | B |
| 1.17 | A | | |
| 1.18 | C | | |
| 1.19 | A | | |
| 1.20 | A | | |
| 1.21 | A | | |
| 1.22 | B | | |
| 1.23 | B | | |
| 1.24 | B | | |
| 1.25 | A | | |
| 1.26 | B | | |
| 1.27 | A | | |
| 1.28 | A | | |
| 1.29 | A | | |
| 1.30 | B | | |
| 1.31 | B | | |
| 1.32 | B | | |
| 1.33 | B | | |
| 1.34 | B | | |
| 1.35 | A | | |
| 1.36 | A | | |
| 1.37 | A | | |
| 1.38 | B | | |
| 1.39 | B | | |
| 1.40 | B | | |

### Equivalents and Scope

**In** the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, e.g., in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications. If there is a conflict between any of the references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims.

## Claims

1. A compound represented by the Formula (I) 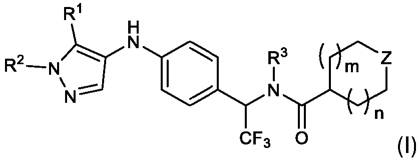 or a stereoisomer and/or a pharmaceutically acceptable salt thereof, wherein:
R¹ is selected from the group consisting of C₁₋₆alkyl, C₁₋₃haloalkyl, C₃₋₆cycloalkyl, and aryl, wherein R¹ may be optionally substituted on one or more available carbons by one, two, three, or more substituents each independently selected from R^{1a};
R² is selected from the group consisting of C₁₋₆alkyl, C₃₋₆ cycloalkyl, aryl, and 5-10 membered heteroaryl, wherein R² may be optionally substituted on one or more available carbons by one, two, three, or more substituents each independently selected from R^{2a};
R³ is hydrogen or C₁₋₃alkyl;
R^{1a} is independently, for each occurrence, hydroxyl or -O-C₁₋₃alkyl;
R^{2a} is independently, for each occurrence, selected from the group consisting of hydroxyl, cyano, C₁₋₆alkyl, and -O-C₁₋₃alkyl;
Z is -C(H)(R^{A})-, -SO₂-, or -NR^{A}-;
R^{A} is -C(O)C₁₋₆alkyl, -C(O)OH, or 5-6 membered heteroaryl;
m is 0 or 1; and
n is 0 or 1.

2. The compound of claim 1, wherein R¹ is selected from the group consisting of - CH(CH₃)₂, CF₃, cyclopropyl, phenyl,

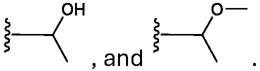

3. The compound of any one of claims 1 and 2, wherein R² is selected from the group consisting of CH₃, cyclopropyl, -C(H)(CH₃)₂, -CH₂C(CH₃)₃, phenyl,

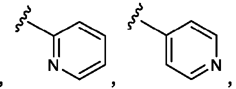

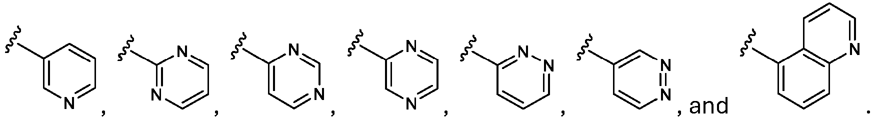

4. The compound of any one of claims 1 to 3, wherein R³ is CH₃.

5. The compound of any one of claims 1 to 4, wherein m is 1 and n is 1.

6. The compound of any one of claims 1 to 4, wherein Z is -C(H)(R^{A})-.

7. The compound of claim 6, wherein m is 1 and n is 1.

8. The compound of claim 7, wherein R^{A} is -C(O)OH or 5-6 membered heteroaryl.

9. The compound of claim 1 represented by Formula (Ic): 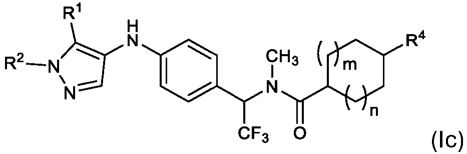 or a stereoisomer and/or a pharmaceutically acceptable salt thereof, wherein:
R¹ is C₁₋₆alkyl or C₁₋₃haloalkyl, wherein the C₁₋₆alkyl may be optionally substituted with - O-C₁₋₃alkyl;
R² is aryl or 5-6 membered heteroaryl, wherein the aryl may be optionally substituted with cyano;
R⁴ is -C(O)OH or 5-6 membered heteroaryl;
m is 0 or 1; and
n is 0 or 1.

10. The compound of claim 9, wherein R¹ is CF₃ or

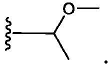

11. The compound of any one of claim 9 or 10, wherein R² is phenyl, 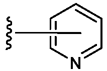 or 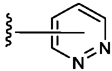 , wherein the phenyl is optionally substituted with cyano.

12. The compound of any one of claims 9 to 11 , wherein m is 1 and n is 1.

13. The compound of claim 12, wherein R⁴ is -C(O)OH or

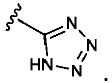

14. The compound of claim 1 selected from any compound set forth in the following Table 1, or a pharmaceutically acceptable salt thereof.
**Table 1. List of compounds.**
| **Cmpd No.** | **Structure** |
|---|---|
| 1.1 | 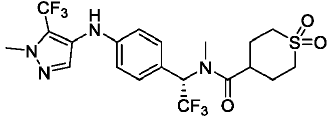 |
| 1.2 | 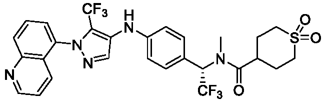 |
| 1.3 | 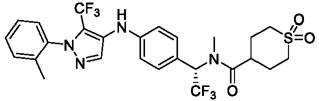 |
| 1.4 | 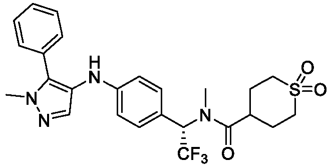 |
| 1.5 | 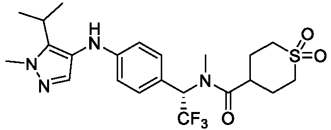 |
| 1.6 | 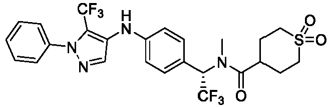 |
| 1.7 | 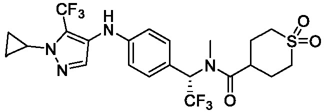 |
| 1.8 | 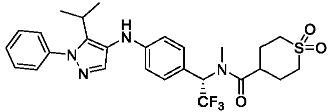 |
| 1.9 | 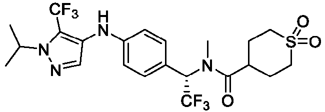 |
| 1.10 | 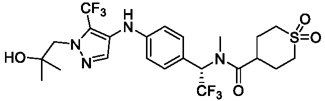 |
| 1.11 | 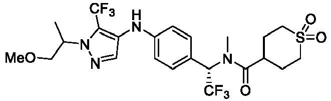 |
| 1.12 | 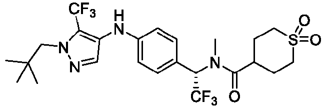 |
| 1.13 | 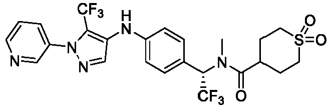 |
| 1.14 | 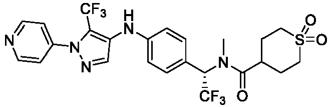 |
| 1.15 | 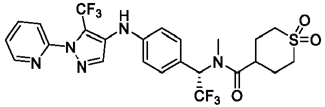 |
| 1.16 | 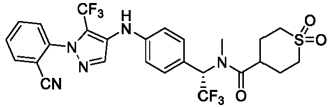 |
| 1.17 | 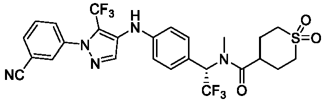 |
| 1.18 | 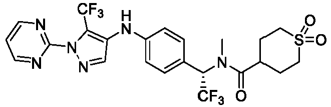 |
| 1.19 | 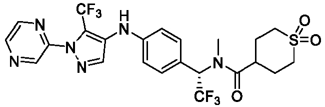 |
| 1.20 | 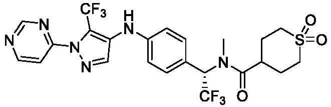 |
| 1.21 | 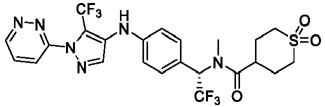 |
| 1.22 | 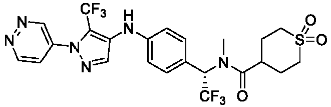 |
| 1.23 | 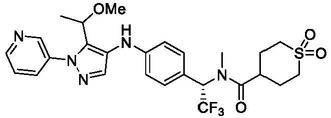 |
| 1.24 | 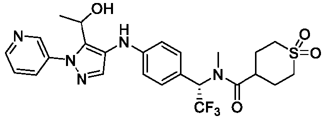 |
| 1.25 | 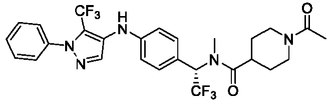 |
| 1.26 | 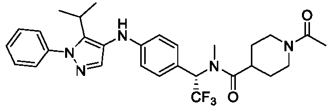 |
| 1.27 | 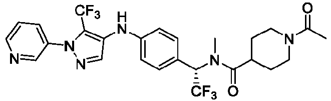 |
| 1.28 | 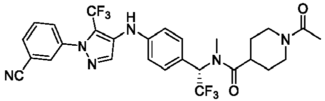 |
| 1.29 | 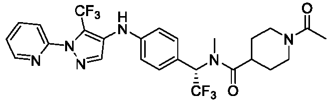 |
| 1.30 | 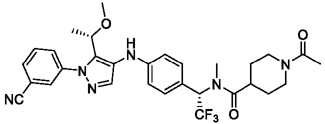 |
| 1.31 | 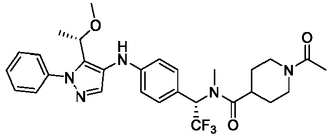 |
| 1.32 | 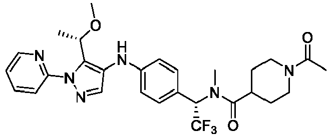 |
| 1.33 | 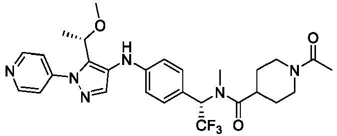 |
| 1.34 | 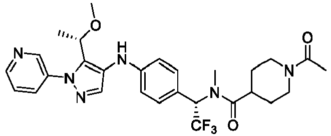 |
| 1.35 | 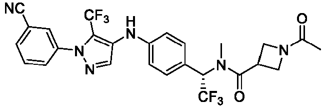 |
| 1.36 | 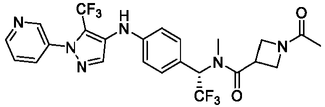 |
| 1.37 | 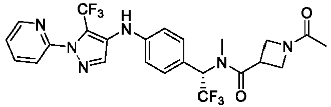 |
| 1.38 | 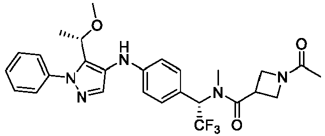 |
| 1.39 | 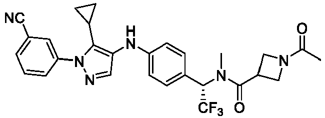 |
| 1.40 | 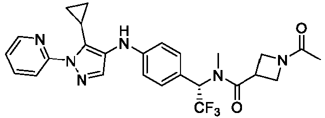 |
| 1.41 | 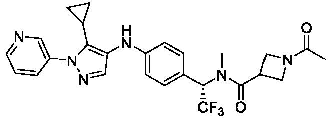 |
| 2.1 | 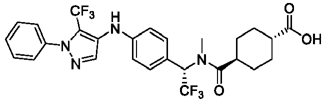 |
| 2.2 | 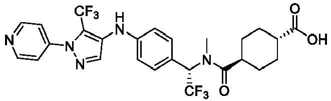 |
| 2.3 | 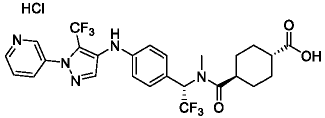 |
| 2.4 | 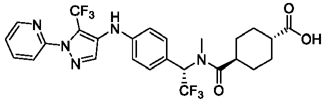 |
| 2.5 | 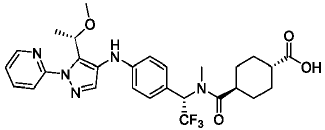 |
| 2.6 | 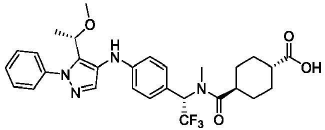 |
| 2.7 | 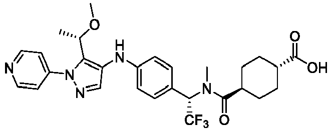 |
| 2.8 | 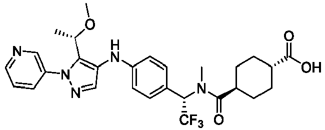 |
| 2.9 | 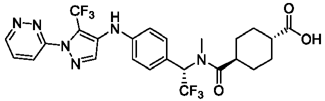 |
| 2.10 | 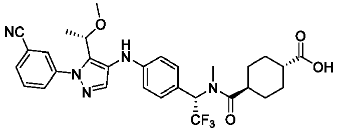 |
| 2.11 | 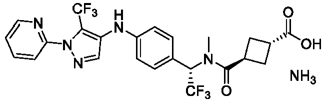 |
| 2.12 | 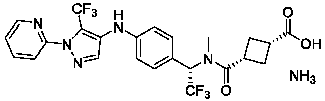 |
| 2.13 | 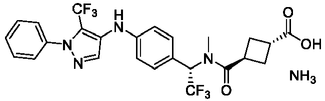 |
| 2.14 | 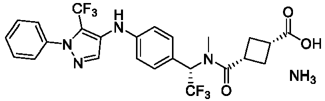 |
| 3.1 | 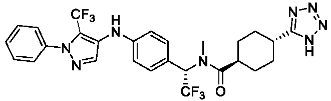 |

15. A pharmaceutical composition comprising a compound of any one of claims 1 to 14 and a pharmaceutically acceptable carrier.

## Patentansprüche

1. Verbindung, die durch die Formel (I) 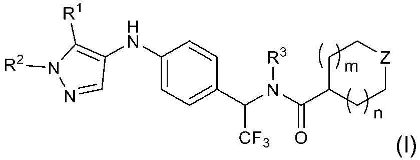 dargestellt ist, oder ein Stereoisomer und/oder ein pharmazeutisch verträgliches Salz davon, wobei:
R¹ aus der Gruppe, bestehend aus C₁₋₆-Alkyl, C₁₋₃-Halogenalkyl, C₃₋₆-Cycloalkyl und Aryl, ausgewählt ist, wobei R¹ gegebenenfalls an einem oder mehreren verfügbaren Kohlenstoffatom(en) durch einen, zwei, drei oder mehr Substituenten, der/die jeweils unabhängig aus R^{1a} ausgewählt ist/sind, substituiert sein kann;
R² aus der Gruppe, bestehend aus C₁₋₆-Alkyl, C₃₋₆-Cycloalkyl, Aryl und 5-10-gliedrigem Heteroaryl, ausgewählt ist, wobei R² gegebenenfalls an einem oder mehreren verfügbaren Kohlenstoffatom(en) durch einen, zwei, drei oder mehr Substituenten, der/die jeweils unabhängig aus R^{2a} ausgewählt ist/sind, substituiert sein kann;
R³ Wasserstoff oder C₁₋₃-Alkyl ist;
R^{1a} unabhängig, bei jedem Auftreten, Hydroxyl oder -O-C₁₋₃-Alkyl ist;
R^{2a} unabhängig, bei jedem Auftreten, aus der Gruppe, bestehend aus Hydroxyl, Cyano, C₁₋₆-Alkyl und -O-C₁₋₃-Alkyl, ausgewählt ist;
Z-C(H)(R^{A})-, -SO₂- oder -NR^{A}- ist;
R^{A}-C(O)C₁₋₆-Alkyl, -C(O)OH oder 5-6-gliedriges Heteroaryl ist;
m 0 oder 1 ist; und
n 0 oder 1 ist.

2. Verbindung nach Anspruch 1, wobei R¹ aus der Gruppe, bestehend aus -CH(CH₃)₂, CF₃, Cyclopropyl, Phenyl, 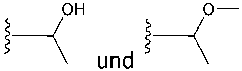 , ausgewählt ist.

3. Verbindung nach einem der Ansprüche 1 und 2, wobei R² aus der Gruppe, bestehend aus CH₃, Cyclopropyl, -C(H)(CH₃)₂, -CH₂C(CH₃)₃, Phenyl, 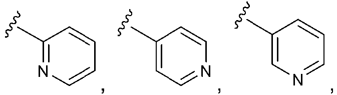 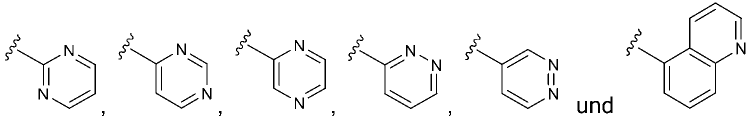 , ausgewählt ist.

4. Verbindung nach einem der Ansprüche 1 bis 3, wobei R³ CH₃ ist.

5. Verbindung nach einem der Ansprüche 1 bis 4, wobei m 1 ist und n 1 ist.

6. Verbindung nach einem der Ansprüche 1 bis 4, wobei Z-C(H)(R^{A})- ist.

7. Verbindung nach Anspruch 6, wobei m 1 ist und n 1 ist.

8. Verbindung nach Anspruch 7, wobei R^{A} -C(O)OH oder 5-6-gliedriges Heteroaryl ist.

9. Verbindung nach Anspruch 1, die durch die Formel (Ic): 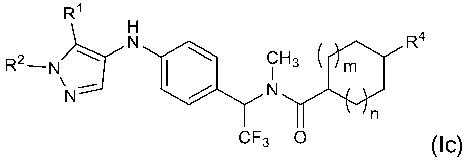 dargestellt ist, oder ein Stereoisomer und/oder ein pharmazeutisch verträgliches Salz davon, wobei:
R¹ C₁₋₆-Alkyl oder C₁₋₃-Halogenalkyl ist, wobei das C₁₋₆-Alkyl gegebenenfalls mit -O-C₁₋₃-Alkyl substituiert sein kann;
R² Aryl oder 5-6-gliedriges Heteroaryl ist, wobei das Aryl gegebenenfalls mit Cyano substituiert sein kann;
R⁴ -C(O)OH oder 5-6-gliedriges Heteroaryl ist;
m 0 oder 1 ist; und
n 0 oder 1 ist.

10. Verbindung nach Anspruch 9, wobei R¹ CF₃ oder 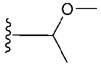 ist.

11. Verbindung nach einem von Anspruch 9 oder 10, wobei R² Phenyl 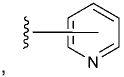 oder 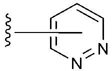 ist, wobei das Phenyl gegebenenfalls mit Cyano substituiert ist.

12. Verbindung nach einem der Ansprüche 9 bis 11, wobei m 1 ist und n 1 ist.

13. Verbindung nach Anspruch 12, wobei R⁴ -C(O)OH oder 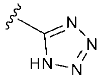 ist.

14. Verbindung nach Anspruch 1, ausgewählt aus jedweder Verbindung, die in der nachstehenden Tabelle 1 angegeben ist, oder einem pharmazeutisch verträglichen Salz davon.
**Tabelle 1. Liste von Verbindungen.**
| **Verb.-Nr.** | **Struktur** |
|---|---|
| 1.1 | 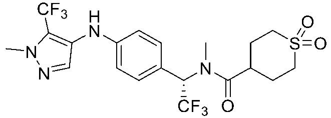 |
| 1.2 | 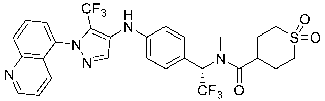 |
| 1.3 | 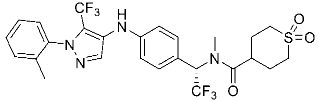 |
| 1.4 | 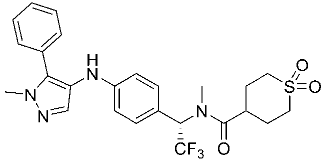 |
| 1.5 | 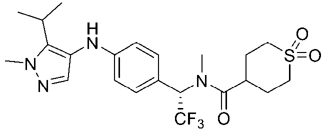 |
| 1.6 | 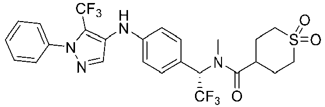 |
| 1.7 | 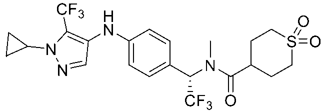 |
| 1.8 | 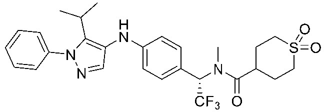 |
| 1.9 | 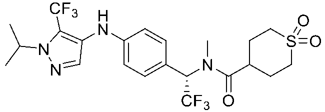 |
| 1.10 | 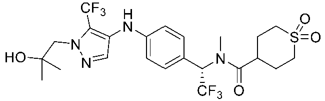 |
| 1.11 | 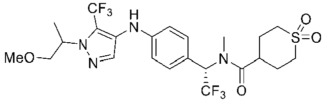 |
| 1.12 | 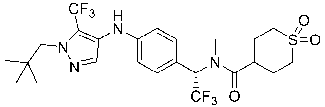 |
| 1.13 | 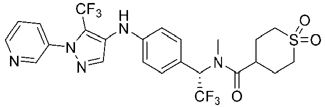 |
| 1.14 | 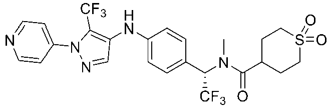 |
| 1.15 | 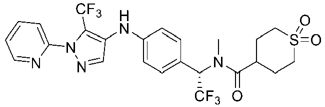 |
| 1.16 | 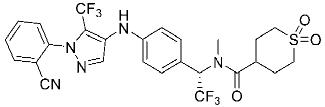 |
| 1.17 | 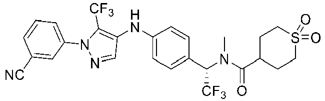 |
| 1.18 | 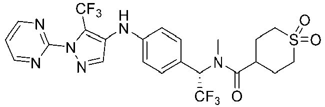 |
| 1.19 | 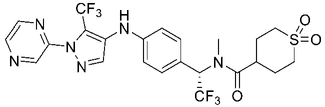 |
| 1.20 | 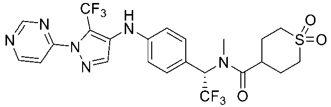 |
| 1.21 | 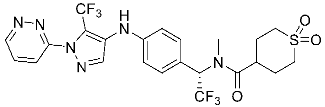 |
| 1.22 | 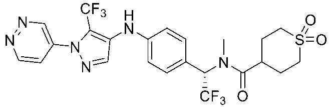 |
| 1.23 | 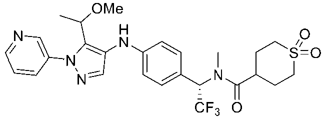 |
| 1.24 | 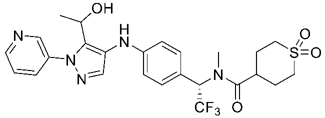 |
| 1.25 | 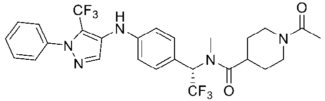 |
| 1.26 | 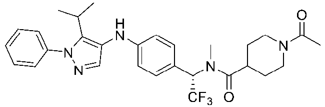 |
| 1.27 | 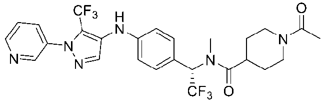 |
| 1.28 | 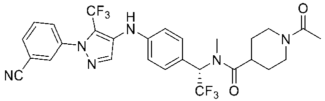 |
| 1.29 | 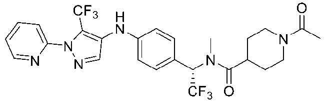 |
| 1.30 | 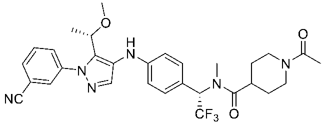 |
| 1.31 | 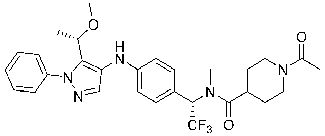 |
| 1.32 | 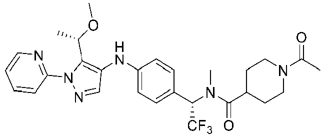 |
| 1.33 | 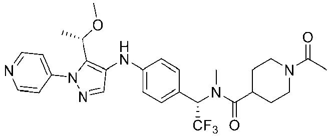 |
| 1.34 | 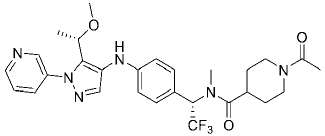 |
| 1.35 | 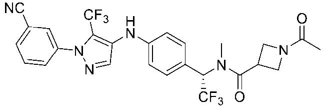 |
| 1.36 | 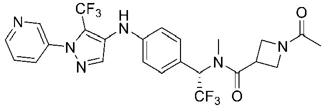 |
| 1.37 | 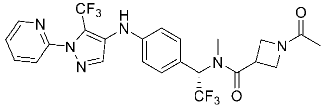 |
| 1.38 | 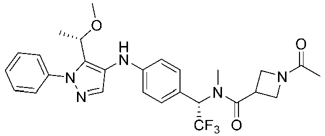 |
| 1.39 | 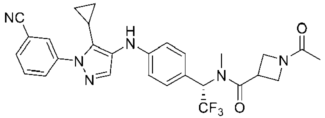 |
| 1.40 | 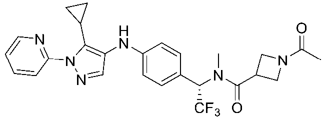 |
| 1.41 | 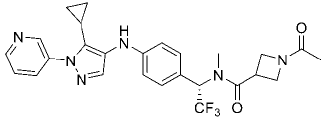 |
| 2.1 | 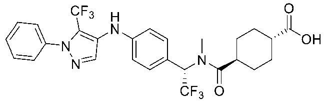 |
| 2.2 | 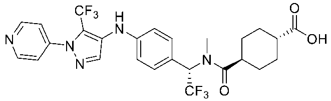 |
| 2.3 | 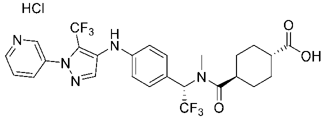 |
| 2.4 | 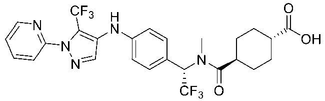 |
| 2.5 | 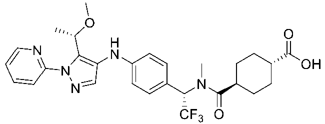 |
| 2.6 | 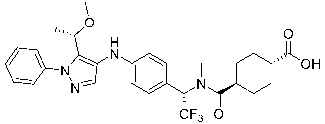 |
| 2.7 | 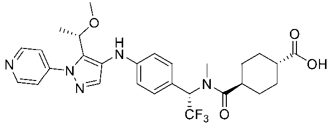 |
| 2.8 | 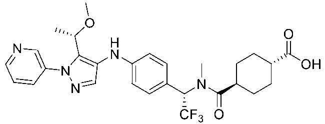 |
| 2.9 | 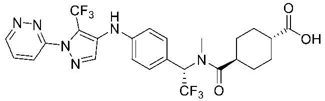 |
| 2.10 | 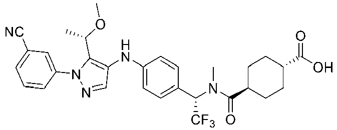 |
| 2.11 | 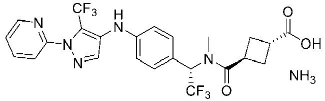 |
| 2.12 | 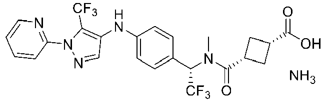 |
| 2.13 | 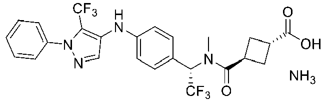 |
| 2.14 | 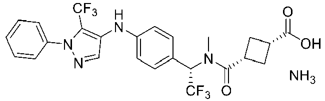 |
| 3.1 | 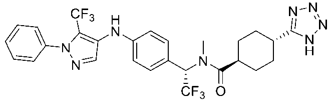 |

15. Pharmazeutische Zusammensetzung, die eine Verbindung nach einem der Ansprüche 1 bis 14 und einen pharmazeutisch verträglichen Träger umfasst.

## Revendications

1. Composé représenté par la Formule (I) 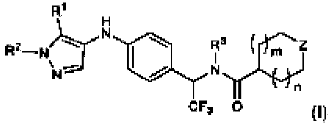 ou stéréoisomère et/ou sel pharmaceutiquement acceptable de celui-ci, dans laquelle :
R¹ est sélectionné parmi le groupe constitué de C₁₋₆alkyle C₁₋₃halogénoalkyle, C₃₋₆cycloalkyle et aryle, dans laquelle R¹ peut être substitué en option sur un ou plusieurs carbones disponibles par un, deux, trois substituants ou plus sélectionnés chacun indépendamment parmi R^{1a};
R² est sélectionné parmi le groupe constitué de C₁₋₆alkyle, C₃₋₆cycloalkyle, aryle et hétéroaryle à 5 à 10 membres, dans laquelle R² peut être substitué en option sur un ou plusieurs carbones disponibles par un, deux, trois substituants ou plus, sélectionnés chacun indépendamment parmi R^{2a} ;
R³ est hydrogène ou C₁₋₃alkyle ;
R^{1a} est indépendamment, pour chaque occurrence, hydroxyle ou -O-C₁₋₃alkyle ;
R^{2a} est indépendamment, pour chaque occurrence, sélectionné parmi le groupe constitué d'hydroxyle, cyano, C₁₋₆alkyle et -O-C₁₋₃alkyle ;
Z est -C(H)(R^{A})-, -SO₂- ou -NR^{A}- ;
R^{A} est -C(O)C₁₋₆alkyle, -C(O)OH ou hétéroaryle à 5 à 6 membres ;
m est 0 ou 1 ; et
n est 0 ou 1.

2. Composé selon la revendication 1, dans lequel R¹ est sélectionné parmi le groupe constitué de -CH(CH₃)₂, CF₃, cyclopropyle, phényle,

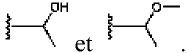

3. Composé selon l'une quelconque des revendications 1 et 2, dans lequel R² est sélectionné parmi le groupe constitué de CH₃, cyclopropyle, -C(H)(CH₃)₂, - CH₂C(CH₃)₃, phényle, 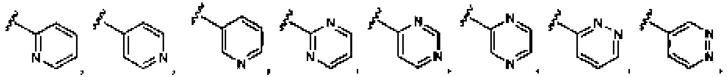 et

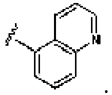

4. Composé selon l'une quelconque des revendications 1 à 3, dans lequel R³ est CH₃.

5. Composé selon l'une quelconque des revendications 1 à 4, dans lequel m est 1 et n est 1.

6. Composé selon l'une quelconque des revendications 1 à 4, dans lequel Z est - C(H)(R^{A})-.

7. Composé selon la revendication 6, dans lequel m est 1 et n est 1.

8. Composé selon la revendication 7, dans lequel R^{A} est -C(O)OH ou hétéroaryle à 5 à 6 membres.

9. Composé selon la revendication 1 représenté par la Formule (Ic) : 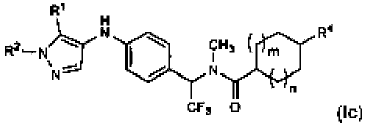 ou stéréoisomère et/ou sel pharmaceutiquement acceptable de celui-ci, dans laquelle :
R¹ est C₁₋₆alkyle ou C₁₋₃halogénoalkyle, dans laquelle le C₁₋₆alkyle peut être substitué en option par -O-C₁₋₃alkyle ;
R² est aryle ou hétéroaryle à 5 à 6 membres, dans laquelle l'aryle peut être substitué en option par cyano ;
R⁴ est -C(O)OH ou hétéroaryle à 5 à 6 membres ;
m est 0 ou 1 ; et
n est 0 ou 1.

10. Composé selon la revendication 9, dans lequel R¹ est CF₃ ou

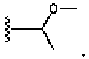

11. Composé selon l'une quelconque de la revendication 9 ou 10, dans lequel R² est
phényle, 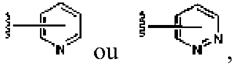 dans laquelle le phényle est substitué en option par cyano.

12. Composé selon l'une quelconque des revendications 9 à 11, dans lequel m est 1 et n est 1.

13. Composé selon la revendication 12, dans lequel R⁴ est -C(O)OH ou

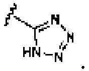

14. Composé selon la revendication 1 sélectionné parmi tout composé quelconque établi dans le Tableau 1 suivant ou sel pharmaceutiquement acceptable de celui-ci.
**Tableau 1. Liste de composés.**
| **N° de composé** | **Structure** |
|---|---|
| 1.1 | 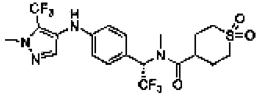 |
| 1.2 | 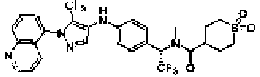 |
| 1.3 | 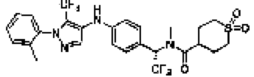 |
| **N° de composé** | **Structure** |
|---|---|
| 1.4 | 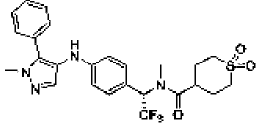 |
| 1.5 | 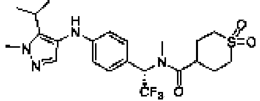 |
| 1.6 | 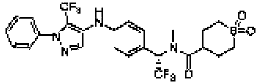 |
| 1.7 | 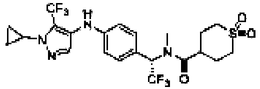 |
| 1.8 | 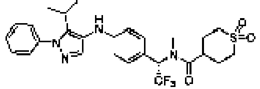 |
| 1.9 | 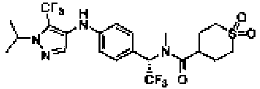 |
| 1.10 | 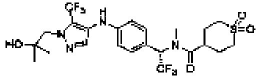 |
| 1.11 | 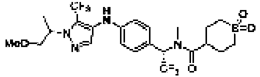 |
| 1.12 | 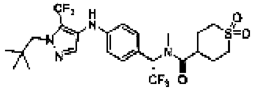 |
| **N° de composé** | **Structure** |
|---|---|
| 1.13 | 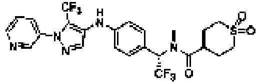 |
| 1.14 | 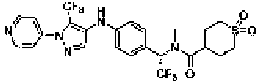 |
| 1.15 | 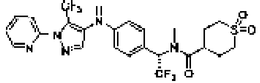 |
| 1.16 | 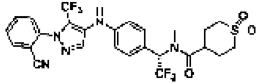 |
| 1.17 | 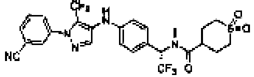 |
| 1.18 | 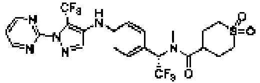 |
| 1.19 | 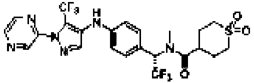 |
| 1.20 | 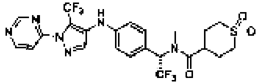 |
| 1.21 | 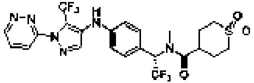 |
| **N° de composé** | **Structure** |
|---|---|
| 1.22 | 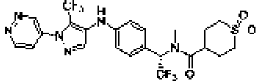 |
| 1.23 | 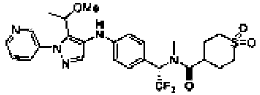 |
| 1.24 | 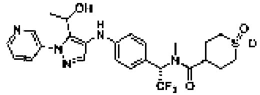 |
| 1.25 | 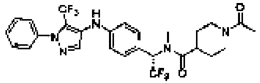 |
| 1.26 | 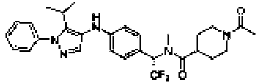 |
| 1.27 | 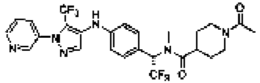 |
| 1.28 | 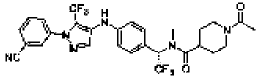 |
| 1.29 | 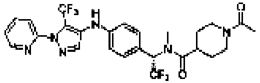 |
| 1.30 | 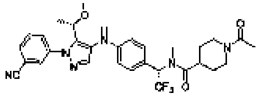 |
| **N° de composé** | **Structure** |
|---|---|
| 1.31 | 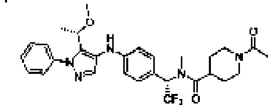 |
| 1.32 | 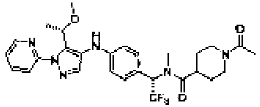 |
| 1.33 | 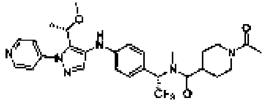 |
| 1.34 | 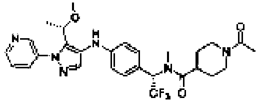 |
| 1.35 | 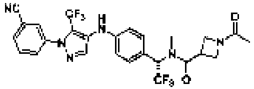 |
| 1.36 | 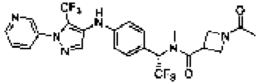 |
| 1.37 | 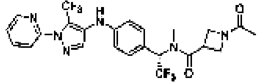 |
| 1.38 | 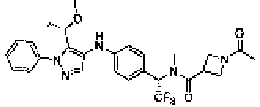 |
| **N° de composé** | **Structure** |
|---|---|
| 1.39 | 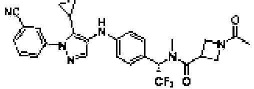 |
| 1.40 | 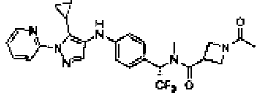 |
| 1.41 | 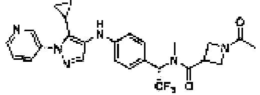 |
| 2.1 | 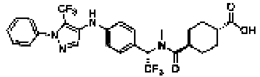 |
| 2.2 | 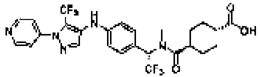 |
| 2.3 | 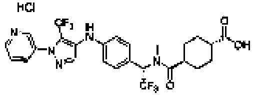 |
| 2.4 | 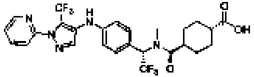 |
| 2.5 | 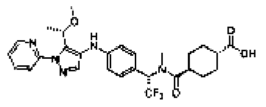 |
| 2.6 | 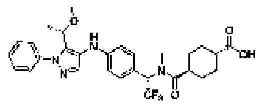 |
| **N° de composé** | **Structure** |
|---|---|
| 2.7 | 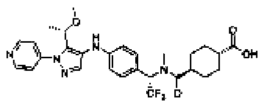 |
| 2.8 | 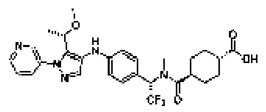 |
| 2.9 | 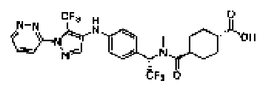 |
| 2.10 | 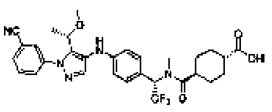 |
| 2.11 | 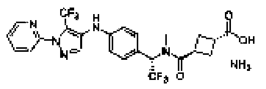 |
| 2.12 | 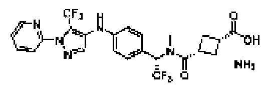 |
| 2.13 | 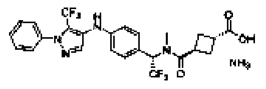 |
| 2.14 | 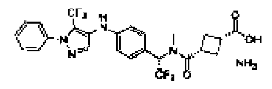 |
| 3.1 | 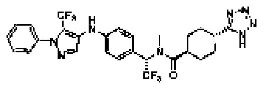 |

15. Composition pharmaceutique comprenant un composé selon l'une quelconque des revendications 1 à 14 et un excipient pharmaceutiquement acceptable.
